(19) Europäisches Patentamt
European Patent Office
Office européen des brevets

(11) EP 4 060 028 A1

(12) **EUROPEAN PATENT APPLICATION**
published in accordance with Art. 153(4) EPC

(43) Date of publication:
**21.09.2022 Bulletin 2022/38**

(21) Application number: **20879069.1**

(22) Date of filing: **23.10.2020**

(51) International Patent Classification (IPC):
***C12N 7/00*** *(2006.01)*

(52) Cooperative Patent Classification (CPC):
**C12N 7/00**

(86) International application number:
**PCT/KR2020/014585**

(87) International publication number:
**WO 2021/080377 (29.04.2021 Gazette 2021/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.10.2019 US 201962924711 P**

(71) Applicant: **Genenmed Co., Ltd.**
**Seoul 04793 (KR)**

(72) Inventor: **LEE, Dongwoo**
**Seoul 01203 (KR)**

(74) Representative: **Plougmann Vingtoft a/s**
**Strandvejen 70**
**2900 Hellerup (DK)**

(54) **HELPER PLASMID-BASED GUTLESS ADENOVIRUS PRODUCTION SYSTEM**

(57) The present invention relates to a helper plasmid-based gutless adenovirus (GLAd) production system, a gutless adenovirus production method using same, a gutless adenovirus produced using same, and a use of gutless adenovirus produced using same.

【Fig 1】

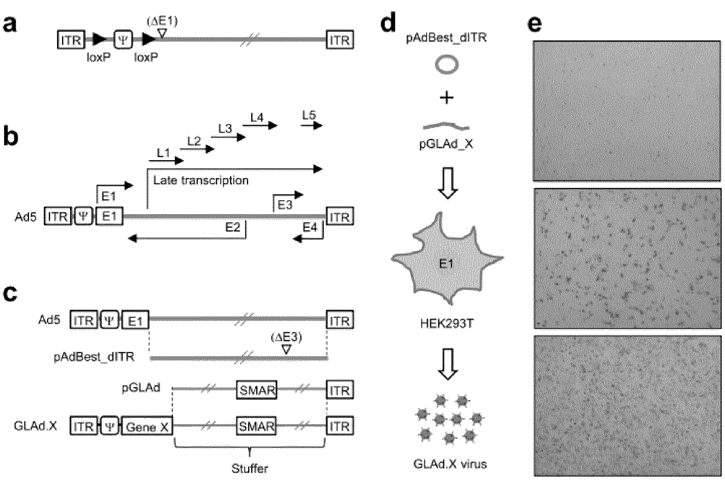


EP 4 060 028 A1

<110> GENENMED CO., LTD.

<120> No more helper adenovirus: production of gutless adenovirus (GLAd) free of adenovirus and replication-competent adenovirus (RCA) contaminants

<130> PP200038

<150> US 62/924,711
<151> 2019-10-23

<160> 82

<170> KoPatentIn 3.0

<210> 1
<211> 27
<212> DNA
<213> Artificial Sequence

<220>
<223> KanColE1_1F

<400> 1
gggccaagga tctgatggcg caggga 27

<210> 2
<211> 40
<212> DNA
<213> Artificial Sequence

<220>
<223> KanColE1_1R

<400> 2
cttggccgca gcggccgagc aaaaggccag caaaaggcca 40

<210> 3
<211> 33
<212> DNA
<213> Artificial Sequence

<220>
<223> KanColE1_2F

<400> 3
cccggatccg cagtgggctt acatggcgat agc 33

<210> 4
<211> 38
<212> DNA
<213> Artificial Sequence

<220>
<223> KanColE1_2R

<400> 4
cccgtataca tcgatttaat taagagcaaa aggccagc 38

<210> 5
<211> 34
<212> DNA
<213> Artificial Sequence

<220>
<223> Cla_delE1_F

<400> 5
gggatcgatt taagggtggg aaagaatata taag 34

<210> 6
<211> 33
<212> DNA
<213> Artificial Sequence

<220>
<223> BstZ_R

<400> 6
cccgtatacg gggacacgga cagccttttc gtc 33

<210> 7
<211> 32
<212> DNA
<213> Artificial Sequence

<220>
<223> KanColE1_3F

<400> 7
ttaattaagc agtgggctta catggcgata gc 32

<210> 8
<211> 38
<212> DNA
<213> Artificial Sequence

<220>
<223> KanColE1_3R

<400> 8
cccggatcca tcgatttaat taagagcaaa aggccagc 38

<210> 9
<211> 34
<212> DNA
<213> Artificial Sequence

<220>
<223> E3_Spe_F

<400> 9
gggactagtt tcgcgccctt tctcaaattt aagc 34

<210> 10
<211> 35
<212> DNA
<213> Artificial Sequence

<220>
<223> delE3_R

<400> 10
gcggatggac aggaacttat aacattcagt cgtag 35

<210> 11
<211> 35
<212> DNA
<213> Artificial Sequence

<220>
<223> delE3_F

<400> 11
ctacgactga atgttataag ttcctgtcca tccgc 35

<210> 12
<211> 34
<212> DNA
<213> Artificial Sequence

<220>
<223> E3_Nde_R

<400> 12
gggcatatgg atacacgggg ttgaaggtat cttc 34

<210> 13
<211> 17
<212> DNA
<213> Artificial Sequence

<220>
<223> Cla_EcoR_S

<400> 13
cgatccggag gcccttg 17

<210> 14
<211> 19
<212> DNA
<213> Artificial Sequence

<220>
<223> Cla_EcoR_AS

<400> 14
aattcaaggg cctccggat 19

<210> 15
<211> 22
<212> DNA
<213> Artificial Sequence

<220>
<223> Avr_F

<400> 15
ctgcctaggc aaaatagcac cc 22

<210> 16
<211> 35
<212> DNA
<213> Artificial Sequence

<220>
<223> Avr_dITR_R

<400> 16
aacgatgtaa gttttagggc ggagtaactt gtatg 35

<210> 17
<211> 35
<212> DNA
<213> Artificial Sequence

<220>
<223> Avr_dITR_F

<400> 17
gccctaaaac ttacatcgtt aattaagcag tgggc 35

<210> 18
<211> 22
<212> DNA
<213> Artificial Sequence

<220>
<223> Rsr_R

<400> 18
tagcggtccg ccacacccag cc 22

<210> 19
<211> 36
<212> DNA

<213> Artificial Sequence

<220>
<223> KanColE1_4F

<400> 19
ggttggcgcg ccctacgtca cccgccccgt tcccac 36

<210> 20
<211> 34
<212> DNA
<213> Artificial Sequence

<220>
<223> KanColE1_4R

<400> 20
cccgagctca aactacataa gacccccacc ttat 34

<210> 21
<211> 30
<212> DNA
<213> Artificial Sequence

<220>
<223> Sac_Pst_Avr_Asc_S

<400> 21
cttaacctgc agatcctcct aggtttttgg 30

<210> 22
<211> 38
<212> DNA
<213> Artificial Sequence

<220>
<223> Sac_Pst_Avr_Asc_AS

<400> 22
cgcgccaaaa acctaggagg atctgcaggt taagagct 38

<210> 23
<211> 55
<212> DNA
<213> Artificial Sequence

<220>
<223> Sac_Hpa_Avr_S

<400> 23
cgggcggcga cctcgcgggt taaccgtcct ttaaaaaagt cgtttctgca agctc 55

<210> 24

<211> 63
<212> DNA
<213> Artificial Sequence

<220>
<223> Sac_Hpa_Avr_AS

<400> 24
ctaggagctt gcagaaacga ctttttttaaa ggacggttaa cccgcgaggt cgccgcccga 60

gct 63

<210> 25
<211> 35
<212> DNA
<213> Artificial Sequence

<220>
<223> SacI/ApaI_S

<400> 25
catggttcca aaatgcccct taaccgggtt gggcc 35

<210> 26
<211> 35
<212> DNA
<213> Artificial Sequence

<220>
<223> SacI/ApaI_AS

<400> 26
caacccggtt aaggggcatt ttggaaccat gagct 35

<210> 27
<211> 31
<212> DNA
<213> Artificial Sequence

<220>
<223> ApaI/AvrII_S

<400> 27
catggttcca aaatgcccct taaccgggtt c 31

<210> 28
<211> 39
<212> DNA
<213> Artificial Sequence

<220>
<223> ApaI/AvrII_AS

<400> 28

ctaggaaccc ggttaagggg cattttggaa ccatgggcc 39

<210> 29
<211> 33
<212> DNA
<213> Artificial Sequence

<220>
<223> pGLAdSMAR_F

<400> 29
tctgggccca aataaactta taaattgtga gag 33

<210> 30
<211> 33
<212> DNA
<213> Artificial Sequence

<220>
<223> pGLAdSMAR_R

<400> 30
cccatgcata tatttaaaga aaaaaaaatt gta 33

<210> 31
<211> 23
<212> DNA
<213> Artificial Sequence

<220>
<223> pCAG_F

<400> 31
gttattaata gtaatcaatt acg 23

<210> 32
<211> 44
<212> DNA
<213> Artificial Sequence

<220>
<223> pCAG_R

<400> 32
cttgggtctc cctatcgccc gccgcgcgct tcgcttttta tagg 44

<210> 33
<211> 38
<212> DNA
<213> Artificial Sequence

<220>
<223> bGint_F

<400> 33
ggcgataggg agacccaagc tggtgagttt ggggaccc 38

<210> 34
<211> 43
<212> DNA
<213> Artificial Sequence

<220>
<223> bGint_R

<400> 34
gggaagcttg ggtcccctgt aggaaaaaga agaaggcatg aac 43

<210> 35
<211> 48
<212> DNA
<213> Artificial Sequence

<220>
<223> Ad5pTP_F

<400> 35
gggaagctta ccatggcctt gagcgtcaac gattgcgcgc gcctgacc 48

<210> 36
<211> 34
<212> DNA
<213> Artificial Sequence

<220>
<223> Ad5pTP_R

<400> 36
ggcgaattcc taaaagcggt gacgcgggcg agcc 34

<210> 37
<211> 24
<212> DNA
<213> Artificial Sequence

<220>
<223> Ad5Fiber_F

<400> 37
cgcgcaagac cgtctgaaga tacc 24

<210> 38
<211> 23
<212> DNA
<213> Artificial Sequence

<220>

<223> Ad5Fiber_R

<400> 38
ggcctgatgt ttgcagggct agc 23

<210> 39
<211> 45
<212> DNA
<213> Artificial Sequence

<220>
<223> N-F

<400> 39
tcttatgtag tttgagctcg gctttggtta tttgatggat tgacc 45

<210> 40
<211> 43
<212> DNA
<213> Artificial Sequence

<220>
<223> N-R

<400> 40
tccggagggg ctcgaggctg gcttaaaaca gtaactcaat atg 43

<210> 41
<211> 43
<212> DNA
<213> Artificial Sequence

<220>
<223> C-F

<400> 41
ctcgagcccc tccggaggag tggccagggc gttctggagg tag 43

<210> 42
<211> 41
<212> DNA
<213> Artificial Sequence

<220>
<223> C-R

<400> 42
cgccaaaaac ctaggtgggc ccaggaagac tcactggttg g 41

<210> 43
<211> 52
<212> DNA
<213> Artificial Sequence

<220>
<223> Nsi-Xho-S

<400> 43
tcgagctaga ctctggggct aaagctgcag tatccatcac actggcggcc gc 52

<210> 44
<211> 60
<212> DNA
<213> Artificial Sequence

<220>
<223> Nsi-Xho-AS

<400> 44
tcgagcggcc gccagtgtga tggatactgc agctttagcc ccagagtcta gctcgatgca 60
60

<210> 45
<211> 36
<212> DNA
<213> Artificial Sequence

<220>
<223> F1-F

<400> 45
cctcctcgag ctagactctg ggctaaagc aatgag 36

<210> 46
<211> 43
<212> DNA
<213> Artificial Sequence

<220>
<223> F1-R

<400> 46
cccacgcgtg cggccgcttg agaggcagag gcaaaggcaa gtg 43

<210> 47
<211> 38
<212> DNA
<213> Artificial Sequence

<220>
<223> F2-F

<400> 47
tcaagcggcc gcgtgctgag actaaaggga tgtgctac 38

<210> 48
<211> 35
<212> DNA
<213> Artificial Sequence

<220>
<223> F2-R

<400> 48
cccgtcgacc atcattctaa ggcctgcctg agcta 35

<210> 49
<211> 35
<212> DNA
<213> Artificial Sequence

<220>
<223> F3-F

<400> 49
cccgtcgacg cactggcttt acaggggccg tctgc 35

<210> 50
<211> 35
<212> DNA
<213> Artificial Sequence

<220>
<223> F3-R

<400> 50
gggcgatcgg gaaggaaacc tacctagcct acaag 35

<210> 51
<211> 35
<212> DNA
<213> Artificial Sequence

<220>
<223> F4-F

<400> 51
gggcgatcgg tagactagga tagcctcaaa ctcct 35

<210> 52
<211> 35
<212> DNA
<213> Artificial Sequence

<220>
<223> F4-R

<400> 52
gggacgcgtg ttaacatact atcagaatag tgata 35

<210> 53
<211> 35
<212> DNA
<213> Artificial Sequence

<220>
<223> F5-F

<400> 53
gggacgcgtt gggtgcaatc ttaccagagc cttac 35

<210> 54
<211> 25
<212> DNA
<213> Artificial Sequence

<220>
<223> F5-R

<400> 54
acggcagttc aaaatcaagt aatac 25

<210> 55
<211> 41
<212> DNA
<213> Artificial Sequence

<220>
<223> SMAR-F

<400> 55
cggcctgggt ggccaaataa acttataaat tgtgagagaa a 41

<210> 56
<211> 35
<212> DNA
<213> Artificial Sequence

<220>
<223> SMAR-R

<400> 56
caggtcgaca tatttaaaga aaaaaaaatt gtatc 35

<210> 57
<211> 103
<212> DNA
<213> Artificial Sequence

<220>
<223> HTTmshR1_S

<400> 57
gatcctgctg ttgacagtga gcgagaccgt gtgaatcatt gtctatagtg aagccacaga 60

tgtatagaca atgattcaca cggtcgtgcc tactgcctcg gag 103

<210> 58
<211> 103
<212> DNA
<213> Artificial Sequence

<220>
<223> HTTmshR1_AS

<400> 58
aattctccga ggcagtaggc acgaccgtgt gaatcattgt ctatacatct gtggcttcac 60

tatagacaat gattcacacg gtctcgctca ctgtcaacag cag 103

<210> 59
<211> 103
<212> DNA
<213> Artificial Sequence

<220>
<223> HTTmshR2_S

<400> 59
gatcctgctg ttgacagtga gcgacagctt gtccaggttt atgaatagtg aagccacaga 60

tgtattcata aacctggaca agctggtgcc tactgcctcg gag 103

<210> 60
<211> 103
<212> DNA
<213> Artificial Sequence

<220>
<223> HTTmshR2_AS

<400> 60
aattctccga ggcagtaggc accagcttgt ccaggtttat gaatacatct gtggcttcac 60

tattcataaa cctggacaag ctgtcgctca ctgtcaacag cag 103

<210> 61
<211> 103
<212> DNA
<213> Artificial Sequence

<220>
<223> HTTmshR3_S

<400> 61
gatcctgctg ttgacagtga gcgaggatac ctgaaatcct gcttttagtg aagccacaga 60

tgtaaaagca ggatttcagg tatccgtgcc tactgcctcg gag 103

<210> 62
<211> 103
<212> DNA
<213> Artificial Sequence

<220>
<223> HTTmshR3_AS

<400> 62
aattctccga ggcagtaggc acggatacct gaaatcctgc ttttacatct gtggcttcac 60

taaaagcagg atttcaggta tcctcgctca ctgtcaacag cag 103

<210> 63
<211> 2905
<212> DNA
<213> Artificial Sequence

<220>
<223> pGT2

<400> 63
gcgcgcgttg acattgatta ttgactagtt attaatagta atcaattacg gggtcattag 60

ttcatagccc atatatggag ttccgcgtta cataacttac ggtaaatggc ccgcctggct 120

gaccgcccaa cgacccccgc ccattgacgt caataatgac gtatgttccc atagtaacgc 180

caatagggac tttccattga cgtcaatggg tggactattt acggtaaact gcccacttgg 240

cagtacatca agtgtatcat atgccaagta cgccccctat tgacgtcaat gacggtaaat 300

ggcccgcctg gcattatgcc cagtacatga ccttatggga ctttcctact tggcagtaca 360

tctacgtatt agtcatcgct attaccatgg tgatgcggtt ttggcagtac atcaatgggc 420

gtggatagcg gtttgactca cggggatttc caagtctcca ccccattgac gtcaatggga 480

gtttgttttg gcaccaaaat caacgggact ttccaaaatg tcgtaacaac tccgccccat 540

tgacgcaaat gggcggtagg cgtgtacggt gggaggtcta tataagcaga gctctctggc 600

taactagaga acccactgct tactggctta tcgaaattaa tacgactcac tatagggaga 660

cccaagcttg gtaccgagct cggatccact agtaacggcc gccagtgtgc tggaattcca 720

gcacactggc ggccgctcga gcatgcatct agagggccct attctatagt gtcacctaaa 780

tgctagagct cgctgatcag cctcgactgt gccttctagt tgccagccat ctgttgtttg 840

cccctccccc gtgccttcct tgaccctgga aggtgccact cccactgtcc tttcctaata 900

aaatgaggaa attgcatcgc attgtctgag taggtgtcat tctattctgg ggggtggggt 960

ggggcaggac agcaaggggg aggattggga agacaatagc aggcatgctg gggatgcggt 1020

gggctctatg gcttctgagg cggaaagaac cagtggcggt aatacggtta tccacagaat 1080

cagggggataa cgcaggaaag aacatgtgag caaaaggcca gcaaaaggcc aggaaccgta 1140

aaaaggccgc gttgctggcg tttttccata ggctccgccc ccctgacgag catcacaaaa 1200

atcgacgctc aagtcagagg tggcgaaacc cgacaggact ataaagatac caggcgtttc 1260

cccctggaag ctccctcgtg cgctctcctg ttccgaccct gccgcttacc ggatacctgt 1320

ccgcctttct cccttcggga agcgtggcgc tttctcatag ctcacgctgt aggtatctca 1380

gttcggtgta ggtcgttcgc tccaagctgg gctgtgtgca cgaacccccc gttcagcccg 1440

accgctgcgc cttatccggt aactatcgtc ttgagtccaa cccggtaaga cacgacttat 1500

cgccactggc agcagccact ggtaacagga ttagcagagc gaggtatgta ggcggtgcta 1560

cagagttctt gaagtggtgg cctaactacg gctacactag aaggacagta tttggtatct 1620

gcgctctgct gaagccagtt accttcggaa aaagagttgg tagctcttga tccggcaaac 1680

aaaccaccgc tggtagcggt ggtttttttg tttgcaagca gcagattacg cgcagaaaaa 1740

aaggatctca agaagatcct ttgatctttt ctacggggtc tgacgctcag tggaacgaaa 1800

actcacgtta agggattttg gtcatgagat tatcaaaaag gatcttcacc tagatccttt 1860

taaattaaaa atgaagtttt aaatcaatct aaagtatata tgagtaacct gaggtcagaa 1920

gaactcgtca agaaggcgat agaaggcgat gcgctgcgaa tcgggagcgg cgataccgta 1980

aagcacgagg aagcggtcag cccattcgcc gccaagctct tcagcaatat cacgggtagc 2040

caacgctatg tcctgatagc ggtccgccac acccagccgg ccacagtcga tgaatccaga 2100

aaagcggcca ttttccacca tgatattcgg caagcaggca tcgccatggg tcacgacgag 2160

atcctcgccg tcgggcatgc tcgccttgag cctggcgaac agttcggctg gcgcgagccc 2220

ctgatgctct tcgtccagat catcctgatc gacaagaccg gcttccatcc gagtacgtgc 2280

tcgctcgatg cgatgtttcg cttggtggtc gaatgggcag gtagccggat caagcgtatg 2340

cagccgccgc attgcatcag ccatgatgga tactttctcg gcaggagcaa ggtgagatga 2400

caggagatcc tgccccggca cttcgcccaa tagcagccag tcccttcccg cttcagtgac 2460

aacgtcgagc acagctgcgc aaggaacgcc cgtcgtggcc agccacgata gccgcgctgc 2520

ctcgtcttgc agttcattca gggcaccgga caggtcggtc ttgacaaaaa gaaccgggcg 2580

cccctgcgct gacagccgga acacggcggc atcagagcag ccgattgtct gttgtgccca 2640

gtcatagccg aatagcctct ccacccaagc ggccggagaa cctgcgtgca atccatcttg 2700

ttcaatcatg cgaaacgatc ctcatcctgt ctcttgatca gagcttgatc ccctgcgcca 2760

tcagatcctt ggcggcaaga aagccatcca gtttactttg cagggcttcc caaccttacc 2820

agagggcgcc ccagctggca attccggttc gcttgctgtc cataaaaccg cccagtctag 2880

ctatcgccat gtaagcccac tgcaa 2905

<210> 64
<211> 3451
<212> DNA
<213> Artificial Sequence

<220>
<223> pBest

<400> 64
ggccgctgcg gccgtttaaa cgccagcaac gcggcctttt tacggttcct ggccttttgc 60

tggccttttg ctcttaatta aatcgcatca tcaataatat accttatttt ggattgaagc 120

caatatgata atgagggggt ggagtttgtg acgtggcgcg gggcgtggga acggggcggg 180

tgacgtagta gtgtggcgga agtgtgatgt tgcaagtgtg gcggaacaca tgtaagcgac 240

ggatgtggca aaagtgacgt ttttggtgtg cgccggtgta cacaggaagt gacaattttc 300

gcgcggtttt aggcggatgt tgtagtaaat ttgggcgtaa ccgagtaaga tttggccatt 360

ttcgcgggaa aactgaataa gaggaagtga aatctgaata attttgtgtt actcatagcg 420

cgtaatattt gtctagggcc gcggggactt tgaccgttta cgtggagact cgcccaggtg 480

tttttctcag gtgttttccg cgttccgggt caaagttggc gttttattat tatagtcagc 540

tgacgtgtag tgtcgagtgt tgacattgat tattgactag ttattaatag taatcaatta 600

cggggtcatt agttcatagc ccatatatgg agttccgcgt tacataactt acggtaaatg 660

gcccgcctgg ctgaccgccc aacgaccccc gcccattgac gtcaataatg acgtatgttc 720

ccatagtaac gccaataggg actttccatt gacgtcaatg ggtggagtat ttacggtaaa 780

ctgcccactt ggcagtacat caagtgtatc atatgccaag tacgccccct attgacgtca 840

atgacggtaa atggcccgcc tggcattatg cccagtacat gaccttatgg gactttccta 900

cttggcagta catctacgta ttagtcatcg ctattaccat ggtgatgcgg ttttggcagt 960

acatcaatgg gcgtggatag cggtttgact cacggggatt tccaagtctc caccccattg 1020

acgtcaatgg gagtttgttt tggcaccaaa atcaacggga ctttccaaaa tgtcgtaaca 1080

actccgcccc attgacgcaa atgggcggta ggcgtgtacg gtgggaggtc tatataagca 1140

gagctctctg gctaactaga gaacccactg cttactggct tatcgaaatt aatacgactc 1200

actataggga cccaagcttg gtaccgagct cggatccact agtaacggcc gccagtgtgc 1260

tggaattctg cagatatcca tcacactggc ggccgctcga gcatgcatct agagggccct 1320

attcaattct ttgtagaggt tttacttgct ttaaaaaacc tcccacacct ccccctgaac 1380

ctgaaacata aaatgaatgc aattgttgtt gttaacttgt ttattgcagc ttataatggt 1440

tacaaataaa gcaatagcat cacaaatttc acaaataaag catttttttc actgcattct 1500

agttgtggtt tgtccaaact catcaatgta tcttatcatg tctggatcat cgatccataa 1560

cttcgtataa tgtatgctat acgaagttat ccagatctcg atttaagggt gggaaagaat 1620

atataaggtg ggggtcttat gtagtttaaa cgctagcagt gggcttacat ggcgatagct 1680

agactgggcg gttttatgga cagcaagcga accggaattg ccagctgggg cgccctctgg 1740

taaggttggg aagccctgca aagtaaactg gatggctttc ttgccgccaa ggatctgatg 1800

gcgcagggga tcaagctctg atcaagagac aggatgagga tcgtttcgca tgattgaaca 1860

agatggattg cacgcaggtt ctccggccgc ttgggtggag aggctattcg gctatgactg 1920

ggcacaacag acaatcggct gctctgatgc cgccgtgttc cggctgtcag cgcaggggcg 1980

cccggttctt tttgtcaaga ccgacctgtc cggtgccctg aatgaactgc aagacgaggc 2040

agcgcggcta tcgtggctgg ccacgacggg cgttccttgc gcagctgtgc tcgacgttgt 2100

cactgaagcg ggaagggact ggctgctatt gggcgaagtg ccggggcagg atctcctgtc 2160

atctcacctt gctcctgccg agaaagtatc catcatggct gatgcaatgc ggcggctgca 2220

tacgcttgat ccggctacct gcccattcga ccaccaagcg aaacatcgca tcgagcgagc 2280

acgtactcgg atggaagccg gtcttgtcga tcaggatgat ctggacgaag agcatcaggg 2340

gctcgcgcca gccgaactgt tcgccaggct caaggcgagc atgcccgacg gcgaggatct 2400

cgtcgtgacc catggcgatg cctgcttgcc gaatatcatg gtggaaaatg gccgcttttc 2460

tggattcatc gactgtggcc ggctgggtgt ggcggaccgc tatcaggaca tagcgttggc 2520

tacccgtgat attgctgaag agcttggcgg cgaatgggct gaccgcttcc tcgtgcttta 2580

cggtatcgcc gctcccgatt cgcagcgcat cgccttctat cgccttcttg acgagttctt 2640

ctgacctcag gttactcata tatactttag attgatttaa aacttcattt ttaatttaaa 2700

aggatctagg tgaagatcct ttttgataat ctcatgacca aaatccctta acgtgagttt 2760

tcgttccact gagcgtcaga ccccgtagaa aagatcaaag gatcttcttg agatcctttt 2820

tttctgcgcg taatctgctg cttgcaaaca aaaaaaccac cgctaccagc ggtggtttgt 2880

ttgccggatc aagagctacc aactcttttt ccgaaggtaa ctggcttcag cagagcgcag 2940

ataccaaata ctgtccttct agtgtagccg tagttaggcc accacttcaa gaactctgta 3000

gcaccgccta catacctcgc tctgctaatc ctgttaccag tggctgctgc cagtggcgat 3060

aagtcgtgtc ttaccgggtt ggactcaaga cgatagttac cggataaggc gcagcggtcg 3120

ggctgaacgg ggggttcgtg cacacagccc agcttggagc gaacgaccta caccgaactg 3180

agatacctac agcgtgagct atgagaaagc gccacgcttc ccgaagggag aaaggcggac 3240

aggtatccgg taagcggcag ggtcggaaca ggagagcgca cgagggagct tccaggggga 3300

aacgcctggt atctttatag tcctgtcggg tttcgccacc tctgacttga gcgtcgattt 3360

ttgtgatgct cgtcaggggg gcggagccta tggaaaaacg ccagcaacgc ggccttttta 3420

cggttcctgg ccttttgctg gccttttgct c 3451

<210> 65
<211> 15804
<212> DNA
<213> Artificial Sequence

<220>
<223> 3rd DNA fragment

<400> 65
gtatacagac ttgagaggcc tgtcctcgag cggtgttccg cggtcctcct cgtatagaaa 60

ctcggaccac tctgagacaa aggctcgcgt ccaggccagc acgaaggagg ctaagtggga 120

ggggtagcgg tcgttgtcca ctagggggtc cactcgctcc agggtgtgaa gacacatgtc 180

gccctcttcg gcatcaagga aggtgattgg tttgtaggtg taggccacgt gaccgggtgt 240

tcctgaaggg gggctataaa aggggggtggg ggcgcgttcg tcctcactct cttccgcatc 300

gctgtctgcg agggccagct gttggggtga gtactccctc tgaaaagcgg gcatgacttc 360

tgcgctaaga ttgtcagttt ccaaaaacga ggaggatttg atattcacct ggcccgcggt 420

gatgcctttg agggtggccg catccatctg gtcagaaaag acaatctttt tgttgtcaag 480

cttggtggca aacgacccgt agagggcgtt ggacagcaac ttggcgatgg agcgcagggt 540

ttggtttttg tcgcgatcgg cgcgctcctt ggccgcgatg tttagctgca cgtattcgcg 600

cgcaacgcac cgccattcgg gaaagacggt ggtgcgctcg tcgggcacca ggtgcacgcg 660

ccaaccgcgg ttgtgcaggg tgacaaggtc aacgctggtg gctacctctc cgcgtaggcg 720

ctcgttggtc cagcagaggc ggccgccctt gcgcgagcag aatggcggta gggggtctag 780

ctgcgtctcg tccggggggt ctgcgtccac ggtaaagacc ccgggcagca ggcgcgcgtc 840

gaagtagtct atcttgcatc cttgcaagtc tagcgcctgc tgccatgcgc gggcggcaag 900

cgcgcgctcg tatgggttga gtgggggacc ccatggcatg gggtgggtga gcgcggaggc 960

gtacatgccg caaatgtcgt aaacgtagag gggctctctg agtattccaa gatatgtagg 1020

gtagcatctt ccaccgcgga tgctggcgcg cacgtaatcg tatagttcgt gcgagggagc 1080

gaggaggtcg ggaccgaggt tgctacgggc gggctgctct gctcggaaga ctatctgcct 1140

gaagatggca tgtgagttgg atgatatggt tggacgctgg aagacgttga agctggcgtc 1200

tgtgagacct accgcgtcac gcacgaagga ggcgtaggag tcgcgcagct tgttgaccag 1260

ctcggcggtg acctgcacgt ctagggcgca gtagtccagg gtttccttga tgatgtcata 1320

cttatcctgt cccttttttt tccacagctc gcggttgagg acaaactctt cgcggtcttt 1380

ccagtactct tggatcggaa acccgtcggc ctccgaacgg taagagccta gcatgtagaa 1440

ctggttgacg gcctggtagg cgcagcatcc cttttctacg ggtagcgcgt atgcctgcgc 1500

ggccttccgg agcgaggtgt gggtgagcgc aaaggtgtcc ctgaccatga ctttgaggta 1560

ctggtatttg aagtcagtgt cgtcgcatcc gccctgctcc cagagcaaaa agtccgtgcg 1620

ctttttggaa cgcggatttg gcagggcgaa ggtgacatcg ttgaagagta tctttcccgc 1680

gcgaggcata aagttgcgtg tgatgcggaa gggtcccggc acctcggaac ggttgttaat 1740

tacctgggcg gcgagcacga tctcgtcaaa gccgttgatg ttgtggccca caatgtaaag 1800

ttccaagaag cgcgggatgc ccttgatgga aggcaatttt ttaagttcct cgtaggtgag 1860

ctcttcaggg gagctgagcc cgtgctctga aagggcccag tctgcaagat gagggttgga 1920

agcgacgaat gagctccaca ggtcacgggc cattagcatt tgcaggtggt cgcgaaaggt 1980

cctaaactgg cgacctatgg ccattttttc tggggtgatg cagtagaagg taagcgggtc 2040

ttgttcccag cggtcccatc caaggttcgc ggctaggtct cgcgcggcag tcactagagg 2100

ctcatctccg ccgaacttca tgaccagcat gaagggcacg agctgcttcc caaaggcccc 2160

catccaagta taggtctcta catcgtaggt gacaaagaga cgctcggtgc gaggatgcga 2220

gccgatcggg aagaactgga tctcccgcca ccaattggag gagtggctat tgatgtggtg 2280

aaagtagaag tccctgcgac gggccgaaca ctcgtgctgg cttttgtaaa aacgtgcgca 2340

gtactggcag cggtgcacgg gctgtacatc ctgcacgagg ttgacctgac gaccgcgcac 2400

aaggaagcag agtgggaatt tgagcccctc gcctggcggg tttggctggt ggtcttctac 2460

ttcggctgct tgtccttgac cgtctggctg ctcgagggga gttacggtgg atcggaccac 2520

cacgccgcgc gagcccaaag tccagatgtc cgcgcgcggc ggtcggagct tgatgacaac 2580

atcgcgcaga tgggagctgt ccatggtctg gagctcccgc ggcgtcaggt caggcgggag 2640

ctcctgcagg tttacctcgc atagacgggt cagggcgcgg gctagatcca ggtgatacct 2700

aatttccagg ggctggttgg tggcggcgtc gatggcttgc aagaggccgc atccccgcgg 2760

cgcgactacg gtaccgcgcg gcgggcggtg ggccgcgggg gtgtccttgg atgatgcatc 2820

taaaagcggt gacgcgggcg agcccccgga ggtagggggg gctccggacc cgccgggaga 2880

gggggcaggg gcacgtcggc gccgcgcgcg ggcaggagct ggtgctgcgc gcgtaggttg 2940

ctggcgaacg cgacgacgcg gcggttgatc tcctgaatct ggcgcctctg cgtgaagacg 3000

acgggcccgg tgagcttgag cctgaaagag agttcgacag aatcaatttc ggtgtcgttg 3060

acggcggcct ggcgcaaaat ctcctgcacg tctcctgagt tgtcttgata ggcgatctcg 3120

gccatgaact gctcgatctc ttcctcctgg agatctccgc gtccggctcg ctccacggtg 3180

gcggcgaggt cgttggaaat gcgggccatg agctgcgaga aggcgttgag gcctccctcg 3240

ttccagacgc ggctgtagac cacgcccccct tcggcatcgc gggcgcgcat gaccacctgc 3300

gcgagattga gctccacgtg ccgggcgaag acggcgtagt ttcgcaggcg ctgaaagagg 3360

tagttgaggg tggtggcggt gtgttctgcc acgaagaagt acataaccca gcgtcgcaac 3420

gtggattcgt tgatatcccc caaggcctca aggcgctcca tggcctcgta gaagtccacg 3480
gcgaagttga aaaactggga gttgcgcgcc gacacggtta actcctcctc cagaagacgg 3540
atgagctcgg cgacagtgtc gcgcacctcg cgctcaaagg ctacaggggc ctcttcttct 3600
tcttcaatct cctcttccat aagggcctcc ccttcttctt cttctggcgg cggtggggga 3660
gggggacac ggcggcgacg acggcgcacc gggaggcggt cgacaaagcg ctcgatcatc 3720
tccccgcggc gacggcgcat ggtctcggtg acggcgcggc cgttctcgcg ggggcgcagt 3780
tggaagacgc cgcccgtcat gtcccggtta tgggttggcg gggggctgcc atgcggcagg 3840
gatacggcgc taacgatgca tctcaacaat tgttgtgtag gtactccgcc gccgagggac 3900
ctgagcgagt ccgcatcgac cggatcggaa aacctctcga gaaaggcgtc taaccagtca 3960
cagtcgcaag gtaggctgag caccgtggcg ggcggcagcg ggcggcggtc ggggttgttt 4020
ctggcggagg tgctgctgat gatgtaatta aagtaggcgg tcttgagacg gcggatggtc 4080
gacagaagca ccatgtcctt gggtccggcc tgctgaatgc gcaggcggtc ggccatgccc 4140
caggcttcgt tttgacatcg gcgcaggtct ttgtagtagt cttgcatgag cctttctacc 4200
ggcacttctt cttctccttc ctcttgtcct gcatctcttg catctatcgc tgcggcggcg 4260
gcggagtttg gccgtaggtg gcgccctctt cctcccatgc gtgtgacccc gaagcccctc 4320
atcggctgaa gcagggctag gtcggcgaca acgcgctcgg ctaatatggc ctgctgcacc 4380
tgcgtgaggg tagactggaa gtcatccatg tccacaaagc ggtggtatgc gcccgtgttg 4440
atggtgtaag tgcagttggc cataacggac cagttaacgg tctggtgacc cggctgcgag 4500
agctcggtgt acctgagacg cgagtaagcc ctcgagtcaa atacgtagtc gttgcaagtc 4560
cgcaccaggt actggtatcc caccaaaaag tgcggcggcg gctggcggta gaggggccag 4620
cgtagggtgg ccggggctcc gggggcgaga tcttccaaca taaggcgatg atatccgtag 4680
atgtacctgg acatccaggt gatgccggcg gcggtggtgg aggcgcgcgg aaagtcgcgg 4740
acgcggttcc agatgttgcg cagcggcaaa aagtgctcca tggtcgggac gctctggccg 4800
gtcaggcgcg cgcaatcgtt gacgctctag accgtgcaaa aggagagcct gtaagcgggc 4860
actcttccgt ggtctggtgg ataaattcgc aagggtatca tggcggacga ccggggttcg 4920
agccccgtat ccggccgtcc gccgtgatcc atgcggttac cgcccgcgtg tcgaacccag 4980
gtgtgcgacg tcagacaacg ggggagtgct ccttttggct tccttccagg cgcggcggct 5040
gctgcgctag ctttttggc cactggccgc gcgcagcgta agcggttagg ctggaaagcg 5100
aaagcattaa gtggctcgct ccctgtagcc ggagggttat tttccaaggg ttgagtcgcg 5160
ggacccccgg ttcgagtctc ggaccggccg gactgcggcg aacgggggtt tgcctccccg 5220
tcatgcaaga ccccgcttgc aaattcctcc ggaaacaggg acgagcccct tttttgcttt 5280
tcccagatgc atccggtgct gcggcagatg cgccccctc ctcagcagcg gcaagagcaa 5340

gagcagcggc agacatgcag ggcaccctcc cctcctccta ccgcgtcagg aggggcgaca 5400

tccgcggttg acgcggcagc agatggtgat tacgaacccc cgcggcgccg ggcccggcac 5460

tacctggact tggaggaggg cgagggcctg gcgcggctag gagcgccctc tcctgagcgg 5520

tacccaaggg tgcagctgaa gcgtgatacg cgtgaggcgt acgtgccgcg gcagaacctg 5580

tttcgcgacc gcgagggaga ggagcccgag gagatgcggg atcgaaagtt ccacgcaggg 5640

cgcgagctgc ggcatggcct gaatcgcgag cggttgctgc gcgaggagga ctttgagccc 5700

gacgcgcgaa ccgggattag tcccgcgcgc gcacacgtgg cggccgccga cctggtaacc 5760

gcatacgagc agacggtgaa ccaggagatt aactttcaaa aaagctttaa caaccacgtg 5820

cgtacgcttg tggcgcgcga ggaggtggct ataggactga tgcatctgtg ggactttgta 5880

agcgcgctgg agcaaaaccc aaatagcaag ccgctcatgg cgcagctgtt ccttatagtg 5940

cagcacagca gggacaacga ggcattcagg gatgcgctgc taaacatagt agagcccgag 6000

ggccgctggc tgctcgattt gataaacatc ctgcagagca tagtggtgca ggagcgcagc 6060

ttgagcctgg ctgacaaggt ggccgccatc aactattcca tgcttagcct gggcaagttt 6120

tacgcccgca agatatacca taccccttac gttcccatag acaaggaggt aaagatcgag 6180

gggttctaca tgcgcatggc gctgaaggtg cttaccttga gcgacgacct gggcgtttat 6240

cgcaacgagc gcatccacaa ggccgtgagc gtgagccggc ggcgcgagct cagcgaccgc 6300

gagctgatgc acagcctgca aagggccctg gctggcacgg gcagcggcga tagagaggcc 6360

gagtcctact ttgacgcggg cgctgacctg cgctgggccc caagccgacg cgccctggag 6420

gcagctgggg ccggacctgg gctggcggtg gcacccgcgc gcgctggcaa cgtcggcggc 6480

gtggaggaat atgacgagga cgatgagtac gagccagagg acggcgagta ctaagcggtg 6540

atgtttctga tcagatgatg caagacgcaa cggacccggc ggtgcgggcg gcgctgcaga 6600

gccagccgtc cggccttaac tccacggacg actggcgcca ggtcatggac cgcatcatgt 6660

cgctgactgc gcgcaatcct gacgcgttcc ggcagcagcc gcaggccaac cggctctccg 6720

caattctgga agcggtggtc ccggcgcgcg caaaccccac gcacgagaag gtgctggcga 6780

tcgtaaacgc gctggccgaa aacagggcca tccggcccga cgaggccggc ctggtctacg 6840

acgcgctgct tcagcgcgtg gctcgttaca acagcggcaa cgtgcagacc aacctggacc 6900

ggctggtggg ggatgtgcgc gaggccgtgg cgcagcgtga gcgcgcgcag cagcagggca 6960

acctgggctc catggttgca ctaaacgcct tcctgagtac acagcccgcc aacgtgccgc 7020

ggggacagga ggactacacc aactttgtga gcgcactgcg gctaatggtg actgagacac 7080

cgcaaagtga ggtgtaccag tctgggccag actatttttt ccagaccagt agacaaggcc 7140

tgcagaccgt aaacctgagc caggctttca aaaacttgca ggggctgtgg ggggtgcggg 7200

ctcccacagg cgaccgcgcg accgtgtcta gcttgctgac gcccaactcg cgcctgttgc 7260
tgctgctaat agcgcccttc acggacagtg gcagcgtgtc ccgggacaca tacctaggtc 7320
acttgctgac actgtaccgc gaggccatag gtcaggcgca tgtggacgag catactttcc 7380
aggagattac aagtgtcagc cgcgcgctgg ggcaggagga cacgggcagc ctggaggcaa 7440
ccctaaacta cctgctgacc aaccggcggc agaagatccc ctcgttgcac agtttaaaca 7500
gcgaggagga gcgcattttg cgctacgtgc agcagagcgt gagccttaac ctgatgcgcg 7560
acggggtaac gcccagcgtg gcgctggaca tgaccgcgcg caacatggaa ccgggcatgt 7620
atgcctcaaa ccggccgttt atcaaccgcc taatggacta cttgcatcgc gcggccgccg 7680
tgaaccccga gtatttcacc aatgccatct tgaacccgca ctggctaccg ccccctggtt 7740
tctacaccgg gggattcgag gtgcccgagg gtaacgatgg attcctctgg gacgacatag 7800
acgacagcgt gttttccccg caaccgcaga ccctgctaga gttgcaacag cgcgagcagg 7860
cagaggcggc gctgcgaaag gaaagcttcc gcaggccaag cagcttgtcc gatctaggcg 7920
ctgcggcccc gcggtcagat gctagtagcc catttccaag cttgataggg tctcttacca 7980
gcactcgcac cacccgcccg cgcctgctgg gcgaggagga gtacctaaac aactcgctgc 8040
tgcagccgca gcgcgaaaaa aacctgcctc cggcatttcc caacaacggg atagagagcc 8100
tagtggacaa gatgagtaga tggaagacgt acgcgcagga gcacagggac gtgccaggcc 8160
cgcgcccgcc cacccgtcgt caaaggcacg accgtcagcg gggtctggtg tgggaggacg 8220
atgactcggc agacgacagc agcgtcctgg atttgggagg gagtggcaac ccgtttgcgc 8280
accttcgccc caggctgggg agaatgtttt aaaaaaaaaa aagcatgatg caaaataaaa 8340
aactcaccaa ggccatggca ccgagcgttg gttttcttgt attcccctta gtatgcggcg 8400
cgcggcgatg tatgaggaag gtcctcctcc ctcctacgag agtgtggtga gcgcggcgcc 8460
agtggcggcg gcgctgggtt ctcccttcga tgctcccctg gacccgccgt ttgtgcctcc 8520
gcggtacctg cggcctaccg gggggagaaa cagcatccgt tactctgagt tggcacccct 8580
attcgacacc acccgtgtgt acctggtgga caacaagtca acggatgtgg catccctgaa 8640
ctaccagaac gaccacagca actttctgac cacggtcatt caaaacaatg actacagccc 8700
gggggaggca agcacacaga ccatcaatct tgacgaccgg tcgcactggg gcggcgacct 8760
gaaaaccatc ctgcatacca acatgccaaa tgtgaacgag ttcatgttta ccaataagtt 8820
taaggcgcgg gtgatggtgt cgcgcttgcc tactaaggac aatcaggtgg agctgaaata 8880
cgagtgggtg gagttcacgc tgcccgaggg caactactcc gagaccatga ccatagacct 8940
tatgaacaac gcgatcgtgg agcactactt gaaagtgggc agacagaacg gggttctgga 9000
aagcgacatc ggggtaaagt ttgacacccg caacttcaga ctggggtttg accccgtcac 9060
tggtcttgtc atgcctgggg tatatacaaa cgaagccttc catccagaca tcattttgct 9120

gccaggatgc ggggtggact tcacccacag ccgcctgagc aacttgttgg gcatccgcaa 9180
gcggcaaccc ttccaggagg gctttaggat cacctacgat gatctggagg gtggtaacat 9240
tcccgcactg ttggatgtgg acgcctacca ggcgagcttg aaagatgaca ccgaacaggg 9300
cgggggtggc gcaggcggca gcaacagcag tggcagcggc gcggaagaga actccaacgc 9360
ggcagccgcg gcaatgcagc cggtggagga catgaacgat catgccattc gcggcgacac 9420
ctttgccaca cgggctgagg agaagcgcgc tgaggccgaa gcagcggccg aagctgccgc 9480
ccccgctgcg caacccgagg tcgagaagcc tcagaagaaa ccggtgatca aacccctgac 9540
agaggacagc aagaaacgca gttacaacct aataagcaat gacagcacct tcacccagta 9600
ccgcagctgg taccttgcat acaactacgg cgaccctcag accggaatcc gctcatggac 9660
cctgctttgc actcctgacg taacctgcgg ctcggagcag gtctactggt cgttgccaga 9720
catgatgcaa gaccccgtga ccttccgctc cacgcgccag atcagcaact ttccggtggt 9780
gggcgccgag ctgttgcccg tgcactccaa gagcttctac aacgaccagg ccgtctactc 9840
ccaactcatc cgccagttta cctctctgac ccacgtgttc aatcgctttc ccgagaacca 9900
gattttggcg cgcccgccag cccccaccat caccaccgtc agtgaaaacg ttcctgctct 9960
cacagatcac gggacgctac cgctgcgcaa cagcatcgga ggagtccagc gagtgaccat 10020
tactgacgcc agacgccgca cctgccccta cgtttacaag gccctgggca tagtctcgcc 10080
gcgcgtccta tcgagccgca ctttttgagc aagcatgtcc atccttatat cgcccagcaa 10140
taacacaggc tggggcctgc gcttcccaag caagatgttt ggcggggcca agaagcgctc 10200
cgaccaacac ccagtgcgcg tgcgcgggca ctaccgcgcg ccctggggcg cgcacaaacg 10260
cggccgcact gggcgcacca ccgtcgatga cgccatcgac gcggtggtgg aggaggcgcg 10320
caactacacg cccacgccgc caccagtgtc cacagtggac gcggccattc agaccgtggt 10380
gcgcggagcc cggcgctatg ctaaaatgaa gagacggcgg aggcgcgtag cacgtcgcca 10440
ccgccgccga cccggcactg ccgcccaacg cgcggcggcg gccctgctta accgcgcacg 10500
tcgcaccggc cgacgggcgg ccatgcgggc cgctcgaagg ctggccgcgg gtattgtcac 10560
tgtgcccccc aggtccaggc gacgagcggc cgccgcagca gccgcggcca ttagtgctat 10620
gactcagggt cgcaggggca acgtgtattg ggtgcgcgac tcggttagcg gcctgcgcgt 10680
gcccgtgcgc acccgccccc cgcgcaacta gattgcaaga aaaaactact tagactcgta 10740
ctgttgtatg tatccagcgg cggcggcgcg caacgaagct atgtccaagc gcaaaatcaa 10800
agaagagatg ctccaggtca tcgcgccgga gatctatggc cccccgaaga aggaagagca 10860
ggattacaag ccccgaaagc taaagcgggt caaaaagaaa aagaaagatg atgatgatga 10920
acttgacgac gaggtggaac tgctgcacgc taccgcgccc aggcgacggg tacagtggaa 10980

aggtcgacgc gtaaaacgtg ttttgcgacc cggcaccacc gtagtcttta cgcccggtga 11040

gcgctccacc cgcacctaca agcgcgtgta tgatgaggtg tacggcgacg aggacctgct 11100

tgagcaggcc aacgagcgcc tcggggagtt tgcctacgga aagcggcata aggacatgct 11160

ggcgttgccg ctggacgagg gcaacccaac acctagccta aagcccgtaa cactgcagca 11220

ggtgctgccc gcgcttgcac cgtccgaaga aaagcgcggc ctaaagcgcg agtctggtga 11280

cttggcaccc accgtgcagc tgatggtacc caagcgccag cgactggaag atgtcttgga 11340

aaaaatgacc gtggaacctg ggctggagcc cgaggtccgc gtgcggccaa tcaagcaggt 11400

ggcgccggga ctgggcgtgc agaccgtgga cgttcagata cccactacca gtagcaccag 11460

tattgccacc gccacagagg gcatggagac acaaacgtcc ccggttgcct cagcggtggc 11520

ggatgccgcg gtgcaggcgg tcgctgcggc cgcgtccaag acctctacgg aggtgcaaac 11580

ggacccgtgg atgtttcgcg tttcagcccc ccggcgcccg cgcggttcga ggaagtacgg 11640

cgccgccagc gcgctactgc ccgaatatgc cctacatcct tccattgcgc ctacccccgg 11700

ctatcgtggc tacacctacc gccccagaag acgagcaact acccgacgcc gaaccaccac 11760

tggaacccgc cgccgccgtc gccgtcgcca gcccgtgctg gccccgattt ccgtgcgcag 11820

ggtggctcgc gaaggaggca ggaccctggt gctgccaaca gcgcgctacc accccagcat 11880

cgtttaaaag ccggtctttg tggttcttgc agatatggcc ctcacctgcc gcctccgttt 11940

cccggtgccg ggattccgag gaagaatgca ccgtaggagg ggcatggccg gccacggcct 12000

gacgggcggc atgcgtcgtg cgcaccaccg gcggcggcgc gcgtcgcacc gtcgcatgcg 12060

cggcggtatc ctgcccctcc ttattccact gatcgccgcg gcgattggcg ccgtgcccgg 12120

aattgcatcc gtggccttgc aggcgcagag acactgatta aaaacaagtt gcatgtggaa 12180

aaatcaaaat aaaaagtctg gactctcacg ctcgcttggt cctgtaacta ttttgtagaa 12240

tggaagacat caactttgcg tctctggccc cgcgacacgg ctcgcgcccg ttcatgggaa 12300

actggcaaga tatcggcacc agcaatatga gcggtggcgc cttcagctgg ggctcgctgt 12360

ggagcggcat taaaaatttc ggttccaccg ttaagaacta tggcagcaag gcctggaaca 12420

gcagcacagg ccagatgctg agggataagt tgaaagagca aaatttccaa caaaaggtgg 12480

tagatggcct ggcctctggc attagcgggg tggtggacct ggccaaccag gcagtgcaaa 12540

ataagattaa cagtaagctt gatccccgcc ctcccgtaga ggagcctcca ccggccgtgg 12600

agacagtgtc tccagagggg cgtggcgaaa agcgtccgcg ccccgacagg gaagaaactc 12660

tggtgacgca aatagacgag cctccctcgt acgaggaggc actaaagcaa ggcctgccca 12720

ccacccgtcc catcgcgccc atggctaccg gagtgctggg ccagcacaca cccgtaacgc 12780

tggacctgcc tccccccgcc gacacccagc agaaacctgt gctgccaggc ccgaccgccg 12840

ttgttgtaac ccgtcctagc cgcgcgtccc tgcgccgcgc cgccagcggt ccgcgatcgt 12900

tgcggcccgt agccagtggc aactggcaaa gcacactgaa cagcatcgtg ggtctggggg 12960
tgcaatccct gaagcgccga cgatgcttct gaatagctaa cgtgtcgtat gtgtgtcatg 13020
tatgcgtcca tgtcgccgcc agaggagctg ctgagccgcc gcgcgcccgc tttccaagat 13080
ggctacccct tcgatgatgc cgcagtggtc ttacatgcac atctcgggcc aggacgcctc 13140
ggagtacctg agccccgggc tggtgcagtt tgcccgcgcc accgagacgt acttcagcct 13200
gaataacaag tttagaaacc ccacggtggc gcctacgcac gacgtgacca cagaccggtc 13260
ccagcgtttg acgctgcggt tcatccctgt ggaccgtgag gatactgcgt actcgtacaa 13320
ggcgcggttc accctagctg tgggtgataa ccgtgtgctg gacatggctt ccacgtactt 13380
tgacatccgc ggcgtgctgg acaggggccc tacttttaag ccctactctg gcactgccta 13440
caacgccctg gctcccaagg gtgccccaaa tccttgcgaa tgggatgaag ctgctactgc 13500
tcttgaaata aacctagaag aagaggacga tgacaacgaa gacgaagtag acgagcaagc 13560
tgagcagcaa aaaactcacg tatttgggca ggcgccttat tctggtataa atattacaaa 13620
ggagggtatt caaataggtg tcgaaggtca aacacctaaa tatgccgata aaacatttca 13680
acctgaacct caaataggag aatctcagtg gtacgaaact gaaattaatc atgcagctgg 13740
gagagtcctt aaaaagacta ccccaatgaa accatgttac ggttcatatg caaaacccac 13800
aaatgaaaat ggagggcaag gcattcttgt aaagcaacaa aatggaaagc tagaaagtca 13860
agtggaaatg caatttttct caactactga ggcgaccgca ggcaatggtg ataacttgac 13920
tcctaaagtg gtattgtaca gtgaagatgt agatatagaa accccagaca ctcatatttc 13980
ttacatgccc actattaagg aaggtaactc acgagaacta atgggccaac aatctatgcc 14040
caacaggcct aattacattg cttttaggga caattttatt ggtctaatgt attacaacag 14100
cacgggtaat atgggtgttc tggcgggcca agcatcgcag ttgaatgctg ttgtagattt 14160
gcaagacaga aacacagagc tttcatacca gcttttgctt gattccattg gtgatagaac 14220
caggtacttt tctatgtgga atcaggctgt tgacagctat gatccagatg ttagaattat 14280
tgaaaatcat ggaactgaag atgaacttcc aaattactgc tttccactgg gaggtgtgat 14340
taatacagag actcttacca aggtaaaacc taaaacaggt caggaaaatg gatgggaaaa 14400
agatgctaca gaattttcag ataaaaatga aataagagtt ggaaataatt ttgccatgga 14460
aatcaatcta aatgccaacc tgtggagaaa tttcctgtac tccaacatag cgctgtattt 14520
gcccgacaag ctaaagtaca gtccttccaa cgtaaaaatt tctgataacc caaacaccta 14580
cgactacatg aacaagcgag tggtggctcc cgggttagtg gactgctaca ttaaccttgg 14640
agcacgctgg tcccttgact atatggacaa cgtcaaccca tttaaccacc accgcaatgc 14700
tggcctgcgc taccgctcaa tgttgctggg caatggtcgc tatgtgccct tccacatcca 14760

ggtgcctcag aagttctttg ccattaaaaa cctccttctc ctgccgggct catacaccta 14820

cgagtggaac ttcaggaagg atgttaacat ggttctgcag agctccctag gaaatgacct 14880

aagggttgac ggagccagca ttaagtttga tagcatttgc ctttacgcca ccttcttccc 14940

catggcccac aacaccgcct ccacgcttga ggccatgctt agaaacgaca ccaacgacca 15000

gtcctttaac gactatctct ccgccgccaa catgctctac cctatacccg ccaacgctac 15060

caacgtgccc atatccatcc cctcccgcaa ctgggcggct ttccgcggct gggccttcac 15120

gcgccttaag actaaggaaa ccccatcact gggctcgggc tacgaccctt attacaccta 15180

ctctggctct ataccctacc tagatggaac cttttacctc aaccacacct ttaagaaggt 15240

ggccattacc tttgactctt ctgtcagctg gcctggcaat gaccgcctgc ttacccccaa 15300

cgagtttgaa attaagcgct cagttgacgg ggagggttac aacgttgccc agtgtaacat 15360

gaccaaagac tggttcctgg tacaaatgct agctaactac aacattggct accagggctt 15420

ctatatccca gagagctaca aggaccgcat gtactccttc tttagaaact tccagcccat 15480

gagccgtcag gtggtggatg atactaaata caaggactac caacaggtgg gcatcctaca 15540

ccaacacaac aactctggat ttgttggcta ccttgccccc accatgcgcg aaggacaggc 15600

ctaccctgct aacttcccct atccgcttat aggcaagacc gcagttgaca gcattaccca 15660

gaaaaagttt ctttgcgatc gcaccctttg gcgcatccca ttctccagta actttatgtc 15720

catgggcgca ctcacagacc tgggccaaaa ccttctctac gccaactccg cccacgcgct 15780

agacatgact tttgaggtgg atcc 15804

<210> 66
<211> 2248
<212> DNA
<213> Artificial Sequence

<220>
<223> 4th DNA fragment

<400> 66
gggatcgatt taagggtggg aaagaatata taaggtgggg gtcttatgta gttttgtatc 60

tgttttgcag cagccgccgc cgccatgagc accaactcgt ttgatggaag cattgtgagc 120

tcatatttga caacgcgcat gcccccatgg gccggggtgc gtcagaatgt gatgggctcc 180

agcattgatg gtcgccccgt cctgcccgca aactctacta ccttgaccta cgagaccgtg 240

tctggaacgc cgttggagac tgcagcctcc gccgccgctt cagccgctgc agccaccgcc 300

cgcgggattg tgactgactt tgctttcctg agcccgcttg caagcagtgc agcttcccgt 360

tcatccgccc gcgatgacaa gttgacggct cttttggcac aattggattc tttgacccgg 420

gaacttaatg tcgtttctca gcagctgttg gatctgcgcc agcaggtttc tgccctgaag 480

gcttcctccc ctcccaatgc ggtttaaaac ataaataaaa aaccagactc tgtttggatt 540

tggatcaagc aagtgtcttg ctgtctttat ttaggggttt tgcgcgcgcg gtaggcccgg 600

gaccagcggt ctcggtcgtt gagggtcctg tgtatttttt ccaggacgtg gtaaaggtga 660

ctctggatgt tcagatacat gggcataagc ccgtctctgg ggtggaggta gcaccactgc 720

agagcttcat gctgcggggt ggtgttgtag atgatccagt cgtagcagga gcgctgggcg 780

tggtgcctaa aaatgtcttt cagtagcaag ctgattgcca ggggcaggcc cttggtgtaa 840

gtgtttacaa agcggttaag ctgggatggg tgcatacgtg gggatatgag atgcatcttg 900

gactgtattt ttaggttggc tatgttccca gccatatccc tccggggatt catgttgtgc 960

agaaccacca gcacagtgta tccggtgcac ttgggaaatt tgtcatgtag cttagaagga 1020

aatgcgtgga agaacttgga gacgcccttg tgacctccaa gattttccat gcattcgtcc 1080

ataatgatgg caatgggccc acgggcggcg gcctgggcga agatatttct gggatcacta 1140

acgtcatagt tgtgttccag gatgagatcg tcataggcca tttttacaaa gcgcgggcgg 1200

agggtgccag actgcggtat aatggttcca tccggcccag gggcgtagtt accctcacag 1260

atttgcattt cccacgcttt gagttcagat ggggggatca tgtctacctg cggggcgatg 1320

aagaaaacgg tttccggggt aggggagatc agctgggaag aaagcaggtt cctgagcagc 1380

tgcgacttac cgcagccggt gggcccgtaa atcacaccta ttaccgggtg caactggtag 1440

ttaagagagc tgcagctgcc gtcatccctg agcagggggg ccacttcgtt aagcatgtcc 1500

ctgactcgca tgttttccct gaccaaatcc gccagaaggc gctcgccgcc cagcgatagc 1560

agttcttgca aggaagcaaa gtttttcaac ggtttgagac cgtccgccgt aggcatgctt 1620

ttgagcgttt gaccaagcag ttccaggcgg tcccacagct cggtcacctg ctctacggca 1680

tctcgatcca gcatatctcc tcgtttcgcg ggttggggcg gctttcgctg tacggcagta 1740

gtcggtgctc gtccagacgg gccagggtca tgtctttcca cgggcgcagg gtcctcgtca 1800

gcgtagtctg ggtcacggtg aaggggtgcg ctccgggctg cgcgctggcc agggtgcgct 1860

tgaggctggt cctgctggtg ctgaagcgct gccggtcttc gccctgcgcg tcggccaggt 1920

agcatttgac catggtgtca tagtccagcc cctccgcggc gtggcccttg gcgcgcagct 1980

tgcccttgga ggaggcgccg cacgaggggc agtgcagact tttgagggcg tagagcttgg 2040

gcgcgagaaa taccgattcc ggggagtagg catccgcgcc gcaggccccg cagacggtct 2100

cgcattccac gagccaggtg agctctggcc gttcggggtc aaaaaccagg tttcccccat 2160

gctttttgat gcgtttctta cctctggttt ccatgagccg gtgtccacgc tcggtgacga 2220

aaaggctgtc cgtgtccccg tatacggg 2248

<210> 67
<211> 12506

<212> DNA
<213> Artificial Sequence

<220>
<223> 5th DNA fragment

<400> 67
ggatcccatg gacgagccca cccttcttta tgttttgttt gaagtctttg acgtggtccg 60

tgtgcaccgg ccgcaccgcg gcgtcatcga aaccgtgtac ctgcgcacgc ccttctcggc 120

cggcaacgcc acaacataaa gaagcaagca acatcaacaa cagctgccgc catgggctcc 180

agtgagcagg aactgaaagc cattgtcaaa gatcttggtt gtgggccata tttttgggc 240

acctatgaca agcgctttcc aggctttgtt tctccacaca agctcgcctg cgccatagtc 300

aatacggccg gtcgcgagac tgggggcgta cactggatgg cctttgcctg gaacccgcac 360

tcaaaaacat gctacctctt tgagcccttt ggcttttctg accagcgact caagcaggtt 420

taccagtttg agtacgagtc actcctgcgc cgtagcgcca ttgcttcttc ccccgaccgc 480

tgtataacgc tggaaaagtc cacccaaagc gtacaggggc ccaactcggc cgcctgtgga 540

ctattctgct gcatgtttct ccacgccttt gccaactggc cccaaactcc catggatcac 600

aaccccacca tgaaccttat taccggggta cccaactcca tgctcaacag tccccaggta 660

cagcccaccc tgcgtcgcaa ccaggaacag ctctacagct tcctggagcg ccactcgccc 720

tacttccgca gccacagtgc gcagattagg agcgccactt ctttttgtca cttgaaaaac 780

atgtaaaaat aatgtactag agacactttc aataaaggca aatgctttta tttgtacact 840

ctcgggtgat tatttacccc cacccttgcc gtctgcgccg tttaaaaatc aaaggggttc 900

tgccgcgcat cgctatgcgc cactggcagg gacacgttgc gatactggtg tttagtgctc 960

cacttaaact caggcacaac catccgcggc agctcggtga agttttcact ccacaggctg 1020

cgcaccatca ccaacgcgtt tagcaggtcg ggcgccgata tcttgaagtc gcagttgggg 1080

cctccgccct gcgcgcgcga gttgcgatac acagggttgc agcactggaa cactatcagc 1140

gccgggtggt gcacgctggc cagcacgctc ttgtcggaga tcagatccgc gtccaggtcc 1200

tccgcgttgc tcagggcgaa cggagtcaac tttggtagct gccttcccaa aaagggcgcg 1260

tgcccaggct ttgagttgca ctcgcaccgt agtggcatca aaaggtgacc gtgcccggtc 1320

tgggcgttag gatacagcgc ctgcataaaa gccttgatct gcttaaaagc cacctgagcc 1380

tttgcgcctt cagagaagaa catgccgcaa gacttgccgg aaaactgatt ggccggacag 1440

gccgcgtcgt gcacgcagca ccttgcgtcg gtgttggaga tctgcaccac atttcggccc 1500

caccggttct tcacgatctt ggccttgcta gactgctcct tcagcgcgcg ctgcccgttt 1560

tcgctcgtca catccatttc aatcacgtgc tccttattta tcataatgct tccgtgtaga 1620

cacttaagct cgccttcgat ctcagcgcag cggtgcagcc acaacgcgca gcccgtgggc 1680

tcgtgatgct tgtaggtcac ctctgcaaac gactgcaggt acgcctgcag gaatcgcccc 1740

atcatcgtca caaaggtctt gttgctggtg aaggtcagct gcaacccgcg gtgctcctcg 1800

ttcagccagg tcttgcatac ggccgccaga gcttccactt ggtcaggcag tagtttgaag 1860

ttcgccttta gatcgttatc cacgtggtac ttgtccatca gcgcgcgcgc agcctccatg 1920

cccttctccc acgcagacac gatcggcaca ctcagcgggt tcatcaccgt aatttcactt 1980

tccgcttcgc tgggctcttc ctcttcctct tgcgtccgca taccacgcgc cactgggtcg 2040

tcttcattca gccgccgcac tgtgcgctta cctcctttgc catgcttgat tagcaccggt 2100

gggttgctga aacccaccat ttgtagcgcc acatcttctc tttcttcctc gctgtccacg 2160

attacctctg gtgatggcgg gcgctcgggc ttgggagaag ggcgcttctt tttcttcttg 2220

ggcgcaatgg ccaaatccgc cgccgaggtc gatggccgcg ggctgggtgt gcgcggcacc 2280

agcgcgtctt gtgatgagtc ttcctcgtcc tcggactcga tacgccgcct catccgcttt 2340

tttgggggcg cccggggagg cggcggcgac ggggacgggg acgacacgtc ctccatggtt 2400

gggggacgtc gcgccgcacc gcgtccgcgc tcgggggtgg tttcgcgctg ctcctcttcc 2460

cgactggcca tttccttctc ctataggcag aaaaagatca tggagtcagt cgagaagaag 2520

gacagcctaa ccgcccccctc tgagttcgcc accaccgcct ccaccgatgc cgccaacgcg 2580

cctaccacct tccccgtcga ggcacccccg cttgaggagg aggaagtgat tatcgagcag 2640

gacccaggtt ttgtaagcga agacgacgag gaccgctcag taccaacaga ggataaaaag 2700

caagaccagg acaacgcaga ggcaaacgag gaacaagtcg ggcgggggga cgaaaggcat 2760

ggcgactacc tagatgtggg agacgacgtg ctgttgaagc atctgcagcg ccagtgcgcc 2820

attatctgcg acgcgttgca agagcgcagc gatgtgcccc tcgccatagc ggatgtcagc 2880

cttgcctacg aacgccacct attctcaccg cgcgtacccc ccaaacgcca agaaaacggc 2940

acatgcgagc ccaacccgcg cctcaacttc taccccgtat ttgccgtgcc agaggtgctt 3000

gccacctatc acatcttttt ccaaaactgc aagatacccc tatcctgccg tgccaaccgc 3060

agccgagcgg acaagcagct ggccttgcgg cagggcgctg tcatacctga tatcgcctcg 3120

ctcaacgaag tgccaaaaat ctttgagggt cttggacgcg acgagaagcg cgcggcaaac 3180

gctctgcaac aggaaaacag cgaaaatgaa agtcactctg gagtgttggt ggaactcgag 3240

ggtgacaacg cgcgcctagc cgtactaaaa cgcagcatcg aggtcaccca ctttgcctac 3300

ccggcactta acctaccccc caaggtcatg agcacagtca tgagtgagct gatcgtgcgc 3360

cgtgcgcagc ccctggagag ggatgcaaat ttgcaagaac aaacagagga gggcctaccc 3420

gcagttggcg acgagcagct agcgcgctgg cttcaaacgc gcgagcctgc cgacttggag 3480

gagcgacgca aactaatgat ggccgcagtg ctcgttaccg tggagcttga gtgcatgcag 3540

cggttctttg ctgacccgga gatgcagcgc aagctagagg aaacattgca ctacaccttt 3600

cgacagggct acgtacgcca ggcctgcaag atctccaacg tggagctctg caacctggtc 3660

tcctaccttg gaattttgca cgaaaaccgc cttgggcaaa acgtgcttca ttccacgctc 3720

aagggcgagg cgcgccgcga ctacgtccgc gactgcgttt acttatttct atgctacacc 3780

tggcagacgg ccatgggcgt ttggcagcag tgcttggagg agtgcaacct caaggagctg 3840

cagaaactgc taaagcaaaa cttgaaggac ctatggacgg ccttcaacga gcgctccgtg 3900

gccgcgcacc tggcggacat cattttcccc gaacgcctgc ttaaaaccct gcaacagggt 3960

ctgccagact tcaccagtca aagcatgttg cagaacttta ggaactttat cctagagcgc 4020

tcaggaatct tgcccgccac ctgctgtgca cttcctagcg actttgtgcc cattaagtac 4080

cgcgaatgcc ctccgccgct ttggggccac tgctaccttc tgcagctagc caactacctt 4140

gcctaccact ctgacataat ggaagacgtg agcggtgacg gtctactgga gtgtcactgt 4200

cgctgcaacc tatgcacccc gcaccgctcc ctggtttgca attcgcagct gcttaacgaa 4260

agtcaaatta tcggtacctt tgagctgcag ggtccctcgc ctgacgaaaa gtccgcggct 4320

ccggggttga aactcactcc ggggctgtgg acgtcggctt accttcgcaa atttgtacct 4380

gaggactacc acgcccacga gattaggttc tacgaagacc aatcccgccc gccaaatgcg 4440

gagcttaccg cctgcgtcat tacccagggc cacattcttg gccaattgca agccatcaac 4500

aaagcccgcc aagagtttct gctacgaaag ggacgggggg tttacttgga cccccagtcc 4560

ggcgaggagc tcaacccaat ccccccgccg ccgcagccct atcagcagca gccgcgggcc 4620

cttgcttccc aggatggcac ccaaaaagaa gctgcagctg ccgccgccac ccacggacga 4680

ggaggaatac tgggacagtc aggcagagga ggttttggac gaggaggagg aggacatgat 4740

ggaagactgg gagagcctag acgaggaagc ttccgaggtc gaagaggtgt cagacgaaac 4800

accgtcaccc tcggtcgcat tcccctcgcc ggcgccccag aaatcggcaa ccggttccag 4860

catggctaca acctccgctc ctcaggcgcc gccggcactg cccgttcgcc gacccaaccg 4920

tagatgggac accactggaa ccagggccgg taagtccaag cagccgccgc cgttagccca 4980

agagcaacaa cagcgccaag gctaccgctc atggcgcggg cacaagaacg ccatagttgc 5040

ttgcttgcaa gactgtgggg gcaacatctc cttcgcccgc cgctttcttc tctaccatca 5100

cggcgtggcc ttcccccgta acatcctgca ttactaccgt catctctaca gcccatactg 5160

caccggcggc agcggcagcg gcagcaacag cagcggccac acagaagcaa aggcgaccgg 5220

atagcaagac tctgacaaag cccaagaaat ccacagcggc ggcagcagca ggaggaggag 5280

cgctgcgtct ggcgcccaac gaacccgtat cgacccgcga gcttagaaac aggatttttc 5340

ccactctgta tgctatattt caacagagca ggggccaaga acaagagctg aaaataaaaa 5400

acaggtctct gcgatccctc acccgcagct gcctgtatca caaaagcgaa gatcagcttc 5460

ggcgcacgct ggaagacgcg gaggctctct tcagtaaata ctgcgcgctg actcttaagg 5520

actagtttcg cgccctttct caaatttaag cgcgaaaact acgtcatctc cagcggccac 5580

acccggcgcc agcacctgtc gtcagcgcca ttatgagcaa ggaaattccc acgccctaca 5640

tgtggagtta ccagccacaa atgggacttg cggctggagc tgcccaagac tactcaaccc 5700

gaataaacta catgagcgcg ggaccccaca tgatatcccg ggtcaacgga atccgcgccc 5760

accgaaaccg aattctcttg gaacaggcgg ctattaccac cacacctcgt aataacctta 5820

atccccgtag ttggcccgct gccctggtgt accaggaaag tcccgctccc accactgtgg 5880

tacttcccag agacgcccag gccgaagttc agatgactaa ctcaggggcg cagcttgcgg 5940

gcggctttcg tcacagggtg cggtcgcccg ggcagggtat aactcacctg acaatcagag 6000

ggcgaggtat tcagctcaac gacgagtcgg tgagctcctc gcttggtctc cgtccggacg 6060

ggacatttca gatcggcggc gccggccgtc cttcattcac gcctcgtcag gcaatcctaa 6120

ctctgcagac ctcgtcctct gagccgcgct ctggaggcat tggaactctg caatttattg 6180

aggagtttgt gccatcggtc tactttaacc ccttctcggg acctcccggc cactatccgg 6240

atcaatttat tcctaacttt gacgcggtaa aggactcggc ggacggctac gactgaatgt 6300

taagtggaga ggcagagcaa ctgcgcctga aacacctggt ccactgtcgc cgccacaagt 6360

gctttgcccg cgactccggt gagttttgct actttgaatt gcccgaggat catatcgagg 6420

gcccggcgca cggcgtccgg cttaccgccc agggagagct tgcccgtagc ctgattcggg 6480

agtttaccca gcgcccctg ctagttgagc gggacagggg accctgtgtt ctcactgtga 6540

tttgcaactg tcctaacctt ggattacatc aagatctttg ttgccatctc tgtgctgagt 6600

ataataaata cagaaattaa aatatactgg ggctcctatc gccatcctgt aaacgccacc 6660

gtcttcaccc gcccaagcaa accaaggcga accttacctg gtacttttaa catctctccc 6720

tctgtgattt acaacagttt caacccagac ggagtgagtc tacgagagaa cctctccgag 6780

ctcagctact ccatcagaaa aaacaccacc ctccttacct gccgggaacg tacgagtgcg 6840

tcaccggccg ctgcaccaca cctaccgcct gaccgtaaac cagacttttt ccggacagac 6900

ctcaataact ctgtttacca gaacaggagg tgagcttaga aaacccttag ggtattaggc 6960

caaaggcgca gctactgtgg ggtttatgaa caattcaagc aactctacgg gctattctaa 7020

ttcaggtttc tctagaatct agaaatggac ggaattatta cagagcagcg cctgctagaa 7080

agacgcaggg cagcggccga gcaacagcgc atgaatcaag agctccaaga catggttaac 7140

ttgcaccagt gcaaaagggg tatcttttgt ctggtaaagc aggccaaagt cacctacgac 7200

agtaatacca ccggacaccg ccttagctac aagttgccaa ccaagcgtca gaaattggtg 7260

gtcatggtgg gagaaaagcc cattaccata actcagcact cggtagaaac cgaaggctgc 7320

attcactcac cttgtcaagg acctgaggat ctctgcaccc ttattaagac cctgtgcggt 7380
ctcaaagatc ttattccctt taactaataa aaaaaaataa taaagcatca cttacttaaa 7440
atcagttagc aaatttctgt ccagtttatt cagcagcacc tccttgccct cctcccagct 7500
ctggtattgc agcttcctcc tggctgcaaa ctttctccac aatctaaatg gaatgtcagt 7560
ttcctcctgt tcctgtccat ccgcacccac tatcttcatg ttgttgcaga tgaagcgcgc 7620
aagaccgtct gaagatacct tcaaccccgt gtatccatat gacacggaaa ccggtcctcc 7680
aactgtgcct tttcttactc ctccctttgt atcccccaat gggtttcaag agagtccccc 7740
tggggtactc tctttgcgcc tatccgaacc tctagttacc tccaatggca tgcttgcgct 7800
caaaatgggc aacggcctct ctctggacga ggccggcaac cttacctccc aaaatgtaac 7860
cactgtgagc ccacctctca aaaaaaccaa gtcaaacata aacctggaaa tatctgcacc 7920
cctcacagtt acctcagaag ccctaactgt ggctgccgcc gcacctctaa tggtcgcggg 7980
caacacactc accatgcaat cacaggcccc gctaaccgtg cacgactcca aacttagcat 8040
tgccacccaa ggacccctca cagtgtcaga aggaaagcta gccctgcaaa catcaggccc 8100
cctcaccacc accgatagca gtacccttac tatcactgcc tcacccccct taactactgc 8160
cactggtagc ttgggcattg acttgaaaga gcccatttat acacaaaatg gaaaactagg 8220
actaaagtac ggggctcctt tgcatgtaac agacgaccta aacactttga ccgtagcaac 8280
tggtccaggt gtgactatta ataatacttc cttgcaaact aaagttactg gagccttggg 8340
ttttgattca caaggcaata tgcaacttaa tgtagcagga ggactaagga ttgattctca 8400
aaacagacgc cttatacttg atgttagtta tccgtttgat gctcaaaacc aactaaatct 8460
aagactagga cagggccctc tttttataaa ctcagcccac aacttggata ttaactacaa 8520
caaaggcctt tacttgttta cagcttcaaa caattccaaa aagcttgagg ttaacctaag 8580
cactgccaag gggttgatgt ttgacgctac agccatagcc attaatgcag gagatgggct 8640
tgaatttggt tcacctaatg caccaaacac aaatcccctc aaaacaaaaa ttggccatgg 8700
cctagaattt gattcaaaca aggctatggt tcctaaacta ggaactggcc ttagttttga 8760
cagcacaggt gccattacag taggaaacaa aaataatgat aagctaactt tgtggaccac 8820
accagctcca tctcctaact gtagactaaa tgcagagaaa gatgctaaac tcactttggt 8880
cttaacaaaa tgtggcagtc aaatacttgc tacagtttca gttttggctg ttaaaggcag 8940
tttggctcca atatctggaa cagttcaaag tgctcatctt attataagat ttgacgaaaa 9000
tggagtgcta ctaaacaatt ccttcctgga cccagaatat tggaacttta gaaatggaga 9060
tcttactgaa ggcacagcct atacaaacgc tgttggattt atgcctaacc tatcagctta 9120
tccaaaatct cacggtaaaa ctgccaaaag taacattgtc agtcaagttt acttaaacgg 9180
agacaaaact aaacctgtaa cactaaccat tacactaaac ggtacacagg aaacaggaga 9240

cacaactcca agtgcatact ctatgtcatt ttcatgggac tggtctggcc acaactacat 9300

taatgaaata tttgccacat cctcttacac tttttcatac attgcccaag aataaagaat 9360

cgtttgtgtt atgtttcaac gtgtttattt ttcaattgca gaaaatttca agtcattttt 9420

cattcagtag tatagcccca ccaccacata gcttatacag atcaccgtac cttaatcaaa 9480

ctcacagaac cctagtattc aacctgccac ctccctccca acacacagag tacacagtcc 9540

tttctccccg gctggcctta aaaagcatca tatcatgggt aacagacata ttcttaggtg 9600

ttatattcca cacggtttcc tgtcgagcca aacgctcatc agtgatatta ataaactccc 9660

cgggcagctc acttaagttc atgtcgctgt ccagctgctg agccacaggc tgctgtccaa 9720

cttgcggttg cttaacgggc ggcgaaggag aagtccacgc ctacatgggg gtagagtcat 9780

aatcgtgcat caggataggg cggtggtgct gcagcagcgc gcgaataaac tgctgccgcc 9840

gccgctccgt cctgcaggaa tacaacatgg cagtggtctc ctcagcgatg attcgcaccg 9900

cccgcagcat aaggcgcctt gtcctccggg cacagcagcg caccctgatc tcacttaaat 9960

cagcacagta actgcagcac agcaccacaa tattgttcaa aatcccacag tgcaaggcgc 10020

tgtatccaaa gctcatggcg gggaccacag aacccacgtg gccatcatac cacaagcgca 10080

ggtagattaa gtggcgaccc ctcataaaca cgctggacat aaacattacc tcttttggca 10140

tgttgtaatt caccacctcc cggtaccata taaacctctg attaaacatg gcgccatcca 10200

ccaccatcct aaaccagctg gccaaaacct gcccgccggc tatacactgc agggaaccgg 10260

gactggaaca atgacagtgg agagcccagg actcgtaacc atggatcatc atgctcgtca 10320

tgatatcaat gttggcacaa cacaggcaca cgtgcataca cttcctcagg attacaagct 10380

cctcccgcgt tagaaccata tcccagggaa caacccattc ctgaatcagc gtaaatccca 10440

cactgcaggg aagacctcgc acgtaactca cgttgtgcat tgtcaaagtg ttacattcgg 10500

gcagcagcgg atgatcctcc agtatggtag cgcgggtttc tgtctcaaaa ggaggtagac 10560

gatccctact gtacggagtg cgccgagaca accgagatcg tgttggtcgt agtgtcatgc 10620

caaatggaac gccggacgta gtcatatttc ctgaagcaaa accaggtgcg ggcgtgacaa 10680

acagatctgc gtctccggtc tcgccgctta gatcgctctg tgtagtagtt gtagtatatc 10740

cactctctca aagcatccag gcgccccctg gcttcgggtt ctatgtaaac tccttcatgc 10800

gccgctgccc tgataacatc caccaccgca gaataagcca cacccagcca acctacacat 10860

tcgttctgcg agtcacacac gggaggagcg ggaagagctg gaagaaccat gttttttttt 10920

ttattccaaa agattatcca aaacctcaaa atgaagatct attaagtgaa cgcgctcccc 10980

tccggtggcg tggtcaaact ctacagccaa agaacagata atggcatttg taagatgttg 11040

cacaatggct tccaaaaggc aaacggccct cacgtccaag tggacgtaaa ggctaaaccc 11100

ttcagggtga atctcctcta taaacattcc agcaccttca accatgccca aataattctc 11160

atctcgccac cttctcaata tatctctaag caaatcccga atattaagtc cggccattgt 11220

aaaaatctgc tccagagcgc cctccacctt cagcctcaag cagcgaatca tgattgcaaa 11280

aattcaggtt cctcacagac ctgtataaga ttcaaaagcg gaacattaac aaaaataccg 11340

cgatcccgta ggtcccttcg cagggccagc tgaacataat cgtgcaggtc tgcacggacc 11400

agcgcggcca cttccccgcc aggaaccatg acaaaagaac ccacactgat tatgacacgc 11460

atactcggag ctatgctaac cagcgtagcc ccgatgtaag cttgttgcat gggcggcgat 11520

ataaaatgca aggtgctgct caaaaaatca ggcaaagcct cgcgcaaaaa agaaagcaca 11580

tcgtagtcat gctcatgcag ataaaggcag gtaagctccg gaaccaccac agaaaaagac 11640

accattttc tctcaaacat gtctgcgggt ttctgcataa acacaaaata aaataacaaa 11700

aaaacattta aacattagaa gcctgtctta caacaggaaa aacaaccctt ataagcataa 11760

gacggactac ggccatgccg gcgtgaccgt aaaaaaactg gtcaccgtga ttaaaaagca 11820

ccaccgacag ctcctcggtc atgtccggag tcataatgta agactcggta aacacatcag 11880

gttgattcac atcggtcagt gctaaaaagc gaccgaaata gcccgggga atacataccc 11940

gcaggcgtag agacaacatt acagccccca taggaggtat aacaaaatta ataggagaga 12000

aaaacacata aacacctgaa aaaccctcct gcctaggcaa aatagcaccc tcccgctcca 12060

gaacaacata cagcgcttcc acagcggcag ccataacagt cagccttacc agtaaaaaag 12120

aaaacctatt aaaaaaacac cactcgacac ggcaccagct caatcagtca cagtgtaaaa 12180

aagggccaag tgcagagcga gtatatatag gactaaaaaa tgacgtaacg gttaaagtcc 12240

acaaaaaaca cccagaaaac cgcacgcgaa cctacgccca gaaacgaaag ccaaaaaacc 12300

cacaacttcc tcaaatcgtc acttccgttt tcccacgtta cgtaacttcc cattttaaga 12360

aaactacaat tcccaacaca tacaagttac tccgccctaa aacctacgtc acccgccccg 12420

ttcccacgcc ccgcgccacg tcacaaactc cacccctca ttatcatatt ggcttcaatc 12480

caaaataagg tatattattg atgatg 12506

<210> 68
<211> 884
<212> DNA
<213> Artificial Sequence

<220>
<223> 6th DNA fragment

<400> 68
gggactagtt tcgcgccctt tctcaaattt aagcgcgaaa actacgtcat ctccagcggc 60

cacacccggc gccagcacct gtcgtcagcg ccattatgag caaggaaatt cccacgccct 120

acatgtggag ttaccagcca caaatgggac ttgcggctgg agctgcccaa gactactcaa 180

cccgaataaa ctacatgagc gcgggacccc acatgatatc ccgggtcaac ggaatccgcg 240

cccaccgaaa ccgaattctc ttggaacagg cggctattac caccacacct cgtaataacc 300

ttaatccccg tagttggccc gctgccctgg tgtaccagga aagtcccgct cccaccactg 360

tggtacttcc cagagacgcc caggccgaag ttcagatgac taactcaggg gcgcagcttg 420

cgggcggctt tcgtcacagg gtgcggtcgc ccgggcaggg tataactcac ctgacaatca 480

gagggcgagg tattcagctc aacgacgagt cggtgagctc ctcgcttggt ctccgtccgg 540

acgggacatt tcagatcggc ggcgccggcc gtccttcatt cacgcctcgt caggcaatcc 600

taactctgca gacctcgtcc tctgagccgc gctctggagg cattggaact ctgcaattta 660

ttgaggagtt tgtgccatcg gtctacttta acccettctc gggacctccc ggccactatc 720

cggatcaatt tattcctaac tttgacgcgg taaaggactc ggcggacggc tacgactgaa 780

tgttataagt tcctgtccat ccgcacccac tatcttcatg ttgttgcaga tgaagcgcgc 840

aagaccgtct gaagatacct tcaaccccgt gtatccatat gccc 884

<210> 69
<211> 18040
<212> DNA
<213> Artificial Sequence

<220>
<223> DNA corresponding to the ClaI-BamHI region of Psi5-Left-Arm-2

<400> 69
atcgatttaa gggtgggaaa gaatatataa ggtgggggtc ttatgtagtt ttgtatctgt 60

tttgcagcag ccgccgccgc catgagcacc aactcgtttg atggaagcat tgtgagctca 120

tatttgacaa cgcgcatgcc cccatgggcc ggggtgcgtc agaatgtgat gggctccagc 180

attgatggtc gccccgtcct gcccgcaaac tctactacct tgacctacga gaccgtgtct 240

ggaacgccgt tggagactgc agcctccgcc gccgcttcag ccgctgcagc caccgcccgc 300

gggattgtga ctgactttgc tttcctgagc ccgcttgcaa gcagtgcagc ttcccgttca 360

tccgcccgcg atgacaagtt gacggctctt ttggcacaat tggattcttt gacccgggaa 420

cttaatgtcg tttctcagca gctgttggat ctgcgccagc aggtttctgc cctgaaggct 480

tcctcccctc ccaatgcggt ttaaaacata aataaaaaac cagactctgt ttggatttgg 540

atcaagcaag tgtcttgctg tctttattta ggggttttgc gcgcgcggta ggcccgggac 600

cagcggtctc ggtcgttgag ggtcctgtgt attttttcca ggacgtggta aaggtgactc 660

tggatgttca gatacatggg cataagcccg tctctggggt ggaggtagca ccactgcaga 720

gcttcatgct gcggggtggt gttgtagatg atccagtcgt agcaggagcg ctgggcgtgg 780

tgcctaaaaa tgtctttcag tagcaagctg attgccaggg gcaggccctt ggtgtaagtg 840
tttacaaagc ggttaagctg ggatgggtgc atacgtgggg atatgagatg catcttggac 900
tgtattttta ggttggctat gttcccagcc atatccctcc ggggattcat gttgtgcaga 960
accaccagca cagtgtatcc ggtgcacttg ggaaatttgt catgtagctt agaaggaaat 1020
gcgtggaaga acttggagac gcccttgtga cctccaagat tttccatgca ttcgtccata 1080
atgatggcaa tgggcccacg ggcggcggcc tgggcgaaga tatttctggg atcactaacg 1140
tcatagttgt gttccaggat gagatcgtca taggccattt ttacaaagcg cgggcggagg 1200
gtgccagact gcggtataat ggttccatcc ggcccagggg cgtagttacc ctcacagatt 1260
tgcatttccc acgctttgag ttcagatggg gggatcatgt ctacctgcgg ggcgatgaag 1320
aaaacggttt ccggggtagg ggagatcagc tgggaagaaa gcaggttcct gagcagctgc 1380
gacttaccgc agccggtggg cccgtaaatc acacctatta ccgggtgcaa ctggtagtta 1440
agagagctgc agctgccgtc atccctgagc aggggggcca cttcgttaag catgtccctg 1500
actcgcatgt tttccctgac caaatccgcc agaaggcgct cgccgcccag cgatagcagt 1560
tcttgcaagg aagcaaagtt tttcaacggt ttgagaccgt ccgccgtagg catgcttttg 1620
agcgtttgac caagcagttc caggcggtcc cacagctcgg tcacctgctc tacggcatct 1680
cgatccagca tatctcctcg tttcgcgggt tggggcggct ttcgctgtac ggcagtagtc 1740
ggtgctcgtc cagacgggcc agggtcatgt ctttccacgg gcgcagggtc ctcgtcagcg 1800
tagtctgggt cacggtgaag ggtgcgctcc cgggctgcgc gctggccagg gtgcgcttga 1860
ggctggtcct gctggtgctg aagcgctgcc ggtcttcgcc ctgcgcgtcg gccaggtagc 1920
atttgaccat ggtgtcatag tccagcccct ccgcggcgtg gcccttggcg cgcagcttgc 1980
ccttggagga ggcgccgcac gaggggcagt gcagactttt gagggcgtag agcttgggcg 2040
cgagaaatac cgattccggg gagtaggcat ccgcgccgca ggccccgcag acggtctcgc 2100
attccacgag ccaggtgagc tctggccgtt cggggtcaaa aaccaggttt cccccatgct 2160
ttttgatgcg tttcttacct ctggtttcca tgagccggtg tccacgctcg gtgacgaaaa 2220
ggctgtccgt gtccccgtat acagacttga gaggcctgtc ctcgagcggt gttccgcggt 2280
cctcctcgta tagaaactcg gaccactctg agacaaaggc tcgcgtccag gccagcacga 2340
aggaggctaa gtgggagggg tagcggtcgt tgtccactag ggggtccact cgctccaggg 2400
tgtgaagaca catgtcgccc tcttcggcat caaggaaggt gattggtttg taggtgtagg 2460
ccacgtgacc gggtgttcct gaaggggggc tataaaaggg ggtgggggcg cgttcgtcct 2520
cactctcttc cgcatcgctg tctgcgaggg ccagctgttg gggtgagtac tccctctgaa 2580
aagcgggcat gacttctgcg ctaagattgt cagtttccaa aaacgaggag gatttgatat 2640
tcacctggcc cgcggtgatg cctttgaggg tggccgcatc catctggtca gaaaagacaa 2700

tctttttgtt gtcaagcttg gtggcaaacg acccgtagag ggcgttggac agcaacttgg 2760
cgatggagcg cagggtttgg tttttgtcgc gatcggcgcg ctccttggcc gcgatgttta 2820
gctgcacgta ttcgcgcgca acgcaccgcc attcgggaaa gacggtggtg cgctcgtcgg 2880
gcaccaggtg cacgcgccaa ccgcggttgt gcagggtgac aaggtcaacg ctggtggcta 2940
cctctccgcg taggcgctcg ttggtccagc agaggcggcc gcccttgcgc gagcagaatg 3000
gcggtagggg gtctagctgc gtctcgtccg gggggtctgc gtccacggta aagaccccgg 3060
gcagcaggcg cgcgtcgaag tagtctatct tgcatccttg caagtctagc gcctgctgcc 3120
atgcgcgggc ggcaagcgcg cgctcgtatg ggttgagtgg gggaccccat ggcatggggt 3180
gggtgagcgc ggaggcgtac atgccgcaaa tgtcgtaaac gtagaggggc tctctgagta 3240
ttccaagata tgtagggtag catcttccac cgcggatgct ggcgcgcacg taatcgtata 3300
gttcgtgcga gggagcgagg aggtcgggac cgaggttgct acgggcgggc tgctctgctc 3360
ggaagactat ctgcctgaag atggcatgtg agttggatga tatggttgga cgctggaaga 3420
cgttgaagct ggcgtctgtg agacctaccg cgtcacgcac gaaggaggcg taggagtcgc 3480
gcagcttgtt gaccagctcg gcggtgacct gcacgtctag ggcgcagtag tccagggttt 3540
ccttgatgat gtcatactta tcctgtccct tttttttcca cagctcgcgg ttgaggacaa 3600
actcttcgcg gtctttccag tactcttgga tcggaaaccc gtcggcctcc gaacggtaag 3660
agcctagcat gtagaactgg ttgacggcct ggtaggcgca gcatcccttt tctacgggta 3720
gcgcgtatgc ctgcgcggcc ttccggagcg aggtgtgggt gagcgcaaag gtgtccctga 3780
ccatgacttt gaggtactgg tatttgaagt cagtgtcgtc gcatccgccc tgctcccaga 3840
gcaaaaagtc cgtgcgcttt ttggaacgcg gatttggcag ggcgaaggtg acatcgttga 3900
agagtatctt tcccgcgcga ggcataaagt tgcgtgtgat gcggaagggt cccggcacct 3960
cggaacggtt gttaattacc tgggcggcga gcacgatctc gtcaaagccg ttgatgttgt 4020
ggcccacaat gtaaagttcc aagaagcgcg ggatgccctt gatggaaggc aatttttttaa 4080
gttcctcgta ggtgagctct tcaggggagc tgagcccgtg ctctgaaagg gcccagtctg 4140
caagatgagg gttggaagcg acgaatgagc tccacaggtc acgggccatt agcatttgca 4200
ggtggtcgcg aaaggtccta aactggcgac ctatggccat tttttctggg gtgatgcagt 4260
agaaggtaag cgggtcttgt tcccagcggt cccatccaag gttcgcggct aggtctcgcg 4320
cggcagtcac tagaggctca tctccgccga acttcatgac cagcatgaag ggcacgagct 4380
gcttcccaaa ggcccccatc caagtatagg tctctacatc gtaggtgaca aagagacgct 4440
cggtgcgagg atgcgagccg atcgggaaga actggatctc ccgccaccaa ttggaggagt 4500
ggctattgat gtggtgaaag tagaagtccc tgcgacgggc cgaacactcg tgctggcttt 4560

tgtaaaaacg tgcgcagtac tggcagcggt gcacgggctg tacatcctgc acgaggttga 4620
cctgacgacc gcgcacaagg aagcagagtg ggaatttgag cccctcgcct ggcgggtttg 4680
gctggtggtc ttctacttcg gctgcttgtc cttgaccgtc tggctgctcg aggggagtta 4740
cggtggatcg gaccaccacg ccgcgcgagc ccaaagtcca gatgtccgcg cgcggcggtc 4800
ggagcttgat gacaacatcg cgcagatggg agctgtccat ggtctggagc tcccgcggcg 4860
tcaggtcagg cgggagctcc tgcaggttta cctcgcatag acgggtcagg gcgcgggcta 4920
gatccaggtg atacctaatt tccaggggct ggttggtggc ggcgtcgatg gcttgcaaga 4980
ggccgcatcc ccgcggcgcg actacggtac cgcgcggcgg gcggtgggcc gcgggggtgt 5040
ccttggatga tgcatctaaa agcggtgacg cgggcgagcc cccggaggta ggggggctc 5100
cggacccgcc gggagagggg gcaggggcac gtcggcgccg cgcgcgggca ggagctggtg 5160
ctgcgcgcgt aggttgctgg cgaacgcgac gacgcggcgg ttgatctcct gaatctggcg 5220
cctctgcgtg aagacgacgg gcccggtgag cttgagcctg aaagagagtt cgacagaatc 5280
aatttcggtg tcgttgacgg cggcctggcg caaaatctcc tgcacgtctc ctgagttgtc 5340
ttgataggcg atctcggcca tgaactgctc gatctcttcc tcctggagat ctccgcgtcc 5400
ggctcgctcc acggtggcgg cgaggtcgtt ggaaatgcgg gccatgagct gcgagaaggc 5460
gttgaggcct ccctcgttcc agacgcggct gtagaccacg ccccttcgg catcgcgggc 5520
gcgcatgacc acctgcgcga gattgagctc cacgtgccgg gcgaagacgg cgtagtttcg 5580
caggcgctga aagaggtagt tgagggtggt ggcggtgtgt tctgccacga agaagtacat 5640
aacccagcgt cgcaacgtgg attcgttgat atcccccaag gcctcaaggc gctccatggc 5700
ctcgtagaag tccacggcga agttgaaaaa ctgggagttg cgcgccgaca cggttaactc 5760
ctcctccaga agacggatga gctcggcgac agtgtcgcgc acctcgcgct caaaggctac 5820
aggggcctct tcttcttctt caatctcctc ttccataagg gcctcccctt cttcttcttc 5880
tggcggcggt gggggagggg ggacacggcg gcgacgacgg cgcaccggga ggcggtcgac 5940
aaagcgctcg atcatctccc cgcggcgacg gcgcatggtc tcggtgacgg cgcggccgtt 6000
ctcgcggggg cgcagttgga agacgccgcc cgtcatgtcc cggttatggg ttggcggggg 6060
gctgccatgc ggcagggata cggcgctaac gatgcatctc aacaattgtt gtgtaggtac 6120
tccgccgccg agggacctga gcgagtccgc atcgaccgga tcggaaaacc tctcgagaaa 6180
ggcgtctaac cagtcacagt cgcaaggtag gctgagcacc gtggcgggcg gcagcgggcg 6240
gcggtcgggg ttgtttctgg cggaggtgct gctgatgatg taattaaagt aggcggtctt 6300
gagacggcgg atggtcgaca gaagcaccat gtccttgggt ccggcctgct gaatgcgcag 6360
gcggtcggcc atgccccagg cttcgttttg acatcggcgc aggtctttgt agtagtcttg 6420
catgagcctt tctaccggca cttcttcttc tccttcctct tgtcctgcat ctcttgcatc 6480

tatcgctgcg gcggcggcgg agtttggccg taggtggcgc cctcttcctc ccatgcgtgt 6540

gaccccgaag cccctcatcg gctgaagcag ggctaggtcg gcgacaacgc gctcggctaa 6600

tatggcctgc tgcacctgcg tgagggtaga ctggaagtca tccatgtcca caaagcggtg 6660

gtatgcgccc gtgttgatgg tgtaagtgca gttggccata acggaccagt taacggtctg 6720

gtgacccggc tgcgagagct cggtgtacct gagacgcgag taagccctcg agtcaaatac 6780

gtagtcgttg caagtccgca ccaggtactg gtatcccacc aaaaagtgcg gcggcggctg 6840

gcggtagagg ggccagcgta gggtggccgg ggctccgggg gcgagatctt ccaacataag 6900

gcgatgatat ccgtagatgt acctggacat ccaggtgatg ccggcggcgg tggtggaggc 6960

gcgcggaaag tcgcggacgc ggttccagat gttgcgcagc ggcaaaaagt gctccatggt 7020

cgggacgctc tggccggtca ggcgcgcgca atcgttgacg ctctagaccg tgcaaaagga 7080

gagcctgtaa gcgggcactc ttccgtggtc tggtggataa attcgcaagg gtatcatggc 7140

ggacgaccgg ggttcgagcc ccgtatccgg ccgtccgccg tgatccatgc ggttaccgcc 7200

cgcgtgtcga acccaggtgt gcgacgtcag acaacggggg agtgctcctt ttggcttcct 7260

tccaggcgcg gcggctgctg cgctagcttt tttggccact ggccgcgcgc agcgtaagcg 7320

gttaggctgg aaagcgaaag cattaagtgg ctcgctccct gtagccggag ggttattttc 7380

caagggttga gtcgcgggac ccccggttcg agtctcggac cggccggact gcggcgaacg 7440

ggggtttgcc tccccgtcat gcaagacccc gcttgcaaat tcctccggaa acagggacga 7500

gcccctttttt tgcttttccc agatgcatcc ggtgctgcgg cagatgcgcc cccctcctca 7560

gcagcggcaa gagcaagagc agcggcagac atgcagggca ccctcccctc ctcctaccgc 7620

gtcaggaggg gcgacatccg cggttgacgc ggcagcagat ggtgattacg aacccccgcg 7680

gcgccgggcc cggcactacc tggacttgga ggagggcgag ggcctggcgc ggctaggagc 7740

gccctctcct gagcggtacc caagggtgca gctgaagcgt gatacgcgtg aggcgtacgt 7800

gccgcggcag aacctgtttc gcgaccgcga gggagaggag cccgaggaga tgcgggatcg 7860

aaagttccac gcagggcgcg agctgcggca tggcctgaat cgcgagcggt tgctgcgcga 7920

ggaggacttt gagcccgacg cgcgaaccgg gattagtccc gcgcgcgcac acgtggcggc 7980

cgccgacctg gtaaccgcat acgagcagac ggtgaaccag gagattaact ttcaaaaaag 8040

ctttaacaac cacgtgcgta cgcttgtggc gcgcgaggag gtggctatag gactgatgca 8100

tctgtgggac tttgtaagcg cgctggagca aaacccaaat agcaagccgc tcatggcgca 8160

gctgttcctt atagtgcagc acagcaggga caacgaggca ttcagggatg cgctgctaaa 8220

catagtagag cccgagggcc gctggctgct cgatttgata aacatcctgc agagcatagt 8280

ggtgcaggag cgcagcttga gcctggctga caaggtggcc gccatcaact attccatgct 8340

tagcctgggc aagttttacg cccgcaagat ataccatacc ccttacgttc ccatagacaa 8400

ggaggtaaag atcgaggggt tctacatgcg catggcgctg aaggtgctta ccttgagcga 8460

cgacctgggc gtttatcgca acgagcgcat ccacaaggcc gtgagcgtga gccggcggcg 8520

cgagctcagc gaccgcgagc tgatgcacag cctgcaaagg gccctggctg gcacgggcag 8580

cggcgataga gaggccgagt cctactttga cgcgggcgct gacctgcgct gggccccaag 8640

ccgacgcgcc ctggaggcag ctggggccgg acctgggctg gcggtggcac ccgcgcgcgc 8700

tggcaacgtc ggcggcgtgg aggaatatga cgaggacgat gagtacgagc cagaggacgg 8760

cgagtactaa gcggtgatgt ttctgatcag atgatgcaag acgcaacgga cccggcggtg 8820

cgggcggcgc tgcagagcca gccgtccggc cttaactcca cggacgactg gcgccaggtc 8880

atggaccgca tcatgtcgct gactgcgcgc aatcctgacg cgttccggca gcagccgcag 8940

gccaaccggc tctccgcaat tctggaagcg gtggtcccgg cgcgcgcaaa ccccacgcac 9000

gagaaggtgc tggcgatcgt aaacgcgctg gccgaaaaca gggccatccg gcccgacgag 9060

gccggcctgg tctacgacgc gctgcttcag cgcgtggctc gttacaacag cggcaacgtg 9120

cagaccaacc tggaccggct ggtgggggat gtgcgcgagg ccgtggcgca gcgtgagcgc 9180

gcgcagcagc agggcaacct gggctccatg gttgcactaa acgccttcct gagtacacag 9240

cccgccaacg tgccgcgggg acaggaggac tacaccaact ttgtgagcgc actgcggcta 9300

atggtgactg agacaccgca aagtgaggtg taccagtctg ggccagacta ttttttccag 9360

accagtagac aaggcctgca gaccgtaaac ctgagccagg ctttcaaaaa cttgcagggg 9420

ctgtgggggg tgcgggctcc cacaggcgac cgcgcgaccg tgtctagctt gctgacgccc 9480

aactcgcgcc tgttgctgct gctaatagcg cccttcacgg acagtggcag cgtgtcccgg 9540

gacacatacc taggtcactt gctgacactg taccgcgagg ccataggtca ggcgcatgtg 9600

gacgagcata ctttccagga gattacaagt gtcagccgcg cgctggggca ggaggacacg 9660

ggcagcctgg aggcaaccct aaactacctg ctgaccaacc ggcggcagaa gatcccctcg 9720

ttgcacagtt taaacagcga ggaggagcgc attttgcgct acgtgcagca gagcgtgagc 9780

cttaacctga tgcgcgacgg ggtaacgccc agcgtggcgc tggacatgac cgcgcgcaac 9840

atggaaccgg gcatgtatgc ctcaaaccgg ccgtttatca accgcctaat ggactacttg 9900

catcgcgcgg ccgccgtgaa ccccgagtat ttcaccaatg ccatcttgaa cccgcactgg 9960

ctaccgcccc ctggtttcta caccggggga ttcgaggtgc ccgagggtaa cgatggattc 10020

ctctgggacg acatagacga cagcgtgttt tccccgcaac cgcagaccct gctagagttg 10080

caacagcgcg agcaggcaga ggcggcgctg cgaaaggaaa gcttccgcag gccaagcagc 10140

ttgtccgatc taggcgctgc ggccccgcgg tcagatgcta gtagcccatt tccaagcttg 10200

atagggtctc ttaccagcac tcgcaccacc cgcccgcgcc tgctgggcga ggaggagtac 10260

ctaaacaact cgctgctgca gccgcagcgc gaaaaaaacc tgcctccggc atttcccaac 10320

aacgggatag agagcctagt ggacaagatg agtagatgga agacgtacgc gcaggagcac 10380

agggacgtgc caggcccgcg cccgcccacc cgtcgtcaaa ggcacgaccg tcagcggggt 10440

ctggtgtggg aggacgatga ctcggcagac gacagcagcg tcctggattt gggagggagt 10500

ggcaacccgt ttgcgcacct tcgccccagg ctggggagaa tgttttaaaa aaaaaaaagc 10560

atgatgcaaa ataaaaaact caccaaggcc atggcaccga gcgttggttt tcttgtattc 10620

cccttagtat gcggcgcgcg gcgatgtatg aggaaggtcc tcctccctcc tacgagagtg 10680

tggtgagcgc ggcgccagtg gcggcggcgc tgggttctcc cttcgatgct cccctggacc 10740

cgccgtttgt gcctccgcgg tacctgcggc ctaccggggg gagaaacagc atccgttact 10800

ctgagttggc acccctattc gacaccaccc gtgtgtacct ggtggacaac aagtcaacgg 10860

atgtggcatc cctgaactac cagaacgacc acagcaactt tctgaccacg gtcattcaaa 10920

acaatgacta cagcccgggg gaggcaagca cacagaccat caatcttgac gaccggtcgc 10980

actggggcgg cgacctgaaa accatcctgc ataccaacat gccaaatgtg aacgagttca 11040

tgtttaccaa taagtttaag gcgcgggtga tggtgtcgcg cttgcctact aaggacaatc 11100

aggtggagct gaaatacgag tgggtggagt tcacgctgcc cgagggcaac tactccgaga 11160

ccatgaccat agaccttatg aacaacgcga tcgtggagca ctacttgaaa gtgggcagac 11220

agaacggggt tctggaaagc gacatcgggg taaagtttga cacccgcaac ttcagactgg 11280

ggtttgaccc cgtcactggt cttgtcatgc ctggggtata tacaaacgaa gccttccatc 11340

cagacatcat tttgctgcca ggatgcgggg tggacttcac ccacagccgc ctgagcaact 11400

tgttgggcat ccgcaagcgg caacccttcc aggagggctt taggatcacc tacgatgatc 11460

tggagggtgg taacattccc gcactgttgg atgtggacgc ctaccaggcg agcttgaaag 11520

atgacaccga acagggcggg ggtggcgcag gcggcagcaa cagcagtggc agcggcgcgg 11580

aagagaactc caacgcggca gccgcggcaa tgcagccggt ggaggacatg aacgatcatg 11640

ccattcgcgg cgacaccttt gccacacggg ctgaggagaa gcgcgctgag gccgaagcag 11700

cggccgaagc tgccgccccc gctgcgcaac ccgaggtcga gaagcctcag aagaaaccgg 11760

tgatcaaacc cctgacagag gacagcaaga aacgcagtta caacctaata agcaatgaca 11820

gcaccttcac ccagtaccgc agctggtacc ttgcatacaa ctacggcgac cctcagaccg 11880

gaatccgctc atggaccctg ctttgcactc ctgacgtaac ctgcggctcg gagcaggtct 11940

actggtcgtt gccagacatg atgcaagacc ccgtgacctt ccgctccacg cgccagatca 12000

gcaactttcc ggtggtgggc gccgagctgt tgcccgtgca ctccaagagc ttctacaacg 12060

accaggccgt ctactcccaa ctcatccgcc agtttacctc tctgacccac gtgttcaatc 12120

gctttcccga gaaccagatt ttggcgcgcc cgccagcccc caccatcacc accgtcagtg 12180

aaaacgttcc tgctctcaca gatcacggga cgctaccgct gcgcaacagc atcggaggag 12240

tccagcgagt gaccattact gacgccagac gccgcacctg cccctacgtt tacaaggccc 12300

tgggcatagt ctcgccgcgc gtcctatcga gccgcacttt ttgagcaagc atgtccatcc 12360

ttatatcgcc cagcaataac acaggctggg gcctgcgctt cccaagcaag atgtttggcg 12420

gggccaagaa gcgctccgac caacacccag tgcgcgtgcg cgggcactac cgcgcgccct 12480

ggggcgcgca caaacgcggc cgcactgggc gcaccaccgt cgatgacgcc atcgacgcgg 12540

tggtggagga ggcgcgcaac tacacgccca cgccgccacc agtgtccaca gtggacgcgg 12600

ccattcagac cgtggtgcgc ggagcccggc gctatgctaa aatgaagaga cggcggaggc 12660

gcgtagcacg tcgccaccgc cgccgacccg gcactgccgc ccaacgcgcg gcggcggccc 12720

tgcttaaccg cgcacgtcgc accggccgac gggcggccat gcgggccgct cgaaggctgg 12780

ccgcgggtat tgtcactgtg ccccccaggt ccaggcgacg agcggccgcc gcagcagccg 12840

cggccattag tgctatgact cagggtcgca ggggcaacgt gtattgggtg cgcgactcgg 12900

ttagcggcct gcgcgtgccc gtgcgcaccc gcccccgcg caactagatt gcaagaaaaa 12960

actacttaga ctcgtactgt tgtatgtatc cagcggcggc ggcgcgcaac gaagctatgt 13020

ccaagcgcaa aatcaaagaa gagatgctcc aggtcatcgc gccggagatc tatggccccc 13080

cgaagaagga agagcaggat tacaagcccc gaaagctaaa gcgggtcaaa aagaaaaaga 13140

aagatgatga tgatgaactt gacgacgagg tggaactgct gcacgctacc gcgcccaggc 13200

gacgggtaca gtggaaaggt cgacgcgtaa aacgtgtttt gcgacccggc accaccgtag 13260

tctttacgcc cggtgagcgc tccacccgca cctacaagcg cgtgtatgat gaggtgtacg 13320

gcgacgagga cctgcttgag caggccaacg agcgcctcgg ggagtttgcc tacggaaagc 13380

ggcataagga catgctggcg ttgccgctgg acgagggcaa cccaacacct agcctaaagc 13440

ccgtaacact gcagcaggtg ctgcccgcgc ttgcaccgtc cgaagaaaag cgcggcctaa 13500

agcgcgagtc tggtgacttg gcacccaccg tgcagctgat ggtacccaag cgccagcgac 13560

tggaagatgt cttggaaaaa atgaccgtgg aacctgggct ggagcccgag gtccgcgtgc 13620

ggccaatcaa gcaggtggcg ccgggactgg gcgtgcagac cgtggacgtt cagataccca 13680

ctaccagtag caccagtatt gccaccgcca cagagggcat ggagacacaa acgtccccgg 13740

ttgcctcagc ggtggcggat gccgcggtgc aggcggtcgc tgcggccgcg tccaagacct 13800

ctacggaggt gcaaacggac ccgtggatgt ttcgcgtttc agccccccgg cgcccgcgcg 13860

gttcgaggaa gtacggcgcc gccagcgcgc tactgcccga atatgcccta catccttcca 13920

ttgcgcctac ccccggctat cgtggctaca cctaccgccc cagaagacga gcaactaccc 13980

gacgccgaac caccactgga acccgccgcc gccgtcgccg tcgccagccc gtgctggccc 14040

cgatttccgt gcgcagggtg gctcgcgaag gaggcaggac cctggtgctg ccaacagcgc 14100

gctaccaccc cagcatcgtt taaaagccgg tctttgtggt tcttgcagat atggccctca 14160

cctgccgcct ccgtttcccg gtgccgggat tccgaggaag aatgcaccgt aggaggggca 14220

tggccggcca cggcctgacg ggcggcatgc gtcgtgcgca ccaccggcgg cggcgcgcgt 14280

cgcaccgtcg catgcgcggc ggtatcctgc ccctccttat tccactgatc gccgcggcga 14340

ttggcgccgt gcccggaatt gcatccgtgg ccttgcaggc gcagagacac tgattaaaaa 14400

caagttgcat gtggaaaaat caaaataaaa agtctggact ctcacgctcg cttggtcctg 14460

taactatttt gtagaatgga agacatcaac tttgcgtctc tggccccgcg acacggctcg 14520

cgcccgttca tgggaaactg gcaagatatc ggcaccagca atatgagcgg tggcgccttc 14580

agctggggct cgctgtggag cggcattaaa aatttcggtt ccaccgttaa gaactatggc 14640

agcaaggcct ggaacagcag cacaggccag atgctgaggg ataagttgaa agagcaaaat 14700

ttccaacaaa aggtggtaga tggcctggcc tctggcatta gcggggtggt ggacctggcc 14760

aaccaggcag tgcaaaataa gattaacagt aagcttgatc cccgccctcc cgtagaggag 14820

cctccaccgg ccgtggagac agtgtctcca gaggggcgtg gcgaaaagcg tccgcgcccc 14880

gacagggaag aaactctggt gacgcaaata gacgagcctc cctcgtacga ggaggcacta 14940

aagcaaggcc tgcccaccac ccgtcccatc gcgcccatgg ctaccggagt gctgggccag 15000

cacacacccg taacgctgga cctgcctccc cccgccgaca cccagcagaa acctgtgctg 15060

ccaggcccga ccgccgttgt tgtaacccgt cctagccgcg cgtccctgcg ccgcgccgcc 15120

agcggtccgc gatcgttgcg gcccgtagcc agtggcaact ggcaaagcac actgaacagc 15180

atcgtgggtc tgggggtgca atccctgaag cgccgacgat gcttctgaat agctaacgtg 15240

tcgtatgtgt gtcatgtatg cgtccatgtc gccgccagag gagctgctga gccgccgcgc 15300

gcccgctttc caagatggct accccttcga tgatgccgca gtggtcttac atgcacatct 15360

cgggccagga cgcctcggag tacctgagcc ccgggctggt gcagtttgcc cgcgccaccg 15420

agacgtactt cagcctgaat aacaagttta gaaaccccac ggtggcgcct acgcacgacg 15480

tgaccacaga ccggtcccag cgtttgacgc tgcggttcat ccctgtggac cgtgaggata 15540

ctgcgtactc gtacaaggcg cggttcaccc tagctgtggg tgataaccgt gtgctggaca 15600

tggcttccac gtactttgac atccgcggcg tgctggacag gggccctact tttaagccct 15660

actctggcac tgcctacaac gccctggctc ccaagggtgc cccaaatcct tgcgaatggg 15720

atgaagctgc tactgctctt gaaataaacc tagaagaaga ggacgatgac aacgaagacg 15780

aagtagacga gcaagctgag cagcaaaaaa ctcacgtatt tgggcaggcg ccttattctg 15840

gtataaatat tacaaaggag ggtattcaaa taggtgtcga aggtcaaaca cctaaatatg 15900

ccgataaaac atttcaacct gaacctcaaa taggagaatc tcagtggtac gaaactgaaa 15960
ttaatcatgc agctgggaga gtccttaaaa agactacccc aatgaaacca tgttacggtt 16020
catatgcaaa acccacaaat gaaaatggag ggcaaggcat tcttgtaaag caacaaaatg 16080
gaaagctaga aagtcaagtg gaaatgcaat ttttctcaac tactgaggcg accgcaggca 16140
atggtgataa cttgactcct aaagtggtat tgtacagtga agatgtagat atagaaaccc 16200
cagacactca tatttcttac atgcccacta ttaaggaagg taactcacga gaactaatgg 16260
gccaacaatc tatgcccaac aggcctaatt acattgcttt tagggacaat tttattggtc 16320
taatgtatta caacagcacg ggtaatatgg gtgttctggc gggccaagca tcgcagttga 16380
atgctgttgt agatttgcaa gacagaaaca cagagctttc ataccagctt ttgcttgatt 16440
ccattggtga tagaaccagg tacttttcta tgtggaatca ggctgttgac agctatgatc 16500
cagatgttag aattattgaa aatcatggaa ctgaagatga acttccaaat tactgctttc 16560
cactgggagg tgtgattaat acagagactc ttaccaaggt aaaacctaaa acaggtcagg 16620
aaaatggatg ggaaaaagat gctacagaat tttcagataa aaatgaaata agagttggaa 16680
ataattttgc catggaaatc aatctaaatg ccaacctgtg gagaaatttc ctgtactcca 16740
acatagcgct gtatttgccc gacaagctaa agtacagtcc ttccaacgta aaaatttctg 16800
ataacccaaa cacctacgac tacatgaaca agcgagtggt ggctcccggg ttagtggact 16860
gctacattaa ccttggagca cgctggtccc ttgactatat ggacaacgtc aacccattta 16920
accaccaccg caatgctggc ctgcgctacc gctcaatgtt gctgggcaat ggtcgctatg 16980
tgcccttcca catccaggtg cctcagaagt tctttgccat taaaaacctc cttctcctgc 17040
cgggctcata cacctacgag tggaacttca ggaaggatgt taacatggtt ctgcagagct 17100
ccctaggaaa tgacctaagg gttgacggag ccagcattaa gtttgatagc atttgccttt 17160
acgccacctt cttccccatg gcccacaaca ccgcctccac gcttgaggcc atgcttagaa 17220
acgacaccaa cgaccagtcc tttaacgact atctctccgc cgccaacatg ctctacccta 17280
tacccgccaa cgctaccaac gtgcccatat ccatcccctc ccgcaactgg gcggctttcc 17340
gcggctgggc cttcacgcgc cttaagacta aggaaacccc atcactgggc tcgggctacg 17400
acccttatta cacctactct ggctctatac cctacctaga tggaaccttt tacctcaacc 17460
acacctttaa gaaggtggcc attacctttg actcttctgt cagctggcct ggcaatgacc 17520
gcctgcttac ccccaacgag tttgaaatta agcgctcagt tgacggggag ggttacaacg 17580
ttgcccagtg taacatgacc aaagactggt tcctggtaca aatgctagct aactacaaca 17640
ttggctacca gggcttctat atcccagaga gctacaagga ccgcatgtac tccttcttta 17700
gaaacttcca gcccatgagc cgtcaggtgg tggatgatac taaatacaag gactaccaac 17760
aggtgggcat cctacaccaa cacaacaact ctggatttgt tggctacctt gcccccacca 17820

tgcgcgaagg acaggcctac cctgctaact tcccctatcc gcttataggc aagaccgcag 17880

ttgacagcat tacccagaaa aagtttcttt gcgatcgcac cctttggcgc atcccattct 17940

ccagtaactt tatgtccatg ggcgcactca cagacctggg ccaaaacctt ctctacgcca 18000

actccgccca cgcgctagac atgacttttg aggtggatcc 18040

<210> 70
<211> 1238
<212> DNA
<213> Artificial Sequence

<220>
<223> 7th DNA fragment

<400> 70
ctgcctaggc aaaatagcac cctcccgctc cagaacaaca tacagcgctt ccacagcggc 60

agccataaca gtcagcctta ccagtaaaaa agaaaaccta ttaaaaaaac accactcgac 120

acggcaccag ctcaatcagt cacagtgtaa aaaagggcca agtgcagagc gagtatatat 180

aggactaaaa aatgacgtaa cggttaaagt ccacaaaaaa cacccagaaa accgcacgcg 240

aacctacgcc cagaaacgaa agccaaaaaa cccacaactt cctcaaatcg tcacttccgt 300

tttcccacgt tacgtcactt cccattttaa gaaaactaca attcccaaca catacaagtt 360

actccgccct aaaacttaca tcgttaatta agcagtgggc ttacatggcg atagctagac 420

tgggcggttt tatggacagc aagcgaaccg gaattgccag ctggggcgcc ctctggtaag 480

gttgggaagc cctgcaaagt aaactggatg gctttcttgc cgccaaggat ctgatggcgc 540

aggggatcaa gctctgatca agagacagga tgaggatcgt ttcgcatgat tgaacaagat 600

ggattgcacg caggttctcc ggccgcttgg gtggagaggc tattcggcta tgactgggca 660

caacagacaa tcggctgctc tgatgccgcc gtgttccggc tgtcagcgca ggggcgcccg 720

gttctttttg tcaagaccga cctgtccggt gccctgaatg aactgcaaga cgaggcagcg 780

cggctatcgt ggctggccac gacgggcgtt ccttgcgcag ctgtgctcga cgttgtcact 840

gaagcgggaa gggactggct gctattgggc gaagtgccgg ggcaggatct cctgtcatct 900

caccttgctc ctgccgagaa agtatccatc atggctgatg caatgcggcg gctgcatacg 960

cttgatccgg ctacctgccc attcgaccac caagcgaaac atcgcatcga gcgagcacgt 1020

actcggatgg aagccggtct tgtcgatcag gatgatctgg acgaagagca tcaggggctc 1080

gcgccagccg aactgttcgc caggctcaag gcgagcatgc ccgacggcga ggatctcgtc 1140

gtgacccatg gcgatgcctg cttgccgaat atcatggtgg aaaatggccg cttttctgga 1200

ttcatcgact gtggccggct gggtgtggcg gaccgcta 1238

<210> 71
<211> 30991
<212> DNA
<213> Artificial Sequence

<220>
<223> pAdBest_dITR

<400> 71
ttaattaagc agtgggctta catggcgata gctagactgg gcggttttat ggacagcaag 60

cgaaccggaa ttgccagctg gggcgccctc tggtaaggtt gggaagccct gcaaagtaaa 120

ctggatggct ttcttgccgc caaggatctg atggcgcagg ggatcaagct ctgatcaaga 180

gacaggatga ggatcgtttc gcatgattga acaagatgga ttgcacgcag gttctccggc 240

cgcttgggtg gagaggctat tcggctatga ctgggcacaa cagacaatcg gctgctctga 300

tgccgccgtg ttccggctgt cagcgcaggg gcgcccggtt ctttttgtca agaccgacct 360

gtccggtgcc ctgaatgaac tgcaagacga ggcagcgcgg ctatcgtggc tggccacgac 420

gggcgttcct tgcgcagctg tgctcgacgt tgtcactgaa gcgggaaggg actggctgct 480

attgggcgaa gtgccggggc aggatctcct gtcatctcac cttgctcctg ccgagaaagt 540

atccatcatg gctgatgcaa tgcggcggct gcatacgctt gatccggcta cctgcccatt 600

cgaccaccaa gcgaaacatc gcatcgagcg agcacgtact cggatggaag ccggtcttgt 660

cgatcaggat gatctggacg aagagcatca ggggctcgcg ccagccgaac tgttcgccag 720

gctcaaggcg agcatgcccg acggcgagga tctcgtcgtg acccatggcg atgcctgctt 780

gccgaatatc atggtggaaa atggccgctt ttctggattc atcgactgtg gccggctggg 840

tgtggcggac cgctatcagg acatagcgtt ggctacccgt gatattgctg aagagcttgg 900

cggcgaatgg gctgaccgct tcctcgtgct ttacggtatc gccgctcccg attcgcagcg 960

catcgccttc tatcgccttc ttgacgagtt cttctgacct caggttactc atatatactt 1020

tagattgatt taaaacttca tttttaattt aaaaggatct aggtgaagat cctttttgat 1080

aatctcatga ccaaaatccc ttaacgtgag ttttcgttcc actgagcgtc agaccccgta 1140

gaaaagatca aaggatcttc ttgagatcct ttttttctgc gcgtaatctg ctgcttgcaa 1200

acaaaaaaac caccgctacc agcggtggtt tgtttgccgg atcaagagct accaactctt 1260

tttccgaagg taactggctt cagcagagcg cagataccaa atactgtcct tctagtgtag 1320

ccgtagttag gccaccactt caagaactct gtagcaccgc ctacatacct cgctctgcta 1380

atcctgttac cagtggctgc tgccagtggc gataagtcgt gtcttaccgg gttggactca 1440

agacgatagt taccggataa ggcgcagcgg tcgggctgaa cggggggttc gtgcacacag 1500

cccagcttgg agcgaacgac ctacaccgaa ctgagatacc tacagcgtga gctatgagaa 1560

agcgccacgc ttcccgaagg gagaaaggcg gacaggtatc cggtaagcgg cagggtcgga 1620

acaggagagc gcacgaggga gcttccaggg ggaaacgcct ggtatcttta tagtcctgtc 1680
gggtttcgcc acctctgact tgagcgtcga tttttgtgat gctcgtcagg ggggcggagc 1740
ctatggaaaa acgccagcaa cgcggccttt ttacggttcc tggccttttg ctggcctttt 1800
gctcttaatt aaatcgattt aagggtggga aagaatatat aaggtggggg tcttatgtag 1860
ttttgtatct gttttgcagc agccgccgcc gccatgagca ccaactcgtt tgatggaagc 1920
attgtgagct catatttgac aacgcgcatg cccccatggg ccggggtgcg tcagaatgtg 1980
atgggctcca gcattgatgg tcgccccgtc ctgcccgcaa actctactac cttgacctac 2040
gagaccgtgt ctggaacgcc gttggagact gcagcctccg ccgccgcttc agccgctgca 2100
gccaccgccc gcgggattgt gactgacttt gctttcctga gcccgcttgc aagcagtgca 2160
gcttcccgtt catccgcccg cgatgacaag ttgacggctc ttttggcaca attggattct 2220
ttgacccggg aacttaatgt cgtttctcag cagctgttgg atctgcgcca gcaggtttct 2280
gccctgaagg cttcctcccc tcccaatgcg gtttaaaaca taaataaaaa accagactct 2340
gtttggattt ggatcaagca agtgtcttgc tgtctttatt taggggtttt gcgcgcgcgg 2400
taggcccggg accagcggtc tcggtcgttg agggtcctgt gtattttttc caggacgtgg 2460
taaaggtgac tctggatgtt cagatacatg ggcataagcc cgtctctggg gtggaggtag 2520
caccactgca gagcttcatg ctgcggggtg gtgttgtaga tgatccagtc gtagcaggag 2580
cgctgggcgt ggtgcctaaa aatgtctttc agtagcaagc tgattgccag gggcaggccc 2640
ttggtgtaag tgtttacaaa gcggttaagc tgggatgggt gcatacgtgg ggatatgaga 2700
tgcatcttgg actgtatttt taggttggct atgttcccag ccatatccct ccggggattc 2760
atgttgtgca gaaccaccag cacagtgtat ccggtgcact tgggaaattt gtcatgtagc 2820
ttagaaggaa atgcgtggaa gaacttggag acgcccttgt gacctccaag attttccatg 2880
cattcgtcca taatgatggc aatgggccca cgggcggcgg cctgggcgaa gatatttctg 2940
ggatcactaa cgtcatagtt gtgttccagg atgagatcgt cataggccat ttttacaaag 3000
cgcgggcgga gggtgccaga ctgcggtata atggttccat ccggcccagg ggcgtagtta 3060
ccctcacaga tttgcatttc ccacgctttg agttcagatg gggggatcat gtctacctgc 3120
ggggcgatga agaaaacggt ttccggggta ggggagatca gctgggaaga aagcaggttc 3180
ctgagcagct gcgacttacc gcagccggtg ggcccgtaaa tcacacctat taccgggtgc 3240
aactggtagt taagagagct gcagctgccg tcatccctga gcaggggggc cacttcgtta 3300
agcatgtccc tgactcgcat gttttccctg accaaatccg ccagaaggcg ctcgccgccc 3360
agcgatagca gttcttgcaa ggaagcaaag tttttcaacg gtttgagacc gtccgccgta 3420
ggcatgcttt tgagcgtttg accaagcagt tccaggcggt cccacagctc ggtcacctgc 3480

tctacggcat ctcgatccag catatctcct cgtttcgcgg gttggggcgg ctttcgctgt 3540
acggcagtag tcggtgctcg tccagacggg ccagggtcat gtctttccac gggcgcaggg 3600
tcctcgtcag cgtagtctgg gtcacggtga aggggtgcgc tccgggctgc gcgctggcca 3660
gggtgcgctt gaggctggtc ctgctggtgc tgaagcgctg ccggtcttcg ccctgcgcgt 3720
cggccaggta gcatttgacc atggtgtcat agtccagccc ctccgcggcg tggcccttgg 3780
cgcgcagctt gcccttggag gaggcgccgc acgaggggca gtgcagactt ttgagggcgt 3840
agagcttggg cgcgagaaat accgattccg gggagtaggc atccgcgccg caggccccgc 3900
agacggtctc gcattccacg agccaggtga gctctggccg ttcggggtca aaaaccaggt 3960
ttcccccatg ctttttgatg cgtttcttac ctctggtttc catgagccgg tgtccacgct 4020
cggtgacgaa aaggctgtcc gtgtccccgt atacagactt gagaggcctg tcctcgagcg 4080
gtgttccgcg gtcctcctcg tatagaaact cggaccactc tgagacaaag gctcgcgtcc 4140
aggccagcac gaaggaggct aagtgggagg ggtagcggtc gttgtccact aggggggtcca 4200
ctcgctccag ggtgtgaaga cacatgtcgc cctcttcggc atcaaggaag gtgattggtt 4260
tgtaggtgta ggccacgtga ccgggtgttc ctgaaggggg gctataaaag gggggtgggg 4320
cgcgttcgtc ctcactctct tccgcatcgc tgtctgcgag ggccagctgt tggggtgagt 4380
actccctctg aaaagcgggc atgacttctg cgctaagatt gtcagtttcc aaaaacgagg 4440
aggatttgat attcacctgg cccgcggtga tgcctttgag ggtggccgca tccatctggt 4500
cagaaaagac aatctttttg ttgtcaagct tggtggcaaa cgacccgtag agggcgttgg 4560
acagcaactt ggcgatggag cgcagggttt ggtttttgtc gcgatcggcg cgctccttgg 4620
ccgcgatgtt tagctgcacg tattcgcgcg caacgcaccg ccattcggga aagacggtgg 4680
tgcgctcgtc gggcaccagg tgcacgcgcc aaccgcggtt gtgcagggtg acaaggtcaa 4740
cgctggtggc tacctctccg cgtaggcgct cgttggtcca gcagaggcgg ccgcccttgc 4800
gcgagcagaa tggcggtagg gggtctagct gcgtctcgtc cggggggtct gcgtccacgg 4860
taaagacccc gggcagcagg cgcgcgtcga agtagtctat cttgcatcct tgcaagtcta 4920
gcgcctgctg ccatgcgcgg gcggcaagcg cgcgctcgta tgggttgagt gggggacccc 4980
atggcatggg gtgggtgagc gcggaggcgt acatgccgca aatgtcgtaa acgtagaggg 5040
gctctctgag tattccaaga tatgtagggt agcatcttcc accgcggatg ctggcgcgca 5100
cgtaatcgta tagttcgtgc gagggagcga ggaggtcggg accgaggttg ctacgggcgg 5160
gctgctctgc tcggaagact atctgcctga agatggcatg tgagttggat gatatggttg 5220
gacgctggaa gacgttgaag ctggcgtctg tgagacctac cgcgtcacgc acgaaggagg 5280
cgtaggagtc gcgcagcttg ttgaccagct cggcggtgac ctgcacgtct agggcgcagt 5340
agtccagggt ttccttgatg atgtcatact tatcctgtcc cttttttttc cacagctcgc 5400

ggttgaggac aaactcttcg cggtctttcc agtactcttg gatcggaaac ccgtcggcct 5460

ccgaacggta agagcctagc atgtagaact ggttgacggc ctggtaggcg cagcatccct 5520

tttctacggg tagcgcgtat gcctgcgcgg ccttccggag cgaggtgtgg gtgagcgcaa 5580

aggtgtccct gaccatgact ttgaggtact ggtatttgaa gtcagtgtcg tcgcatccgc 5640

cctgctccca gagcaaaaag tccgtgcgct ttttggaacg cggatttggc agggcgaagg 5700

tgacatcgtt gaagagtatc tttcccgcgc gaggcataaa gttgcgtgtg atgcggaagg 5760

gtcccggcac ctcggaacgg ttgttaatta cctgggcggc gagcacgatc tcgtcaaagc 5820

cgttgatgtt gtggcccaca atgtaaagtt ccaagaagcg cgggatgccc ttgatggaag 5880

gcaatttttt aagttcctcg taggtgagct cttcagggga gctgagcccg tgctctgaaa 5940

gggcccagtc tgcaagatga gggttggaag cgacgaatga gctccacagg tcacgggcca 6000

ttagcatttg caggtggtcg cgaaaggtcc taaactggcg acctatggcc atttttctg 6060

gggtgatgca gtagaaggta agcgggtctt gttcccagcg gtcccatcca aggttcgcgg 6120

ctaggtctcg cgcggcagtc actagaggct catctccgcc gaacttcatg accagcatga 6180

agggcacgag ctgcttccca aaggccccca tccaagtata ggtctctaca tcgtaggtga 6240

caaagagacg ctcggtgcga ggatgcgagc cgatcgggaa gaactggatc tcccgccacc 6300

aattggagga gtggctattg atgtggtgaa agtagaagtc cctgcgacgg gccgaacact 6360

cgtgctggct tttgtaaaaa cgtgcgcagt actggcagcg gtgcacgggc tgtacatcct 6420

gcacgaggtt gacctgacga ccgcgcacaa ggaagcagag tgggaatttg agcccctcgc 6480

ctggcgggtt tggctggtgg tcttctactt cggctgcttg tccttgaccg tctggctgct 6540

cgaggggagt tacggtggat cggaccacca cgccgcgcga gcccaaagtc cagatgtccg 6600

cgcgcggcgg tcggagcttg atgacaacat cgcgcagatg ggagctgtcc atggtctgga 6660

gctcccgcgg cgtcaggtca ggcgggagct cctgcaggtt tacctcgcat agacgggtca 6720

gggcgcgggc tagatccagg tgatacctaa tttccagggg ctggttggtg gcggcgtcga 6780

tggcttgcaa gaggccgcat ccccgcggcg cgactacggt accgcgcggc gggcggtggg 6840

ccgcgggggt gtccttggat gatgcatcta aaagcggtga cgcgggcgag ccccggagg 6900

taggggggc tccggacccg ccgggagagg gggcaggggc acgtcggcgc cgcgcgcggg 6960

caggagctgg tgctgcgcgc gtaggttgct ggcgaacgcg acgacgcggc ggttgatctc 7020

ctgaatctgg cgcctctgcg tgaagacgac gggcccggtg agcttgagcc tgaaagagag 7080

ttcgacagaa tcaatttcgg tgtcgttgac ggcggcctgg cgcaaaatct cctgcacgtc 7140

tcctgagttg tcttgatagg cgatctcggc catgaactgc tcgatctctt cctcctggag 7200

atctccgcgt ccggctcgct ccacggtggc ggcgaggtcg ttggaaatgc gggccatgag 7260

ctgcgagaag gcgttgaggc ctccctcgtt ccagacgcgg ctgtagacca cgccccccttc 7320
ggcatcgcgg gcgcgcatga ccacctgcgc gagattgagc tccacgtgcc gggcgaagac 7380
ggcgtagttt cgcaggcgct gaaagaggta gttgagggtg gtggcggtgt gttctgccac 7440
gaagaagtac ataacccagc gtcgcaacgt ggattcgttg atatccccca aggcctcaag 7500
gcgctccatg gcctcgtaga agtccacggc gaagttgaaa aactgggagt tgcgcgccga 7560
cacggttaac tcctcctcca gaagacggat gagctcggcg acagtgtcgc gcacctcgcg 7620
ctcaaaggct acaggggcct cttcttcttc ttcaatctcc tcttccataa gggcctcccc 7680
ttcttcttct tctggcggcg gtgggggagg ggggacacgg cggcgacgac ggcgcaccgg 7740
gaggcggtcg acaaagcgct cgatcatctc cccgcggcga cggcgcatgg tctcggtgac 7800
ggcgcggccg ttctcgcggg ggcgcagttg gaagacgccg cccgtcatgt cccggttatg 7860
ggttggcggg gggctgccat gcggcaggga tacggcgcta acgatgcatc tcaacaattg 7920
ttgtgtaggt actccgccgc cgagggacct gagcgagtcc gcatcgaccg gatcggaaaa 7980
cctctcgaga aaggcgtcta accagtcaca gtcgcaaggt aggctgagca ccgtggcggg 8040
cggcagcggg cggcggtcgg ggttgtttct ggcggaggtg ctgctgatga tgtaattaaa 8100
gtaggcggtc ttgagacggc ggatggtcga cagaagcacc atgtccttgg gtccggcctg 8160
ctgaatgcgc aggcggtcgg ccatgcccca ggcttcgttt tgacatcggc gcaggtcttt 8220
gtagtagtct tgcatgagcc tttctaccgg cacttcttct tctccttcct cttgtcctgc 8280
atctcttgca tctatcgctg cggcggcggc ggagtttggc cgtaggtggc gccctcttcc 8340
tcccatgcgt gtgaccccga agcccctcat cggctgaagc agggctaggt cggcgacaac 8400
gcgctcggct aatatggcct gctgcacctg cgtgagggta gactggaagt catccatgtc 8460
cacaaagcgg tggtatgcgc ccgtgttgat ggtgtaagtg cagttggcca taacggacca 8520
gttaacggtc tggtgacccg gctgcgagag ctcggtgtac ctgagacgcg agtaagccct 8580
cgagtcaaat acgtagtcgt tgcaagtccg caccaggtac tggtatccca ccaaaaagtg 8640
cggcggcggc tggcggtaga ggggccagcg tagggtggcc ggggctccgg gggcgagatc 8700
ttccaacata aggcgatgat atccgtagat gtacctggac atccaggtga tgccggcggc 8760
ggtggtggag gcgcgcggaa agtcgcggac gcggttccag atgttgcgca gcggcaaaaa 8820
gtgctccatg gtcgggacgc tctggccggt caggcgcgcg caatcgttga cgctctagac 8880
cgtgcaaaag gagagcctgt aagcgggcac tcttccgtgg tctggtggat aaattcgcaa 8940
gggtatcatg gcggacgacc ggggttcgag ccccgtatcc ggccgtccgc cgtgatccat 9000
gcggttaccg cccgcgtgtc gaacccaggt gtgcgacgtc agacaacggg ggagtgctcc 9060
ttttggcttc cttccaggcg cggcggctgc tgcgctagct tttttggcca ctggccgcgc 9120
gcagcgtaag cggttaggct ggaaagcgaa agcattaagt ggctcgctcc ctgtagccgg 9180

agggttattt tccaagggtt gagtcgcggg acccccggtt cgagtctcgg accggccgga 9240
ctgcggcgaa cgggggtttg cctccccgtc atgcaagacc ccgcttgcaa attcctccgg 9300
aaacagggac gagccccttt tttgcttttc ccagatgcat ccggtgctgc ggcagatgcg 9360
ccccctcct cagcagcggc aagagcaaga gcagcggcag acatgcaggg caccctcccc 9420
tcctcctacc gcgtcaggag gggcgacatc cgcggttgac gcggcagcag atggtgatta 9480
cgaacccccg cggcgccggg cccggcacta cctggacttg gaggagggcg agggcctggc 9540
gcggctagga gcgccctctc ctgagcggta cccaagggtg cagctgaagc gtgatacgcg 9600
tgaggcgtac gtgccgcggc agaacctgtt tcgcgaccgc gagggagagg agcccgagga 9660
gatgcgggat cgaaagttcc acgcagggcg cgagctgcgg catggcctga atcgcgagcg 9720
gttgctgcgc gaggaggact ttgagcccga cgcgcgaacc gggattagtc ccgcgcgcgc 9780
acacgtggcg gccgccgacc tggtaaccgc atacgagcag acggtgaacc aggagattaa 9840
ctttcaaaaa agctttaaca accacgtgcg tacgcttgtg gcgcgcgagg aggtggctat 9900
aggactgatg catctgtggg actttgtaag cgcgctggag caaaacccaa atagcaagcc 9960
gctcatggcg cagctgttcc ttatagtgca gcacagcagg gacaacgagg cattcaggga 10020
tgcgctgcta aacatagtag agcccgaggg ccgctggctg ctcgatttga taaacatcct 10080
gcagagcata gtggtgcagg agcgcagctt gagcctggct gacaaggtgg ccgccatcaa 10140
ctattccatg cttagcctgg gcaagtttta cgcccgcaag atataccata ccccttacgt 10200
tcccatagac aaggaggtaa agatcgaggg gttctacatg cgcatggcgc tgaaggtgct 10260
taccttgagc gacgacctgg gcgtttatcg caacgagcgc atccacaagg ccgtgagcgt 10320
gagccggcgg cgcgagctca gcgaccgcga gctgatgcac agcctgcaaa gggccctggc 10380
tggcacgggc agcggcgata gagaggccga gtcctacttt gacgcgggcg ctgacctgcg 10440
ctgggcccca agccgacgcg ccctggaggc agctggggcc ggacctgggc tggcggtggc 10500
acccgcgcgc gctggcaacg tcggcggcgt ggaggaatat gacgaggacg atgagtacga 10560
gccagaggac ggcgagtact aagcggtgat gtttctgatc agatgatgca agacgcaacg 10620
gacccggcgg tgcgggcggc gctgcagagc cagccgtccg gccttaactc cacggacgac 10680
tggcgccagg tcatggaccg catcatgtcg ctgactgcgc gcaatcctga cgcgttccgg 10740
cagcagccgc aggccaaccg gctctccgca attctggaag cggtggtccc ggcgcgcgca 10800
aaccccacgc acgagaaggt gctggcgatc gtaaacgcgc tggccgaaaa cagggccatc 10860
cggcccgacg aggccggcct ggtctacgac gcgctgcttc agcgcgtggc tcgttacaac 10920
agcggcaacg tgcagaccaa cctggaccgg ctggtggggg atgtgcgcga ggccgtggcg 10980
cagcgtgagc gcgcgcagca gcagggcaac ctgggctcca tggttgcact aaacgccttc 11040

ctgagtacac agcccgccaa cgtgccgcgg ggacaggagg actacaccaa ctttgtgagc 11100
gcactgcggc taatggtgac tgagacaccg caaagtgagg tgtaccagtc tgggccagac 11160
tattttttcc agaccagtag acaaggcctg cagaccgtaa acctgagcca ggctttcaaa 11220
aacttgcagg ggctgtgggg ggtgcgggct cccacaggcg accgcgcgac cgtgtctagc 11280
ttgctgacgc ccaactcgcg cctgttgctg ctgctaatag cgcccttcac ggacagtggc 11340
agcgtgtccc gggacacata cctaggtcac ttgctgacac tgtaccgcga ggccataggt 11400
caggcgcatg tggacgagca tactttccag gagattacaa gtgtcagccg cgcgctgggg 11460
caggaggaca cgggcagcct ggaggcaacc ctaaactacc tgctgaccaa ccggcggcag 11520
aagatcccct cgttgcacag tttaaacagc gaggaggagc gcattttgcg ctacgtgcag 11580
cagagcgtga gccttaacct gatgcgcgac ggggtaacgc ccagcgtggc gctggacatg 11640
accgcgcgca acatggaacc gggcatgtat gcctcaaacc ggccgtttat caaccgccta 11700
atggactact tgcatcgcgc ggccgccgtg aaccccgagt atttcaccaa tgccatcttg 11760
aacccgcact ggctaccgcc ccctggtttc tacaccgggg gattcgaggt gcccgagggt 11820
aacgatggat tcctctggga cgacatagac gacagcgtgt tttccccgca accgcagacc 11880
ctgctagagt tgcaacagcg cgagcaggca gaggcggcgc tgcgaaagga aagcttccgc 11940
aggccaagca gcttgtccga tctaggcgct gcggccccgc ggtcagatgc tagtagccca 12000
tttccaagct tgatagggtc tcttaccagc actcgcacca cccgcccgcg cctgctgggc 12060
gaggaggagt acctaaacaa ctcgctgctg cagccgcagc gcgaaaaaaa cctgcctccg 12120
gcatttccca acaacgggat agagagccta gtggacaaga tgagtagatg gaagacgtac 12180
gcgcaggagc acagggacgt gccaggcccg cgcccgccca cccgtcgtca aaggcacgac 12240
cgtcagcggg gtctggtgtg ggaggacgat gactcggcag acgacagcag cgtcctggat 12300
ttgggaggga gtggcaaccc gtttgcgcac cttcgcccca ggctggggag aatgttttaa 12360
aaaaaaaaaa gcatgatgca aaataaaaaa ctcaccaagg ccatggcacc gagcgttggt 12420
tttcttgtat tccccttagt atgcggcgcg cggcgatgta tgaggaaggt cctcctccct 12480
cctacgagag tgtggtgagc gcggcgccag tggcggcggc gctgggttct cccttcgatg 12540
ctcccctgga cccgccgttt gtgcctccgc ggtacctgcg gcctaccggg gggagaaaca 12600
gcatccgtta ctctgagttg gcacccctat tcgacaccac ccgtgtgtac ctggtggaca 12660
acaagtcaac ggatgtggca tccctgaact accagaacga ccacagcaac tttctgacca 12720
cggtcattca aaacaatgac tacagcccgg gggaggcaag cacacagacc atcaatcttg 12780
acgaccggtc gcactggggc ggcgacctga aaaccatcct gcataccaac atgccaaatg 12840
tgaacgagtt catgtttacc aataagttta aggcgcgggt gatggtgtcg cgcttgccta 12900
ctaaggacaa tcaggtggag ctgaaatacg agtgggtgga gttcacgctg cccgagggca 12960

actactccga gaccatgacc atagacctta tgaacaacgc gatcgtggag cactacttga 13020
aagtgggcag acagaacggg gttctggaaa gcgacatcgg ggtaaagttt gacacccgca 13080
acttcagact ggggtttgac cccgtcactg gtcttgtcat gcctggggta tatacaaacg 13140
aagccttcca tccagacatc attttgctgc caggatgcgg ggtggacttc acccacagcc 13200
gcctgagcaa cttgttgggc atccgcaagc ggcaaccctt ccaggagggc tttaggatca 13260
cctacgatga tctggagggt ggtaacattc ccgcactgtt ggatgtggac gcctaccagg 13320
cgagcttgaa agatgacacc gaacagggcg gggtggcgc aggcggcagc aacagcagtg 13380
gcagcggcgc ggaagagaac tccaacgcgg cagccgcggc aatgcagccg gtggaggaca 13440
tgaacgatca tgccattcgc ggcgacacct ttgccacacg ggctgaggag aagcgcgctg 13500
aggccgaagc agcggccgaa gctgccgccc ccgctgcgca acccgaggtc gagaagcctc 13560
agaagaaacc ggtgatcaaa cccctgacag aggacagcaa gaaacgcagt tacaacctaa 13620
taagcaatga cagcaccttc acccagtacc gcagctggta ccttgcatac aactacggcg 13680
accctcagac cggaatccgc tcatggaccc tgctttgcac tcctgacgta acctgcggct 13740
cggagcaggt ctactggtcg ttgccagaca tgatgcaaga ccccgtgacc ttccgctcca 13800
cgcgccagat cagcaacttt ccggtggtgg gcgccgagct gttgcccgtg cactccaaga 13860
gcttctacaa cgaccaggcc gtctactccc aactcatccg ccagtttacc tctctgaccc 13920
acgtgttcaa tcgctttccc gagaaccaga ttttggcgcg cccgccagcc cccaccatca 13980
ccaccgtcag tgaaaacgtt cctgctctca cagatcacgg gacgctaccg ctgcgcaaca 14040
gcatcggagg agtccagcga gtgaccatta ctgacgccag acgccgcacc tgcccctacg 14100
tttacaaggc cctgggcata gtctcgccgc gcgtcctatc gagccgcact ttttgagcaa 14160
gcatgtccat ccttatatcg cccagcaata acacaggctg gggcctgcgc ttcccaagca 14220
agatgtttgg cggggccaag aagcgctccg accaacaccc agtgcgcgtg cgcgggcact 14280
accgcgcgcc ctggggcgcg cacaaacgcg gccgcactgg gcgcaccacc gtcgatgacg 14340
ccatcgacgc ggtggtggag gaggcgcgca actacacgcc cacgccgcca ccagtgtcca 14400
cagtggacgc ggccattcag accgtggtgc gcggagcccg gcgctatgct aaaatgaaga 14460
gacggcggag gcgcgtagca cgtcgccacc gccgccgacc cggcactgcc gcccaacgcg 14520
cggcggcggc cctgcttaac cgcgcacgtc gcaccggccg acgggcggcc atgcgggccg 14580
ctcgaaggct ggccgcgggt attgtcactg tgccccccag gtccaggcga cgagcggccg 14640
ccgcagcagc cgcggccatt agtgctatga ctcagggtcg caggggcaac gtgtattggg 14700
tgcgcgactc ggttagcggc ctgcgcgtgc ccgtgcgcac ccgccccccg cgcaactaga 14760
ttgcaagaaa aaactactta gactcgtact gttgtatgta tccagcggcg gcggcgcgca 14820

acgaagctat gtccaagcgc aaaatcaaag aagagatgct ccaggtcatc gcgccggaga 14880

tctatggccc cccgaagaag gaagagcagg attacaagcc ccgaaagcta aagcgggtca 14940

aaaagaaaaa gaaagatgat gatgatgaac ttgacgacga ggtggaactg ctgcacgcta 15000

ccgcgcccag gcgacgggta cagtggaaag gtcgacgcgt aaaacgtgtt ttgcgacccg 15060

gcaccaccgt agtctttacg cccggtgagc gctccacccg cacctacaag cgcgtgtatg 15120

atgaggtgta cggcgacgag gacctgcttg agcaggccaa cgagcgcctc ggggagtttg 15180

cctacggaaa gcggcataag gacatgctgg cgttgccgct ggacgagggc aacccaacac 15240

ctagcctaaa gcccgtaaca ctgcagcagg tgctgcccgc gcttgcaccg tccgaagaaa 15300

agcgcggcct aaagcgcgag tctggtgact tggcacccac cgtgcagctg atggtaccca 15360

agcgccagcg actggaagat gtcttggaaa aaatgaccgt ggaacctggg ctggagcccg 15420

aggtccgcgt gcggccaatc aagcaggtgg cgccgggact gggcgtgcag accgtggacg 15480

ttcagatacc cactaccagt agcaccagta ttgccaccgc cacagagggc atggagacac 15540

aaacgtcccc ggttgcctca gcggtggcgg atgccgcggt gcaggcggtc gctgcggccg 15600

cgtccaagac ctctacggag gtgcaaacgg acccgtggat gtttcgcgtt tcagcccccc 15660

ggcgcccgcg cggttcgagg aagtacggcg ccgccagcgc gctactgccc gaatatgccc 15720

tacatccttc cattgcgcct acccccggct atcgtggcta cacctaccgc cccagaagac 15780

gagcaactac ccgacgccga accaccactg gaacccgccg ccgccgtcgc cgtcgccagc 15840

ccgtgctggc cccgatttcc gtgcgcaggg tggctcgcga aggaggcagg accctggtgc 15900

tgccaacagc gcgctaccac cccagcatcg tttaaaagcc ggtctttgtg gttcttgcag 15960

atatggccct cacctgccgc ctccgtttcc cggtgccggg attccgagga agaatgcacc 16020

gtaggagggg catggccggc cacggcctga cgggcggcat gcgtcgtgcg caccaccggc 16080

ggcggcgcgc gtcgcaccgt cgcatgcgcg gcggtatcct gcccctcctt attccactga 16140

tcgccgcggc gattggcgcc gtgcccggaa ttgcatccgt ggccttgcag gcgcagagac 16200

actgattaaa aacaagttgc atgtggaaaa atcaaaataa aaagtctgga ctctcacgct 16260

cgcttggtcc tgtaactatt ttgtagaatg gaagacatca actttgcgtc tctggccccg 16320

cgacacggct cgcgcccgtt catgggaaac tggcaagata tcggcaccag caatatgagc 16380

ggtggcgcct tcagctgggg ctcgctgtgg agcggcatta aaaatttcgg ttccaccgtt 16440

aagaactatg gcagcaaggc ctggaacagc agcacaggcc agatgctgag ggataagttg 16500

aaagagcaaa atttccaaca aaaggtggta gatggcctgg cctctggcat tagcggggtg 16560

gtggacctgg ccaaccaggc agtgcaaaat aagattaaca gtaagcttga tccccgccct 16620

cccgtagagg agcctccacc ggccgtggag acagtgtctc cagaggggcg tggcgaaaag 16680

cgtccgcgcc ccgacaggga agaaactctg gtgacgcaaa tagacgagcc tccctcgtac 16740

gaggaggcac taaagcaagg cctgcccacc acccgtccca tcgcgcccat ggctaccgga 16800

gtgctgggcc agcacacacc cgtaacgctg gacctgcctc cccccgccga cacccagcag 16860

aaacctgtgc tgccaggccc gaccgccgtt gttgtaaccc gtcctagccg cgcgtccctg 16920

cgccgcgccg ccagcggtcc gcgatcgttg cggcccgtag ccagtggcaa ctggcaaagc 16980

acactgaaca gcatcgtggg tctgggggtg caatccctga agcgccgacg atgcttctga 17040

atagctaacg tgtcgtatgt gtgtcatgta tgcgtccatg tcgccgccag aggagctgct 17100

gagccgccgc gcgcccgctt tccaagatgg ctaccccttc gatgatgccg cagtggtctt 17160

acatgcacat ctcgggccag gacgcctcgg agtacctgag ccccgggctg gtgcagtttg 17220

cccgcgccac cgagacgtac ttcagcctga ataacaagtt tagaaacccc acggtggcgc 17280

ctacgcacga cgtgaccaca gaccggtccc agcgtttgac gctgcggttc atccctgtgg 17340

accgtgagga tactgcgtac tcgtacaagg cgcggttcac cctagctgtg ggtgataacc 17400

gtgtgctgga catggcttcc acgtactttg acatccgcgg cgtgctggac aggggcccta 17460

cttttaagcc ctactctggc actgcctaca acgccctggc tcccaagggt gccccaaatc 17520

cttgcgaatg ggatgaagct gctactgctc ttgaaataaa cctagaagaa gaggacgatg 17580

acaacgaaga cgaagtagac gagcaagctg agcagcaaaa aactcacgta tttgggcagg 17640

cgccttattc tggtataaat attacaaagg agggtattca aataggtgtc gaaggtcaaa 17700

cacctaaata tgccgataaa acatttcaac ctgaacctca aataggagaa tctcagtggt 17760

acgaaactga aattaatcat gcagctggga gagtccttaa aaagactacc ccaatgaaac 17820

catgttacgg ttcatatgca aaacccacaa atgaaaatgg agggcaaggc attcttgtaa 17880

agcaacaaaa tggaaagcta gaaagtcaag tggaaatgca atttttctca actactgagg 17940

cgaccgcagg caatggtgat aacttgactc ctaaagtggt attgtacagt gaagatgtag 18000

atatagaaac cccagacact catatttctt acatgcccac tattaaggaa ggtaactcac 18060

gagaactaat gggccaacaa tctatgccca acaggcctaa ttacattgct tttagggaca 18120

attttattgg tctaatgtat tacaacagca cgggtaatat gggtgttctg gcgggccaag 18180

catcgcagtt gaatgctgtt gtagatttgc aagacagaaa cacagagctt tcataccagc 18240

ttttgcttga ttccattggt gatagaacca ggtacttttc tatgtggaat caggctgttg 18300

acagctatga tccagatgtt agaattattg aaaatcatgg aactgaagat gaacttccaa 18360

attactgctt tccactggga ggtgtgatta atacagagac tcttaccaag gtaaaaccta 18420

aaacaggtca ggaaaatgga tgggaaaaag atgctacaga attttcagat aaaaatgaaa 18480

taagagttgg aaataatttt gccatggaaa tcaatctaaa tgccaacctg tggagaaatt 18540

tcctgtactc caacatagcg ctgtatttgc ccgacaagct aaagtacagt ccttccaacg 18600

taaaaatttc tgataaccca aacacctacg actacatgaa caagcgagtg gtggctcccg 18660
ggttagtgga ctgctacatt aaccttggag cacgctggtc ccttgactat atggacaacg 18720
tcaacccatt taaccaccac cgcaatgctg gcctgcgcta ccgctcaatg ttgctgggca 18780
atggtcgcta tgtgcccttc cacatccagg tgcctcagaa gttctttgcc attaaaaacc 18840
tccttctcct gccgggctca tacacctacg agtggaactt caggaaggat gttaacatgg 18900
ttctgcagag ctccctagga aatgacctaa gggttgacgg agccagcatt aagtttgata 18960
gcatttgcct ttacgccacc ttcttcccca tggcccacaa caccgcctcc acgcttgagg 19020
ccatgcttag aaacgacacc aacgaccagt cctttaacga ctatctctcc gccgccaaca 19080
tgctctaccc tatacccgcc aacgctacca acgtgcccat atccatcccc tcccgcaact 19140
gggcggcttt ccgcggctgg gccttcacgc gccttaagac taaggaaacc ccatcactgg 19200
gctcgggcta cgacccttat tacacctact ctggctctat accctaccta gatggaacct 19260
tttacctcaa ccacaccttt aagaaggtgg ccattacctt tgactcttct gtcagctggc 19320
ctggcaatga ccgcctgctt acccccaacg agtttgaaat taagcgctca gttgacgggg 19380
agggttacaa cgttgcccag tgtaacatga ccaaagactg gttcctggta caaatgctag 19440
ctaactacaa cattggctac cagggcttct atatcccaga gagctacaag gaccgcatgt 19500
actccttctt tagaaacttc cagcccatga gccgtcaggt ggtggatgat actaaataca 19560
aggactacca acaggtgggc atcctacacc aacacaacaa ctctggattt gttggctacc 19620
ttgcccccac catgcgcgaa ggacaggcct accctgctaa cttcccctat ccgcttatag 19680
gcaagaccgc agttgacagc attacccaga aaaagtttct ttgcgatcgc accctttggc 19740
gcatcccatt ctccagtaac tttatgtcca tgggcgcact cacagacctg ggccaaaacc 19800
ttctctacgc caactccgcc cacgcgctag acatgacttt tgaggtggat cccatggacg 19860
agcccaccct tctttatgtt ttgtttgaag tctttgacgt ggtccgtgtg caccggccgc 19920
accgcggcgt catcgaaacc gtgtacctgc gcacgccctt ctcggccggc aacgccacaa 19980
cataaagaag caagcaacat caacaacagc tgccgccatg ggctccagtg agcaggaact 20040
gaaagccatt gtcaaagatc ttggttgtgg gccatatttt ttgggcacct atgacaagcg 20100
ctttccaggc tttgtttctc cacacaagct cgcctgcgcc atagtcaata cggccggtcg 20160
cgagactggg ggcgtacact ggatggcctt tgcctggaac ccgcactcaa aaacatgcta 20220
cctctttgag ccctttggct tttctgacca gcgactcaag caggtttacc agtttgagta 20280
cgagtcactc ctgcgccgta gcgccattgc ttcttccccc gaccgctgta taacgctgga 20340
aaagtccacc caaagcgtac aggggcccaa ctcggccgcc tgtggactat tctgctgcat 20400
gtttctccac gcctttgcca actggcccca aactcccatg gatcacaacc ccaccatgaa 20460
ccttattacc ggggtaccca actccatgct caacagtccc caggtacagc ccaccctgcg 20520

tcgcaaccag gaacagctct acagcttcct ggagcgccac tcgccctact tccgcagcca 20580

cagtgcgcag attaggagcg ccacttcttt ttgtcacttg aaaaacatgt aaaaataatg 20640

tactagagac actttcaata aaggcaaatg cttttatttg tacactctcg ggtgattatt 20700

tacccccacc cttgccgtct gcgccgttta aaaatcaaag ggttctgccg gcgcatcgct 20760

atgcgccact ggcagggaca cgttgcgata ctggtgttta gtgctccact taaactcagg 20820

cacaaccatc cgcggcagct cggtgaagtt ttcactccac aggctgcgca ccatcaccaa 20880

cgcgtttagc aggtcgggcg ccgatatctt gaagtcgcag ttggggcctc cgccctgcgc 20940

gcgcgagttg cgatacacag ggttgcagca ctggaacact atcagcgccg ggtggtgcac 21000

gctggccagc acgctcttgt cggagatcag atccgcgtcc aggtcctccg cgttgctcag 21060

ggcgaacgga gtcaactttg gtagctgcct tcccaaaaag ggcgcgtgcc caggctttga 21120

gttgcactcg caccgtagtg gcatcaaaag gtgaccgtgc ccggtctggg cgttaggata 21180

cagcgcctgc ataaaagcct tgatctgctt aaaagccacc tgagcctttg cgccttcaga 21240

gaagaacatg ccgcaagact tgccggaaaa ctgattggcc ggacaggccg cgtcgtgcac 21300

gcagcacctt gcgtcggtgt tggagatctg caccacattt cggccccacc ggttcttcac 21360

gatcttggcc ttgctagact gctccttcag cgcgcgctgc ccgttttcgc tcgtcacatc 21420

catttcaatc acgtgctcct tatttatcat aatgcttccg tgtagacact taagctcgcc 21480

ttcgatctca gcgcagcggt gcagccacaa cgcgcagccc gtgggctcgt gatgcttgta 21540

ggtcacctct gcaaacgact gcaggtacgc ctgcaggaat cgccccatca tcgtcacaaa 21600

ggtcttgttg ctggtgaagg tcagctgcaa cccgcggtgc tcctcgttca gccaggtctt 21660

gcatacggcc gccagagctt ccacttggtc aggcagtagt ttgaagttcg cctttagatc 21720

gttatccacg tggtacttgt ccatcagcgc gcgcgcagcc tccatgccct tctcccacgc 21780

agacacgatc ggcacactca gcgggttcat caccgtaatt tcactttccg cttcgctggg 21840

ctcttcctct tcctcttgcg tccgcatacc acgcgccact gggtcgtctt cattcagccg 21900

ccgcactgtg cgcttacctc ctttgccatg cttgattagc accggtgggt tgctgaaacc 21960

caccatttgt agcgccacat cttctctttc ttcctcgctg tccacgatta cctctggtga 22020

tggcgggcgc tcgggcttgg gagaagggcg cttctttttc ttcttgggcg caatggccaa 22080

atccgccgcc gaggtcgatg gccgcgggct gggtgtgcgc ggcaccagcg cgtcttgtga 22140

tgagtcttcc tcgtcctcgg actcgatacg ccgcctcatc cgcttttttg ggggcgcccg 22200

gggaggcggc ggcgacgggg acggggacga cacgtcctcc atggttgggg gacgtcgcgc 22260

cgcaccgcgt ccgcgctcgg gggtggtttc gcgctgctcc tcttcccgac tggccatttc 22320

cttctcctat aggcagaaaa agatcatgga gtcagtcgag aagaaggaca gcctaaccgc 22380

cccctctgag ttcgccacca ccgcctccac cgatgccgcc aacgcgccta ccaccttccc 22440

cgtcgaggca cccccgcttg aggaggagga agtgattatc gagcaggacc caggttttgt 22500

aagcgaagac gacgaggacc gctcagtacc aacagaggat aaaaagcaag accaggacaa 22560

cgcagaggca aacgaggaac aagtcgggcg gggggacgaa aggcatggcg actacctaga 22620

tgtgggagac gacgtgctgt tgaagcatct gcagcgccag tgcgccatta tctgcgacgc 22680

gttgcaagag cgcagcgatg tgcccctcgc catagcggat gtcagccttg cctacgaacg 22740

ccacctattc tcaccgcgcg taccccccaa acgccaagaa aacggcacat gcgagcccaa 22800

cccgcgcctc aacttctacc ccgtatttgc cgtgccagag gtgcttgcca cctatcacat 22860

ctttttccaa aactgcaaga tacccctatc ctgccgtgcc aaccgcagcc gagcggacaa 22920

gcagctggcc ttgcggcagg gcgctgtcat acctgatatc gcctcgctca acgaagtgcc 22980

aaaaatcttt gagggtcttg gacgcgacga gaagcgcgcg gcaaacgctc tgcaacagga 23040

aaacagcgaa aatgaaagtc actctggagt gttggtggaa ctcgagggtg acaacgcgcg 23100

cctagccgta ctaaaacgca gcatcgaggt cacccacttt gcctacccgg cacttaacct 23160

accccccaag gtcatgagca cagtcatgag tgagctgatc gtgcgccgtg cgcagcccct 23220

ggagagggat gcaaatttgc aagaacaaac agaggagggc ctacccgcag ttggcgacga 23280

gcagctagcg cgctggcttc aaacgcgcga gcctgccgac ttggaggagc gacgcaaact 23340

aatgatggcc gcagtgctcg ttaccgtgga gcttgagtgc atgcagcggt tctttgctga 23400

cccggagatg cagcgcaagc tagaggaaac attgcactac acctttcgac agggctacgt 23460

acgccaggcc tgcaagatct ccaacgtgga gctctgcaac ctggtctcct accttggaat 23520

tttgcacgaa aaccgccttg ggcaaaacgt gcttcattcc acgctcaagg gcgaggcgcg 23580

ccgcgactac gtccgcgact gcgtttactt atttctatgc tacacctggc agacggccat 23640

gggcgtttgg cagcagtgct tggaggagtg caacctcaag gagctgcaga aactgctaaa 23700

gcaaaacttg aaggacctat ggacggcctt caacgagcgc tccgtggccg cgcacctggc 23760

ggacatcatt ttccccgaac gcctgcttaa aaccctgcaa cagggtctgc cagacttcac 23820

cagtcaaagc atgttgcaga actttaggaa ctttatccta gagcgctcag gaatcttgcc 23880

cgccacctgc tgtgcacttc ctagcgactt tgtgcccatt aagtaccgcg aatgccctcc 23940

gccgctttgg ggccactgct accttctgca gctagccaac taccttgcct accactctga 24000

cataatggaa gacgtgagcg gtgacggtct actggagtgt cactgtcgct gcaacctatg 24060

caccccgcac cgctccctgg tttgcaattc gcagctgctt aacgaaagtc aaattatcgg 24120

tacctttgag ctgcagggtc cctcgcctga cgaaaagtcc gcggctccgg ggttgaaact 24180

cactccgggg ctgtggacgt cggcttacct tcgcaaattt gtacctgagg actaccacgc 24240

ccacgagatt aggttctacg aagaccaatc ccgcccgcca aatgcggagc ttaccgcctg 24300

cgtcattacc cagggccaca ttcttggcca attgcaagcc atcaacaaag cccgccaaga 24360

gtttctgcta cgaaagggac gggggtttta cttggacccc cagtccggcg aggagctcaa 24420

cccaatcccc ccgccgccgc agccctatca gcagcagccg cgggcccttg cttcccagga 24480

tggcacccaa aaagaagctg cagctgccgc cgccacccac ggacgaggag gaatactggg 24540

acagtcaggc agaggaggtt ttggacgagg aggaggagga catgatggaa gactgggaga 24600

gcctagacga ggaagcttcc gaggtcgaag aggtgtcaga cgaaacaccg tcaccctcgg 24660

tcgcattccc ctcgccggcg ccccagaaat cggcaaccgg ttccagcatg gctacaacct 24720

ccgctcctca ggcgccgccg gcactgcccg ttcgccgacc caaccgtaga tgggacacca 24780

ctggaaccag ggccggtaag tccaagcagc cgccgccgtt agcccaagag caacaacagc 24840

gccaaggcta ccgctcatgg cgcgggcaca agaacgccat agttgcttgc ttgcaagact 24900

gtgggggcaa catctccttc gcccgccgct ttcttctcta ccatcacggc gtggccttcc 24960

cccgtaacat cctgcattac taccgtcatc tctacagccc atactgcacc ggcggcagcg 25020

gcagcggcag caacagcagc ggccacacag aagcaaaggc gaccggatag caagactctg 25080

acaaagccca agaaatccac agcggcggca gcagcaggag gaggagcgct gcgtctggcg 25140

cccaacgaac ccgtatcgac ccgcgagctt agaaacagga tttttcccac tctgtatgct 25200

atatttcaac agagcagggg ccaagaacaa gagctgaaaa taaaaaacag gtctctgcga 25260

tccctcaccc gcagctgcct gtatcacaaa agcgaagatc agcttcggcg cacgctggaa 25320

gacgcggagg ctctcttcag taaatactgc gcgctgactc ttaaggacta gtttcgcgcc 25380

ctttctcaaa tttaagcgcg aaaactacgt catctccagc ggccacaccc ggcgccagca 25440

cctgtcgtca gcgccattat gagcaaggaa attcccacgc cctacatgtg gagttaccag 25500

ccacaaatgg gacttgcggc tggagctgcc caagactact caacccgaat aaactacatg 25560

agcgcgggac cccacatgat atcccgggtc aacggaatcc gcgcccaccg aaaccgaatt 25620

ctcttggaac aggcggctat taccaccaca cctcgtaata accttaatcc ccgtagttgg 25680

cccgctgccc tggtgtacca ggaaagtccc gctcccacca ctgtggtact tcccagagac 25740

gcccaggccg aagttcagat gactaactca ggggcgcagc ttgcgggcgg ctttcgtcac 25800

agggtgcggt cgcccgggca gggtataact cacctgacaa tcagagggcg aggtattcag 25860

ctcaacgacg agtcggtgag ctcctcgctt ggtctccgtc cggacgggac atttcagatc 25920

ggcggcgccg gccgtccttc attcacgcct cgtcaggcaa tcctaactct gcagacctcg 25980

tcctctgagc cgcgctctgg aggcattgga actctgcaat ttattgagga gtttgtgcca 26040

tcggtctact ttaacccctt ctcgggacct cccggccact atccggatca atttattcct 26100

aactttgacg cggtaaagga ctcggcggac ggctacgact gaatgttagt tcctgtccat 26160

ccgcacccac tatcttcatg ttgttgcaga tgaagcgcgc aagaccgtct gaagatacct 26220
tcaaccccgt gtatccatat gacacggaaa ccggtcctcc aactgtgcct tttcttactc 26280
ctccctttgt atcccccaat gggtttcaag agagtccccc tggggtactc tctttgcgcc 26340
tatccgaacc tctagttacc tccaatggca tgcttgcgct caaaatgggc aacggcctct 26400
ctctggacga ggccggcaac cttacctccc aaaatgtaac cactgtgagc ccacctctca 26460
aaaaaaccaa gtcaaacata aacctggaaa tatctgcacc cctcacagtt acctcagaag 26520
ccctaactgt ggctgccgcc gcacctctaa tggtcgcggg caacacactc accatgcaat 26580
cacaggcccc gctaaccgtg cacgactcca aacttagcat tgccacccaa ggacccctca 26640
cagtgtcaga aggaaagcta gccctgcaaa catcaggccc cctcaccacc accgatagca 26700
gtacccttac tatcactgcc tcacccccctc taactactgc cactggtagc ttgggcattg 26760
acttgaaaga gcccatttat acacaaaatg gaaaactagg actaaagtac ggggctcctt 26820
tgcatgtaac agacgaccta aacactttga ccgtagcaac tggtccaggt gtgactatta 26880
ataatacttc cttgcaaact aaagttactg gagccttggg ttttgattca caaggcaata 26940
tgcaacttaa tgtagcagga ggactaagga ttgattctca aaacagacgc cttatacttg 27000
atgttagtta tccgtttgat gctcaaaacc aactaaatct aagactagga cagggccctc 27060
tttttataaa ctcagcccac aacttggata ttaactacaa caaaggcctt tacttgttta 27120
cagcttcaaa caattccaaa aagcttgagg ttaacctaag cactgccaag gggttgatgt 27180
ttgacgctac agccatagcc attaatgcag gagatgggct tgaatttggt tcacctaatg 27240
caccaaacac aaatcccctc aaaacaaaaa ttggccatgg cctagaattt gattcaaaca 27300
aggctatggt tcctaaacta ggaactggcc ttagttttga cagcacaggt gccattacag 27360
taggaaacaa aaataatgat aagctaactt tgtggaccac accagctcca tctcctaact 27420
gtagactaaa tgcagagaaa gatgctaaac tcactttggt cttaacaaaa tgtggcagtc 27480
aaatacttgc tacagtttca gttttggctg ttaaaggcag tttggctcca atatctggaa 27540
cagttcaaag tgctcatctt attataagat ttgacgaaaa tggagtgcta ctaaacaatt 27600
ccttcctgga cccagaatat tggaacttta gaaatggaga tcttactgaa ggcacagcct 27660
atacaaacgc tgttggattt atgcctaacc tatcagctta tccaaaatct cacggtaaaa 27720
ctgccaaaag taacattgtc agtcaagttt acttaaacgg agacaaaact aaacctgtaa 27780
cactaaccat tacactaaac ggtacacagg aaacaggaga cacaactcca agtgcatact 27840
ctatgtcatt ttcatgggac tggtctggcc acaactacat taatgaaata tttgccacat 27900
cctcttacac tttttcatac attgcccaag aataaagaat cgtttgtgtt atgtttcaac 27960
gtgtttattt ttcaattgca gaaaatttca agtcattttt cattcagtag tatagcccca 28020
ccaccacata gcttatacag atcaccgtac cttaatcaaa ctcacagaac cctagtattc 28080

aacctgccac ctccctccca acacacagag tacacagtcc tttctccccg gctggcctta 28140

aaaagcatca tatcatgggt aacagacata ttcttaggtg ttatattcca cacggtttcc 28200

tgtcgagcca aacgctcatc agtgatatta ataaactccc cgggcagctc acttaagttc 28260

atgtcgctgt ccagctgctg agccacaggc tgctgtccaa cttgcggttg cttaacgggc 28320

ggcgaaggag aagtccacgc ctacatgggg gtagagtcat aatcgtgcat caggataggg 28380

cggtggtgct gcagcagcgc gcgaataaac tgctgccgcc gccgctccgt cctgcaggaa 28440

tacaacatgg cagtggtctc ctcagcgatg attcgcaccg cccgcagcat aaggcgcctt 28500

gtcctccggg cacagcagcg caccctgatc tcacttaaat cagcacagta actgcagcac 28560

agcaccacaa tattgttcaa aatcccacag tgcaaggcgc tgtatccaaa gctcatggcg 28620

gggaccacag aacccacgtg gccatcatac cacaagcgca ggtagattaa gtggcgaccc 28680

ctcataaaca cgctggacat aaacattacc tcttttggca tgttgtaatt caccacctcc 28740

cggtaccata taaacctctg attaaacatg gcgccatcca ccaccatcct aaaccagctg 28800

gccaaaacct gcccgccggc tatacactgc agggaaccgg gactggaaca atgacagtgg 28860

agagcccagg actcgtaacc atggatcatc atgctcgtca tgatatcaat gttggcacaa 28920

cacaggcaca cgtgcataca cttcctcagg attacaagct cctcccgcgt tagaaccata 28980

tcccagggaa caacccattc ctgaatcagc gtaaatccca cactgcaggg aagacctcgc 29040

acgtaactca cgttgtgcat tgtcaaagtg ttacattcgg gcagcagcgg atgatcctcc 29100

agtatggtag cgcgggtttc tgtctcaaaa ggaggtagac gatccctact gtacggagtg 29160

cgccgagaca accgagatcg tgttggtcgt agtgtcatgc caaatggaac gccggacgta 29220

gtcatatttc ctgaagcaaa accaggtgcg ggcgtgacaa acagatctgc gtctccggtc 29280

tcgccgctta gatcgctctg tgtagtagtt gtagtatatc cactctctca aagcatccag 29340

gcgccccctg gcttcgggtt ctatgtaaac tccttcatgc gccgctgccc tgataacatc 29400

caccaccgca gaataagcca cacccagcca acctacacat tcgttctgcg agtcacacac 29460

gggaggagcg ggaagagctg gaagaaccat gttttttttt ttattccaaa agattatcca 29520

aaacctcaaa atgaagatct attaagtgaa cgcgctcccc tccggtggcg tggtcaaact 29580

ctacagccaa agaacagata atggcatttg taagatgttg cacaatggct tccaaaaggc 29640

aaacggccct cacgtccaag tggacgtaaa ggctaaaccc ttcagggtga atctcctcta 29700

taaacattcc agcaccttca accatgccca aataattctc atctcgccac cttctcaata 29760

tatctctaag caaatcccga atattaagtc cggccattgt aaaaatctgc tccagagcgc 29820

cctccacctt cagcctcaag cagcgaatca tgattgcaaa aattcaggtt cctcacagac 29880

ctgtataaga ttcaaaagcg gaacattaac aaaaataccg cgatcccgta ggtcccttcg 29940

cagggccagc tgaacataat cgtgcaggtc tgcacggacc agcgcggcca cttccccgcc 30000

aggaaccatg acaaaagaac ccacactgat tatgacacgc atactcggag ctatgctaac 30060

cagcgtagcc ccgatgtaag cttgttgcat gggcggcgat ataaaatgca aggtgctgct 30120

caaaaaatca ggcaaagcct cgcgcaaaaa agaaagcaca tcgtagtcat gctcatgcag 30180

ataaaggcag gtaagctccg gaaccaccac agaaaaagac accatttttc tctcaaacat 30240

gtctgcgggt ttctgcataa acacaaaata aaataacaaa aaaacattta aacattagaa 30300

gcctgtctta caacaggaaa aacaaccctt ataagcataa gacggactac ggccatgccg 30360

gcgtgaccgt aaaaaaactg gtcaccgtga ttaaaaagca ccaccgacag ctcctcggtc 30420

atgtccggag tcataatgta agactcggta aacacatcag gttgattcac atcggtcagt 30480

gctaaaaagc gaccgaaata gcccggggga atacataccc gcaggcgtag agacaacatt 30540

acagccccca taggaggtat aacaaaatta ataggagaga aaaacacata aacacctgaa 30600

aaaccctcct gcctaggcaa aatagcaccc tcccgctcca gaacaacata cagcgcttcc 30660

acagcggcag ccataacagt cagccttacc agtaaaaaag aaaacctatt aaaaaaacac 30720

cactcgacac ggcaccagct caatcagtca cagtgtaaaa aagggccaag tgcagagcga 30780

gtatatatag gactaaaaaa tgacgtaacg gttaaagtcc acaaaaaaca cccagaaaac 30840

cgcacgcgaa cctacgccca gaaacgaaag ccaaaaaacc cacaacttcc tcaaatcgtc 30900

acttccgttt tcccacgtta cgtaacttcc cattttaaga aaactacaat tcccaacaca 30960

tacaagttac tccgccctaa aacttacatc g 30991

<210> 72
<211> 1995
<212> DNA
<213> Artificial Sequence

<220>
<223> 8th DNA fragment

<400> 72
ggttggcgcg ccctacgtca cccgccccgt tcccacgccc cgcgccacgt cacaaactcc 60

accccctcat tatcatattg gcttcaatcc aaaataaggt atattattga tgatgttaca 120

tcgttaatta agcagtgggc ttacatggcg atagctagac tgggcggttt tatggacagc 180

aagcgaaccg gaattgccag ctggggcgcc ctctggtaag gttgggaagc cctgcaaagt 240

aaactggatg gctttcttgc cgccaaggat ctgatggcgc aggggatcaa gctctgatca 300

agagacagga tgaggatcgt ttcgcatgat tgaacaagat ggattgcacg caggttctcc 360

ggccgcttgg gtggagaggc tattcggcta tgactgggca caacagacaa tcggctgctc 420

tgatgccgcc gtgttccggc tgtcagcgca ggggcgcccg gttctttttg tcaagaccga 480

cctgtccggt gccctgaatg aactgcaaga cgaggcagcg cggctatcgt ggctggccac 540

gacgggcgtt ccttgcgcag ctgtgctcga cgttgtcact gaagcgggaa gggactggct 600

gctattgggc gaagtgccgg ggcaggatct cctgtcatct caccttgctc ctgccgagaa 660

agtatccatc atggctgatg caatgcggcg gctgcatacg cttgatccgg ctacctgccc 720

attcgaccac caagcgaaac atcgcatcga gcgagcacgt actcggatgg aagccggtct 780

tgtcgatcag gatgatctgg acgaagagca tcaggggctc gcgccagccg aactgttcgc 840

caggctcaag gcgagcatgc ccgacggcga ggatctcgtc gtgacccatg gcgatgcctg 900

cttgccgaat atcatggtgg aaaatggccg cttttctgga ttcatcgact gtggccggct 960

gggtgtggcg gaccgctatc aggacatagc gttggctacc cgtgatattg ctgaagagct 1020

tggcggcgaa tgggctgacc gcttcctcgt gctttacggt atcgccgctc ccgattcgca 1080

gcgcatcgcc ttctatcgcc ttcttgacga gttcttctga cctcaggtta ctcatatata 1140

ctttagattg atttaaaact tcatttttaa tttaaaagga tctaggtgaa gatccttttt 1200

gataatctca tgaccaaaat cccttaacgt gagttttcgt tccactgagc gtcagacccc 1260

gtagaaaaga tcaaaggatc ttcttgagat cctttttttc tgcgcgtaat ctgctgcttg 1320

caaacaaaaa aaccaccgct accagcggtg gtttgtttgc cggatcaaga gctaccaact 1380

ctttttccga aggtaactgg cttcagcaga gcgcagatac caaatactgt ccttctagtg 1440

tagccgtagt taggccacca cttcaagaac tctgtagcac cgcctacata cctcgctctg 1500

ctaatcctgt taccagtggc tgctgccagt ggcgataagt cgtgtcttac cgggttggac 1560

tcaagacgat agttaccgga taaggcgcag cggtcgggct gaacgggggg ttcgtgcaca 1620

cagcccagct tggagcgaac gacctacacc gaactgagat acctacagcg tgagctatga 1680

gaaagcgcca cgcttcccga agggagaaag gcggacaggt atccggtaag cggcagggtc 1740

ggaacaggag agcgcacgag ggagcttcca gggggaaacg cctggtatct ttatagtcct 1800

gtcgggtttc gccacctctg acttgagcgt cgatttttgt gatgctcgtc aggggggcgg 1860

agcctatgga aaaacgccag caacgcggcc tttttacggt tcctggcctt ttgctggcct 1920

tttgctctta attaaatcga tttaagggtg ggaaagaata tataaggtgg gggtcttatg 1980

tagtttgagc tcggg 1995

<210> 73
<211> 26877
<212> DNA
<213> Artificial Sequence

<220>
<223> pGLAd

<400> 73

ttaattaagc agtgggctta catggcgata gctagactgg gcggttttat ggacagcaag 60
cgaaccggaa ttgccagctg gggcgccctc tggtaaggtt gggaagccct gcaaagtaaa 120
ctggatggct ttcttgccgc caaggatctg atggcgcagg ggatcaagct ctgatcaaga 180
gacaggatga ggatcgtttc gcatgattga acaagatgga ttgcacgcag gttctccggc 240
cgcttgggtg gagaggctat tcggctatga ctgggcacaa cagacaatcg gctgctctga 300
tgccgccgtg ttccggctgt cagcgcaggg gcgcccggtt ctttttgtca agaccgacct 360
gtccggtgcc ctgaatgaac tgcaagacga ggcagcgcgg ctatcgtggc tggccacgac 420
gggcgttcct tgcgcagctg tgctcgacgt tgtcactgaa gcgggaaggg actggctgct 480
attgggcgaa gtgccggggc aggatctcct gtcatctcac cttgctcctg ccgagaaagt 540
atccatcatg gctgatgcaa tgcggcggct gcatacgctt gatccggcta cctgcccatt 600
cgaccaccaa gcgaaacatc gcatcgagcg agcacgtact cggatggaag ccggtcttgt 660
cgatcaggat gatctggacg aagagcatca ggggctcgcg ccagccgaac tgttcgccag 720
gctcaaggcg agcatgcccg acggcgagga tctcgtcgtg acccatggcg atgcctgctt 780
gccgaatatc atggtggaaa atggccgctt ttctggattc atcgactgtg gccggctggg 840
tgtggcggac cgctatcagg acatagcgtt ggctacccgt gatattgctg aagagcttgg 900
cggcgaatgg gctgaccgct tcctcgtgct ttacggtatc gccgctcccg attcgcagcg 960
catcgccttc tatcgccttc ttgacgagtt cttctgacct caggttactc atatatactt 1020
tagattgatt taaaacttca tttttaattt aaaaggatct aggtgaagat cctttttgat 1080
aatctcatga ccaaaatccc ttaacgtgag ttttcgttcc actgagcgtc agaccccgta 1140
gaaaagatca aaggatcttc ttgagatcct ttttttctgc gcgtaatctg ctgcttgcaa 1200
acaaaaaaac caccgctacc agcggtggtt tgtttgccgg atcaagagct accaactctt 1260
tttccgaagg taactggctt cagcagagcg cagataccaa atactgtcct tctagtgtag 1320
ccgtagttag gccaccactt caagaactct gtagcaccgc ctacatacct cgctctgcta 1380
atcctgttac cagtggctgc tgccagtggc gataagtcgt gtcttaccgg gttggactca 1440
agacgatagt taccggataa ggcgcagcgg tcgggctgaa cggggggttc gtgcacacag 1500
cccagcttgg agcgaacgac ctacaccgaa ctgagatacc tacagcgtga gctatgagaa 1560
agcgccacgc ttcccgaagg gagaaaggcg gacaggtatc cggtaagcgg cagggtcgga 1620
acaggagagc gcacgaggga gcttccaggg ggaaacgcct ggtatcttta tagtcctgtc 1680
gggtttcgcc acctctgact tgagcgtcga tttttgtgat gctcgtcagg ggggcggagc 1740
ctatggaaaa acgccagcaa cgcggccttt ttacggttcc tggccttttg ctggcctttt 1800
gctcttaatt aaatcgattt aagggtggga aagaatatat aaggtggggg tcttatgtag 1860
tttgagctcg ggcggcgacc tcgcgggttt tcgctattta tgaaaatttt ccggtttaag 1920

gcgtttccgt tcttcttcgt cataacttaa tgtttttatt taaaataccc tctgaaaaga 1980
aaggaaacga caggtgctga aagcgaggct ttttggcctc tgtcgtttcc tttctctgtt 2040
tttgtccgtg gaatgaacaa tggaagtcaa caaaaagcag ctggctgaca ttttcggtgc 2100
gagtatccgt accattcaga actggcagga acagggaatg cccgttctgc gaggcggtgg 2160
caagggtaat gaggtgcttt atgactctgc cgccgtcata aaatggtatg ccgaaaggga 2220
tgctgaaatt gagaacgaaa agctgcgccg ggaggttgaa gaactgcggc aggccagcga 2280
ggcagatctc cagccaggaa ctattgagta cgaacgccat cgacttacgc gtgcgcaggc 2340
cgacgcacag gaactgaaga atgccagaga ctccgctgaa gtggtggaaa ccgcattctg 2400
tactttcgtg ctgtcgcgga tcgcaggtga aattgccagt attctcgacg ggctccccct 2460
gtcggtgcag cggcgttttc cggaactgga aaaccgacat gttgatttcc tgaaacggga 2520
tatcatcaaa gccatgaaca aagcagccgc gctggatgaa ctgataccgg ggttgctgag 2580
tgaatatatc gaacagtcag gttaacaggc tgcggcattt tgtccgcgcc gggcttcgct 2640
cactgttcag gccggagcca cagaccgccg ttgaatgggc ggatgctaat tactatctcc 2700
cgaaagaatc cgcataccag gaagggcgct gggaaacact gccctttcag cgggccatca 2760
tgaatgcgat gggcagcgac tacatccgtg aggtgaatgt ggtgaagtct gcccgtgtcg 2820
gttattccaa aatgctgctg ggtgtttatg cctactttat agagcataag cagcgcaaca 2880
cccttatctg gttgccgacg gatggtgatg ccgagaactt tatgaaaacc cacgttgagc 2940
cgactattcg tgatattccg tcgctgctgg cgctggcccc gtggtatggc aaaaagcacc 3000
gggataacac gctcaccatg aagcgtttca ctaatgggcg tggcttctgg tgcctgggcg 3060
gtaaagcggc aaaaaactac cgtgaaaagt cggtggatgt ggcgggttat gatgaacttg 3120
ctgcttttga tgatgatatt gaacaggaag gctctccgac gttcctgggt gacaagcgta 3180
ttgaaggctc ggtctggcca aagtccatcc gtggctccac gccaaaagtg agaggcacct 3240
gtcagattga gcgtgcagcc agtgaatccc cgcattttat gcgttttcat gttgcctgcc 3300
cgcattgcgg ggaggagcag tatcttaaat ttggcgacaa agagacgccg tttggcctca 3360
aatggacgcc ggatgacccc tccagcgtgt tttatctctg cgagcataat gcctgcgtca 3420
tccgccagca ggagctggac tttactgatg cccgttatat ctgcgaaaag accgggatct 3480
ggacccgtga tggcattctc tggttttcgt catccggtga agagattgag ccacctgaca 3540
gtgtgacctt tcacatctgg acagcgtaca gcccgttcac cacctgggtg cagattgtca 3600
aagactggat gaaaacgaaa ggggatacgg gaaaacgtaa aaccttcgta aacaccacgc 3660
tcggtgagac gtgggaggcg aaaattggcg aacgtccgga tgctgaagtg atggcagagc 3720
ggaaagagca ttattcagcg cccgttcctg accgtgtggc ttacctgacc gccggtatcg 3780

actcccagct ggaccgctac gaaatgcgcg tatggggatg ggggccgggt gaggaaagct 3840
ggctgattga ccggcagatt attatgggcc gccacgacga tgaacagacg ctgctgcgtg 3900
tggatgaggc catcaataaa acctataccc gccggaatgg tgcagaaatg tcgatatccc 3960
gtatctgctg ggatactggc gggattgacc cgaccattgt gtatgaacgc tcgaaaaaac 4020
atgggctgtt ccgggtgatc cccattaaag ggcatccgt ctacggaaag ccggtggcca 4080
gcatgccacg taagcgaaac aaaaacgggg tttaccttac cgaaatcggt acggataccg 4140
cgaaagagca gatttataac cgcttcacac tgacgccgga aggggatgaa ccgcttcccg 4200
gtgccgttca cttcccgaat aacccggata tttttgatct gaccgaagcg cagcagctga 4260
ctgctgaaga gcaggtcgaa aaatgggtgg atggcaggaa aaaaatactg tgggacagca 4320
aaaagcgacg caatgaggca ctcgactgct tcgtttatgc gctggcggcg ctgcgcatca 4380
gtatttcccg ctggcagctg gatctcagtg cgctgctggc gagcctgcag gaagaggatg 4440
gtgcagcaac caacaagaaa acactggcag attacgcccg tgccttatcc ggagaggatg 4500
aatgacgcga caggaagaac ttgccgctgc ccgtgcggca ctgcatgacc tgatgacagg 4560
taaacgggtg gcaacagtac agaaagacgg acgaagggtg gagtttacgg ccacttccgt 4620
gtctgacctg aaaaaatata ttgcagagct ggaagtgcag accggcatga cacagcgacg 4680
caggggacct gcaggatttt atgtatgaaa acgcccacca ttcccaccct tctggggccg 4740
gacggcatga catcgctgcg cgaatatgcc ggttatcacg gcggtggcag cggatttgga 4800
gggcagttgc ggtcgtggaa cccaccgagt gaaagtgtgg atgcagccct gttgcccaac 4860
tttacccgtg gcaatgcccg cgcagacgat ctggtacgca ataacggcta tgccgccaac 4920
gccatccagc tgcatcagga tcatatcgtc gggtcttttt tccggctcag tcatcgccca 4980
agctggcgct atctgggcat cggggaggaa gaagcccgtg ccttttcccg cgaggttgaa 5040
gcggcatgga aagagtttgc cgaggatgac tgctgctgca ttgacgttga gcgaaaacgc 5100
acgtttacca tgatgattcg ggaaggtgtg gccatgcacg cctttaacgg tgaactgttc 5160
gttcaggcca cctgggatac cagttcgtcg cggcttttcc ggacacagtt ccggatggtc 5220
agcccgaagc gcatcagcaa cccgaacaat accggcgaca gccggaactg ccgtgccggt 5280
gtgcagatta atgacagcgg tgcggcgctg ggatattacg tcagcgagga cgggtatcct 5340
ggctggatgc cgcagaaatg gacatggata ccccgtgagt tacccggcgg gcgcgcctcg 5400
ttcattcacg tttttgaacc cgtggaggac gggcagactc gcggtgcaaa tgtgttttac 5460
agcgtgatgg agcagatgaa gatgctcgac acgctgcaga acacgcagct gcagagcgcc 5520
attgtgaagg cgatgtatgc cgccaccatt gagagtgagc tggatacgca gtcagcgatg 5580
gattttattc tgggcgcgaa cagtcaggag cagcgggaaa ggctgaccgg ctggattggt 5640
gaaattgccg cgtattacgc cgcagcgccg gtccggctgg gaggcgcaaa agtaccgcac 5700

ctgatgccgg gtgactcact gaacctgcag acggctcagg atacggataa cggctactcc 5760
gtgtttgagc agtcactgct gcggtatatc gctgccgggc tgggtgtctc gtatgagcag 5820
ctttcccgga attacgccca gatgagctac tccacggcac gggccagtgc gaacgagtcg 5880
tgggcgtact ttatggggcg gcgaaaattc gtcgcatccc gtcaggcgag ccagatgttt 5940
ctgtgctggc tggaagaggc catcgttcgc cgcgtggtga cgttaccttc aaaagcgcgc 6000
ttcagttttc aggaagcccg cagtgcctgg gggaactgcg actggatagg ctccggtcgt 6060
atggccatcg cgatggtctg aaagaagttc aggaagcggt gatgctgata gaagccggac 6120
tgagtaccta cgagaaagag tgcgcaaaac gcggtgacga ctatcaggaa atttttgccc 6180
agcaggtccg tgaaacgatg gagcgccgtg cagccggtct taaaccgccc gcctgggcgg 6240
ctgcagcatt tgaatccggg ctgcgacaat caacagagga ggagaagagt gacagcagag 6300
ctgcgtaatc tcccgcatat tgccagcatg gcctttaatg agccgctgat gcttgaaccc 6360
gcctatgcgc gggttttctt ttgtgcgctt gcaggccagc ttgggatcag cagcctgacg 6420
gatgcggtgt ccggcgacag cctgactgcc caggaggcac tcgcgacgct ggcattatcc 6480
ggtgatgatg acggaccacg acaggcccgc agttatcagg tcatgaacgg catcgccgtg 6540
ctgccggtgt ccggcacgct ggtcagccgg acgcgggcgc tgcagccgta ctcggggatg 6600
accggttaca acggcattat cgcccgtctg caacaggctg ccagcgatcc gatggtggac 6660
ggcattctgc tcgatatgga cacgcccggc gggatggtgg cgggggcatt tgactgcgct 6720
gacatcatcg cccgtgtgcg tgacataaaa ccggtatggg cgcttgccaa cgacatgaac 6780
tgcagtgcag gtcagttgct tgccagtgcc gcctcccggc gtctggtcac gcagaccgcc 6840
cggacaggct ccatcggcgt catgatggct cacagtaatt acggtgctgc gctggagaaa 6900
cagggtgtgg aaatcacgct gatttacagc ggcagccata aggtggatgg caacccctac 6960
agccatcttc cggatgacgt ccgggagaca ctgcagtccc ggatggacgc aacccgccag 7020
atgtttgcgc agaaggtgtc ggcatatacc ggcctgtccg tgcaggttgt gctggatacc 7080
gaggctgcag tgtacagcgg tcaggaggcc attgatgccg gactggctga tgaacttgtt 7140
aacagcaccg atgcgatcac cgtcatgcgt gatgcactgg atgcacgtaa atcccgtctc 7200
tcaggagggc gaatgaccaa agagactcaa tcaacaactg tttcagccac tgcttcgcag 7260
gctgacgtta ctgacgtggt gccagcgacg gagggcgaga acgccagcgc ggcgcagccg 7320
gacgtgaacg cgcagatcac cgcagcggtt gcggcagaaa acagccgcat tatggggatc 7380
ctcaactgtg aggaggctca cggacgcgaa gaacaggcac gcgtgctggc agaaaccccc 7440
ggtatgaccg tgaaaacggc ccgccgcatt ctggccgcag caccacagag tgcacaggcg 7500
cgcagtgaca ctgcgctgga tcgtctgatg cagggggcac cggcaccgct ggctgcaggt 7560

aacccggcat ctgatgccgt taacgatttg ctgaacacac cagtgtaagg gatgtttatg 7620

acgagcaaag aaacctttac ccattaccag ccgcagggca acagtgaccc ggctcatacc 7680

gcaaccgcgc ccggcggatt gagtgcgaaa gcgcctgcaa tgaccccgct gatgctggac 7740

acctccagcc gtaagctggt tgcgtgggat ggcaccaccg acggtgctgc cgttggcatt 7800

cttgcggttg ctgctgacca gaccagcacc acgctgacgt tctacaagtc cggcacgttc 7860

cgttatgagg atgtgctctg gccggaggct gccagcgacg agacgaaaaa acggaccgcg 7920

tttgccggaa cggcaatcag catcgtttaa ctttaccctt catcactaaa ggccgcctgt 7980

gcggcttttt ttacgggatt tttttatgtc gatgtacaca accgcccaac tgctggcggc 8040

aaatgagcag aaatttaagt ttgatccgct gtttctgcgt ctctttttcc gtgagagcta 8100

tcccttcacc acggagaaag tctatctctc acaaattccg ggactggtaa acatggcgct 8160

gtacgtttcg ccgattgttt ccggtgaggt tatccgttcc cgtggcggct ccacctctga 8220

atttacgccg ggatatgtca agccgaagca tgaagtgaat ccgcagatga ccctgcgtcg 8280

cctgccggat gaagatccgc agaatctggc ggacccggct taccgccgcc gtcgcatcat 8340

catgcagaac atgcgtgacg aagagctggc cattgctcag gtcgaagaga tgcaggcagt 8400

ttctgccgtg cttaagggca aatacaccat gaccggtgaa gccttcgatc cggttgaggt 8460

ggatatgggc cgcagtgagg agaataacat cacgcagtcc ggcggcacgg agtggagcaa 8520

gcgtgacaag tccacgtatg acccgaccga cgatatcgaa gcctacgcgc tgaacgccag 8580

cggtgtggtg aatatcatcg tgttcgatcc gaaaggctgg gcgctgttcc gttccttcaa 8640

agccgtcaag gagaagctgg atacccgtcg tggctctaat tccgagctgg agacagcggt 8700

gaaagacctg ggcaaagcgg tgtcctataa ggggatgtat ggcgatgtgg ccatcgtcgt 8760

gtattccgga cagtacgtgg aaaacggcgt caaaaagaac ttcctgccgg acaacacgat 8820

ggtgctgggg aacactcagg cacgcggtct gcgcacctat ggctgcattc aggatgcgga 8880

cgcacagcgc gaaggcatta acgcctctgc ccgttacccg aaaaactggg tgaccaccgg 8940

cgatccggcg cgtgagttca ccatgattca gtcagcaccg ctgatgctgc tggctgaccc 9000

tgatgagttc gtgtccgtac aactggcgta atcatggccc ttcggggcca ttgtttctct 9060

gtggaggagt ccatgacgaa agatgaactg attgcccgtc tccgctcgct gggtgaacaa 9120

ctgaaccgtg atgtcagcct gacggggacg aaagaagaac tggcgctccg tgtggcagag 9180

ctgaaagagg agcttgatga cacggatgaa actgccggtc aggacacccc tctcagccgg 9240

gaaaatgtgc tgaccggaca tgaaaatgag gtgggatcag cgcagccgga taccgtgatt 9300

ctggatacgt ctgaactggt cacggtcgtg gcactggtga agctgcatac tgatgcactt 9360

cacgccacgc gggatgaacc tgtggcattt gtgctgccgg gaacggcgtt tcgtgtctct 9420

gccggtgtgg cagccgaaat gacagagcgc ggcctggcca gaatgcaata acgggaggcg 9480

ctgtggctga tttcgataac ctgttcgatg ctgccattgc ccgcgccgat gaaacgatac 9540
gcgggtacat gggaacgtca gccaccatta catccggtga gcagtcaggt gcggtgatac 9600
gtggtgtttt tgatgaccct gaaaatatca gctatgccgg acagggcgtg cgcgttgaag 9660
gctccagccc gtccctgttt gtccggactg atgaggtgcg gcagctgcgg cgtggagaca 9720
cgctgaccat cggtgaggaa aatttctggg tagatcgggt ttcgccggat gatggcggaa 9780
gttgtcatct ctggcttgga cggggcgtac cgcctgccgt taaccgtcgc cgctgaaagg 9840
gggatgtatg gccataaaag gtcttgagca ggccgttgaa aacctcagcc gtatcagcaa 9900
aacggcggtg cctggtgccg ccgcaatggc cattaaccgc gttgcttcat ccgcgatatc 9960
gcagtcggcg tcacaggttg cccgtgagac aaaggtacgc cggaaactgg taaaggaaag 10020
ggccaggctg aaaagggcca cggtcaaaaa tccgcaggcc agaatcaaag ttaaccgggg 10080
ggatttgccc gtaatcaagc tgggtaatgc gcgggttgtc ctttcgcgcc gcaggcgtcg 10140
taaaaagggg cagcgttcat ccctgaaagg tggcggcagc gtgcttgtgg tgggtaaccg 10200
tcgtattccc ggcgcgttta ttcagcaact gaaaaatggc cggtggcatg tcatgcagcg 10260
tgtggctggg aaaaaccgtt accccattga tgtggtgaaa atcccgatgg cggtgccgct 10320
gaccacggcg tttaaacaaa atattgagcg gatacggcgt gaacgtcttc cgaaagagct 10380
gggctatgcg ctgcagcatc aactgaggat ggtaataaag cgatgaaaca tactgaactc 10440
cgtgcagccg tactggatgc actggagaag catgacaccg ggcgacgtt ttttgatggt 10500
cgccccgctg tttttgatga ggcggatttt ccggcagttg ccgtttatct caccggcgct 10560
gaatacacgg gcgaagagct ggacagcgat acctggcagg cggagctgca tatcgaagtt 10620
ttcctgcctg ctcaggtgcc ggattcagag ctggatgcgt ggatggagtc ccggatttat 10680
ccggtgatga gcgatatccc ggcactgtca gatttgatca ccagtatggt ggccagcggc 10740
tatgactacc ggcgcgacga tgatgcgggc ttgtggagtt cagccgatct gacttatgtc 10800
attacctatg aaatgtgagg acgctatgcc tgtaccaaat cctacaatgc cggtgaaagg 10860
tgccgggacc accctgtggg tttataaggg gagcggtgac ccttacgcga atccgctttc 10920
agacgttgac tggtcgcgtc tggcaaaagt taaagacctg acgcccggcg aactgaccgc 10980
tgagtcctat gacgacagct atctcgatga tgaagatgca gactggactg cgaccgggca 11040
ggggcagaaa tctgccggag ataccagctt cacgctggcg tggatgcccg gagagcaggg 11100
gcagcaggcg ctgctggcgt ggtttaatga aggcgatacc cgtgcctata aaatccgctt 11160
cccgaacggc acggtcgatg tgttccgtgg ctgggtcagc agtatcggta aggcggtgac 11220
ggcgaaggaa gtgatcaccc gcacggtgaa agtcaccaat gtgggacgtc cgtcgatggc 11280
agaagatcgc agcacggtaa cagcggcaac cggcatgacc gtgacgcctg ccagcacctc 11340

ggtggtgaaa gggcagagca ccacgctgac cgtggccttc cagccggagg gcgtaaccga 11400
caagagcttt cgtgcggtgt ctgcggataa aacaaaagcc accgtgtcgg tcagtggtat 11460
gaccatcacc gtgaacggcg ttgctgcagg caaggtcaac attccggttg tatccggtaa 11520
tggtgagttt gctgcggttg cagaaattac cgtcaccgcc agttaatccg gagagtcagc 11580
gatgttcctg aaaaccgaat catttgaaca taacggtgtg accgtcacgc tttctgaact 11640
gtcagccctg cagcgcattg agcatctcgc cctgatgaaa cggcaggcag aacaggcgga 11700
gtcagacagc aaccggaagt ttactgtgga agacgccatc agaaccggcg cgtttctggt 11760
ggcgatgtcc ctgtggcata accatccgca gaagacgcag atgccgtcca tgaatgaagc 11820
cgttaaacag attgagcagg aagtgcttac cacctggccc acggaggcaa tttctcatgc 11880
tgaaaacgtg gtgtaccggc tgtctggtat gtatgagttt gtggtgaata atgcccctga 11940
acagacagag gacgccgggc ccaaataaac ttataaattg tgagagaaat taatgaatgt 12000
ctaagttaat gcagaaacgg agagacatac tatattcatg aactaaaaga cttaatattg 12060
tgaaggtata ctttctttcc acataaattt gtagtcaata tgttcacccc aaaaaagctg 12120
tttgttaact tgccaacctc attctaaaat gtatatagaa gcccaaaaga caataacaaa 12180
aatattcttg tagaacaaaa tgggaaagaa tgttccacta aatatcaaga tttagagcaa 12240
agcatgagat gtgtggggat agacagtgag gctgataaaa tagagtagag ctcagaaaca 12300
gacccattga tatatgtaag tgacctatga aaaaaatatg gcattttaca atgggaaaat 12360
gatgatcttt ttcttttta gaaaaacagg gaaatatatt tatatgtaaa aaataaaagg 12420
gaacccatat gtcataccat acacacaaaa aaattccagt gaattataag tctaaatgga 12480
gaaggcaaaa ctttaaatct tttagaaaat aatatagaag catgccatca tgacttcagt 12540
gtagagaaaa atttcttatg actcaaagtc ctaaccacaa agaaaagatt gttaattaga 12600
ttgcatgaat attaagactt atttttaaaa ttaaaaaacc attaagaaaa gtcaggccat 12660
agaatgacag aaaatatttg caacacccca gtaaagagaa ttgtaatatg cagattataa 12720
aaagaagtct tacaaatcag taaaaaataa aactagacaa aaatttgaac agatgaaaga 12780
gaaactctaa ataatcatta cacatgagaa actcaatctc agaaatcaga gaactatcat 12840
tgcatataca ctaaattaga gaaatattaa aaggctaagt aacatctgtg gcaatattga 12900
tggtatataa ccttgatatg atgtgatgag aacagtactt taccccatgg gcttcctccc 12960
caaaccctta ccccagtata aatcatgaca aatatacttt aaaaaccatt accctatatc 13020
taaccagtac tcctcaaaac tgtcaaggtc atcaaaaata agaaaagtct gaggaactgt 13080
caaaactaag aggaacccaa ggagacatga gaattatatg taatgtggca ttctgaatga 13140
gatcccagaa cagaaaaaga acagtagcta aaaaactaat gaaatataaa taaagtttga 13200
actttagttt tttttaaaaa agagtagcat taacacggca aagccatttt catatttttc 13260

ttgaacatta agtacaagtc tataattaaa aattttttaa atgtagtctg gaacattgcc 13320

agaaacagaa gtacaacagc tatctgtgct gtcgcctaac tatccatagc tgattggtct 13380

aaaatgagat acatcaacgc tcctccatgt tttttgtttt ctttttaaat gaaaaacttt 13440

atttttaag aggagtttca ggttcatagc aaaattgaga ggaaggtaca ttcaagctga 13500

ggaagttttc ctctattcct agtttactga gagattgcat catgaatggg tgttaaattt 13560

tgtcaaatgc tttttctgtg tctatcaata tgaccatgtg attttcttct ttaacctgtt 13620

gatgggacaa attacgttaa ttgattttca aacgttgaac caccccttaca tatctggaat 13680

aaattctact tggttgtggt gtatattttt tgatacattc ttggattctt tttgctaata 13740

ttttgttgaa aatgtttgta tctttgttca tgagagatat tggtctgttg ttttcttttc 13800

ttgtaatgtc attttctagt tccggtatta aggtaatgct ggcctagttg aatgatttag 13860

gaagtattcc ctctgcttct gtcttctgaa agagattgta gaaagttgat acaatttttt 13920

tttctttaaa tatatgcatc cgctggattt cagtctgctg aaccggcgcg aggctgacga 13980

agagcctgaa gatgatgtgc tgatgcagaa agcggcaggg cttgccggag gtgtccgctt 14040

tggcccggac gggaatgaag ttatccccgc ttccccggat gtggcggaca tgacggagga 14100

tgacgtaatg ctgatgacag tatcagaagg gatcgcagga ggagtccggt atggctgaac 14160

cggtaggcga tctggtcgtt gatttgagtc tggatgcggc cagatttgac gagcagatgg 14220

ccagagtcag gcgtcatttt tctggtacgg aaagtgatgc gaaaaaaaca gcggcagtcg 14280

ttgaacagtc gctgagccga caggcgctgg ctgcacagaa agcggggatt tccgtcgggc 14340

agtataaagc cgccatgcgt atgctgcctg cacagttcac cgacgtggcc acgcagcttg 14400

caggcgggca aagtccgtgg ctgatcctgc tgcaacaggg ggggcaggtg aaggactcct 14460

tcggcgggat gatccccatg ttcaggggggc ttgccggtgc gatcaccctg ccgatggtgg 14520

gggccacctc gctggcggtg gcgaccggtg cgctggcgta tgcctggtat cagggcaact 14580

caaccctgtc cgatttcaac aaaacgctgg tcctttccgg caatcaggcg ggactgacgg 14640

cagatcgtat gctggtcctg tccagagccg ggcaggcggc agggctgacg tttaaccaga 14700

ccagcgagtc actcagcgca ctggttaagg cgggggtaag cggtgaggct cagattgcgt 14760

ccatcagcca gagtgtggcg cgtttctcct ctgcatccgg cgtggaggtg gacaaggtcg 14820

ctgaagcctt cgggaagctg accacagacc cgacgtcggg gctgacggcg atggctcgcc 14880

agttccataa cgtgtcggcg gagcagattg cgtatgttgc tcagttgcag cgttccggcg 14940

atgaagccgg ggcattgcag gcggcgaacg aggccgcaac gaaagggttt gatgaccaga 15000

cccgccgcct gaaagagaac atgggcacgc tggagacctg ggcagacagg actgcgcggg 15060

cattcaaatc catgtgggat gcggtgctgg atattggtcg tcctgatacc gcgcaggaga 15120

tgctgattaa ggcagaggct gcgtataaga aagcagacga catctggaat ctgcgcaagg 15180
atgattattt tgttaacgat gaagcgcggg cgcgttactg ggatgatcgt gaaaaggccc 15240
gtcttgcgct tgaagccgcc cgaaagaagg ctgagcagca gactcaacag gacaaaaatg 15300
cgcagcagca gagcgatacc gaagcgtcac ggctgaaata taccgaagag gcgcagaagg 15360
cttacgaacg gctgcagacg ccgctggaga aatataccgc ccgtcaggaa gaactgaaca 15420
aggcactgaa agacgggaaa atcctgcagg cggattacaa cacgctgatg gcggcggcga 15480
aaaaggatta tgaagcgacg ctgaaaaagc cgaaacagtc cagcgtgaag gtgtctgcgg 15540
gcgatcgtca ggaagacagt gctcatgctg ccctgctgac gcttcaggca gaactccgga 15600
cgctggagaa gcatgccgga gcaaatgaga aaatcagcca gcagcgccgg gatttgtgga 15660
aggcggagag tcagttcgcg gtactggagg aggcggcgca acgtcgccag ctgtctgcac 15720
aggagaaatc cctgctggcg cataaagatg agacgctgga gtacaaacgc cagctggctg 15780
cacttggcga caaggttacg tatcaggagc gcctgaacgc gctggcgcag caggcggata 15840
aattcgcaca gcagcaacgg gcaaaacggg ccgccattga tgcgaaaagc cgggggctga 15900
ctgaccggca ggcagaacgg gaagccacgg aacagcgcct gaaggaacag tatggcgata 15960
atccgctggc gctgaataac gtcatgtcag agcagaaaaa gacctgggcg gctgaagacc 16020
agcttcgcgg gaactggatg gcaggcctga agtccggctg gagtgagtgg gaagagagcg 16080
ccacggacag tatgtcgcag gtaaaaagtg cagccacgca gacctttgat ggtattgcac 16140
agaatatggc ggcgatgctg accggcagtg agcagaactg gcgcagcttc acccgttccg 16200
tgctgtccat gatgacagaa attctgctta agcaggcaat ggtggggatt gtcgggagta 16260
tcggcagcgc cattggcggg gctgttggtg gcggcgcatc cgcgtcaggc ggtacagcca 16320
ttcaggccgc tgcggcgaaa ttccattttg caaccggagg atttacggga accggcggca 16380
aatatgagcc agcggggatt gttcaccgtg gtgagtttgt cttcacgaag gaggcaacca 16440
gccggattgg cgtggggaat ctttaccggc tgatgcgcgg ctatgccacc ggcggttatg 16500
tcggtacacc gggcagcatg gcagacagcc ggtcgcaggc gtccgggacg tttgagcaga 16560
ataaccatgt ggtgattaac aacgacggca cgaacgggca gataggtccg gctgctctga 16620
aggcggtgta tgacatggcc cgcaagggtg cccgtgatga aattcagaca cagatgcgtg 16680
atggtggcct gttctccgga ggtggacgat gaagaccttc cgctggaaag tgaaacccgg 16740
tatggatgtg gcttcggtcc cttctgtaag aaaggtgcgc tttggtgatg gctattctca 16800
gcgagcgcct gccgggctga atgccaacct gaaaacgtac agcgtgacgc tttctgtccc 16860
ccgtgaggag gccacggtac tggagtcgtt tctggaagag cacgggggct ggaaatcctt 16920
tctgtggacg ccgccttatg agtggcggca gataaaggtg acctgcgcaa aatggtcgtc 16980
gcgggtcagt atgctgcgtg ttgagttcag cgcagagttt gaacaggtgg tgaactgatg 17040

caggatatcc ggcaggaaac actgaatgaa tgcacccgtg cggagcagtc ggccagcgtg 17100
gtgctctggg aaatcgacct gacagaggtc ggtggagaac gttatttttt ctgtaatgag 17160
cagaacgaaa aaggtgagcc ggtcacctgg caggggcgac agtatcagcc gtatcccatt 17220
caggggagcg gttttgaact gaatggcaaa ggcaccagta cgcgccccac gctgacggtt 17280
tctaacctgt acggtatggt caccgggatg gcggaagata tgcagagtct ggtcggcgga 17340
acggtggtcc ggcgtaaggt ttacgcccgt tttctggatg cggtgaactt cgtcaacgga 17400
aacagttacg ccgatccgga gcaggaggtg atcagccgct ggcgcattga gcagtgcagc 17460
gaactgagcg cggtgagtgc ctcctttgta ctgtccacgc cgacggaaac ggatggcgct 17520
gtttttccgg gacgtatcat gctggccaac acctgcacct ggacctatcg cggtgacgag 17580
tgcggttata gcggtccggc tgtcgcggat gaatatgacc agccaacgtc cgatatcacg 17640
aaggataaat gcagcaaatg cctgagcggt tgtaagttcc gcaataacgt cggcaacttt 17700
ggcggcttcc tttccattaa caaactttcg cagtaaatcc catgacacag acagaatcag 17760
cgattctggc gcacgcccgg cgatgtgcgc cagcggagtc gtgcggcttc gtggtaagca 17820
cgccggaggg ggaaagatat ttcccctgcg tgaatatctc cggtgagccg gaggctattt 17880
ccgtatgtcg ccggaagact ggctgcaggc agaaatgcag ggtgagattg tggcgctggt 17940
ccacagccac cccggtggtc tgccctggct gagtgaggcc gaccggcggc tgcaggtgca 18000
gagtgatttg ccgtggtggc tggtctgccg gggggacgatt cataagttcc gctgtgtgcc 18060
gcatctcacc gggcggcgct ttgagcacgg tgtgacggac tgttacacac tgttccggga 18120
tgcttatcat ctggcgggga ttgagatgcc ggactttcat cgtgaggatg actggtggcg 18180
taacggccag aatctctatc tggataatct ggaggcgacg gggctgtatc aggtgccgtt 18240
gtcagcggca cagccgggcg atgtgctgct gtgctgtttt ggttcatcag tgccgaatca 18300
cgccgcaatt tactgcggcg acggcgagct gctgcaccat attcctgaac aactgagcaa 18360
acgagagagg tacaccgaca aatggcagcg acgcacacac tccctctggc gtcaccgggc 18420
atggcgcgca tctgccttta cggggattta caacgatttg gtcgccgcat cgaccttcgt 18480
gtgaaaacgg gggctgaagc catccgggca ctggccacac agctcccggc gtttcgtcag 18540
aaactgagcg acggctggta tcaggtacgg attgccgggc gggacgtcag cacgtccggg 18600
ttaacggcgc agttacatga gactctgcct gatggcgctg taattcatat tgttcccaga 18660
gtcgccgggg ccaagtcagg tggcgtattc cagattgtcc tgggggctgc cgccattgcc 18720
ggatcattct ttaccgccgg agccaccctt gcagcatggg gggcagccat tggggccggt 18780
ggtatgaccg gcatcctgtt ttctctcggt gccagtatgg tgctcggtgg tgtggcgcag 18840
atgctggcac cgaaagccag aactccccgt atacagacaa cggataacgg taagcagaac 18900

acctatttct cctcactgga taacatggtt gcccagggca atgttctgcc tgttctgtac 18960
ggggaaatgc gcgtggggtc acgcgtggtt tctcaggaga tcagcacggc agacgaaggg 19020
gacggtggtc aggttgtggt gattggtcgc tgatgcaaaa tgttttatgt gaaaccgcct 19080
gcgggcggtt ttgtcattta tggagcgtga ggaatgggta aaggaagcag taaggggcat 19140
accccgcgcg aagcgaagga caacctgaag tccacgcagt tgctgagtgt gatcgcgatg 19200
ccatcagcga agggccgatt gaaggtccgg tggatggctt aaaaagcgtg ctgctgaaca 19260
gtacgccggt gctggacact gaggggaata ccaacatatc cggtgtcacg gtggtgttcc 19320
gggctggtga gcaggagcag actccgccgg agggatttga atcctccggc tccgagacgg 19380
tgctgggtac ggaagtgaaa tatgacacgc cgatcacccg caccattacg tctgcaaaca 19440
tcgaccgtct gcgctttacc ttcggtgtac aggcactggt ggaaaccacc tcaaagggtg 19500
acaggaatcc gtcggaagtc cgcctgctgg ttcagataca acgtaacggt ggctgggtga 19560
cggaaaaaga catcaccatt aagggcaaaa ccacctcgca gtatctggcc tcggtggtga 19620
tgggtaacct gccgccgcgc ccgtttaata tccggatgcg caggatgacg ccggacagca 19680
ccacagacca gctgcagaac aaaacgctct ggtcgtcata cactgaaatc atcgcgatgt 19740
gaaacagtgc tacccgaaca cggcactggt cggcgtgcag gtggactcgg agcagttcgg 19800
cagccagcag gtgagccgta attatcatct gcgcgggcgt attctgcagg tgccgtcgaa 19860
ctataacccg cagacgcggc aatacagcgg tatctgggac ggaacgttta aaccggcata 19920
cagcaacaac atggcctggt gtctgtggga tatgctgacc catccgcgct acggcatggg 19980
gaaacgtctt ggtgcggcgg atgtggataa atgggcgctg tatgtcatcg gccagtactg 20040
cgaccagtca gtgccggacg gctttggcgg cacggagccg cgcatcacct gtaatgcgta 20100
cctgaccaca cagcgtaagg cgtgggatgt gctcagcgat ttctgctcgg cgatgcgctg 20160
tatgccggta tggaacgggc agacgctgac gttcgtgcag gaccgaccgt cggataagac 20220
gtggacctat aaccgcagta atgtggtgat gccggatgat ggcgcgccgt tccgctacag 20280
cttcagcgcc ctgaaggacc gccataatgc cgttgaggtg aactggattg acccgaacaa 20340
cggctgggag acggcgacag agcttgttga agatacgcag gccattgccc gttacggtcg 20400
taatgttacg aagatggatg cctttggctg taccagccgg gggcaggcac accgcgccgg 20460
gctgtggctg attaaaacag aactgctgga aacgcagacc gtggatttca gcgtcggcgc 20520
agaagggctt cgccatgtac cgggcgatgt tattgaaatc tgcgatgatg actatgccgg 20580
tatcagcacc ggtggtcgtg tgctggcggt gaacagccag acccggacgc tgacgctcga 20640
ccgtgaaatc acgctgccat cctccggtac cgcgctgata agcctggttg acggaagtgg 20700
caatccggtc agcgtggagg ttcagtccgt caccgacggc gtgaaggtaa aagtgagccg 20760
tgttcctgac ggtgttgctg aatacagcgt atgggagctg aagctgccga cgctgcgcca 20820

gcgactgttc cgctgcgtga gtatccgtga gaacgacgac ggcacgtatg ccatcaccgc 20880
cgtgcagcat gtgccggaaa aagaggccat cgtggataac ggggcgcact ttgacggcga 20940
acagagtggc acggtgaatg gtgtcacgcc gccagcggtg cagcacctga ccgcagaagt 21000
cactgcagac agcggggaat atcaggtgct ggcgcgatgg gacacaccga aggtggtgaa 21060
gggcgtgagt ttcctgctcc gtctgaccgt aacagcggac gacggcagtg agcggctggt 21120
cagcacggcc cggacgacgg aaaccacata ccgcttcacg caactggcgc tggggaacta 21180
caggctgaca gtccgggcgg taaatgcgtg gggcagcag ggcgatccgg cgtcggtatc 21240
gttccggatt gccgcaccgg cagcaccgtc gaggattgag ctgacgccgg gctattttca 21300
gataaccgcc acgccgcatc ttgccgttta tgacccgacg gtacagtttg agttctggtt 21360
ctcggaaaag cagattgcgg atatcagaca ggttgaaacc agcacgcgtt atcttggtac 21420
ggcgctgtac tggatagccg ccagtatcaa tatcaaaccg ggccatgatt attactttta 21480
tatccgcagt gtgaacaccg ttggcaaatc ggcattcgtg gaggccgtcg gtcgggcgag 21540
cgatgatgcg gaaggttacc tggatttttt caaaggcaag ataaccgaat cccatctcgg 21600
caaggagctg ctggaaaaag tcgagctgac ggaggataac gccagcagac tggaggagtt 21660
ttcgaaagag tggaaggatg ccagtgataa gtggaatgcc atgtgggctg tcaaaattga 21720
gcagaccaaa gacggcaaac attatgtcgc gggtattggc ctcagcatgg aggacacgga 21780
ggaaggcaaa ctgagccagt ttctggttgc cgccaatcgt atcgcattta ttgacccggc 21840
aaacgggaat gaaacgccga tgtttgtggc gcagggcaac cagatattca tgaacgacgt 21900
gttcctgaag cgcctgacgg cccccaccat taccagcggc ggcaatcctc cggccttttc 21960
cctgacaccg gacggaaagc tgaccgctaa aaatgcggat atcagtggca gtgtgaatgc 22020
gaactccggg acgctcagta atgtgacgat agctgaaaac tgtacgataa acggtacgct 22080
gagggcggaa aaaatcgtcg ggacattgt aaaggcggcg agcgcggctt ttccgcgcca 22140
gcgtgaaagc agtgtggact ggccgtcagg tacccgtact gtcaccgtga ccgatgacca 22200
tccttttgat cgccagatag tggtgcttcc gctgacgttt cgcggaagta agcgtactgt 22260
cagcggcagg acaacgtatt cgatgtgtta tctgaaagta ctgatgaacg gtgcggtgat 22320
ttatgatggc gcggcgaacg aggcggtaca ggtgttctcc cgtattgttg acatgccagc 22380
gggtcgggga aacgtgatcc tgacgttcac gcttacgtcc acacggcatt cggcagatat 22440
tccgccgtat acgtttgcca gcgatgtgca ggttatggtg attaagaaac aggcgctggg 22500
catcagcgtg gtctgagtgt gttacagagg ttcgtccggg aacgggcgtt ttattataaa 22560
acagtgagag gtgaacgatg cgtaatgtgt gtattgccgt tgctgtcttt gccgcacttg 22620
cggtgacagt cactccggcc cgtgcggaag gtggacatgg tacgtttacg gtgggctatt 22680

ttcaagtgaa accgggtaca ttgccgtcgt tgtcgggcgg ggataccggt gtgagtcatc 22740

tgaaagggat taacgtgaag taccgttatg agctgacgga cagtgtgggg gtgatggctt 22800

ccctggggtt cgccgcgtcg aaaaagagca gcacagtgat gaccggggag gatacgtttc 22860

actatgagag cctgcgtgga cgttatgtga gcgtgatggc cggaccggtt ttacaaatca 22920

gtaagcaggt cagtgcgtac gccatggccg gagtggctca cagtcggtgg tccggcagta 22980

caatggatta ccgtaagacg gaaatcactc ccgggtatat gaaagagacg accactgcca 23040

gggacgaaag tgcaatgcgg catacctcag tggcgtggag tgcaggtata cagattaatc 23100

cggcagcgtc cgtcgttgtt gatattgctt atgaaggctc cggcagtggc gactggcgta 23160

ctgacggatt catcgttggg gtcggttata aattctgatt agccaggtaa cacagtgtta 23220

tgacagcccg ccggaaccgg tgggcttttt tgtggggtga atatggcagt aaagatttca 23280

ggagtcctga aagacggcac aggaaaaccg gtacagaact gcaccattca gctgaaagcc 23340

agacgtaaca gcaccacggt ggtggtgaac acggtgggct cagagaatcc ggatgaagcc 23400

gggcgttaca gcatggatgt ggagtacggt cagtacagtg tcatcctgca ggttgacggt 23460

tttccaccat cgcacgccgg gaccatcacc gtgtatgaag attcacaacc ggggacgctg 23520

aatgattttc tctgtgccat gacggaggat gatgcccggc cggaggtgct gcgtcgtctt 23580

gaactgatgg tggaagaggt ggcgcgtaac gcgtccgtgg tggcacagag tacggcagac 23640

gcgaagaaat cagccggcga tgccagtgca tcagctgctc aggtcgcggc ccttgtgact 23700

gatgcaactg actcagcacg cgccgccagc acgtccgccg gacaggctgc atcgtcagct 23760

caggaagcgt cctccggcgc agaagcggca tcagcaaagg ccactgaagc ggaaaaaagt 23820

gccgcagccg cagagtcctc aaaaaacgcg gcggccacca gtgccggtgc ggcgaaaacg 23880

tcagaaacga atgctgcagc gtcacaacaa tcagccgcca cgtctgcctc caccgcggcc 23940

acgaaagcgt cagaggccgc cacttcagca cgagatgcgg tggcctcaaa agaggcagca 24000

aaatcatcag aaacgaacgc atcatcaagt gccggtcgtg cagcttcctc ggcaacggcg 24060

gcagaaaatt ctgccagggc ggcaaaaacg tccgagacga atgccaggtc atctgaaaca 24120

gcagcggaac ggagcgcctc tgccgcggca gacgcaaaaa cagcggcggc ggggagtgcg 24180

tcaacggcat ccacgaaggc gacagaggct gcgggaagtg cggtatcagc atcgcagagc 24240

aaaagtgcgg cagaagcggc ggcaatacgt gcaaaaaatt cggcaaaacg tgcagaagat 24300

atagcttcag ctgtcgcgct tgaggatgcg gacacaacga gaaaggggat agtgcagctc 24360

agcagtgcaa ccaacagcac gtctgaaacg cttgctgcaa cgccaaaggc ggttaaggtg 24420

gtaatggatg aaacgaacag aaaagcccac tggacagtcc ggcactgacc ggaacgccaa 24480

cagcaccaac cgcgctcagg ggaacaaaca atacccagat tgcgaacacc gcttttgtac 24540

tggccgcgat tgcagatgtt atcgacgcgt cacctgacgc actgaatacg ctgaatgaac 24600

tggccgcagc gctcgggaat gatccagatt ttgctaccac catgactaac gcgcttgcgg 24660
gtaaacaacc gaagaatgcg acactgacgg cgctggcagg gctttccacg gcgaaaaata 24720
aattaccgta ttttgcggaa aatgatgccg ccagcctgac tgaactgact caggttggca 24780
gggatattct ggcaaaaaat tccgttgcag atgttcttga ataccttggg gccggtgaga 24840
attcggcctt tccggcaggt gcgccgatcc cgtggccatc agatatcgtt ccgtctggct 24900
acgtcctgat gcaggggcag gcgtttgaca aatcagccta cccaaaactt gctgtcgcgt 24960
atccatcggg tgtgcttcct gatatgcgag gctggacaat caaggggaaa cccgccagcg 25020
gtcgtgctgt attgtctcag gaacaggatg gaattaagtc gcacacccac agtgccagtg 25080
catccggtac ggatttgggg acgaaaacca catcgtcgtt tgattacggg acgaaaacaa 25140
caggcagttt cgattacggc accaaatcga cgaataacac gggggctcat gctcacagtc 25200
tgagcggttc aacaggggcc gcgggtgctc atgcccacac aagtggttta aggatgaaca 25260
gttctggctg gagtcagtat ggaacagcaa ccattacagg aagtttatcc acagttaaag 25320
gaaccagcac acagggtatt gcttatttat cgaaaacgga cagtcagggc agccacagtc 25380
actcattgtc cggtacagcc gtgagtgccg gtgcacatgc gcatacagtt ggtattggtg 25440
cgcaccagca tccggttgtt atcggtgctc atgcccattc tttcagtatt ggttcacacg 25500
gacacaccat caccgttaac gctgcgggta acgcggaaaa caccgtcaaa aacattgcat 25560
ttaactatat tgtgaggctt gcataatggc attcagaatg agtgaacaac cacggaccat 25620
aaaaatttat aatctgctgg ccggaactaa tgaatttatt ggtgaaggtg acgcatatat 25680
tccgcctcat accggtctgc ctgcaaacag taccgatatt gcaccgccag atattccggc 25740
tggctttgtg gctgttttca acagtgatga ggcatcgtgg catctcgttg aagaccatcg 25800
gggtaaaacc gtctatgacg tggcttccgg cgacgcgtta tttatttctg aactcggtcc 25860
gttaccggaa aattttacct ggttatcgcc gggaggggaa tatcagaagt ggaacggcac 25920
agcctgggtg aaggatacgg aagcagaaaa actgttccgg atccgggagg cggaagaaac 25980
aaaaaaaagc ctgatgcagg tagccagtga gcatattgcg ccgcttcagg atgctgcaga 26040
tctggaaatt gcaacgaagg aagaaacctc gttgctggaa gcctggaaga agtatcgggt 26100
gttgctgaac cgtgttgata catcaactgc acctgatatt gagtggcctg ctgtccctgt 26160
tatggagtaa tcgttttgtg atatgccgca gaaacgttgt atgaaataac gttctgcggt 26220
tagttagtat attgtaaagc tgagtattgg tttatttggc gattattatc ttcaggagaa 26280
taatggaagt tctatgactc aattgttcat agtgtttaca tcaccgccaa ttgcttttaa 26340
gactgaacgc atgaaatatg gtttttcgtc atgttttgag tctgctgttg atatttctaa 26400
agtcggtttt ttttcttcgt tttctctaac tattttccat gaaatacatt tttgattatt 26460

atttgaatca attccaatta cctgaagtct ttcatctata attggcattg tatgtattgg 26520

tttattggag tagatgcttg cttttctgag ccatagctct gatatccaaa tgaagccata 26580

ggcatttgtt attttggctc tgtcagctgc ataacgccaa aaaatatatt tatctgcttg 26640

atcttcaaat gttgtattga ttaaatcaat tggatggaat tgtttatcat aaaaaattaa 26700

tgtttgaatg tgataaccgt cctttaaaaa agtcgtttct gcaagctcct aggtttttgg 26760

cgcgccctac gtcacccgcc ccgttcccac gccccgcgcc acgtcacaaa ctccaccccc 26820

tcattatcat attggcttca atccaaaata aggtatatta ttgatgatgt tacatcg 26877

<210> 74
<211> 26597
<212> DNA
<213> Artificial Sequence

<220>
<223> pGLAd3

<400> 74
ttaattaagc agtgggctta catggcgata gctagactgg gcggttttat ggacagcaag 60

cgaaccggaa ttgccagctg gggcgccctc tggtaaggtt gggaagccct gcaaagtaaa 120

ctggatggct ttcttgccgc caaggatctg atggcgcagg ggatcaagct ctgatcaaga 180

gacaggatga ggatcgtttc gcatgattga acaagatgga ttgcacgcag gttctccggc 240

cgcttgggtg gagaggctat tcggctatga ctgggcacaa cagacaatcg gctgctctga 300

tgccgccgtg ttccggctgt cagcgcaggg gcgcccggtt ctttttgtca agaccgacct 360

gtccggtgcc ctgaatgaac tgcaagacga ggcagcgcgg ctatcgtggc tggccacgac 420

gggcgttcct tgcgcagctg tgctcgacgt tgtcactgaa gcgggaaggg actggctgct 480

attgggcgaa gtgccggggc aggatctcct gtcatctcac cttgctcctg ccgagaaagt 540

atccatcatg gctgatgcaa tgcggcggct gcatacgctt gatccggcta cctgcccatt 600

cgaccaccaa gcgaaacatc gcatcgagcg agcacgtact cggatggaag ccggtcttgt 660

cgatcaggat gatctggacg aagagcatca ggggctcgcg ccagccgaac tgttcgccag 720

gctcaaggcg agcatgcccg acggcgagga tctcgtcgtg acccatggcg atgcctgctt 780

gccgaatatc atggtggaaa atggccgctt ttctggattc atcgactgtg gccggctggg 840

tgtggcggac cgctatcagg acatagcgtt ggctacccgt gatattgctg aagagcttgg 900

cggcgaatgg gctgaccgct tcctcgtgct ttacggtatc gccgctcccg attcgcagcg 960

catcgccttc tatcgccttc ttgacgagtt cttctgacct caggttactc atatatactt 1020

tagattgatt taaaacttca tttttaattt aaaaggatct aggtgaagat cctttttgat 1080

aatctcatga ccaaaatccc ttaacgtgag ttttcgttcc actgagcgtc agaccccgta 1140

gaaaagatca aaggatcttc ttgagatcct ttttttctgc gcgtaatctg ctgcttgcaa 1200

acaaaaaaac caccgctacc agcggtggtt tgtttgccgg atcaagagct accaactctt 1260

tttccgaagg taactggctt cagcagagcg cagataccaa atactgtcct tctagtgtag 1320

ccgtagttag gccaccactt caagaactct gtagcaccgc ctacatacct cgctctgcta 1380

atcctgttac cagtggctgc tgccagtggc gataagtcgt gtcttaccgg gttggactca 1440

agacgatagt taccggataa ggcgcagcgg tcgggctgaa cggggggttc gtgcacacag 1500

cccagcttgg agcgaacgac ctacaccgaa ctgagatacc tacagcgtga gctatgagaa 1560

agcgccacgc ttcccgaagg gagaaaggcg gacaggtatc cggtaagcgg cagggtcgga 1620

acaggagagc gcacgaggga gcttccaggg ggaaacgcct ggtatcttta tagtcctgtc 1680

gggtttcgcc acctctgact tgagcgtcga tttttgtgat gctcgtcagg ggggcggagc 1740

ctatggaaaa acgccagcaa cgcggccttt ttacggttcc tggccttttg ctggcctttt 1800

gctcttaatt aaatcgattt aagggtggga aagaatatat aaggtggggg tcttatgtag 1860

tttgagctcg gctttggtta tttgatggat tgacctagtt ctcaggcagc cacctgtgtg 1920

gactaaatac acccatttct tctccattct gaaggatgtg ctgaaagccg ccttggacag 1980

tgagaaaagc aaacatggaa gcctgctgta ggatcacctt acaccatcag atacccactt 2040

cccacccccg gcttcctgaa ctttctgtaa ttactcatgc cagtgactag ggctcatgtg 2100

ggccatccca ggaaggagac tagtggctgc cattctgtcc tggtctttgt gtctcccgag 2160

gaggagtgta ggtggtgagg tcacaggcta agccaggcca tttaaggaaa gctgcctcag 2220

ctccagccca gccctctctg ccaggtggat atggaaatcc cggtttactc tctggggcct 2280

tttattggct gaaatgctca ccttggtatc tgttacacat tcccttccaa gagcagagct 2340

agataagcca ggtgtggaga caatcagctc ttgaaatctc ccagaagctg ccaaacccct 2400

aaaatcagca agagacaccc ctcatagtcc agactcagct gttttattct gcctctccct 2460

gatctctttt cagggcataa agttttccaa ggagaagaat gaaatcagta tagcaggtca 2520

ttgagaaagt atagaaaggc tcaggttgaa cttcagtgtc tgctaccaat gtgtcataaa 2580

taagtcattt tactactgtg agactcagtt tacccatttg ttgagtatga aaaaaaatca 2640

tatatatttg ttatgctata tgtatcaatg catcgagcta gactctgggg ctaaagcaat 2700

gagtaaaacg tatttggctg aacataaata tgggaggggg gtggaatgca tgaatgtaaa 2760

gaatgctggc ataagtagga ccagtgagtt gggtgtggca gtgcaccttg gagacagagc 2820

caggcggacc tctgagttca aggacagtca tcagggctcc acagagaaat cctgtccaaa 2880

aaaaaaaaaa aaaatcagtg gggaaaggca cctgccatcg aggctgaaga cctgaatttg 2940

gtccacagga tccacgtggt aggaaagaaa gaacttccaa gggttattct ctaacctcca 3000

tacttatatg tgagtgtaca cttaagtaat gtttgtttgt ttgttttgtt cctgttatgt 3060

tttgtttgtt tttaaagcca agtctggtgg ttcaggcatt taatcatagc acccagaagg 3120

cagagttcaa gggcagcctg gtctacatag gaaattgtag gcctatacag tgaaaccctg 3180

tttcaaagga agattaaagg aaaaaagtct tgcactcttc cacaccctga ccaaagtcct 3240

atagtgaaac tctggtcccc tccctcatca ctgctattgc caaactcagt ctccctctat 3300

gacccacatc atgatagcca gggctagacc gtttgtaaag ataaatcagc cttgtctgga 3360

taaataactc caggaaggag aaactgccgg ctgtaaagaa ccttagcagt cgtattcccc 3420

aaaaccacct atataagcct caagtgcccc tccacctttc acagacaccc ctcccctacg 3480

ggagagtatc tgtcctgcac tgtttgaaat aaacagtcct gtctgttgag gtcagtcttc 3540

agtctttttt tctgcttcca gtctcttaag ttgcttcaga aatttaccaa gaatcactaa 3600

ataaagccag ctgtgatggt ccacatctgt attccagtgc ttcgaaggct gaggcagagg 3660

gtctcaagtg tgaggctagc ctgggctgcc tgtggaaccc tgcctttttt tggggagcag 3720

gcttttaaaa aatatttatc acggtgaaat ataaggcagg aggattgtta gaagttcaat 3780

aaggacaatc tgattctgta gtggggttca ggctagctag ggctacatac taaacaaggt 3840

ctcaaccttg ttttagtaag aggaggagga ggaaacagaa ggaaaaaaaa gtaatttctt 3900

aggcacccag taggcctgca agtcttgttt tgaacaaatg aatggatgca tgaatcaatc 3960

tttgctacct ttcaggctct attctgtgtc cccagagcac ccccggccca ggaaagtgag 4020

cagggcacgg taggtctgct ggaatctctg attgtacttg tgagggctct gcctagcagt 4080

ctccctggaa gctgagcctg gccttagcct cttccttctc tctcagagct tctgaagcca 4140

cagaagggac gcatcaaact cggacctaga aatgaacgat tagtcttttt tagtgatttt 4200

ttttcttccg tctttatttt ttgacactca ctgggtagcc ttggctggcc cagagctccc 4260

taggttggtc agagatctgc ctgcctcctg cctgggtgtt gatatacagg gattcaccac 4320

tctatgctct tacgagctgg cttcgaactc atcaggctta gaatgtcggg tcgtctcact 4380

tatacctcct aagtgttgga ataaccagtg tgcaccaccc aagagcccct agctgggact 4440

cttgccaggc ctgtcttctc taagccttag tatccccatc tccaaaatgg gtataaccat 4500

atgcatacgt atcctaaggg catataccaa aagttcagtg aatacagtag agtatgtagg 4560

accccctgcg ggctagcagt agaagccttt ccctttccat tataccettg gttctaaagt 4620

tggttgaaag ttcccctaag ctctctcccg cccagcaaac cagtcaaacc agtttcctca 4680

ttccccgggc ttctgtacta agcaaacact gctttgcaaa cggttgacca ggagcttgcc 4740

tttgttagag aaggaagtcc aatggtgttt tcttccttgc aatgccggga gtgggtttgc 4800

tgtgccttag aaagggttca aactgggctg gaagtagggc cccagagtag ctgggggttg 4860

gggattcagg atggcctcaa gtttgttgtt ccccaaaccc tcactttttc tagaactatt 4920

tcataataca catgcttaag gatacccatt tgttgaccca ttgcaaaatg ctctctgacc 4980

cagcttctga gactggttca ccccccatgg ataagaattg agtcatctgg tgccattcac 5040

aaatacagat taacctccct cccccaattt actgaacctc aaccaaaaaa agatccgcct 5100

gcctctgcct cccaagtgct aggattttaa agggccacgg taccacacgg atggacacaa 5160

cattcatttt cctgacagac caggagtcag gtcacaggtg tttgactagt aaacacccgg 5220

tgccaaccag ggtgtgggca tttggctatc taccctgagg cccaagatcc aagagcaccc 5280

gtggtccatg gtgctagggg acccggaaat gtagccagtg catcagagat ccagcctctg 5340

tcctcaggga agttcagaaa catctctgct tactgagact tctgcagggt atttgggaag 5400

aggggccagc acagcaggca tgatcagttt tacatgctgg gcctttgtaa gtaagaggat 5460

gtgtccaggc acagtaaagg aaagtctccc ccaggacctt gggccttttt atccactggt 5520

gaggcttttt ttctctttct gttccccct ccccacctcc cgggctttcc agacagagtt 5580

tccctgtgta ttcattcctg gctgtcctgg aacttgctag gtagaccggg ctggcctcga 5640

actcagtatg aacaaatgtt tacttatggc tggcactgag acttcctctt caccctgact 5700

cctcacccag agcttcccct gaacagaatt ctcttggtta gccttctcaa gatcttctgt 5760

ggaaaaggca gagatagtca tggggtgtgg ctctgtggtc aggctaaacc taatgagtgt 5820

gaggccctga ctttgattcc tagcattgag gaaaagcaga aacaggaatc ttacccaatt 5880

ttcctctgcc tgttaccaga ctcaaggctg tcccgtggag cccattgccc ttcctgagaa 5940

acccacttcc tttcctgagg ctaggaaaga catccctcat ctctgtgcct caatctcatt 6000

gatttttgtg gtgttaaaat tcagttattt gtctttgaga gggctcccct ccccccttttg 6060

cctttcacat cctttccctt ctttattgct atgcagagga ccctaactcc taggcaggcc 6120

ctctaccctt gagatacagc tccatcctct tgctttttaa aaacttataa ttcgagacag 6180

ggtctcactg ctacccaggc tggttttgag cttactctgc agcctcctgc atcagcccac 6240

cgccttgtac gtgagatggt gggcctgaga ctcagcctct tgaccttcac atacatctga 6300

tacgtgctat ctgaacaggc ctgtcttact cagttttctg ggtcctgttg gggggtgagg 6360

tggggagggg agatcttcgt tcaggcatgg agaggtggga aggaaaccag tccacacctc 6420

taacaagtga gttccaacta ggacttgctt agggcacata aggcagacag acgtgggaga 6480

tgtgagcggc ggggaagcga tgatgcttag atgctaccac gaggacctga tgtggtgtgc 6540

ctgaagatga acctgttccc catgagccag ctcataaatg gggaccagca ccggcccctg 6600

tgggataatt ggcctcgtcc tagataataa ttacttagct ttgtaattag gagagcttgt 6660

aagaagccga aagtgtttcg gatttatttt acctggacaa gttcaagact aaactaacac 6720

ctgttcctgt ggtttggcat gcagagccat ctgtttccaa aggaggtcct gcagcagctt 6780

tttttggggt tctcctccct gttccttccc ttctctcccc acatcgcctt ttctccgtcc 6840

agtggtcagt gtgtgtgtgt gtgtgtgtgt gtgtgtgtgt gttaatgaga cagggtctca 6900
ttatgtagcc ttgggcctgg aactcactat gtagatgtgg ctggcttcaa actcagagag 6960
gcatatctat ccacttgcct ttgcctctgc ctctcaagcg gccgcgtgct gagactaaag 7020
ggatgtgcta ctactcctgg ccagagtgat aagtactcat cctgtttaaa gttgcctggg 7080
attccctgtt tgacactgaa taaaaatcct gcccatgact tccaaggctc tgggtccctc 7140
cttcccccgc agcccatggc ttgggccttc attttaaccc cactccccac atgtgcttcg 7200
ttcagatgct ttgcttcctg attgtactca gaataccatg gcagctcggg ctgaggctgg 7260
ttttgaactc ttaatactcc tgtccgggag tagtagcatc tcaggtgtgt ccaatcacac 7320
tctatgtatt ccttgtttgt gatgtctgta tcactcaccc gtgcccttca aagctttgct 7380
aagatgttgc ctgggcaatg aggaccttct ctttttgctt tatttttctg tagcccccca 7440
cctccccgtt ttttgggtgg ttttggtagg gatgagacat taagatgttg cctgggcaat 7500
gaggaccttc tctttttgct ttatttttct gtagcccccc acctccccgt tttttgggtg 7560
gttttggtag ggatgagaca acagggcctt atgcaggtta tgcaagtacc ctattgctga 7620
tccacatccc agcctttctg gtattttcca ccattgaacc tgtcatgtgg tttgtgtatt 7680
tcctcagttc atagtcttct tccataaaaa cataaatgtg gccggacgtg gtggcgcatg 7740
cctttaatcc cagcactcgg gaggcagagg caggtggatt tctgagttcg aggccagcct 7800
ggtctacaaa gtgagttcca ggacagccag ggctatagag aaaccctgtc tcaaaaccaa 7860
aaaaaaaaaa aaaaaaaaaa aaaaaccaaa accataaatg tgaggaggtc agggtgttct 7920
gtgtttgttg gttcttgagc tgtacttcag gccgtaacaa gttcttagac ctcatctgtt 7980
gaatgagtga acggaggagt gagaggtcca gaaggtaaat atggcgctgt acagttcttt 8040
ttagaagctg tgctgtgggt tttgtttttt ctaagataaa atggggtcca gtgacatggc 8100
tcagagtaaa tgtaaatgtt gtaccttttg gcatcctggg ttttattttt ccagaaccca 8160
tttatcctgt gacttccaga tgcgcaccat accccctgta aacccccccc cccccaacac 8220
acacacacac caaactagca aacagataca gggctgtcag cgtgtgagca catgaggaag 8280
ggatgctatg ttctcaggcc ctgtgtttct tcttggcacc ccctggtttc tctgagccct 8340
gcacagctac agtgtaacct tacctaattg tcttgttaca aagaagtttg ctgcccagag 8400
tctaaaggtc agatgagtca cttgggcctc acacttctgg gtgtatggaa gcaccagatg 8460
gaagtctcaa ggtacacccg acaccggctg cctttccgtc gtcaggcagc acattcctga 8520
agcaggctct ctcagctgtg ccctctcaac cttcatcctg cagcccatta attgttcaag 8580
gccttctagt tcttggcagg tctgagcatc ttggaaggag ctagaagaga gatgtagaaa 8640
gctcaaagac aaaaatgcaa agctaccccc atcagatacc agctcttggg tctattgacc 8700

agccaatatc cattgatagt ggagttctac atccatatgg aagatacacc attctctttc 8760
atggacagga gtgccaaatc agaatacagg acattacatc tttttgagac aaggtcttac 8820
catgtagcct ttgtagacca gctatgtaga ccatgctgcc ctggaaccca cagagatttg 8880
ctagcttctg cctcccaagt gctaagatta gtgacacaca cctccactta cacagtacac 8940
acccaggaaa aaagtaaaac ataaataaaa agcaaaaaat tgttgtgaaa tagctcagta 9000
ttagagtcct tgtctaagat atatgggctc caggcttgcc catccatcta ttttctgaga 9060
ctggattttt ctgtgtagcc ctggctgtcc tgggtcttac tttgtagacc aggctgacct 9120
tgaactcaga gatccacctg cctctgcctc tggagctctg gggttaaagg tagaccccac 9180
cagccatggg cttaagccca gtatgggttt aaaaacatgg aaccgattgg actttgcata 9240
aagttagatt ggatcctgtg ggtgctgacc tcccatgctt tcaagcactt attggtagca 9300
ttcactgaac aagcctgagg gcgggaacag ccctggcatc tttcaaaggg tatccggttc 9360
aaaccagaat cgaatgctct tttcctagcc accccacctc ttggcttgtt gcctaaggaa 9420
aatggctcta gcctggttct ccagtgttct catggcagtc tggagagatt gtccatttgt 9480
atcacaacct tttgggcgct gcccttggtg acccgctgct gatgtggaca ccagcaagac 9540
ttgagactgc agacctcctc ccccacagat ttctcacaat gcccgtgtgg gagtgtttga 9600
aaagggaaca agctgaccaa cctgtttgtg ggaaagtcgt ggctttccta tcagaaaacg 9660
tcacactctg agtcaggagt gggacatacc tgggtacttt aacaggtggg gcagggtcct 9720
gagtcataga gaggtctcac ctgggaaggt ttctctttca aagagtgaat caaccatgac 9780
atcacccatc tggatctaca ccctttcaag ccgtctctct ccttagcaaa caggaatcta 9840
aaagaggaat aataaggtga aagcaaactg gaaataggac agaacagaca agggaaaaag 9900
ttaaagagaa agcttaagag acgcagacac agacgtttac atgcaaacag ggggtccata 9960
aaaacaaaac cggaagccgt aatatatgta catggcctgt aagattagaa acaaatgtcc 10020
tggcacaaca ttatgggaca acttcccaag ctgctgttga gtttgttttg tgttggccgg 10080
catgggccct taaactgagt ggtgtgtttc tctcttagag aatttttcat gaactagttt 10140
ttcatttgca agtggttagt agtttgagat agcttctagg ttaggggtgg ggccgtgtgt 10200
ccactttact cagctctaat gtcccccgtt tggtgcagac ccctgcagac cctatgcgtc 10260
ctgccacagt ctctgagttc atgcctgcat tgttcaaatt gtgtttcctt gatgtcctcc 10320
aatctcctct ttgctgggct ccctggggcc cccaaggtga gggatttggc agaggtattc 10380
attccattta gggttgattg ttccaaggtc cctcactgta cgtgttattt gtcccttctc 10440
cctggcttcc tgctcaggag ttctgcagac atggctggtg tttagtagct tcttggtgaa 10500
gttcaggtag ccagtttcat gaccacccag ctctcaggcc tgactaagcc cgtggagaaa 10560
gcccataaaa taaaggggt gaacttctaa tctacatctg aaaggagaac ccctgtctac 10620

atctacctgt taccctagcc atcggggaga ctgaggcaga aggatctagc ctggaaattt 10680
agggtgagtc tgggcagcat taggagacta tttcacagtg aagaaggaag gaactcactc 10740
acacccttgg tgcacctcac aggtctctgg gctcagattg gagtctttgg ttttgaaatt 10800
tcctcatcat cggccgcctc agctggtttt tcttctggag tctgttacgc cttctcccct 10860
ttcccccctt ctcttgctaa tcctttcgct tgaacaatag gagtccttaa aacctgggag 10920
ggaagtaaat acctttcaga tgagaccctg tcttaagagg gggaaaaaaa aaacaaaaaa 10980
accctaggga ccgggtcacc aagtgtcagg ttaatcagca gatgtcaaga tgactcctga 11040
gtgcttgctc tttcttcagc tggtgaaatt acagcggcaa catagaatga cagtcagcag 11100
ggagggagga gagggcctgg gtggccaaat aaacttataa attgtgagag aaattaatga 11160
atgtctaagt taatgcagaa acggagagac atactatatt catgaactaa aagacttaat 11220
attgtgaagg tatactttct ttccacataa atttgtagtc aatatgttca ccccaaaaaa 11280
gctgtttgtt aacttgccaa cctcattcta aaatgtatat agaagcccaa aagacaataa 11340
caaaaatatt cttgtagaac aaaatgggaa agaatgttcc actaaatatc aagatttaga 11400
gcaaagcatg agatgtgtgg ggatagacag tgaggctgat aaaatagagt agagctcaga 11460
aacagaccca ttgatatatg taagtgacct atgaaaaaaa tatggcattt tacaatggga 11520
aaatgatgat ctttttcttt tttagaaaaa cagggaaata tatttatatg taaaaaataa 11580
aagggaaccc atatgtcata ccatacacac aaaaaaattc cagtgaatta taagtctaaa 11640
tggagaaggc aaaactttaa atcttttaga aaataatata gaagcatgcc atcatgactt 11700
cagtgtagag aaaaatttct tatgactcaa agtcctaacc acaaagaaaa gattgttaat 11760
tagattgcat gaatattaag acttattttt aaaattaaaa aaccattaag aaaagtcagg 11820
ccatagaatg acagaaaata tttgcaacac cccagtaaag agaattgtaa tatgcagatt 11880
ataaaaagaa gtcttacaaa tcagtaaaaa ataaaactag acaaaaattt gaacagatga 11940
aagagaaact ctaaataatc attacacatg agaaactcaa tctcagaaat cagagaacta 12000
tcattgcata tacactaaat tagagaaata ttaaaaggct aagtaacatc tgtggcaata 12060
ttgatggtat ataaccttga tatgatgtga tgagaacagt actttacccc atgggcttcc 12120
tccccaaacc cttaccccag tataaatcat gacaaatata ctttaaaaac cattacccta 12180
tatctaacca gtactcctca aaactgtcaa ggtcatcaaa aataagaaaa gtctgaggaa 12240
ctgtcaaaac taagaggaac ccaaggagac atgagaatta tatgtaatgt ggcattctga 12300
atgagatccc agaacagaaa aagaacagta gctaaaaaac taatgaaata taaataaagt 12360
ttgaacttta gtttttttta aaaaagagta gcattaacac ggcaaagcca ttttcatatt 12420
tttcttgaac attaagtaca agtctataat taaaaatttt ttaaatgtag tctggaacat 12480

tgccagaaac agaagtacaa cagctatctg tgctgtcgcc taactatcca tagctgattg 12540
gtctaaaatg agatacatca acgctcctcc atgtttttg ttttcttttt aaatgaaaaa 12600
ctttattttt taagaggagt ttcaggttca tagcaaaatt gagaggaagg tacattcaag 12660
ctgaggaagt tttcctctat tcctagttta ctgagagatt gcatcatgaa tgggtgttaa 12720
attttgtcaa atgctttttc tgtgtctatc aatatgacca tgtgattttc ttctttaacc 12780
tgttgatggg acaaattacg ttaattgatt ttcaaacgtt gaaccaccct tacatatctg 12840
gaataaattc tacttggttg tggtgtatat tttttgatac attcttggat tctttttgct 12900
aatattttgt tgaaaatgtt tgtatctttg ttcatgagag atattggtct gttgttttct 12960
tttcttgtaa tgtcattttc tagttccggt attaaggtaa tgctggccta gttgaatgat 13020
ttaggaagta ttccctctgc ttctgtcttc tgaaagagat tgtagaaagt tgatacaatt 13080
ttttttttctt taaatatgtc gacgcactgg ctttacaggg gccgtctgct ttgtctgtag 13140
atatggtgat aggagtctga tttagaaact tccttgttct cctcttcccg aatccttcct 13200
cctgaacaat aggaatacct aagtgcacaa gtgcaggtgc ccaggaggcc ctgagacaac 13260
ccccccctccc cagctgcgag ccttctccgg tggtgctggg aatcgaactg gagtcctctg 13320
caagaacagt atgtgctctt aaccactgca ccatctcttc agccccctct agttctttag 13380
tttgtacata aattagcaag ttcagggaat gcagaagaac tggccagggg tcagagccag 13440
actggaaaca tttttcacca aacaatagct acagtgttca tggcgtgcct ccattgaata 13500
ctaaactagc tgttgatgtg tgactttgat ttttaatctt tgcaccaact ttagttacta 13560
tatagataat tccatttcca aatgatatat agtggaagct tgtaaaagct gcttactatc 13620
ccagggtccc cagccgagat ctacatgata aagctactct caacccagcc tacattcccc 13680
ctccctgtgc tcagccctac ctggggttgt tcactttcaa gctcccagga aggaacctga 13740
gcgctccctg cccccatgca gtccatttct atctctggtc ctttgggtac ttggagcctc 13800
aagctcctga ctgctaatga agcctgtaac agaagggcat tacttactgc ccgccttgtg 13860
ctcccttgaa atctggtcac ctggaggtgg aagtgggcag tgcttccgtg cttccgtgct 13920
tccgctttct cccagctcct tttaatcact attttttttt tttcttttcc aaattactac 13980
ctaggagttt tctagtaatc actttggtgg gagtgggagg gggagtggag gcagttaact 14040
ctgtgagtgc caaggacagt tgttggacgg tggtagtgat ggaggggagg gtgttcacgg 14100
tgagggaact ctgtggagtt acgtagctgg caacaatggg gagagacgga gccgcaccac 14160
cacagacgct ttgcatgagc aactattttg ttttctgtag atgttttcca aaccttaaac 14220
tctgttaagt ctgaaactgg gaggggaacc tctccaccaa aatctaaata tggatcagag 14280
ataaggaaac ttccatagtc tccagctccc tccctgactc agtggcctct gtgaccaact 14340
ccacgtgact ctagtggaca ccttcctggt ggtttggctt tttttttttt tccccatttc 14400

atcttttaaa tggagttttc catgaataag aatataaaac tgcgggggct ggtgagatgg 14460

atcagcaggt aagagcacag aggtcctgag ttcaaatccc agcaaccact tggtggctca 14520

ccatcggtaa cgagatctga ctccctcttc tagagtgtct gaagatagct acagtgtact 14580

tacatataat aaataaacaa atcttaaaaa aaagaatata aaactgcttc cttcccaaca 14640

ggccttggat agttttgtct gtgggaacag cttcccatag gaaaggtgct tttgctactt 14700

ccgaccgtca cacactcctc ctctgcaccc cgtgagggcc tcccaggagt tttgtgttgg 14760

tcacaagcct ccccatgtcc caagggggtc tggagaggga tctgaggtgg tggaaagtgc 14820

tggggtgcca agttatcaga caatgggcac attgagaatg caggtggggc caggcctgtg 14880

tgggctggag cagctgtcgg agcctcaggg ggtgcagctc atgcgcagag ggaagaggca 14940

gtctctctgc tggctgagag gggagggcca cagtaagcaa aggagggggg tgatccccccc 15000

ctcacaagat tccagagcaa agggatgttt gaacgatgac tcacatgaca ggggcggggt 15060

ctgtgagcaa acttcctgtt ggaagcttgt tcttggttcc tgactgtcct tcccaggtca 15120

tagagactgt ctgcaatagg gctggggcct gttaggatgc catcgaggtg ggaaatcctg 15180

gagagcttgg agtagccagc aggccctctt ccctctcctg ccccaggcta gctggaaacc 15240

cgcgaggagg aagggtgtgt tgttccctat cccatggaga ggcttgcatg gtcagaccag 15300

gcctggtgtg acctctgggg ctgctctgtg actgccctgt tctgtgactg cccttctccc 15360

tcccagccct ccccttccat ttgcttctag attcgtctag gagcccatct cagcaaatgg 15420

cagcatcctc gcttaccaga catcttgttt gtttgtttgt ttgttcattc attctgggcc 15480

tgaagatgga gagctgtggt aggggagagc tcagaggctg gggcagcct taagggcaca 15540

gccaggacgg agcctctgag caaagggcag tgtgtgtaga gtcaggtagg tggtgggcag 15600

caggagacag gaggagcaca gaggtaggtg gcactgtaca gaggccctgg caccattttg 15660

tgacaaggac tctcagaccc ctctagccta ttcccccacc cccaccccca ttacaagcag 15720

tagggtctta atagctaatg gcttggacac cagcctctgg ctgctctggt tcccagctgg 15780

tcagagccct tgttctcctt tgggttctga tccaggtggc acagggtggg ggccctgatt 15840

cacattcttg atagtgccgc taggttccct gctaccagct ggcacattct gtcttccttt 15900

ccagcaggta cttggctggg cttcctgccc agctgctaga cctactgtta ggtcagctca 15960

ttggatgctt cctccaggaa gccttctcta atctccattc ccttggcttc ttttcaagag 16020

cagaagttca ctggcttctc ctgtttcccc agagtagcac ctggtgtctc aagtactcgc 16080

taagcagtta ggagaggtgt gaactgggcc tcagggaccc cagagacagg aggggaggtc 16140

attacaggaa agtcggattc ttttcctggc tttgttctta taaccatcgt gtgtgtgtgt 16200

gtgtgtgtgt gtgtgtgtgt gtgtgtgtgt gtgtgtgtgt acgtgtgtgt acgtgtgtgt 16260

gtgtgtacat gcgtgcgcgc acacgtgcac gcggctgatg ccctctttct ggccaccctt 16320
tatttatttg taatatttat ttatttattt atttatttat ttttggtttt tttgagacag 16380
ggtttctcgt atagccctgg ctatcctgaa acccacttta tagaccaggc tggccttgaa 16440
ctcagaaatc tgcctgcctc tgcctcccga gtgctgggat taaaggtgtg cgccaccact 16500
gcccggcttc tgttcactct taagcgtacc caacagccca tcctgcacca gcccagagag 16560
tctgcagagt cccctgtgaa aatgtgggca acctgttcct cctggcatca ggtggggagg 16620
agaggcagat tatgcagaat caatcaatag caatcaatag gtccaatgcg gaggagacaa 16680
aggattgaga tgagacaggg ggcgtcccac gtccaattcc tttattgctg cagtcctttc 16740
tgcccctctt tacaaacagg agactgaggt ttagaaaggc aggctagggt agtgggtgga 16800
agggtgaggt gaacctacag gggagactaa ggagccagat gaatggggaa agggaggcag 16860
cctcttaatc cttttgtctg tagctccatc tttggaaatg ctgagtaatt aatcaattgt 16920
taaagggctt tctgccgaat tctggggaaa gcctttactt ttaatgagct gctagtctgt 16980
ctcttcagtg ctgggaatga gtctagagcc acctgtctcc taggcaaaca ctttactgct 17040
aaactactaa gctctgctcc cccacactcc gggttagcct ttacatctgt atctgtctgc 17100
ctctgttggg atttcaggtg tgtgccaccc cctgtgctcc tcagtgtatt tctacagggt 17160
ggtggtacct aaggaaaaat ccattcctag ggactctgac gtaaatattt gggatgaggt 17220
ctgtcaagga ctgggcattt taacttcact tatttttata gccctgaggg tggacatggg 17280
acctcttgta ggctaggtag gtttccttcc cgatcgccca agggcgaatt ccagcacact 17340
ggcggccgtt actagcggta gactaggata gcctcaaact cctaacggtc tgcctgcctc 17400
tacctcttgg cctagggctg tggactgtct ttttggtcct actataagga tcatctggac 17460
cagaacgcac gttctcaagc ctacgctggg gagtgcctca cctagaaagc ttaaaagcgt 17520
agactctcgc tggatgtggt ggctcaggcc tttgatccca gtactcagga ggcagaagca 17580
ggtagatctc tgtgagttcc aggacatcca gggctacata gagagacatt gtttgccccc 17640
aaagcacaga ccctgacagg cctgtccatc ttaaggaacg gaggccagag acctgttggt 17700
agttgatggt ttgatggttt atttagaaac tctccctgat ggttcttgac ttggggagtg 17760
aggagaagcc acttcaagtt gtagacaagg tgtgtgtgtg tgtgtgtgtg tgtgtgcaag 17820
tgtgccaatg tgtaagtgtg tctgtatgcc ttgcacacac acagttgttt caagtgtgct 17880
atgtagccaa ggataacact gaactctgaa ttccccttta tctgtttatg ggcacacatt 17940
tccccccctga gggatataaa ttctccttga agtgttgatt cacccctgaa gtagaatcag 18000
gtctaaagtg gggatgaatc tcagggtcct tccaggtaga aatgtagggc aacaatgaca 18060
gtacactgta ccctcctcct ccctgtcctt ctccccgctt ctttttttctt tttgagctct 18120
gagtgactgg aagtcatgta gattggctgg tcccgatctt gtgcctttgc ttcccgggtg 18180

ctgagagtcg tgtgttctga agaacagatt taagcaggca agcaggctat gtaagatgtg 18240

tgacagaatt ccagagttga agaggctgga tggccaaggg acctggctct gtccatccag 18300

ccttcagtct gccttttctt ctcgctggga gagtggcttg gttaatccac accagtgggt 18360

gactttacaa attaactccc gtgaccttgg actgttccat ccataaacac ggctccctag 18420

tttatgagtt gcccagccca gtgttaaaaa tctacggtga actaagccct gggagaagcc 18480

agtgtgaaac tcaaactgag cagagaccag atcaaacatg gagataagac agctacagtt 18540

tcacttttgg gcagggccaa gaatttcagg actttcttta ctacagatgg ggcgttattt 18600

tgctttccta tgggtgtgtg tgtgtgtgtg ctgatttagt ttatgattca ttgattttgg 18660

ggtttgatag caggtctagg gaacacataa tgcgttacag atatttgatt ttaaatgtat 18720

tcctggtact ttttgggggg gggttgttta ttgttttgag gtttctcttt tcccccggct 18780

gtcctagaac tcatgctata gactagactg tcctcaaact caagatttgc ctgcttctgt 18840

ttcctgagta ttggaattaa aggcacgcac caccactgcc aagttgtttg cctggtattt 18900

tagaaagggc aattagtcat gagcctgagg gtttttcaca tttatttgtt ttgcttgtaa 18960

caagttaacc agtgacaggt ttactttagt tctctctgtc tctgtctttg tgtctctgtc 19020

tcttatcttt ccttgacagg ggggtctcat gtagcttcgg ttgaccccaa actcactatt 19080

ttagccacca gggcctttag cacctagtct tctgactgta agtcctctga gtgctggggt 19140

cacaggtcta accttctcat tgaggaaact ttggcctcta tctctggtca tcaatactga 19200

tctggagatc tctgagatac tattactgtg gaaggccatt tgattctctt atgatggagc 19260

ctgatgagcc agaccagaga atggctccta ttaggctcat aaactggaat gcttactcac 19320

tggggagtgg catcatttga aaggattaga aggattaggg gtgtggcctt gttgggggaa 19380

gtatgtcact ggaggtgggc tttgaggttt caaaagccca tgtcaagacc agtgtgtgtg 19440

tgtgtgtgtg tgtatgtgtc tgtctgtctg tctgtctgcc tctttggatc aggttgtagc 19500

tctcagctac tgctccagca ccattcaagc caccatgatg attaatgggc caaacttttg 19560

aaactgttag acaagtcccc agttaaacgc ttttttggat atgagttcct taagtcacat 19620

gcctcttcag caatagaaca gtgatattgg acaaccagga gcgtgctgga aaagtgagaa 19680

atttagaatt tttcccagca gtgggaattt cctgtgtcct gatgccgatg ccctcttttc 19740

aagactaaac tgagaacttt gctcctttgc tgcttccctc cttccatcct tacctgctcc 19800

agtgaagaat ttgttgacat ccgtatctca ggcacaaggg ggaaaacaaa gggaaaataa 19860

agataagagg aggagggctg agtggcaaag tgagtgccat acagcttaga actctgcaga 19920

aagcagacca caaatttgcc tctgttcctg ccaggcaaaa taaagaggga aatctgatca 19980

gccctcctgc tggacttcag tgtgctggcc attagtgggg tgcctggctc ctccagagag 20040

ttcttgcttc attgtagctc ccaccactct aatgtgctag acttcccatt tgcaattgtc 20100
tttactagat aaccaactaa tgcttgggtg gacaacattg cacaatagag ggcagagatt 20160
tcagtcatgt gtgtcaccac ctacacagct ccatgaggag catttttgca tctttgcact 20220
cccccactcc ccgggtaaga tctgtcttgt ataatgcata gcataactat gtaatggtgg 20280
ggattttact aatgctattc actacccagc actctgccgc ctcagggtca cgtgtttact 20340
attccctctg ctgggcacct ttgccttggc atatagctta taactccagg ctctgttaaa 20400
gcttccccca tagtggagtc taccacataa taaaatgtac agttcctttt tcatagcctt 20460
tattataatc cagcttaata ctgtatgcta tttgattgct tctccaacat cccccccctc 20520
caaatgtagg ctccttgaag gtgcaacctg ctttatttca ttggttgctg atagaaaatt 20580
ttcaagtgat cctccccacc ccgccccccct cacactcgct gcatgagatc aggtgctggg 20640
aattgaaccc agcaccttac ctgctacaca tgctaggcaa gtgctctacc atcgaggctc 20700
caggaatatg gatctctctc cttttctctc cccactcctc cagttacagt tcttttgcct 20760
tgacttcact tgaactagct ctgtagacca ggttggcctc aaagactcag gcgtccactt 20820
gcctcccaaa gtgctgggat taaaggtgtg ccatttcagg catcagccag agatttaaaa 20880
agaaaaaaaa aaatccagat ataaaaagaa tactgtttat ctccactttt agaaaattgg 20940
ggagggatat agtgcagaga tagagtacct agcatgtggg tggtcctgga ttgaagagag 21000
tagtctgcaa gagaaaaagc aaaagagatt ctctaataga ggaaggactc catgagaaga 21060
gtagaaagaa ggttctagaa caaccaaggc agggagtctc cagacagaat gagcacagtg 21120
tgccgagaag gaaaaggcca aggcccgtga ctgagatcct tgagggtctg taggtgctgc 21180
acagttatgg aaatgatatc cagggtccag cctctcagtt ctgaagactg ggtaactgcc 21240
tctgtatcca tcagcttctg ccaagatgga gtgatgagtc tgtgttcaaa ccgattcttc 21300
cttctggcca agcatacggt gggtgtggtg cactggtgtg tgcacgtgag tagtaagtga 21360
gtgcgttcct tgttccttca gcccaggagc ttctgggtcc tttggaaaca attgcaggag 21420
accagaataa cacagctcat tcatcactgg gtatggctca gcccagcatc ttgtgtcaga 21480
aacgatccga ggggcatgtt tgatggggat tagcgctgct ctcaatatcc tgccttttgg 21540
tcccagctga ctctgacatt ccttgctggt gagttcctgc tcatttcagt tctagccctg 21600
gagcagcagt cagtattaaa gcaaacactt attctgtgtg tcacagtgcc aaggggaact 21660
atcactattc tgatagtatg ttaacacgcg ttgggtgcaa tcttaccaga gccttacccc 21720
atagcttcta ggtatgcact atatagttca ttgtcttgaa acctgatgta gcttcctcaa 21780
aagtcactat ggaaatggac tgacacaagc caccggccag tgggagaatg gggtttgaag 21840
cttagcgact gtaagttcat catgtaatgt aggaggggga agtgtggtgt tagtaatacc 21900
tatagtctta gttcttgtgg agttgaggca ggaaaatccc ttgagcctaa gtgaagatga 21960

ggtaacatag caggaccctg tgtctgtgtg tgtgtgtgtg tgtgtgtgtg tgtgtgtgtg 22020
tgtgtgtgtg tgtgactgac tgagatagtc tcaatatatg tagccttgtc tgccctggaa 22080
ctcttctcat ggccttgaac tcctagagat ctttttgcct ctgtttctgc ttctgccttc 22140
catgtgctgg gactaaaggc atatgccacc atgccaggtc ccaggtccct gtctctttta 22200
aaaaataaag cagaaagaaa caccatactt ggggctggag agatggctca gcggttaaga 22260
gcactgactg ttcttatgaa ggtcctgagt tcaaatccca acaaccacat gatggcttac 22320
aaccatctgt aacgaaatct gatgctctct tctggagtgt ctgaagacac actacagtgt 22380
acttacatat aataaataaa taaataaata aataaataaa taaatattaa aaaaaaagaa 22440
acaccacact taaactgatg ccttacaaga acttcgtacg tgttttgtac ctgttactgt 22500
gcagctgtgg tcgggacaag ggttatcaga gagagatcta tctggtagag tttgagctct 22560
agatgtcagt ggatgtggca tgtcacgggc ttcggctttg tgccttccta ctttgcagct 22620
gggtgtaatt ttctgcattc acctggtgtt tcttgcagca gaaatcatca cacccataag 22680
agaagtagcc tctgagttaa tcttaagtga ggtagacctc tgggctatgc ttaagttgtc 22740
cgctaattca tcacttgtgc tggaaggaat gtgcactctc caaacagatg ctgtagagga 22800
ctgttatctc caccttgcag tgaagtgaac tgaggttctg agatttaagg acaaaggttc 22860
ctgtcggggg aaggacaggg agaggtgtgt gtgggggtg ggactgggag agaaagggac 22920
aggcaggttt gagacagggc ttctcagtgt agcctaggct tacctggaat tcacagagat 22980
ccacctgtct ctgagtcatg ggtgctggac atttgtttat tttaaagatt tatttattta 23040
ttttatatga gtacagtgta gctgtcttca gacacactag aagagggcat cagataccat 23100
tacagttggt tgtgagtcac catgtggttg ctgggaattg aactcgggac ctccggtaga 23160
gcagtcagtg ctcttaactg ctgagccacc tctccagccc aggatatttg ttttaagagt 23220
gatagaacta ggaagctgga ggaatagggc agtcgataaa gtgcttacct taagttcaaa 23280
gacatgcttt tctgctgctg cttgtcctcc tcctcccctt ccctctttct cttcttcctc 23340
ctcctcctct tttctttcaa gatagggtct ctctctacat agctcttgct gtcctagaac 23400
cctatctgta aaccaggcta gcctcaaact cttacctgct cccgactcct gaggagacag 23460
gttctcaaac agaccctttt gagacgggct ggacttgaac tcttgacctt cctgccttac 23520
ttattctgct agtgctggca ccacaggcag ctgcctggtt ttgtgttaga cctttttagt 23580
ggtggtcaga ggtgccttgg gcagcagctg cagagccacg tgggcatagg gaaaatgccc 23640
aggggcaggg cagaggccat cagggcagcg gagtgagcag ctctcatggc acgtcagcct 23700
ccatcgtgtc ctcggtgtgc acatggttgg atgctcgctt tgagttcttc tgtaggatct 23760
tcccttatcc ccaaatgttt tagttataaa aaaaaaaaaa aaaaaaaaaa aacaacttcc 23820

cagaggtgca aggagccacc tccttctgca gttcattctg tgaatggagc ttgttgcttt 23880
gtgtcaggca tttgcaagtg cgggtcactc agaggttgag aagtcctcac cctggtgctt 23940
cctggaggct gggggggagga gggcagcggg atggggggaa aggttggcct cccaagtgct 24000
gcaatgaaag tcaggcacca ctacacctgg ctaattttat ttttaaaaac ttctgtatat 24060
gtgtggccat gcacacattt agaggccaga aggtgtcgga tccccagagg tggagttaca 24120
gatggtcatg agccacctaa catgggtgct gggaaccgaa tgtgggtcct ctgcaagaac 24180
agtgcacacc tttgactgct gagccatctt tccagcccca gcttgttcaa ttccaaacaa 24240
taagtcagta tttaggaaaa caaagcattc catgagtcag gataagatgc ctgatgcccc 24300
ttctgaacca tgctgcccgt tctcatgggt aggagattta cagtatcatt tgctgtctag 24360
tccatattca aacaccccca actatcccaa gaatgtttta tttagtgtag gaacttaaac 24420
gcaaggcatc tcagacatga cctccaattc tgtacagtca agaatgtaaa gaaggggagg 24480
tagtcctgcc tttgatcagc ctgcctatcc ctctttcttc tccctcttgg gggttgagcc 24540
aggaagcatc ttattggatt ataggagtca agattctggt tttgtgtttt taagcacatg 24600
gatttcatat aagctacatc tatgcgcaca aatagccaca cacagagctc aagtttgtaa 24660
tttaaaaaga catataatgt ctccccctgt aataaaggca agctggcgtt tctggatctg 24720
aagtgtctgg tagtgtctgc atgttacttc cacaaggaac aaagggcttt gatgcagatg 24780
ctgctgcagt tagtcatgaa ggtcaagccc ccaggtctgt ctgtccttta cagacttctt 24840
tcctcaaagc gggcaatcta ggagagaaag ctgattctgt ttgtccctgg aggcaggcct 24900
ggagctcaac tgtatagacc aggctggcct cagacgtgtg gcagttctgc ctgggcctca 24960
cgtgtggtgg attgttttcc atccaggttt ggagtcacag gcttgtaatc ataaaagttg 25020
gaagtgcagt tagtacactg cagaatggtt tagcagcaca gcatacttac tgctcagcag 25080
aggcaagagg aacgttggaa gtttgaagct agcctagtcc acacagtgag ttctggttta 25140
tccagagctc aagctgtgct tgtctgctgt gtatgaaacc ctggacttgt tctgcagtgc 25200
tgcataaact ggtcatggtg gtccattcca gcacaaggac atttttgcta agtcgtaagc 25260
ctaggctaca tgaggcactg tctcaaaaaa aaattttttt tttaaggtag agaataatag 25320
agaagggaat ccagcatcta cttctgccca aacatatata tcttcttcac tcccaggaac 25380
acacacgcca cacctgaaaa cagctattag cagtactagg cagggaacta tatgcatacc 25440
tcttagatgg ctgagtttaa ggccaacctg ggccacgcag gcaattcaaa gccagccaag 25500
gctatgcata tatagtgagg ctatctggga aagatggaaa acaaatgaaa catcagactg 25560
aggcagagcg ggacacttga cctctcacgt cacaagcttc tggacttaca gtgggtaaca 25620
tcctgctaag ttggaagtgc agttggaaca ctgcaaaatg gtttagcaat acagcatagt 25680
gcccagtggc cgagtgctgc aggctgcaga ctgctagcct gggacccaga tgcaatcttt 25740

atagtctcca tcaaacatgt attacttgat tttgaactgc cgtatccatc acactggcgg 25800

ccgctcgagc ccctccggag gagtggccag ggcgttctgg aggtagaaac tggttattgc 25860

tacacaagat ggagactata tccaaaccac tgaattttgt gttattaaca gtagttttga 25920

ttttaaaaat tgattgccaa ggacaaatac taattacttt taaatattac ctaccagttt 25980

ccttttgctt tgcttttatg ttttaaaaat tatttatttt atgtatatga gtacaatata 26040

gatgtcttca gaagagggca tcagagatct cattacagat ggctgtgagc caccacgtgg 26100

ttgctgggaa ttgaactcag gacctctgga agagcagcca gtgcttttag ctgctgagcc 26160

atctctccag tcccttgttt ttgttattat tatattttat ctacttttgg gtttgtgtct 26220

gtgcacacat accacgttgt gcacatggag gtttgaaaac atgagcgtct tctcctgccg 26280

tcattttact tggctgcatg cctttgcccg ctgagccatc ttgccagcct ttttattttt 26340

gtttcctttt gtccttgttg agataggttc tcactatgga gcccagacta gctttgaact 26400

ccctactcca acctgtccta tctctcaggc aatgacctaa accaaccagt gagtcttcct 26460

gggcccacct aggtttttgg cgcgccctac gtcacccgcc ccgttcccac gccccgcgcc 26520

acgtcacaaa ctccaccccc tcattatcat attggcttca atccaaaata aggtatatta 26580

ttgatgatgt tacatcg 26597

<210> 75
<211> 4056
<212> DNA
<213> Artificial Sequence

<220>
<223> pBest4

<400> 75
ggccgctgcg gccgtttaaa cgccagcaac gcggcctttt tacggttcct ggccttttgc 60

tggccttttg ctcttaatta aatcgcatca tcaataatat accttatttt ggattgaagc 120

caatatgata atgagggggt ggagtttgtg acgtggcgcg gggcgtggga acggggcggg 180

tgacgtagta gtgtggcgga agtgtgatgt tgcaagtgtg gcggaacaca tgtaagcgac 240

ggatgtggca aaagtgacgt ttttggtgtg cgccggtgta cacaggaagt gacaattttc 300

gcgcggtttt aggcggatgt tgtagtaaat ttgggcgtaa ccgagtaaga tttggccatt 360

ttcgcgggaa aactgaataa gaggaagtga aatctgaata attttgtgtt actcatagcg 420

cgtaatattt gtctagggcc gcggggactt tgaccgttta cgtggagact cgcccaggtg 480

tttttctcag gtgttttccg cgttccgggt caaagttggc gttttattat tatagtcagc 540

tgacgtgtag tgtcgagtgt tgacattgat tattgactag ttattaatag taatcaatta 600

cggggtcatt agttcatagc ccatatatgg agttccgcgt tacataactt acggtaaatg 660

gcccgcctgg ctgaccgccc aacgaccccc gcccattgac gtcaataatg acgtatgttc 720

ccatagtaac gccaataggg actttccatt gacgtcaatg ggtggagtat ttacggtaaa 780

ctgcccactt ggcagtacat caagtgtatc atatgccaag tacgccccct attgacgtca 840

atgacggtaa atggcccgcc tggcattgtg cccagtacat gaccttatgg gactttccta 900

cttggcagta catctacgta ttagtcatcg ctattaccat ggtcgaggtg agccccacgt 960

tctgcttcac tctccccatc tcccccccct ccccaccccc aattttgtat ttatttattt 1020

tttaattatt ttgtgcagcg atgggggcgg gggggggggg ggggcgcgcg ccaggcgggg 1080

cggggcgggg cgaggggcgg ggcggggcga ggcggagagg tgcggcggca gccaatcaga 1140

gcggcgcgct ccgaaagttt ccttttatgg cgaggcggcg gcggcggcgg ccctataaaa 1200

agcgaagcgc gcggcgggcg atagggagac ccaagctggt gagtttgggg acccttgatt 1260

gttctttctt tttcgctatt gtaaaattca tgttatatgg agggggcaaa gttttcaggg 1320

tgttgtttag aacgggaaga tgtcccttgt atcaccatgg accctcatga taattttgtt 1380

tctttcactt tctactctgt tgacaaccat tgtctcctct tattttcttt tcattttctg 1440

taactttttc gttaaacttt agcttgcatt tgtaacgaat ttttaaattc acttttgttt 1500

atttgtcaga ttgtaagtac tttctctaat cacttttttt tcaaggcaat cagggtatat 1560

tatattgtac ttcagcacag ttttagagaa caattgttat aattaaatga taaggtagaa 1620

tatttctgca tataaattct ggctggcgtg gaaatattct tattggtaga aacaactaca 1680

tcctggtcat catcctgcct ttctctttat ggttacaatg atatacactg tttgagatga 1740

ggataaaata ctctgagtcc aaaccgggcc cctctgctaa ccatgttcat gccttcttct 1800

ttttcctaca ggggacccaa gcttggtacc gagctcggat ccactagtaa cggccgccag 1860

tgtgctggaa ttctgcagat atccatcaca ctggcggccg ctcgagcatg catctagagg 1920

gccctattca attctttgta gaggttttac ttgctttaaa aaacctccca cacctccccc 1980

tgaacctgaa acataaaatg aatgcaattg ttgttgttaa cttgtttatt gcagcttata 2040

atggttacaa ataaagcaat agcatcacaa atttcacaaa taaagcattt ttttcactgc 2100

attctagttg tggtttgtcc aaactcatca atgtatctta tcatgtctgg atcatcgatc 2160

cataacttcg tataatgtat gctatacgaa gttatccaga tctcgattta agggtgggaa 2220

agaatatata aggtgggggt cttatgtagt ttaaacgcta gcagtgggct tacatggcga 2280

tagctagact gggcggtttt atggacagca agcgaaccgg aattgccagc tggggcgccc 2340

tctggtaagg ttgggaagcc ctgcaaagta aactggatgg ctttcttgcc gccaaggatc 2400

tgatggcgca ggggatcaag ctctgatcaa gagacaggat gaggatcgtt tcgcatgatt 2460

gaacaagatg gattgcacgc aggttctccg gccgcttggg tggagaggct attcggctat 2520

gactgggcac aacagacaat cggctgctct gatgccgccg tgttccggct gtcagcgcag 2580

gggcgcccgg ttctttttgt caagaccgac ctgtccggtg ccctgaatga actgcaagac 2640

gaggcagcgc ggctatcgtg gctggccacg acgggcgttc cttgcgcagc tgtgctcgac 2700

gttgtcactg aagcgggaag ggactggctg ctattgggcg aagtgccggg gcaggatctc 2760

ctgtcatctc accttgctcc tgccgagaaa gtatccatca tggctgatgc aatgcggcgg 2820

ctgcatacgc ttgatccggc tacctgccca ttcgaccacc aagcgaaaca tcgcatcgag 2880

cgagcacgta ctcggatgga agccggtctt gtcgatcagg atgatctgga cgaagagcat 2940

caggggctcg cgccagccga actgttcgcc aggctcaagg cgagcatgcc cgacggcgag 3000

gatctcgtcg tgacccatgg cgatgcctgc ttgccgaata tcatggtgga aaatggccgc 3060

ttttctggat tcatcgactg tggccggctg ggtgtggcgg accgctatca ggacatagcg 3120

ttggctaccc gtgatattgc tgaagagctt ggcggcgaat gggctgaccg cttcctcgtg 3180

ctttacggta tcgccgctcc cgattcgcag cgcatcgcct tctatcgcct tcttgacgag 3240

ttcttctgac ctcaggttac tcatatatac tttagattga tttaaaactt catttttaat 3300

ttaaaaggat ctaggtgaag atcctttttg ataatctcat gaccaaaatc ccttaacgtg 3360

agttttcgtt ccactgagcg tcagaccccg tagaaaagat caaaggatct tcttgagatc 3420

cttttttct gcgcgtaatc tgctgcttgc aaacaaaaaa accaccgcta ccagcggtgg 3480

tttgtttgcc ggatcaagag ctaccaactc tttttccgaa ggtaactggc ttcagcagag 3540

cgcagatacc aaatactgtc cttctagtgt agccgtagtt aggccaccac ttcaagaact 3600

ctgtagcacc gcctacatac ctcgctctgc taatcctgtt accagtggct gctgccagtg 3660

gcgataagtc gtgtcttacc gggttggact caagacgata gttaccggat aaggcgcagc 3720

ggtcgggctg aacggggggt tcgtgcacac agcccagctt ggagcgaacg acctacaccg 3780

aactgagata cctacagcgt gagctatgag aaagcgccac gcttcccgaa gggagaaagg 3840

cggacaggta tccggtaagc ggcagggtcg gaacaggaga gcgcacgagg gagcttccag 3900

ggggaaacgc ctggtatctt tatagtcctg tcgggtttcg ccacctctga cttgagcgtc 3960

gatttttgtg atgctcgtca ggggggcgga gcctatggaa aaacgccagc aacgcggcct 4020

ttttacggtt cctggccttt tgctggcctt ttgctc 4056

<210> 76
<211> 5624
<212> DNA
<213> Artificial Sequence

<220>
<223> pAd5pTP

<400> 76

ggatcccctg aggggggcccc catgggctag aggatccggc ctcggcctct gcataaataa 60
aaaaaattag tcagccatga gcttggccca ttgcatacgt tgtatccata tcataatatg 120
tacatttata ttggctcatg tccaacatta ccgccatgtt gacattgatt attgactagt 180
tattaatagt aatcaattac ggggtcatta gttcatagcc catatatgga gttccgcgtt 240
acataactta cggtaaatgg cccgcctggc tgaccgccca acgacccccg cccattgacg 300
tcaataatga cgtatgttcc catagtaacg ccaataggga ctttccattg acgtcaatgg 360
gtggagtatt tacggtaaac tgcccacttg gcagtacatc aagtgtatca tatgccaagt 420
acgcccccta ttgacgtcaa tgacggtaaa tggcccgcct ggcattatgc ccagtacatg 480
accttatggg actttcctac ttggcagtac atctacgtat tagtcatcgc tattaccatg 540
gtgatgcggt tttggcagta catcaatggg cgtggatagc ggtttgactc acggggattt 600
ccaagtctcc accccattga cgtcaatggg agtttgtttt ggcaccaaaa tcaacgggac 660
tttccaaaat gtcgtaacaa ctccgcccca ttgacgcaaa tgggcggtag gcgtgtacgg 720
tgggaggtct atataagcag agctcgttta gtgaaccgtc agatcgcctg gagacgccat 780
ccacgctgtt ttgacctcca tagaagacac cgggaccgat ccagcctccc ctcgaagctt 840
accatggcct tgagcgtcaa cgattgcgcg cgcctgaccg gccagagcgt cccgaccatg 900
gagcactttt tgccgctgcg caacatctgg aaccgcgtcc gcgactttcc gcgcgcctcc 960
accaccgccg ccggcatcac ctggatgtcc aggtacatct acggatatca tcgccttatg 1020
ttggaagatc tcgcccccgg agccccggcc accctacgct ggcccctcta ccgccagccg 1080
ccgccgcact ttttggtggg ataccagtac ctggtgcgga cttgcaacga ctacgtattt 1140
gactcgaggg cttactcgcg tctcaggtac accgagctct cgcagccggg tcaccagacc 1200
gttaactggt ccgttatggc caactgcact tacaccatca acacgggcgc ataccaccgc 1260
tttgtggaca tggatgactt ccagtctacc ctcacgcagg tgcagcaggc catattagcc 1320
gagcgcgttg tcgccgacct agccctgctt cagccgatga ggggcttcgg ggtcacacgc 1380
atgggaggaa gagggcgcca cctacggcca aactccgccg ccgccgcagc gatagatgca 1440
agagatgcag gacaagagga aggagaagaa gaagtgccgg tagaaaggct catgcaagac 1500
tactacaaag acctgcgccg atgtcaaaac gaagcctggg gcatggccga ccgcctgcgc 1560
attcagcagg ccggacccaa ggacatggtg cttctgtcga ccatccgccg tctcaagacc 1620
gcctacttta attacatcat cagcagcacc tccgccagaa acaaccccga ccgccgcccg 1680
ctgccgcccg ccacggtgct cagcctacct tgcgactgtg actggttaga cgcctttctc 1740
gagaggtttt ccgatccggt cgatgcggac tcgctcaggt ccctcggcgg cggagtacct 1800
acacaacaat tgttgagatg catcgttagc gccgtatccc tgccgcatgg cagccccccg 1860
ccaacccata accgggacat gacgggcggc gtcttccaac tgcgcccccg cgagaacggc 1920

cgcgccgtca ccgagaccat gcgccgtcgc cgcggggaga tgatcgagcg ctttgtcgac 1980
cgcctcccgg tgcgccgtcg tcgccgccgt gtcccccctc ccccaccgcc gccagaagaa 2040
gaagaagggg aggcccttat ggaagaggag attgaagaag aagaagaggc ccctgtagcc 2100
tttgagcgcg aggtgcgcga cactgtcgcc gagctcatcc gtcttctgga ggaggagtta 2160
accgtgtcgg cgcgcaactc ccagtttttc aacttcgccg tggacttcta cgaggccatg 2220
gagcgccttg aggccttggg ggatatcaac gaatccacgt tgcgacgctg ggttatgtac 2280
ttcttcgtgg cagaacacac cgccaccacc ctcaactacc tctttcagcg cctgcgaaac 2340
tacgccgtct tcgcccggca cgtggagctc aatctcgcgc aggtggtcat gcgcgcccgc 2400
gatgccgaag gggcgtggt ctacagccgc gtctggaacg agggaggcct caacgccttc 2460
tcgcagctca tggcccgcat ttccaacgac ctcgccgcca ccgtggagcg agccggacgc 2520
ggagatctcc aggaggaaga gatcgagcag ttcatggccg agatcgccta tcaagacaac 2580
tcaggagacg tgcaggagat tttgcgccag gccgccgtca acgacaccga aattgattct 2640
gtcgaactct ctttcaggtt caagctcacc gggcccgtcg tcttcacgca gaggcgccag 2700
attcaggaga tcaaccgccg cgtcgtcgcg ttcgccagca acctacgcgc gcagcaccag 2760
ctcctgcccg cgcgcggcgc cgacgtgccc ctgcccctc tcccggcggg tccggagccc 2820
cccctacctc cgggggctcg cccgcgtcac cgcttttagg aattcacccc accagtgcag 2880
gctgcctatc agaaagtggt ggctggtgtg gctaatgccc tggcccacaa gtatcactaa 2940
gctcgctttc ttgctgtcca atttctatta aaggttcctt tgttccctaa gtccaactac 3000
taaactgggg gatattatga agggccttga gcatctggat tctgcctaat aaaaaacatt 3060
tattttcatt gcaatgatgt atttaaatta tttctgaata ttttactaaa aagggaatgt 3120
gggaggtcag tgcatttaaa acataaagaa atgaagagct agttcaaacc ttgggaaaat 3180
acactatatc ttaaactcca tgaaagaagg tgaggctgca aacagctaat gcacattggc 3240
aacagcccct gatgcctatg ccttattcat ccctcagaaa aggattcaag tagaggcttg 3300
atttggaggt taaagttttg ctatgctgta ttttacatta cttattgttt tagctgtcct 3360
catgaatgtc ttttcactac ccatttgctt atcctgcatc tctcagcctt gactccactc 3420
agttctcttg cttagagata ccacctttcc cctgaagtgt tccttccatg ttttacggcg 3480
agatggtttc tcctcgcctg gccactcagc cttagttgtc tctgttgtct tatagaggtc 3540
tacttgaaga aggaaaaaca gggggcatgg tttgactgtc ctgtgagccc ttcttccctg 3600
cctcccccac tcacagtgac ccggaatccc tcgacatggc agtctagcac tagtgcggcc 3660
gcatcccgcc cctaactccg cccagttccg cccattctcc gccccatggc tgactaattt 3720
tttttattta tgcagaggcc gaggccgcct cggcctctga gctattccag aagtagtgag 3780

gaggcttttt tggaggccgg atcctgccca gatctgagca aaaggccagc aaaaggccag 3840

gaaccgtaaa aaggccgcgt tgctggcgtt tttccatagg ctccgccccc ctgacgagca 3900

tcacaaaaat cgacgctcaa gtcagaggtg gcgaaacccg acaggactat aaagatacca 3960

ggcgtttccc cctggaagct ccctcgtgcg ctctcctgtt ccgaccctgc cgcttaccgg 4020

atacctgtcc gcctttctcc cttcgggaag cgtggcgctt tctcatagct cacgctgtag 4080

gtatctcagt tcggtgtagg tcgttcgctc caagctgggc tgtgtgcacg aacccccgt 4140

tcagcccgac cgctgcgcct tatccggtaa ctatcgtctt gagtccaacc cggtaagaca 4200

cgacttatcg ccactggcag cagccactgg taacaggatt agcagagcga ggtatgtagg 4260

cggtgctaca gagttcttga agtggtggcc taactacggc tacactagaa gaacagtatt 4320

tggtatctgc gctctgctga agccagttac cttcggaaaa agagttggta gctcttgatc 4380

cggcaaacaa accaccgctg gtagcggtgg tttttttgtt tgcaagcagc agattacgcg 4440

cagaaaaaaa ggatctcaag aagatccttt gatcttttct acggggtctg acgctcagtg 4500

gaacgaaaac tcacgttaag ggattttggt catgagatta tcaaaaagga tcttcaccta 4560

gatcctttta aattaaaaat gaagttttaa atcaatctaa agtatatatg agtaaacttg 4620

gtctgacagt taccaatgct taatcagtga ggcacctatc tcagcgatct gtctatttcg 4680

ttcatccata gttgcctgac tccccgtcgt gtagataact acgatacggg agggcttacc 4740

atctggcccc agtgctgcaa tgataccgcg agacccacgc tcaccggctc cagatttatc 4800

agcaataaac cagccagccg gaagggccga gcgcagaagt ggtcctgcaa ctttatccgc 4860

ctccatccag tctattaatt gttgccggga agctagagta agtagttcgc cagttaatag 4920

tttgcgcaac gttgttgcca ttgctacagg catcgtggtg tcacgctcgt cgtttggtat 4980

ggcttcattc agctccggtt cccaacgatc aaggcgagtt acatgatccc ccatgttgtg 5040

caaaaaagcg gttagctcct tcggtcctcc gatcgttgtc agaagtaagt tggccgcagt 5100

gttatcactc atggttatgg cagcactgca taattctctt actgtcatgc catccgtaag 5160

atgcttttct gtgactggtg agtactcaac caagtcattc tgagaatagt gtatgcggcg 5220

accgagttgc tcttgcccgg cgtcaatacg ggataatacc gcgccacata gcagaacttt 5280

aaaagtgctc atcattggaa aacgttcttc ggggcgaaaa ctctcaagga tcttaccgct 5340

gttgagatcc agttcgatgt aacccactcg tgcacccaac tgatcttcag catcttttac 5400

tttcaccagc gtttctgggt gagcaaaaac aggaaggcaa aatgccgcaa aaaagggaat 5460

aagggcgaca cggaaatgtt gaatactcat actcttcctt tttcaatatt attgaagcat 5520

ttatcagggt tattgtctca tgagcggata catatttgaa tgtatttaga aaaataaaca 5580

aataggggtt ccgcgcacat ttccccgaaa agtgccacct gacg 5624

<210> 77
<211> 16392
<212> DNA
<213> Artificial Sequence

<220>
<223> pGLAd4

<400> 77
ttaattaagc agtgggctta catggcgata gctagactgg gcggttttat ggacagcaag 60

cgaaccggaa ttgccagctg gggcgccctc tggtaaggtt gggaagccct gcaaagtaaa 120

ctggatggct ttcttgccgc caaggatctg atggcgcagg ggatcaagct ctgatcaaga 180

gacaggatga ggatcgtttc gcatgattga acaagatgga ttgcacgcag gttctccggc 240

cgcttgggtg gagaggctat tcggctatga ctgggcacaa cagacaatcg gctgctctga 300

tgccgccgtg ttccggctgt cagcgcaggg gcgcccggtt ctttttgtca agaccgacct 360

gtccggtgcc ctgaatgaac tgcaagacga ggcagcgcgg ctatcgtggc tggccacgac 420

gggcgttcct tgcgcagctg tgctcgacgt tgtcactgaa gcgggaaggg actggctgct 480

attgggcgaa gtgccggggc aggatctcct gtcatctcac cttgctcctg ccgagaaagt 540

atccatcatg gctgatgcaa tgcggcggct gcatacgctt gatccggcta cctgcccatt 600

cgaccaccaa gcgaaacatc gcatcgagcg agcacgtact cggatggaag ccggtcttgt 660

cgatcaggat gatctggacg aagagcatca ggggctcgcg ccagccgaac tgttcgccag 720

gctcaaggcg agcatgcccg acggcgagga tctcgtcgtg acccatggcg atgcctgctt 780

gccgaatatc atggtggaaa atggccgctt ttctggattc atcgactgtg gccggctggg 840

tgtggcggac cgctatcagg acatagcgtt ggctacccgt gatattgctg aagagcttgg 900

cggcgaatgg gctgaccgct tcctcgtgct ttacggtatc gccgctcccg attcgcagcg 960

catcgccttc tatcgccttc ttgacgagtt cttctgacct caggttactc atatatactt 1020

tagattgatt taaaacttca tttttaattt aaaaggatct aggtgaagat cctttttgat 1080

aatctcatga ccaaaatccc ttaacgtgag ttttcgttcc actgagcgtc agaccccgta 1140

gaaaagatca aaggatcttc ttgagatcct ttttttctgc gcgtaatctg ctgcttgcaa 1200

acaaaaaaac caccgctacc agcggtggtt tgtttgccgg atcaagagct accaactctt 1260

tttccgaagg taactggctt cagcagagcg cagataccaa atactgtcct tctagtgtag 1320

ccgtagttag gccaccactt caagaactct gtagcaccgc ctacatacct cgctctgcta 1380

atcctgttac cagtggctgc tgccagtggc gataagtcgt gtcttaccgg gttggactca 1440

agacgatagt taccggataa ggcgcagcgg tcgggctgaa cggggggttc gtgcacacag 1500

cccagcttgg agcgaacgac ctacaccgaa ctgagatacc tacagcgtga gctatgagaa 1560

agcgccacgc ttcccgaagg gagaaaggcg gacaggtatc cggtaagcgg cagggtcgga 1620

acaggagagc gcacgaggga gcttccaggg ggaaacgcct ggtatcttta tagtcctgtc 1680

gggtttcgcc acctctgact tgagcgtcga tttttgtgat gctcgtcagg ggggcggagc 1740

ctatggaaaa acgccagcaa cgcggccttt ttacggttcc tggccttttg ctggcctttt 1800

gctcttaatt aaatcgattt aagggtggga aagaatatat aaggtggggg tcttatgtag 1860

tttgagctcg gctttggtta tttgatggat tgacctagtt ctcaggcagc cacctgtgtg 1920

gactaaatac acccatttct tctccattct gaaggatgtg ctgaaagccg ccttggacag 1980

tgagaaaagc aaacatggaa gcctgctgta ggatcacctt acaccatcag atacccactt 2040

cccacccccg gcttcctgaa ctttctgtaa ttactcatgc cagtgactag ggctcatgtg 2100

ggccatccca ggaaggagac tagtggctgc cattctgtcc tggtctttgt gtctcccgag 2160

gaggagtgta ggtggtgagg tcacaggcta agccaggcca tttaaggaaa gctgcctcag 2220

ctccagccca gccctctctg ccaggtggat atggaaatcc cggtttactc tctggggcct 2280

tttattggct gaaatgctca ccttggtatc tgttacacat tcccttccaa gagcagagct 2340

agataagcca ggtgtggaga caatcagctc ttgaaatctc ccagaagctg ccaaacccct 2400

aaaatcagca agagacaccc ctcatagtcc agactcagct gttttattct gcctctccct 2460

gatctctttt cagggcataa agttttccaa ggagaagaat gaaatcagta tagcaggtca 2520

ttgagaaagt atagaaaggc tcaggttgaa cttcagtgtc tgctaccaat gtgtcataaa 2580

taagtcattt tactactgtg agactcagtt tacccatttg ttgagtatga aaaaaaatca 2640

tatatatttg ttatgctata tgtatcaatg catcgagcta gactctgggg ctaaagcaat 2700

gagtaaaacg tatttggctg aacataaata tgggaggggg gtggaatgca tgaatgtaaa 2760

gaatgctggc ataagtagga ccagtgagtt gggtgtggca gtgcaccttg gagacagagc 2820

caggcggacc tctgagttca aggacagtca tcagggctcc acagagaaat cctgtccaaa 2880

aaaaaaaaaa aaaatcagtg gggaaaggca cctgccatcg aggctgaaga cctgaatttg 2940

gtccacagga tccacgtggt aggaaagaaa gaacttccaa gggttattct ctaacctcca 3000

tacttatatg tgagtgtaca cttaagtaat gtttgtttgt ttgttttgtt cctgttatgt 3060

tttgtttgtt tttaaagcca agtctggtgg ttcaggcatt taatcatagc acccagaagg 3120

cagagttcaa gggcagcctg gtctacatag gaaattgtag gcctatacag tgaaaccctg 3180

tttcaaagga agattaaagg aaaaaagtct tgcactcttc cacaccctga ccaaagtcct 3240

atagtgaaac tctggtcccc tccctcatca ctgctattgc caaactcagt ctccctctat 3300

gacccacatc atgatagcca gggctagacc gtttgtaaag ataaatcagc cttgtctgga 3360

taaataactc caggaaggag aaactgccgg ctgtaaagaa ccttagcagt cgtattcccc 3420

aaaaccacct atataagcct caagtgcccc tccacctttc acagacaccc ctcccctacg 3480

ggagagtatc tgtcctgcac tgtttgaaat aaacagtcct gtctgttgag gtcagtcttc 3540
agtctttttt tctgcttcca gtctcttaag ttgcttcaga aatttaccaa gaatcactaa 3600
ataaagccag ctgtgatggt ccacatctgt attccagtgc ttcgaaggct gaggcagagg 3660
gtctcaagtg tgaggctagc ctgggctgcc tgtggaaccc tgcctttttt tggggagcag 3720
gcttttaaaa aatatttatc acggtgaaat ataaggcagg aggattgtta gaagttcaat 3780
aaggacaatc tgattctgta gtggggttca ggctagctag ggctacatac taaacaaggt 3840
ctcaaccttg ttttagtaag aggaggagga ggaaacagaa ggaaaaaaaa gtaatttctt 3900
aggcacccag taggcctgca agtcttgttt tgaacaaatg aatggatgca tgaatcaatc 3960
tttgctacct ttcaggctct attctgtgtc cccagagcac ccccggccca ggaaagtgag 4020
cagggcacgg taggtctgct ggaatctctg attgtacttg tgagggctct gcctagcagt 4080
ctccctggaa gctgagcctg gccttagcct cttccttctc tctcagagct tctgaagcca 4140
cagaagggac gcatcaaact cggacctaga aatgaacgat tagtcttttt tagtgatttt 4200
ttttcttccg tctttatttt ttgacactca ctgggtagcc ttggctggcc cagagctccc 4260
taggttggtc agagatctgc ctgcctcctg cctgggtgtt gatatacagg gattcaccac 4320
tctatgctct tacgagctgg cttcgaactc atcaggctta gaatgtcggg tcgtctcact 4380
tatacctcct aagtgttgga ataaccagtg tgcaccaccc aagagcccct agctgggact 4440
cttgccaggc ctgtcttctc taagccttag tatccccatc tccaaaatgg gtataaccat 4500
atgcatacgt atcctaaggg catataccaa aagttcagtg aatacagtag agtatgtagg 4560
accccctgcg ggctagcagt agaagccttt ccctttccat tatacccttg gttctaaagt 4620
tggttgaaag ttcccctaag ctctctcccg cccagcaaac cagtcaaacc agtttcctca 4680
ttccccgggc ttctgtacta agcaaacact gctttgcaaa cggttgacca ggagcttgcc 4740
tttgttagag aaggaagtcc aatggtgttt tcttccttgc aatgccggga gtgggtttgc 4800
tgtgccttag aaagggttca aactgggctg gaagtagggc cccagagtag ctgggggttg 4860
gggattcagg atggcctcaa gtttgttgtt ccccaaaccc tcactttttc tagaactatt 4920
tcataataca catgcttaag gatacccatt tgttgaccca ttgcaaaatg ctctctgacc 4980
cagcttctga gactggttca ccccccatgg ataagaattg agtcatctgg tgccattcac 5040
aaatacagat taacctccct cccccaattt actgaacctc aaccaaaaaa agatccgcct 5100
gcctctgcct cccaagtgct aggattttaa agggccacgg taccacacgg atggacacaa 5160
cattcatttt cctgacagac caggagtcag gtcacaggtg tttgactagt aaacacccgg 5220
tgccaaccag ggtgtgggca tttggctatc taccctgagg cccaagatcc aagagcaccc 5280
gtggtccatg gtgctagggg acccggaaat gtagccagtg catcagagat ccagcctctg 5340
tcctcaggga agttcagaaa catctctgct tactgagact tctgcagggt atttgggaag 5400

agggggccagc acagcaggca tgatcagttt tacatgctgg gcctttgtaa gtaagaggat 5460
gtgtccaggc acagtaaagg aaagtctccc ccaggacctt gggccttttt atccactggt 5520
gaggcttttt ttctctttct gttccccccct ccccacctcc cgggctttcc agacagagtt 5580
tccctgtgta ttcattcctg gctgtcctgg aacttgctag gtagaccggg ctggcctcga 5640
actcagtatg aacaaatgtt tacttatggc tggcactgag acttcctctt caccctgact 5700
cctcacccag agcttcccct gaacagaatt ctcttggtta gccttctcaa gatcttctgt 5760
ggaaaaggca gagatagtca tggggtgtgg ctctgtggtc aggctaaacc taatgagtgt 5820
gaggccctga ctttgattcc tagcattgag gaaaagcaga aacaggaatc ttacccaatt 5880
ttcctctgcc tgttaccaga ctcaaggctg tcccgtggag cccattgccc ttcctgagaa 5940
acccacttcc tttcctgagg ctaggaaaga catccctcat ctctgtgcct caatctcatt 6000
gatttttgtg gtgttaaaat tcagttattt gtctttgaga gggctcccct cccccctttg 6060
cctttcacat cctttccctt ctttattgct atgcagagga ccctaactcc taggcaggcc 6120
ctctaccctt gagatacagc tccatcctct tgctttttaa aaacttataa ttcgagacag 6180
ggtctcactg ctacccaggc tggttttgag cttactctgc agcctcctgc atcagcccac 6240
cgccttgtac gtgagatggt gggcctgaga ctcagcctct tgaccttcac atacatctga 6300
tacgtgctat ctgaacaggc ctgtcttact cagttttctg ggtcctgttg gggggtgagg 6360
tggggagggg agatcttcgt tcaggcatgg agaggtggga aggaaaccag tccacacctc 6420
taacaagtga gttccaacta ggacttgctt agggcacata aggcagacag acgtgggaga 6480
tgtgagcggc ggggaagcga tgatgcttag atgctaccac gaggacctga tgtggtgtgc 6540
ctgaagatga acctgttccc catgagccag ctcataaatg gggaccagca ccggcccctg 6600
tgggataatt ggcctcgtcc tagataataa ttacttagct ttgtaattag gagagcttgt 6660
aagaagccga aagtgtttcg gatttatttt acctggacaa gttcaagact aaactaacac 6720
ctgttcctgt ggtttggcat gcagagccat ctgtttccaa aggaggtcct gcagcagctt 6780
tttttggggt tctcctccct gttccttccc ttctctcccc acatcgcctt ttctccgtcc 6840
agtggtcagt gtgtgtgtgt gtgtgtgtgt gtgtgtgtgt gttaatgaga cagggtctca 6900
ttatgtagcc ttgggcctgg aactcactat gtagatgtgg ctggcttcaa actcagagag 6960
gcatatctat ccacttgcct ttgcctctgc ctctcaagcg gccgcgtgct gagactaaag 7020
ggatgtgcta ctactcctgg ccagagtgat aagtactcat cctgtttaaa gttgcctggg 7080
attccctgtt tgacactgaa taaaaatcct gcccatgact tccaaggctc tgggtccctc 7140
cttcccccgc agcccatggc ttgggccttc attttaaccc cactccccac atgtgcttcg 7200
ttcagatgct ttgcttcctg attgtactca gaataccatg gcagctcggg ctgaggctgg 7260

ttttgaactc ttaatactcc tgtccgggag tagtagcatc tcaggtgtgt ccaatcacac 7320

tctatgtatt ccttgtttgt gatgtctgta tcactcaccc gtgcccttca aagctttgct 7380

aagatgttgc ctgggcaatg aggaccttct ctttttgctt tatttttctg tagcccccca 7440

cctccccgtt ttttgggtgg ttttggtagg gatgagacat taagatgttg cctgggcaat 7500

gaggaccttc tctttttgct ttatttttct gtagcccccc acctccccgt tttttgggtg 7560

gttttggtag ggatgagaca acagggcctt atgcaggtta tgcaagtacc ctattgctga 7620

tccacatccc agcctttctg gtattttcca ccattgaacc tgtcatgtgg tttgtgtatt 7680

tcctcagttc atagtcttct tccataaaaa cataaatgtg gccggacgtg gtggcgcatg 7740

cctttaatcc cagcactcgg gaggcagagg caggtggatt tctgagttcg aggccagcct 7800

ggtctacaaa gtgagttcca ggacagccag ggctatagag aaaccctgtc tcaaaaccaa 7860

aaaaaaaaaa aaaaaaaaaa aaaaaccaaa accataaatg tgaggaggtc agggtgttct 7920

gtgtttgttg gttcttgagc tgtacttcag gccgtaacaa gttcttagac ctcatctgtt 7980

gaatgagtga acggaggagt gagaggtcca gaaggtaaat atggcgctgt acagttcttt 8040

ttagaagctg tgctgtgggt tttgtttttt ctaagataaa atggggtcca gtgacatggc 8100

tcagagtaaa tgtaaatgtt gtaccttttg gcatcctggg ttttattttt ccagaaccca 8160

tttatcctgt gacttccaga tgcgcaccat accccctgta aacccccccc cccccaacac 8220

acacacacac caaactagca aacagataca gggctgtcag cgtgtgagca catgaggaag 8280

ggatgctatg ttctcaggcc ctgtgtttct tcttggcacc ccctggtttc tctgagccct 8340

gcacagctac agtgtaacct tacctaattg tcttgttaca aagaagtttg ctgcccagag 8400

tctaaaggtc agatgagtca cttgggcctc acacttctgg gtgtatggaa gcaccagatg 8460

gaagtctcaa ggtacacccg acaccggctg cctttccgtc gtcaggcagc acattcctga 8520

agcaggctct ctcagctgtg ccctctcaac cttcatcctg cagcccatta attgttcaag 8580

gccttctagt tcttggcagg tctgagcatc ttggaaggag ctagaagaga gatgtagaaa 8640

gctcaaagac aaaaatgcaa agctaccccc atcagatacc agctcttggg tctattgacc 8700

agccaatatc cattgatagt ggagttctac atccatatgg aagatacacc attctctttc 8760

atggacagga gtgccaaatc agaatacagg acattacatc tttttgagac aaggtcttac 8820

catgtagcct ttgtagacca gctatgtaga ccatgctgcc ctggaaccca cagagatttg 8880

ctagcttctg cctcccaagt gctaagatta gtgacacaca cctccactta cacagtacac 8940

acccaggaaa aaagtaaaac ataaataaaa agcaaaaaat tgttgtgaaa tagctcagta 9000

ttagagtcct tgtctaagat atatgggctc caggcttgcc catccatcta ttttctgaga 9060

ctggattttt ctgtgtagcc ctggctgtcc tgggtcttac tttgtagacc aggctgacct 9120

tgaactcaga gatccacctg cctctgcctc tggagctctg gggttaaagg tagaccccac 9180

cagccatggg cttaagccca gtatgggttt aaaaacatgg aaccgattgg actttgcata 9240
aagttagatt ggatcctgtg ggtgctgacc tcccatgctt tcaagcactt attggtagca 9300
ttcactgaac aagcctgagg gcgggaacag ccctggcatc tttcaaaggg tatccggttc 9360
aaaccagaat cgaatgctct tttcctagcc accccacctc ttggcttgtt gcctaaggaa 9420
aatggctcta gcctggttct ccagtgttct catggcagtc tggagagatt gtccatttgt 9480
atcacaacct tttgggcgct gcccttggtg acccgctgct gatgtggaca ccagcaagac 9540
ttgagactgc agacctcctc ccccacagat ttctcacaat gcccgtgtgg gagtgtttga 9600
aaagggaaca agctgaccaa cctgtttgtg ggaaagtcgt ggctttccta tcagaaaacg 9660
tcacactctg agtcaggagt gggacatacc tgggtacttt aacaggtggg gcagggtcct 9720
gagtcataga gaggtctcac ctgggaaggt ttctctttca aagagtgaat caaccatgac 9780
atcacccatc tggatctaca ccctttcaag ccgtctctct ccttagcaaa caggaatcta 9840
aaagaggaat aataaggtga aagcaaactg gaaataggac agaacagaca agggaaaaag 9900
ttaaagagaa agcttaagag acgcagacac agacgtttac atgcaaacag ggggtccata 9960
aaaacaaaac cggaagccgt aatatatgta catggcctgt aagattagaa acaaatgtcc 10020
tggcacaaca ttatgggaca acttcccaag ctgctgttga gtttgttttg tgttggccgg 10080
catgggccct taaactgagt ggtgtgtttc tctcttagag aatttttcat gaactagttt 10140
ttcatttgca agtggttagt agtttgagat agcttctagg ttaggggtgg ggccgtgtgt 10200
ccactttact cagctctaat gtcccccgtt tggtgcagac ccctgcagac cctatgcgtc 10260
ctgccacagt ctctgagttc atgcctgcat tgttcaaatt gtgtttcctt gatgtcctcc 10320
aatctcctct ttgctgggct ccctggggcc cccaaggtga gggatttggc agaggtattc 10380
attccattta gggttgattg ttccaaggtc cctcactgta cgtgttattt gtcccttctc 10440
cctggcttcc tgctcaggag ttctgcagac atggctggtg tttagtagct tcttggtgaa 10500
gttcaggtag ccagtttcat gaccacccag ctctcaggcc tgactaagcc cgtggagaaa 10560
gcccataaaa taaagggggt gaacttctaa tctacatctg aaaggagaac ccctgtctac 10620
atctacctgt taccctagcc atcggggaga ctgaggcaga aggatctagc ctggaaattt 10680
agggtgagtc tgggcagcat taggagacta tttcacagtg aagaaggaag gaactcactc 10740
acacccttgg tgcacctcac aggtctctgg gctcagattg gagtctttgg ttttgaaatt 10800
tcctcatcat cggccgcctc agctggtttt tcttctggag tctgttacgc cttctcccct 10860
ttcccccctt ctcttgctaa tcctttcgct tgaacaatag gagtccttaa aacctgggag 10920
ggaagtaaat acctttcaga tgagaccctg tcttaagagg gggaaaaaaa aaacaaaaaa 10980
accctaggga ccgggtcacc aagtgtcagg ttaatcagca gatgtcaaga tgactcctga 11040

gtgcttgctc tttcttcagc tggtgaaatt acagcggcaa catagaatga cagtcagcag 11100

ggagggagga gagggcctgg gtggccaaat aaacttataa attgtgagag aaattaatga 11160

atgtctaagt taatgcagaa acggagagac atactatatt catgaactaa aagacttaat 11220

attgtgaagg tatactttct ttccacataa atttgtagtc aatatgttca ccccaaaaaa 11280

gctgtttgtt aacttgccaa cctcattcta aaatgtatat agaagcccaa aagacaataa 11340

caaaaatatt cttgtagaac aaaatgggaa agaatgttcc actaaatatc aagatttaga 11400

gcaaagcatg agatgtgtgg ggatagacag tgaggctgat aaaatagagt agagctcaga 11460

aacagaccca ttgatatatg taagtgacct atgaaaaaaa tatggcattt tacaatggga 11520

aaatgatgat ctttttcttt tttagaaaaa cagggaaata tatttatatg taaaaaataa 11580

aagggaaccc atatgtcata ccatacacac aaaaaaattc cagtgaatta taagtctaaa 11640

tggagaaggc aaaactttaa atcttttaga aaataatata gaagcatgcc atcatgactt 11700

cagtgtagag aaaaatttct tatgactcaa agtcctaacc acaaagaaaa gattgttaat 11760

tagattgcat gaatattaag acttattttt aaaattaaaa aaccattaag aaaagtcagg 11820

ccatagaatg acagaaaata tttgcaacac cccagtaaag agaattgtaa tatgcagatt 11880

ataaaaagaa gtcttacaaa tcagtaaaaa ataaaactag acaaaaattt gaacagatga 11940

aagagaaact ctaaataatc attacacatg agaaactcaa tctcagaaat cagagaacta 12000

tcattgcata tacactaaat tagagaaata ttaaaaggct aagtaacatc tgtggcaata 12060

ttgatggtat ataaccttga tatgatgtga tgagaacagt actttacccc atgggcttcc 12120

tccccaaacc cttaccccag tataaatcat gacaaatata ctttaaaaac cattacccta 12180

tatctaacca gtactcctca aaactgtcaa ggtcatcaaa aataagaaaa gtctgaggaa 12240

ctgtcaaaac taagaggaac ccaaggagac atgagaatta tatgtaatgt ggcattctga 12300

atgagatccc agaacagaaa aagaacagta gctaaaaaac taatgaaata taaataaagt 12360

ttgaacttta gtttttttta aaaaagagta gcattaacac ggcaaagcca ttttcatatt 12420

tttcttgaac attaagtaca agtctataat taaaaatttt ttaaatgtag tctggaacat 12480

tgccagaaac agaagtacaa cagctatctg tgctgtcgcc taactatcca tagctgattg 12540

gtctaaaatg agatacatca acgctcctcc atgtttttttg ttttcttttt aaatgaaaaa 12600

ctttattttt taagaggagt ttcaggttca tagcaaaatt gagaggaagg tacattcaag 12660

ctgaggaagt tttcctctat tcctagttta ctgagagatt gcatcatgaa tgggtgttaa 12720

attttgtcaa atgctttttc tgtgtctatc aatatgacca tgtgattttc ttctttaacc 12780

tgttgatggg acaaattacg ttaattgatt ttcaaacgtt gaaccaccct tacatatctg 12840

gaataaattc tacttggttg tggtgtatat tttttgatac attcttggat tctttttgct 12900

aatattttgt tgaaaatgtt tgtatctttg ttcatgagag atattggtct gttgttttct 12960

tttcttgtaa tgtcattttc tagttccggt attaaggtaa tgctggccta gttgaatgat 13020
ttaggaagta ttccctctgc ttctgtcttc tgaaagagat tgtagaaagt tgatacaatt 13080
tttttttctt taaatatgtc gacgcactgg ctttacaggg gccgtctgct ttgtctgtag 13140
atatggtgat aggagtctga tttagaaact tccttgttct cctcttcccg aatccttcct 13200
cctgaacaat aggaatacct aagtgcacaa gtgcaggtgc ccaggaggcc ctgagacaac 13260
ccccccctccc cagctgcgag ccttctccgg tggtgctggg aatcgaactg gagtcctctg 13320
caagaacagt atgtgctctt aaccactgca ccatctcttc agccccctct agttctttag 13380
tttgtacata aattagcaag ttcagggaat gcagaagaac tggccagggg tcagagccag 13440
actggaaaca tttttcacca aacaatagct acagtgttca tggcgtgcct ccattgaata 13500
ctaaactagc tgttgatgtg tgactttgat ttttaatctt tgcaccaact ttagttacta 13560
tatagataat tccatttcca aatgatatat agtggaagct tgtaaaagct gcttactatc 13620
ccagggtccc cagccgagat ctacatgata aagctactct caacccagcc tacattcccc 13680
ctccctgtgc tcagccctac ctggggttgt tcactttcaa gctcccagga aggaacctga 13740
gcgctccctg cccccatgca gtccatttct atctctggtc ctttgggtac ttggagcctc 13800
aagctcctga ctgctaatga agcctgtaac agaagggcat tacttactgc ccgccttgtg 13860
ctcccttgaa atctggtcac ctggaggtgg aagtgggcag tgcttccgtg cttccgtgct 13920
tccgctttct cccagctcct tttaatcact attttttttt tttcttttcc aaattactac 13980
ctaggagttt tctagtaatc actttggtgg gagtgggagg gggagtggag gcagttaact 14040
ctgtgagtgc caaggacagt tgttggacgg tggtagtgat ggaggggagg gtgttcacgg 14100
tgagggaact ctgtggagtt acgtagctgg caacaatggg gagagacgga gccgcaccac 14160
cacagacgct ttgcatgagc aactattttg ttttctgtag atgttttcca aaccttaaac 14220
tctgttaagt ctgaaactgg gaggggaacc tctccaccaa aatctaaata tggatcagag 14280
ataaggaaac ttccatagtc tccagctccc tccctgactc agtggcctct gtgaccaact 14340
ccacgtgact ctagtggaca ccttcctggt ggtttggctt tttttttttt tccccatttc 14400
atcttttaaa tggagttttc catgaataag aatataaaac tgcgggggct ggtgagatgg 14460
atcagcaggt aagagcacag aggtcctgag ttcaaatccc agcaaccact tggtggctca 14520
ccatcggtaa cgagatctga ctccctcttc tagagtgtct gaagatagct acagtgtact 14580
tacatataat aaataaacaa atcttaaaaa aaagaatata aaactgcttc cttcccaaca 14640
ggccttggat agttttgtct gtgggaacag cttcccatag gaaaggtgct tttgctactt 14700
ccgaccgtca cacactcctc ctctgcaccc cgtgagggcc tcccaggagt tttgtgttgg 14760
tcacaagcct ccccatgtcc caagggggtc tggagaggga tctgaggtgg tggaaagtgc 14820

tggggtgcca agttatcaga caatgggcac attgagaatg caggtggggc caggcctgtg 14880

tgggctggag cagctgtcgg agcctcaggg ggtgcagctc atgcgcagag ggaagaggca 14940

gtctctctgc tggctgagag gggagggcca cagtaagcaa aggagggggg tgatcccccc 15000

ctcacaagat tccagagcaa agggatgttt gaacgatgac tcacatgaca ggggcggggt 15060

ctgtgagcaa acttcctgtt ggaagcttgt tcttggttcc tgactgtcct tcccaggtca 15120

tagagactgt ctgcaatagg gctggggcct gttaggatgc catcgaggtg ggaaatcctg 15180

gagagcttgg agtagccagc aggccctctt ccctctcctg ccccaggcta gctggaaacc 15240

cgcgaggagg aagggtgtgt tgttccctat cccatggaga ggcttgcatg gtcagaccag 15300

gcctggtgtg acctctgggg ctgctctgtg actgccctgt tctgtgactg cccttctccc 15360

tcccagccct ccccttccat ttgcttctag attcgtctag gagcccatct cagcaaatgg 15420

cagcatcctc gcttaccaga catcttgttt gtttgtttgt ttgttcattc attctgggcc 15480

tgaagatgga gagctgtggt aggggagagc tcagaggctg gggcagcct taagggcaca 15540

gccaggacgg agcctctgag caaagggcag tgtgtgtaga gtcaggtagg tggtgggcag 15600

caggagacag gaggagcaca gaggtaggtg gcactgtaca gaggccctgg caccattttg 15660

tgacaaggac tctcagaccc ctctagccta ttcccccacc cccacccca ttacaagcag 15720

tagggtctta atagctaatg gcttggacac cagcctctgg ctgctctggt tcccagctgg 15780

tcagagccct tgttctcctt tgggttctga tccaggtggc acagggtggg ggccctgatt 15840

cacattcttg atagtgccgc taggttccct gctaccagct ggcacattct gtcttccttt 15900

ccagcaggta cttggctggg cttcctgccc agctgctaga cctactgtta ggtcagctca 15960

ttggatgctt cctccaggaa gccttctcta atctccattc ccttggcttc ttttcaagag 16020

cagaagttca ctggcttctc ctgtttcccc agagtagcac ctggtgtctc aagtactcgc 16080

taagcagtta ggagaggtgt gaactgggcc tcagggaccc cagagacagg aggggaggtc 16140

attacaggaa agtcggattc ttttcctggc tttgttctta taaccatcgt gtgtgtgtgt 16200

gtgtgtgtgt gtgtgtgtgt gtgtgtgtgt gtgtgtgtgt acgtgtgtgt acgtgtgtgt 16260

gtgtgtacat gcgtgcgcgc cctacgtcac ccgccccgtt cccacgcccc gcgccacgtc 16320

acaaactcca ccccctcatt atcatattgg cttcaatcca aaataaggta tattattgat 16380

gatgttacat cg 16392

<210> 78
<211> 30119
<212> DNA
<213> Artificial Sequence

<220>
<223> pGLAd4_coHTT.HTTmshR1/3

<400> 78
ttaattaagc agtgggctta catggcgata gctagactgg gcggttttat ggacagcaag 60

cgaaccggaa ttgccagctg gggcgccctc tggtaaggtt gggaagccct gcaaagtaaa 120

ctggatggct ttcttgccgc caaggatctg atggcgcagg ggatcaagct ctgatcaaga 180

gacaggatga ggatcgtttc gcatgattga acaagatgga ttgcacgcag gttctccggc 240

cgcttgggtg gagaggctat tcggctatga ctgggcacaa cagacaatcg gctgctctga 300

tgccgccgtg ttccggctgt cagcgcaggg gcgcccggtt ctttttgtca agaccgacct 360

gtccggtgcc ctgaatgaac tgcaagacga ggcagcgcgg ctatcgtggc tggccacgac 420

gggcgttcct tgcgcagctg tgctcgacgt tgtcactgaa gcgggaaggg actggctgct 480

attgggcgaa gtgccggggc aggatctcct gtcatctcac cttgctcctg ccgagaaagt 540

atccatcatg gctgatgcaa tgcggcggct gcatacgctt gatccggcta cctgcccatt 600

cgaccaccaa gcgaaacatc gcatcgagcg agcacgtact cggatggaag ccggtcttgt 660

cgatcaggat gatctggacg aagagcatca ggggctcgcg ccagccgaac tgttcgccag 720

gctcaaggcg agcatgcccg acggcgagga tctcgtcgtg acccatggcg atgcctgctt 780

gccgaatatc atggtggaaa atggccgctt ttctggattc atcgactgtg gccggctggg 840

tgtggcggac cgctatcagg acatagcgtt ggctacccgt gatattgctg aagagcttgg 900

cggcgaatgg gctgaccgct tcctcgtgct ttacggtatc gccgctcccg attcgcagcg 960

catcgccttc tatcgccttc ttgacgagtt cttctgacct caggttactc atatatactt 1020

tagattgatt taaaacttca tttttaattt aaaaggatct aggtgaagat cctttttgat 1080

aatctcatga ccaaaatccc ttaacgtgag ttttcgttcc actgagcgtc agaccccgta 1140

gaaaagatca aaggatcttc ttgagatcct ttttttctgc gcgtaatctg ctgcttgcaa 1200

acaaaaaaac caccgctacc agcggtggtt tgtttgccgg atcaagagct accaactctt 1260

tttccgaagg taactggctt cagcagagcg cagataccaa atactgtcct tctagtgtag 1320

ccgtagttag gccaccactt caagaactct gtagcaccgc ctacatacct cgctctgcta 1380

atcctgttac cagtggctgc tgccagtggc gataagtcgt gtcttaccgg gttggactca 1440

agacgatagt taccggataa ggcgcagcgg tcgggctgaa cggggggttc gtgcacacag 1500

cccagcttgg agcgaacgac ctacaccgaa ctgagatacc tacagcgtga gctatgagaa 1560

agcgccacgc ttcccgaagg gagaaaggcg gacaggtatc cggtaagcgg cagggtcgga 1620

acaggagagc gcacgaggga gcttccaggg ggaaacgcct ggtatcttta tagtcctgtc 1680

gggtttcgcc acctctgact tgagcgtcga tttttgtgat gctcgtcagg ggggcggagc 1740

ctatggaaaa acgccagcaa cgcggccttt ttacggttcc tggccttttg ctggcctttt 1800

gctcttaatt aaatcgcatc atcaataata taccttattt tggattgaag ccaatatgat 1860

aatgaggggg tggagtttgt gacgtggcgc ggggcgtggg aacggggcgg gtgacgtagt 1920

agtgtggcgg aagtgtgatg ttgcaagtgt ggcggaacac atgtaagcga cggatgtggc 1980

aaaagtgacg tttttggtgt gcgccggtgt acacaggaag tgacaatttt cgcgcggttt 2040

taggcggatg ttgtagtaaa tttgggcgta accgagtaag atttggccat tttcgcggga 2100

aaactgaata agaggaagtg aaatctgaat aattttgtgt tactcatagc gcgtaatatt 2160

tgtctagggc cgcggggact ttgaccgttt acgtggagac tcgcccaggt gtttttctca 2220

ggtgttttcc gcgttccggg tcaaagttgg cgttttatta ttatagtcag ctgacgtgta 2280

gtgtcgagtg ttgacattga ttattgacta gttattaata gtaatcaatt acggggtcat 2340

tagttcatag cccatatatg gagttccgcg ttacataact tacggtaaat ggcccgcctg 2400

gctgaccgcc caacgacccc cgcccattga cgtcaataat gacgtatgtt cccatagtaa 2460

cgccaatagg gactttccat tgacgtcaat gggtggagta tttacggtaa actgcccact 2520

tggcagtaca tcaagtgtat catatgccaa gtacgccccc tattgacgtc aatgacggta 2580

aatggcccgc ctggcattgt gcccagtaca tgaccttatg ggactttcct acttggcagt 2640

acatctacgt attagtcatc gctattacca tggtcgaggt gagccccacg ttctgcttca 2700

ctctccccat ctcccccccc tccccacccc caattttgta tttatttatt ttttaattat 2760

tttgtgcagc gatgggggcg gggggggggg ggggcgcgc gccaggcggg gcggggcggg 2820

gcgaggggcg gggcggggcg aggcggagag gtgcggcggc agccaatcag agcggcgcgc 2880

tccgaaagtt tccttttatg gcgaggcggc ggcggcggcg gccctataaa aagcgaagcg 2940

cgcggcgggc gatagggaga cccaagctgg tgagtttggg gacccttgat tgttctttct 3000

ttttcgctat tgtaaaattc atgttatatg gagggggcaa agttttcagg gtgttgttta 3060

gaacgggaag atgtcccttg tatcaccatg gaccctcatg ataattttgt ttctttcact 3120

ttctactctg ttgacaacca ttgtctcctc ttattttctt ttcattttct gtaacttttt 3180

cgttaaactt tagcttgcat ttgtaacgaa tttttaaatt cacttttgtt tatttgtcag 3240

attgtaagta ctttctctaa tcactttttt ttcaaggcaa tcagggtata ttatattgta 3300

cttcagcaca gttttagaga acaattgtta taattaaatg ataaggtaga atatttctgc 3360

atataaattc tggctggcgt ggaaatattc ttattggtag aaacaactac atcctggtca 3420

tcatcctgcc tttctcttta tggttacaat gatatacact gtttgagatg aggataaaat 3480

actctgagtc caaaccgggc ccctctgcta accatgttca tgccttcttc tttttcctac 3540

aggggaccca agcttggtac cgagctcgga tccaccatgg ctactctgga aaaactgatg 3600

aaggcatttg agtctctgaa gagcttccag caacaacagc aacaacagca acagcaacag 3660

cagcaacaac agcagcaaca gcaacaacaa cagccacctc ctcctccacc tccacctcca 3720

ccccctcagc tgccccagcc accacctcag gcacagcctc tgctgccaca gccccagcca 3780

ccccctcctc ctcctccacc acctccagga ccagcagtgg ccgaggagcc tctgcacaga 3840

ccaaagaagg agctgagcgc cacaaagaag gatagggtga accactgtct gaccatctgc 3900

gagaatatcg tggcccagtc tgtgaggaac agccctgagt ttcagaagct gctgggcatc 3960

gccatggagc tgttcctgct gtgctctgac gatgccgaga gcgatgtgcg gatggtggcc 4020

gacgagtgtc tgaacaaagt gatcaaggcc ctgatggaca gcaacctgcc cagactgcag 4080

ctggagctgt acaaggagat caagaagaat ggagcaccta ggtccctgag ggccgccctg 4140

tggcgcttcg ccgagctggc ccacctggtg cggcctcaga agtgtagacc atatctggtg 4200

aacctgctgc cctgcctgac acggacctcc aagagacctg aggagtctgt gcaggagacc 4260

ctggcagcag cagtgcctaa gatcatggcc agctttggca atttcgccaa cgacaatgag 4320

atcaaggtgc tgctgaaggc ctttatcgcc aacctgaaga gctcctctcc aaccatcagg 4380

agaacagcag caggaagcgc cgtgtccatc tgccagcaca gcaggcgcac ccagtacttc 4440

tattcctggc tgctgaatgt gctgctggga ctgctggtgc cagtggagga tgagcactcc 4500

acactgctga tcctgggcgt gctgctgacc ctgcggtacc tggtgcctct gctgcagcag 4560

caggtgaagg acacatccct gaagggctct tttggcgtga ccagaaagga gatggaggtg 4620

tccccctctg ccgagcagct ggtgcaggtg tatgagctga ccctgcacca cacacagcac 4680

caggaccaca acgtggtgac aggcgccctg gagctgctgc agcagctgtt ccggacccca 4740

cctccagagc tgctgcagac actgaccgca gtgggaggaa tcggacagct gaccgcagca 4800

aaggaggaga gcggaggccg gagcagatcc ggatctatcg tggagctgat cgcaggagga 4860

ggaagctcct gcagccccgt gctgtccagg aagcagaagg gcaaggtgct gctgggagag 4920

gaggaggccc tggaggacga tagcgagtcc cgctctgacg tgagcagctc cgccctgaca 4980

gccagcgtga aggacgagat ctccggagag ctggcagcat ctagcggcgt gtctacccct 5040

ggaagcgccg gccacgacat catcacagag cagccaagaa gccagcacac cctgcaggcc 5100

gattccgtgg acctggcctc ttgtgatctg acctcctctg ccacagatgg cgacgaggag 5160

gacatcctga gccacagctc ctctcaggtg tccgccgtgc cttctgatcc agccatggat 5220

ctgaatgacg gaacccaggc cagctcccca atctccgact ctagccagac cacaaccgag 5280

ggccctgata gcgccgtgac accatccgac tcctctgaga tcgtgctgga tggcaccgac 5340

aaccagtacc tgggactgca gatcggacag ccacaggatg aggacgagga ggcaaccggc 5400

atcctgcctg atgaggccag cgaggccttt cggaatagct ccatggcact gcagcaggca 5460

cacctgctga agaacatgtc tcactgcaga cagcctagcg attctagcgt ggacaagttc 5520

gtgctgaggg atgaggcaac agagccaggc gaccaggaga ataagccttg ccggatcaag 5580

ggcgacatcg gccagtctac cgacgatgac agcgccccac tggtgcactg cgtgcggctg 5640

ctgagcgcct ccttcctgct gaccggaggc aagaacgtgc tggtgcccga tcgggacgtg 5700

agagtgtctg tgaaggccct ggccctgagc tgcgtgggag cagccgtggc cctgcaccct 5760

gagtctttct ttagcaagct gtacaaggtg ccactggata caaccgagta ccccgaggag 5820

cagtatgtga gcgacatcct gaattatatc gatcacggcg acccacaggt gcggggagca 5880

accgccatcc tgtgcggcac actgatctgc tctatcctgt ctaggagccg ctttcacgtg 5940

ggcgactgga tgggcacaat caggacactg accggcaaca ccttctccct ggccgattgc 6000

atccccctgc tgcgcaagac actgaaggac gagtcctctg tgacctgtaa gctggcctgc 6060

acagccgtga gaaattgcgt gatgtccctg tgcagctcct cttactctga gctgggcctg 6120

cagctgatca tcgatgtgct gaccctgaga aacagctcct attggctggt gaggaccgag 6180

ctgctggaga cactggccga gatcgacttt agactggtga gcttcctgga ggccaaggcc 6240

gagaatctgc acaggggagc acaccactac accggactgc tgaagctgca ggagcgcgtg 6300

ctgaacaacg tggtcatcca cctgctgggc gatgaggacc cacgggtgag acacgtggca 6360

gcagccagcc tgatcaggct ggtgcctaag ctgttttaca agtgtgatca gggacaggca 6420

gacccagtgg tggcagtggc aagggatcag tctagcgtgt atctgaagct gctgatgcac 6480

gagacccagc ccccttccca cttctccgtg tctacaatca ccaggatcta ccgcggctat 6540

aacctgctgc cctccatcac agacgtgacc atggagaaca atctgtctag agtgatcgcc 6600

gccgtgtccc acgagctgat cacctctaca acccgcgccc tgacatttgg ctgctgtgag 6660

gccctgtgcc tgctgtccac agccttcccc gtgtgcatct ggtctctggg atggcactgc 6720

ggagtgccac cactgagcgc ctccgatgag tccaggaagt cttgtaccgt gggcatggcc 6780

accatgatcc tgacactgct gtcctctgcc tggtttccac tggatctgag cgcccaccag 6840

gacgcactga tcctggcagg aaatctgctg gcagcaagcg ccccaaagtc cctgcggagc 6900

tcctgggcat ccgaggagga ggcaaaccca gcagcaacca agcaggagga agtgtggcca 6960

gccctgggcg acagagccct ggtgccaatg gtggagcagc tgttcagcca cctgctgaaa 7020

gtgatcaaca tctgcgcaca cgtgctggat gacgtggcac ctggaccagc catcaaggcc 7080

gccctgcctt ccctgacaaa ccctccatct ctgagcccaa tccggagaaa gggcaaggag 7140

aaggagccag gagagcaggc ctctgtgcca ctgagcccta agaagggaag cgaggcctcc 7200

gccgcatcta ggcagagcga cacctccggc ccagtgacaa ccagcaagtc tagctccctg 7260

ggcagctttt accacctgcc ctcctatctg aagctgcacg atgtgctgaa ggccacccac 7320

gccaattaca aggtgacact ggacctgcag aactccaccg agaagtttgg cggcttcctg 7380

aggtctgccc tggatgtgct gagccagatc ctggagctgg ccacactgca ggacatcggc 7440

aagtgcgtgg aggagatcct gggctatctg aagtcttgct tcagccgcga gccaatgatg 7500

gccaccgtgt gcgtgcagca gctgctgaag accctgtttg gcacaaatct ggccagccag 7560
ttcgatggcc tgtctagcaa cccatccaag tctcagggaa gggcacagag actgggatcc 7620
tctagcgtga ggcctggcct gtaccactat tgctttatgg ccccatacac acacttcacc 7680
caggccctgg cagacgcctc cctgcgcaat atggtgcagg ccgagcagga gaacgataca 7740
tctggctggt ttgacgtgct gcagaaggtg agcacccagc tgaagacaaa cctgacctcc 7800
gtgacaaaga atagagccga taagaacgcc atccacaatc acatcaggct gttcgagccc 7860
ctggtcatca aggccctgaa gcagtataca accacaacct gcgtgcagct gcagaagcag 7920
gtgctggacc tgctggccca gctggtgcag ctgagggtga attactgtct gctggattcc 7980
gaccaggtgt ttatcggctt cgtgctgaag cagtttgagt atatcgaagt gggccagttc 8040
cgggagtctg aggccatcat ccctaacatc ttcttttcc tggtgctgct gtcctacgag 8100
agatatcact ctaagcagat catcggcatc ccaaagatca tccagctgtg cgacggcatc 8160
atggcaagcg gaaggaaggc agtgacccac gcaatccccg ccctgcagcc tatcgtgcac 8220
gatctgtttg tgctgcgcgg cacaaataag gcagacgcag gcaaggagct ggagacccag 8280
aaggaggtgg tggtgtccat gctgctgcgc ctgatccagt accaccaggt gctggagatg 8340
ttcatcctgg tgctgcagca gtgtcacaag gagaacgagg ataagtggaa gaggctgtcc 8400
cgccagatcg ccgacatcat cctgcccatg ctggccaagc agcagatgca catcgattct 8460
cacgaggccc tgggcgtgct gaataccctg tttgagatcc tggcaccatc ctctctgagg 8520
cctgtggaca tgctgctgag atctatgttc gtgaccccta acacaatggc cagcgtgtcc 8580
acagtgcagc tgtggatcag cggcatcctg gccatcctgc gggtgctgat ctctcagagc 8640
accgaggata tcgtgctgag cagaatccag gagctgtcct tttctccata cctgatctcc 8700
tgcaccgtga tcaataggct gcgcgatggc gacagcacat ccaccctgga ggagcacagc 8760
gagggcaagc agatcaagaa cctgcccgag gagacatttt cccggttcct gctgcagctg 8820
gtgggcatcc tgctggagga catcgtgacc aagcagctga aggtggagat gagcgagcag 8880
cagcacacat tctattgtca ggagctgggc accctgctga tgtgcctgat ccacatcttt 8940
aagtccggaa tgttcaggag gatcaccgca gcagcaacaa ggctgtttag aagcgatggc 9000
tgcggcggct ccttctacac actggactct ctgaatctgc gggccagaag catgatcaca 9060
acccaccccg ccctggtgct gctgtggtgt cagatcctgc tgctggtgaa ccacaccgac 9120
tataggtggt gggcagaggt gcagcagaca ccaaagaggc acagcctgag ctccaccaag 9180
ctgctgtccc ctcagatgtc tggagaggag gaggattccg acctggcagc aaagctgggc 9240
atgtgcaaca gagagatcgt gcggagaggc gccctgatcc tgttctgtga ttacgtgtgc 9300
cagaatctgc acgacagcga gcacctgacc tggctgatcg tgaaccacat ccaggatctg 9360
atctctctga gccacgagcc ccctgtgcag gactttatct ccgccgtgca ccggaacagc 9420

gccgcatctg gcctgttcat ccaggccatc cagagcagat gcgagaacct gtccacaccc 9480

accatgctga agaagaccct gcagtgtctg gagggcatcc acctgtccca gtctggcgcc 9540

gtgctgacac tgtatgtgga taggctgctg tgcaccccct ttagggtgct ggcccgcatg 9600

gtggacatcc tggcctgtag gcgcgtggag atgctgctgg ccgccaatct gcagtctagc 9660

atggcccagc tgcctatgga ggagctgaac cgcatccagg agtacctgca gtcctctgga 9720

ctggcacaga ggcaccagcg cctgtattct ctgctggata ggttccgcct gagcaccatg 9780

caggacagcc tgtccccatc tccacccgtg agctcccacc cactggatgg cgacggacac 9840

gtgtccctgg agacagtgtc tcccgataag gactggtacg tgcacctggt gaagagccag 9900

tgctggacca ggagcgattc cgccctgctg gagggagcag agctggtgaa tcgcatccct 9960

gccgaggaca tgaacgcctt tatgatgaac agcgagttca atctgtccct gctggcccca 10020

tgtctgtctc tgggcatgag cgagatctcc ggaggacaga agagcgccct gtttgaggcc 10080

gccagggagg tgacactggc ccgcgtgtcc ggaaccgtgc agcagctgcc agcagtgcac 10140

cacgtgtttc agccagagct gccagcagag cctgcagcat actggtctaa gctgaacgat 10200

ctgttcggcg acgccgccct gtaccagagc ctgcctacac tggcaagagc cctggcacag 10260

tatctggtgg tggtgagcaa gctgccatcc cacctgcacc tgcctccaga gaaggagaag 10320

gatatcgtga agttcgtggt ggccaccctg gaggccctga gctggcacct gatccacgag 10380

cagatcccac tgtccctgga tctgcaggca ggactggact gctgttgcct ggccctgcag 10440

ctgccaggac tgtggtctgt ggtgtctagc accgagtttg tgacacacgc ctgtagcctg 10500

atctactgcg tgcacttcat cctggaggca gtggcagtgc agcctggaga gcagctgctg 10560

tccccagagc ggagaacaaa tacccccaag gccatctctg aggaggagga ggaggtggac 10620

ccaaacacac agaatcccaa gtatatcacc gcagcatgcg agatggtggc agagatggtc 10680

gagtctctgc agagcgtgct ggccctggga cacaagcgga acagcggagt gcctgccttc 10740

ctgacaccac tgctgcgcaa tatcatcatc tctctggccc ggctgcccct ggtgaacagc 10800

tacaccagag tgcccccctct ggtgtggaag ctgggatggt ccccaaagcc tggaggcgat 10860

tttggcaccg ccttcccaga gatccccgtg gagtttctgc aggagaagga ggtgtttaag 10920

gagttcatct atcgcatcaa tacactgggc tggaccagcc ggacacagtt cgaggagacc 10980

tgggccacac tgctgggcgt gctggtgacc cagcctctgg tcatggagca ggaggagtcc 11040

ccaccagagg aggacaccga gaggacacag atcaacgtgc tggccgtgca ggccatcaca 11100

tctctggtgc tgagcgccat gaccgtgcca gtggcaggaa atcctgccgt gagctgcctg 11160

gagcagcagc caagaaacaa gcccctgaag gccctggata cacggttcgg cagaaagctg 11220

tccatcatcc ggggcatcgt ggagcaggag atccaggcca tggtgtctaa gagagagaac 11280

atcgccaccc accacctgta ccaggcctgg gacccagtgc cctctctgag cccagcaaca 11340

accggcgccc tgatcagcca cgagaagctg ctgctgcaga tcaatcctga gagggagctg 11400

ggctccatgt cttataagct gggccaggtg tccatccaca gcgtgtggct gggcaactcc 11460

atcacacccc tgcgcgagga ggagtgggat gaagaggagg aggaggaggc agacgcacct 11520

gcaccatcct ctcctccaac ctcccctgtg aacagcagga agcaccgcgc cggcgtggat 11580

atccacagct gttcccagtt cctgctggag ctgtacagca gatggatcct gcctagctcc 11640

tctgccaggc gcacaccagc catcctgatc tctgaggtgg tgcgcagcct gctggtggtg 11700

tccgacctgt ttaccgagcg gaaccagttc gagctgatgt acgtgacact gaccgagctg 11760

cggagagtgc acccaagcga ggatgagatc ctggcccagt atctggtgcc agcaacatgc 11820

aaggcagcag ccgtgctggg aatggacaag gcagtggcag agcccgtgag caggctgctg 11880

gagagcaccc tgcgcagctc ccacctgcca tccagggtgg gcgccctgca cggcgtgctg 11940

tacgtgctgg agtgtgatct gctggatgac accgccaagc agctgatccc cgtgatctcc 12000

gactatctgc tgtctaatct gaagggcatc gcccactgcg tgaacatcca cagccagcag 12060

cacgtgctgg tcatgtgcgc cacagccttt tacctgatcg agaactatcc actggacgtg 12120

ggccccgagt tctctgccag catcatccag atgtgcggcg tgatgctgtc cggctctgag 12180

gagagcaccc catccatcat ctaccactgt gcactgaggg gactggagag actgctgctg 12240

agcgagcagc tgtccaggct ggatgccgag agcctggtga agctgtccgt ggacagagtg 12300

aatgtgcaca gcccacacag ggcaatggcc gccctgggac tgatgctgac ctgcatgtat 12360

acaggcaagg agaaggtgtc tccaggaagg accagcgatc caaaccctgc agcacctgac 12420

agcgagtccg tgatcgtggc catggagcgc gtgtccgtgc tgtttgatcg gatcagaaag 12480

ggcttcccat gtgaggcaag ggtggtggca cgcatcctgc cacagtttct ggatgacttt 12540

ttcccccctc aggacatcat gaacaaagtg atcggcgagt tcctgtctaa ccagcagcca 12600

tacccccagt ttatggccac cgtggtgtat aaggtgttcc agaccctgca cagcacaggc 12660

cagtctagca tggtgcgcga ttgggtcatg ctgtctctga gcaacttcac ccagagggca 12720

cctgtggcaa tggcaacctg gtccctgtct tgctttttcg tgagcgcctc cacatctcct 12780

tgggtggccg ccatcctgcc acacgtgatc agccggatgg gcaagctgga gcaggtggat 12840

gtgaacctgt tttgtctggt ggccaccgac ttctacagac accagatcga ggaggagctg 12900

gacaggaggg cattccagtc cgtgctggag gtggtggccg ccccaggctc cccataccat 12960

aggctgctga cctgtctgag gaatgtgcat aaagtgacta cctgttgatg aattctgcag 13020

atatccatca cactggcggc cgctcgagca tgcatctaga gggccctatt caattctttg 13080

tagaggtttt acttgcttta aaaaacctcc cacacctccc cctgaacctg aaacataaaa 13140

tgaatgcaat tgttgttgtt aacttgttta ttgcagctta taatggttac aaataaagca 13200

atagcatcac aaatttcaca aataaagcat ttttttcact gcattctagt tgtggtttgt 13260
ccaaactcat caatgtatct tatcatgtct ggatcatcga tccataactt cgtataatgt 13320
atgctatacg aagttatcca gatctcgatt taagggtggg aaagaatata taaggtgggg 13380
gtcttatgta gtttgagctc ggctttggtt atttgatgga ttgacctagt tctcaggcag 13440
ccacctgtgt ggactaaata cacccatttc ttctccattc tgaaggatgt gctgaaagcc 13500
gccttggaca gtgagaaaag caaacatgga agcctgctgt aggatcacct tacaccatca 13560
gatacccact tcccaccccc ggcttcctga actttctgta attactcatg ccagtgacta 13620
gggctcatgt gggccatccc aggaaggaga ctagtggctg ccattctgtc ctggtctttg 13680
tgtctcccga ggaggagtgt aggtggtgag gtcacaggct aagccaggcc atttaaggaa 13740
agctgcctca gctccagccc agccctctct gccaggtgga tatggaaatc ccggtttact 13800
ctctggggcc ttttattggc tgaaatgctc accttggtat ctgttacaca ttcccttcca 13860
agagcagagc tagataagcc aggtgtggag acaatcagct cttgaaatct cccagaagct 13920
gccaaacccc taaaatcagc aagagacacc cctcatagtc cagactcagc tgttttattc 13980
tgcctctccc tgatctcttt tcagggcata aagttttcca aggagaagaa tgaaatcagt 14040
atagcaggtc attgagaaag tatagaaagg ctcaggttga acttcagtgt ctgctaccaa 14100
tgtgtcataa ataagtcatt ttactactgt gagactcagt ttacccattt gttgagtatg 14160
aaaaaaaatc atatatattt gttatgctat atgtatcaat gcatcgagct agactctggg 14220
gctaaagcaa tgagtaaaac gtatttggct gaacataaat atgggagggg ggtggaatgc 14280
atgaatgtaa agaatgctgg cataagtagg accagtgagt tgggtgtggc agtgcacctt 14340
ggagacagag ccaggcggac ctctgagttc aaggacagtc atcagggctc cacagagaaa 14400
tcctgtccaa aaaaaaaaaa aaaaatcagt ggggaaaggc acctgccatc gaggctgaag 14460
acctgaattt ggtccacagg atccacgtgg taggaaagaa agaacttcca agggttattc 14520
tctaacctcc atacttatat gtgagtgtac acttaagtaa tgtttgtttg tttgttttgt 14580
tcctgttatg ttttgtttgt ttttaaagcc aagtctggtg gttcaggcat ttaatcatag 14640
cacccagaag gcagagttca agggcagcct ggtctacata ggaaattgta ggcctataca 14700
gtgaaaccct gtttcaaagg aagattaaag gaaaaaagtc ttgcactctt ccacaccctg 14760
accaaagtcc tatagtgaaa ctctggtccc ctccctcatc actgctattg ccaaactcag 14820
tctccctcta tgacccacat catgatagcc agggctagac cgtttgtaaa gataaatcag 14880
ccttgtctgg ataaataact ccaggaagga gaaactgccg gctgtaaaga accttagcag 14940
tcgtattccc caaaaccacc tatataagcc tcaagtgccc ctccaccttt cacagacacc 15000
cctcccctac gggagagtat ctgtcctgca ctgtttgaaa taaacagtcc tgtctgttga 15060

ggtcagtctt cagtcttttt ttctgcttcc agtctcttaa gttgcttcag aaatttacca 15120

agaatcacta aataaagcca gctgtgatgg tccacatctg tattccagtg cttcgaaggc 15180

tgaggcagag ggtctcaagt gtgaggctag cctgggctgc ctgtggaacc ctgccttttt 15240

ttggggagca ggcttttaaa aaatatttat cacggtgaaa tataaggcag gaggattgtt 15300

agaagttcaa taaggacaat ctgattctgt agtggggttc aggctagcta gggctacata 15360

ctaaacaagg tctcaacctt gttttagtaa gaggaggagg aggaaacaga aggaaaaaaa 15420

agtaatttct taggcaccca gtaggcctgc aagtcttgtt ttgaacaaat gaatggatgc 15480

atgaatcaat ctttgctacc tttcaggctc tattctgtgt ccccagagca cccccggccc 15540

aggaaagtga gcagggcacg gtaggtctgc tggaatctct gattgtactt gtgagggctc 15600

tgcctagcag tctccctgga agctgagcct ggccttagcc tcttccttct ctctcagagc 15660

ttctgaagcc acagaaggga cgcatcaaac tcggacctag aaatgaacga ttagtctttt 15720

ttagtgattt tttttcttcc gtctttattt tttgacactc actgggtagc cttggctggc 15780

ccagagctcc ctaggttggt cagagatctg cctgcctcct gcctgggtgt tgatatacag 15840

ggattcacca ctctatgctc ttacgagctg gcttcgaact catcaggctt agaatgtcgg 15900

gtcgtctcac ttatacctcc taagtgttgg aataaccagt gtgcaccacc caagagcccc 15960

tagctgggac tcttgccagg cctgtcttct ctaagcctta gtatccccat ctccaaaatg 16020

ggtataacca tatgcatacg tatcctaagg gcatatacca aaagttcagt gaatacagta 16080

gagtatgtag gacccccctgc gggctagcag tagaagcctt tccctttcca ttataccctt 16140

ggttctaaag ttggttgaaa gttcccctaa gctctctccc gcccagcaaa ccagtcaaac 16200

cagtttcctc attccccggg cttctgtact aagcaaacac tgctttgcaa acggttgacc 16260

aggagcttgc ctttgttaga gaaggaagtc caatggtgtt ttcttccttg caatgccggg 16320

agtgggtttg ctgtgcctta gaaagggttc aaactgggct ggaagtaggg ccccagagta 16380

gctgggggtt ggggattcag gatggcctca agtttgttgt tccccaaacc ctcacttttt 16440

ctagaactat ttcataatac acatgcttaa ggatacccat ttgttgaccc attgcaaaat 16500

gctctctgac ccagcttctg agactggttc accccccatg gataagaatt gagtcatctg 16560

gtgccattca caaatacaga ttaacctccc tcccccaatt tactgaacct caaccaaaaa 16620

aagatccgcc tgcctctgcc tcccaagtgc taggatttta aagggccacg gtacgacatt 16680

gattattgac tagttattaa tagtaatcaa ttacggggtc attagttcat agcccatata 16740

tggagttccg cgttacataa cttacggtaa atggcccgcc tggctgaccg cccaacgacc 16800

cccgcccatt gacgtcaata atgacgtatg ttcccatagt aacgccaata gggactttcc 16860

attgacgtca atgggtggac tatttacggt aaactgccca cttggcagta catcaagtgt 16920

atcatatgcc aagtacgccc cctattgacg tcaatgacgg taaatggccc gcctggcatt 16980

atgcccagta catgacctta tgggactttc ctacttggca gtacatctac gtattagtca 17040

tcgctattac catggtgatg cggttttggc agtacatcaa tgggcgtgga tagcggtttg 17100

actcacgggg atttccaagt ctccacccca ttgacgtcaa tgggagtttg ttttggcacc 17160

aaaatcaacg ggactttcca aaatgtcgta acaactccgc cccattgacg caaatgggcg 17220

gtaggcgtgt acggtgggag gtctatataa gcagagctct ctggctaact agagaaccca 17280

ctgcttactg gcttatcgaa attaatacga ctcactatag ggagacccaa gcttggtacc 17340

gagctcggat cctgctgttg acagtgagcg agaccgtgtg aatcattgtc tatagtgaag 17400

ccacagatgt atagacaatg attcacacgg tcgtgcctac tgcctcggag aattccagca 17460

cactggcggc cgctcgagca tgcatctaga gggccctatt ctatagtgtc acctaaatgc 17520

tagagctcgc tgatcagcct cgactgtgcc ttctagttgc cagccatctg ttgtttgccc 17580

ctcccccgtg ccttccttga ccctggaagg tgccactccc actgtccttt cctaataaaa 17640

tgaggaaatt gcatcgcatt gtctgagtag gtgtcattct attctggggg gtggggtggg 17700

gcaggacagc aagggggagg attgggaaga caatagcagg catgctgggg atgcggtggg 17760

ctctatgggt accacacgga tggacacaac attcattttc ctgacagacc aggagtcagg 17820

tcacaggtgt ttgactagta aacacccggt gccaaccagg gtgtgggcat ttggctatct 17880

accctgaggc ccaagatcca agagcacccg tggtccatgg tgctagggga cccggaaatg 17940

tagccagtgc atcagagatc cagcctctgt cctcagggaa gttcagaaac atctctgctt 18000

actgagactt ctgcagggta tttgggaaga ggggccagca cagcaggcat gatcagtttt 18060

acatgctggg cctttgtaag taagaggatg tgtccaggca cagtaaagga aagtctcccc 18120

caggaccttg ggccttttta tccactggtg aggctttttt tctctttctg ttcccccctc 18180

cccacctccc gggctttcca gacagagttt ccctgtgtat tcattcctgg ctgtcctgga 18240

acttgctagg tagaccgggc tggcctcgaa ctcagtatga acaaatgttt acttatggct 18300

ggcactgaga cttcctcttc accctgactc ctcacccaga gcttcccctg aacagaattc 18360

tcttggttag ccttctcaag atcttctgtg gaaaaggcag agatagtcat ggggtgtggc 18420

tctgtggtca ggctaaacct aatgagtgtg aggccctgac tttgattcct agcattgagg 18480

aaaagcagaa acaggaatct tacccaattt tcctctgcct gttaccagac tcaaggctgt 18540

cccgtggagc ccattgccct tcctgagaaa cccacttcct ttcctgaggc taggaaagac 18600

atccctcatc tctgtgcctc aatctcattg atttttgtgg tgttaaaatt cagttatttg 18660

tctttgagag ggctcccctc ccccctttgc ctttcacatc ctttcccttc tttattgcta 18720

tgcagaggac cctaactcct aggcaggccc tctacccttg agatacagct ccatcctctt 18780

gctttttaaa aacttataat tcgagacagg gtctcactgc tacccaggct ggttttgagc 18840

ttactctgca gcctcctgca tcagcccacc gccttgtacg tgagatggtg ggcctgagac 18900
tcagcctctt gaccttcaca tacatctgat acgtgctatc tgaacaggcc tgtcttactc 18960
agttttctgg gtcctgttgg ggggtgaggt ggggagggga gatcttcgtt caggcatgga 19020
gaggtgggaa ggaaaccagt ccacacctct aacaagtgag ttccaactag gacttgctta 19080
gggcacataa ggcagacaga cgtgggagat gtgagcggcg gggaagcgat gatgcttaga 19140
tgctaccacg aggacctgat gtggtgtgcc tgaagatgaa cctgttcccc atgagccagc 19200
tcataaatgg ggaccagcac cggcccctgt gggataattg gcctcgtcct agataataat 19260
tacttagctt tgtaattagg agagcttgta agaagccgaa agtgtttcgg atttatttta 19320
cctggacaag ttcaagacta aactaacacc tgttcctgtg gtttggcatg cagagccatc 19380
tgtttccaaa ggaggtcctg cagcagcttt ttttggggtt ctcctccctg ttccttccct 19440
tctctcccca catcgccttt tctccgtcca gtggtcagtg tgtgtgtgtg tgtgtgtgtg 19500
tgtgtgtgtg ttaatgagac agggtctcat tatgtagcct tgggcctgga actcactatg 19560
tagatgtggc tggcttcaaa ctcagagagg catatctatc cacttgcctt tgcctctgcc 19620
tctcaagcgg ccgcgtgctg agactaaagg gatgtgctac tactcctggc cagagtgata 19680
agtactcatc ctgtttaaag ttgcctggga ttccctgttt gacactgaat aaaaatcctg 19740
cccatgactt ccaaggctct gggtccctcc ttcccccgca gcccatggct tgggccttca 19800
ttttaacccc actccccaca tggacattga ttattgacta gttattaata gtaatcaatt 19860
acggggtcat tagttcatag cccatatatg gagttccgcg ttacataact tacggtaaat 19920
ggcccgcctg gctgaccgcc caacgacccc cgcccattga cgtcaataat gacgtatgtt 19980
cccatagtaa cgccaatagg gactttccat tgacgtcaat gggtggacta tttacggtaa 20040
actgcccact tggcagtaca tcaagtgtat catatgccaa gtacgccccc tattgacgtc 20100
aatgacggta aatggcccgc ctggcattat gcccagtaca tgaccttatg ggactttcct 20160
acttggcagt acatctacgt attagtcatc gctattacca tggtgatgcg gttttggcag 20220
tacatcaatg ggcgtggata gcggtttgac tcacggggat ttccaagtct ccaccccatt 20280
gacgtcaatg ggagtttgtt ttggcaccaa aatcaacggg actttccaaa atgtcgtaac 20340
aactccgccc cattgacgca aatgggcggt aggcgtgtac ggtgggaggt ctatataagc 20400
agagctctct ggctaactag agaacccact gcttactggc ttatcgaaat taatacgact 20460
cactataggg agacccaagc ttggtaccga gctcggatcc tgctgttgac agtgagcgag 20520
gatacctgaa atcctgcttt tagtgaagcc acagatgtaa aagcaggatt tcaggtatcc 20580
gtgcctactg cctcggagaa ttccagcaca ctggcggccg ctcgagcatg catctagagg 20640
gccctattct atagtgtcac ctaaatgcta gagctcgctg atcagcctcg actgtgcctt 20700
ctagttgcca gccatctgtt gtttgcccct cccccgtgcc ttccttgacc ctggaaggtg 20760

ccactcccac tgtcctttcc taataaaatg aggaaattgc atcgcattgt ctgagtaggt 20820

gtcattctat tctggggggt ggggtggggc aggacagcaa gggggaggat tgggaagaca 20880

atagcaggca tgctggggat gcggtgggct ctatggcatg tgcttcgttc agatgctttg 20940

cttcctgatt gtactcagaa taccatggca gctcgggctg aggctggttt tgaactctta 21000

atactcctgt ccgggagtag tagcatctca ggtgtgtcca atcacactct atgtattcct 21060

tgtttgtgat gtctgtatca ctcacccgtg cccttcaaag ctttgctaag atgttgcctg 21120

ggcaatgagg accttctctt tttgctttat ttttctgtag ccccccacct ccccgttttt 21180

tgggtggttt tggtagggat gagacattaa gatgttgcct gggcaatgag gaccttctct 21240

ttttgcttta tttttctgta gccccccacc tccccgtttt ttgggtggtt ttggtaggga 21300

tgagacaaca gggccttatg caggttatgc aagtacccta ttgctgatcc acatcccagc 21360

ctttctggta ttttccacca ttgaacctgt catgtggttt gtgtatttcc tcagttcata 21420

gtcttcttcc ataaaaacat aaatgtggcc ggacgtggtg gcgcatgcct ttaatcccag 21480

cactcgggag gcagaggcag gtggatttct gagttcgagg ccagcctggt ctacaaagtg 21540

agttccagga cagccagggc tatagagaaa ccctgtctca aaaccaaaaa aaaaaaaaaa 21600

aaaaaaaaaa aaccaaaacc ataaatgtga ggaggtcagg gtgttctgtg tttgttggtt 21660

cttgagctgt acttcaggcc gtaacaagtt cttagacctc atctgttgaa tgagtgaacg 21720

gaggagtgag aggtccagaa ggtaaatatg gcgctgtaca gttcttttta gaagctgtgc 21780

tgtgggtttt gtttttttcta agataaaatg gggtccagtg acatggctca gagtaaatgt 21840

aaatgttgta ccttttggca tcctgggttt tatttttcca gaacccattt atcctgtgac 21900

ttccagatgc gcaccatacc ccctgtaaac cccccccccc ccaacacaca cacacaccaa 21960

actagcaaac agatacaggg ctgtcagcgt gtgagcacat gaggaaggga tgctatgttc 22020

tcaggccctg tgtttcttct tggcaccccc tggtttctct gagccctgca cagctacagt 22080

gtaaccttac ctaattgtct tgttacaaag aagtttgctg cccagagtct aaaggtcaga 22140

tgagtcactt gggcctcaca cttctgggtg tatggaagca ccagatggaa gtctcaaggt 22200

acacccgaca ccggctgcct ttccgtcgtc aggcagcaca ttcctgaagc aggctctctc 22260

agctgtgccc tctcaacctt catcctgcag cccattaatt gttcaaggcc ttctagttct 22320

tggcaggtct gagcatcttg gaaggagcta gaagagagat gtagaaagct caaagacaaa 22380

aatgcaaagc taccccccatc agataccagc tcttgggtct attgaccagc caatatccat 22440

tgatagtgga gttctacatc catatggaag atacaccatt ctctttcatg gacaggagtg 22500

ccaaatcaga atacaggaca ttacatcttt ttgagacaag gtcttaccat gtagcctttg 22560

tagaccagct atgtagacca tgctgccctg gaacccacag agatttgcta gcttctgcct 22620

cccaagtgct aagattagtg acacacacct ccacttacac agtacacacc caggaaaaaa 22680

gtaaaacata aataaaaagc aaaaaattgt tgtgaaatag ctcagtatta gagtccttgt 22740

ctaagatata tgggctccag gcttgcccat ccatctattt tctgagactg gatttttctg 22800

tgtagccctg gctgtcctgg gtcttacttt gtagaccagg ctgaccttga actcagagat 22860

ccacctgcct ctgcctctgg agctctgggg ttaaaggtag accccaccag ccatgggctt 22920

aagcccagta tgggtttaaa aacatggaac cgattggact ttgcataaag ttagattgga 22980

tcctgtgggt gctgacctcc catgctttca agcacttatt ggtagcattc actgaacaag 23040

cctgagggcg ggaacagccc tggcatcttt caaagggtat ccggttcaaa ccagaatcga 23100

atgctctttt cctagccacc ccacctcttg gcttgttgcc taaggaaaat ggctctagcc 23160

tggttctcca gtgttctcat ggcagtctgg agagattgtc catttgtatc acaacctttt 23220

gggcgctgcc cttggtgacc cgctgctgat gtggacacca gcaagacttg agactgcaga 23280

cctcctcccc cacagatttc tcacaatgcc cgtgtgggag tgtttgaaaa gggaacaagc 23340

tgaccaacct gtttgtggga aagtcgtggc tttcctatca gaaaacgtca cactctgagt 23400

caggagtggg acatacctgg gtactttaac aggtggggca gggtcctgag tcatagagag 23460

gtctcacctg ggaaggtttc tctttcaaag agtgaatcaa ccatgacatc acccatctgg 23520

atctacaccc tttcaagccg tctctctcct tagcaaacag gaatctaaaa gaggaataat 23580

aaggtgaaag caaactggaa ataggacaga acagacaagg gaaaaagtta aagagaaagc 23640

ttaagagacg cagacacaga cgtttacatg caaacagggg gtccataaaa acaaaaccgg 23700

aagccgtaat atatgtacat ggcctgtaag attagaaaca aatgtcctgg cacaacatta 23760

tgggacaact tcccaagctg ctgttgagtt tgttttgtgt tggccggcat gggcccttaa 23820

actgagtggt gtgtttctct cttagagaat ttttcatgaa ctagtttttc atttgcaagt 23880

ggttagtagt ttgagatagc ttctaggtta ggggtggggc cgtgtgtcca ctttactcag 23940

ctctaatgtc ccccgtttgg tgcagacccc tgcagaccct atgcgtcctg ccacagtctc 24000

tgagttcatg cctgcattgt tcaaattgtg tttccttgat gtcctccaat ctcctctttg 24060

ctgggctccc tggggccccc aaggtgaggg atttggcaga ggtattcatt ccatttaggg 24120

ttgattgttc caaggtccct cactgtacgt gttatttgtc ccttctccct ggcttcctgc 24180

tcaggagttc tgcagacatg gctggtgttt agtagcttct tggtgaagtt caggtagcca 24240

gtttcatgac cacccagctc tcaggcctga ctaagcccgt ggagaaagcc cataaaataa 24300

agggggtgaa cttctaatct acatctgaaa ggagaacccc tgtctacatc tacctgttac 24360

cctagccatc ggggagactg aggcagaagg atctagcctg gaaatttagg gtgagtctgg 24420

gcagcattag gagactattt cacagtgaag aaggaaggaa ctcactcaca cccttggtgc 24480

acctcacagg tctctgggct cagattggag tctttggttt tgaaatttcc tcatcatcgg 24540

ccgcctcagc tggtttttct tctggagtct gttacgcctt ctcccctttc cccccttctc 24600

ttgctaatcc tttcgcttga acaataggag tccttaaaac ctgggaggga agtaaatacc 24660

tttcagatga gaccctgtct taagaggggg aaaaaaaaaa caaaaaaacc ctagggaccg 24720

ggtcaccaag tgtcaggtta atcagcagat gtcaagatga ctcctgagtg cttgctcttt 24780

cttcagctgg tgaaattaca gcggcaacat agaatgacag tcagcaggga gggaggagag 24840

ggcctgggtg gccaaataaa cttataaatt gtgagagaaa ttaatgaatg tctaagttaa 24900

tgcagaaacg gagagacata ctatattcat gaactaaaag acttaatatt gtgaaggtat 24960

actttctttc cacataaatt tgtagtcaat atgttcaccc caaaaaagct gtttgttaac 25020

ttgccaacct cattctaaaa tgtatataga agcccaaaag acaataacaa aaatattctt 25080

gtagaacaaa atgggaaaga atgttccact aaatatcaag atttagagca aagcatgaga 25140

tgtgtgggga tagacagtga ggctgataaa atagagtaga gctcagaaac agacccattg 25200

atatatgtaa gtgacctatg aaaaaaatat ggcattttac aatgggaaaa tgatgatctt 25260

tttcttttt agaaaaacag ggaaatatat ttatatgtaa aaaataaaag ggaacccata 25320

tgtcatacca tacacacaaa aaaattccag tgaattataa gtctaaatgg agaaggcaaa 25380

actttaaatc ttttagaaaa taatatagaa gcatgccatc atgacttcag tgtagagaaa 25440

aatttcttat gactcaaagt cctaaccaca aagaaaagat tgttaattag attgcatgaa 25500

tattaagact tatttttaaa attaaaaaac cattaagaaa agtcaggcca tagaatgaca 25560

gaaaatattt gcaacacccc agtaaagaga attgtaatat gcagattata aaaagaagtc 25620

ttacaaatca gtaaaaaata aaactagaca aaaatttgaa cagatgaaag agaaactcta 25680

aataatcatt acacatgaga aactcaatct cagaaatcag agaactatca ttgcatatac 25740

actaaattag agaaatatta aaaggctaag taacatctgt ggcaatattg atggtatata 25800

accttgatat gatgtgatga gaacagtact ttaccccatg ggcttcctcc ccaaaccctt 25860

accccagtat aaatcatgac aaatatactt taaaaaccat taccctatat ctaaccagta 25920

ctcctcaaaa ctgtcaaggt catcaaaaat aagaaaagtc tgaggaactg tcaaaactaa 25980

gaggaaccca aggagacatg agaattatat gtaatgtggc attctgaatg agatcccaga 26040

acagaaaaag aacagtagct aaaaaactaa tgaaatataa ataaagtttg aactttagtt 26100

ttttttaaaa aagagtagca ttaacacggc aaagccattt tcatattttt cttgaacatt 26160

aagtacaagt ctataattaa aaatttttta aatgtagtct ggaacattgc cagaaacaga 26220

agtacaacag ctatctgtgc tgtcgcctaa ctatccatag ctgattggtc taaaatgaga 26280

tacatcaacg ctcctccatg ttttttgttt tctttttaaa tgaaaaactt tatttttaa 26340

gaggagtttc aggttcatag caaaattgag aggaaggtac attcaagctg aggaagtttt 26400

cctctattcc tagtttactg agagattgca tcatgaatgg gtgttaaatt ttgtcaaatg 26460
ctttttctgt gtctatcaat atgaccatgt gattttcttc tttaacctgt tgatgggaca 26520
aattacgtta attgattttc aaacgttgaa ccacccttac atatctggaa taaattctac 26580
ttggttgtgg tgtatatttt ttgatacatt cttggattct ttttgctaat attttgttga 26640
aaatgtttgt atctttgttc atgagagata ttggtctgtt gttttctttt cttgtaatgt 26700
cattttctag ttccggtatt aaggtaatgc tggcctagtt gaatgattta ggaagtattc 26760
cctctgcttc tgtcttctga aagagattgt agaaagttga tacaattttt ttttctttaa 26820
atatgtcgac gcactggctt tacaggggcc gtctgctttg tctgtagata tggtgatagg 26880
agtctgattt agaaacttcc ttgttctcct cttcccgaat ccttcctcct gaacaatagg 26940
aatacctaag tgcacaagtg caggtgccca ggaggccctg agacaacccc ccctccccag 27000
ctgcgagcct tctccggtgg tgctgggaat cgaactggag tcctctgcaa gaacagtatg 27060
tgctcttaac cactgcacca tctcttcagc cccctctagt tctttagttt gtacataaat 27120
tagcaagttc agggaatgca gaagaactgg ccaggggtca gagccagact ggaaacattt 27180
ttcaccaaac aatagctaca gtgttcatgg cgtgcctcca ttgaatacta aactagctgt 27240
tgatgtgtga ctttgatttt taatctttgc accaacttta gttactatat agataattcc 27300
atttccaaat gatatatagt ggaagcttgt aaaagctgct tactatccca gggtccccag 27360
ccgagatcta catgataaag ctactctcaa cccagcctac attccccctc cctgtgctca 27420
gccctacctg ggttgttca ctttcaagct cccaggaagg aacctgagcg ctccctgccc 27480
ccatgcagtc catttctatc tctggtcctt tgggtacttg gagcctcaag ctcctgactg 27540
ctaatgaagc ctgtaacaga agggcattac ttactgcccg ccttgtgctc ccttgaaatc 27600
tggtcacctg gaggtggaag tgggcagtgc ttccgtgctt ccgtgcttcc gctttctccc 27660
agctcctttt aatcactatt tttttttttt cttttccaaa ttactaccta ggagttttct 27720
agtaatcact ttggtgggag tgggaggggg agtggaggca gttaactctg tgagtgccaa 27780
ggacagttgt tggacggtgg tagtgatgga ggggagggtg ttcacggtga gggaactctg 27840
tggagttacg tagctggcaa caatggggag agacggagcc gcaccaccac agacgctttg 27900
catgagcaac tattttgttt tctgtagatg ttttccaaac cttaaactct gttaagtctg 27960
aaactgggag gggaacctct ccaccaaaat ctaaatatgg atcagagata aggaaacttc 28020
catagtctcc agctccctcc ctgactcagt ggcctctgtg accaactcca cgtgactcta 28080
gtggacacct tcctggtggt ttggcttttt tttttttcc ccatttcatc ttttaaatgg 28140
agttttccat gaataagaat ataaaactgc gggggctggt gagatggatc agcaggtaag 28200
agcacagagg tcctgagttc aaatcccagc aaccacttgg tggctcacca tcggtaacga 28260
gatctgactc cctcttctag agtgtctgaa gatagctaca gtgtacttac atataataaa 28320

taaacaaatc ttaaaaaaaa gaatataaaa ctgcttcctt cccaacaggc cttggatagt 28380

tttgtctgtg ggaacagctt cccataggaa aggtgctttt gctacttccg accgtcacac 28440

actcctcctc tgcaccccgt gagggcctcc caggagtttt gtgttggtca caagcctccc 28500

catgtcccaa gggggtctgg agagggatct gaggtggtgg aaagtgctgg ggtgccaagt 28560

tatcagacaa tgggcacatt gagaatgcag gtggggccag gcctgtgtgg gctggagcag 28620

ctgtcggagc ctcagggggt gcagctcatg cgcagaggga agaggcagtc tctctgctgg 28680

ctgagagggg agggccacag taagcaaagg agggggtga tcccccctc acaagattcc 28740

agagcaaagg gatgtttgaa cgatgactca catgacaggg gcggggtctg tgagcaaact 28800

tcctgttgga agcttgttct tggttcctga ctgtccttcc caggtcatag agactgtctg 28860

caatagggct ggggcctgtt aggatgccat cgaggtggga aatcctggag agcttggagt 28920

agccagcagg ccctcttccc tctcctgccc caggctagct ggaaacccgc gaggaggaag 28980

ggtgtgttgt tccctatccc atggagaggc ttgcatggtc agaccaggcc tggtgtgacc 29040

tctggggctg ctctgtgact gccctgttct gtgactgccc ttctccctcc cagccctccc 29100

cttccatttg cttctagatt cgtctaggag cccatctcag caaatggcag catcctcgct 29160

taccagacat cttgtttgtt tgtttgtttg ttcattcatt ctgggcctga agatggagag 29220

ctgtggtagg ggagagctca gaggctgggg gcagccttaa gggcacagcc aggacggagc 29280

ctctgagcaa agggcagtgt gtgtagagtc aggtaggtgg tgggcagcag gagacaggag 29340

gagcacagag gtaggtggca ctgtacagag gccctggcac cattttgtga caaggactct 29400

cagacccctc tagcctattc ccccaccccc acccccatta caagcagtag ggtcttaata 29460

gctaatggct tggacaccag cctctggctg ctctggttcc cagctggtca gagcccttgt 29520

tctcctttgg gttctgatcc aggtggcaca gggtgggggc cctgattcac attcttgata 29580

gtgccgctag gttccctgct accagctggc acattctgtc ttcctttcca gcaggtactt 29640

ggctgggctt cctgcccagc tgctagacct actgttaggt cagctcattg gatgcttcct 29700

ccaggaagcc ttctctaatc tccattccct tggcttcttt tcaagagcag aagttcactg 29760

gcttctcctg tttccccaga gtagcacctg gtgtctcaag tactcgctaa gcagttagga 29820

gaggtgtgaa ctgggcctca gggaccccag agacaggagg ggaggtcatt acaggaaagt 29880

cggattcttt tcctggcttt gttcttataa ccatcgtgtg tgtgtgtgtg tgtgtgtgtg 29940

tgtgtgtgtg tgtgtgtgtg tgtgtgtacg tgtgtgtacg tgtgtgtgtg tgtacatgcg 30000

tgcgcgccct acgtcacccg ccccgttccc acgccccgcg ccacgtcaca aactccaccc 30060

cctcattatc atattggctt caatccaaaa taaggtatat tattgatgat gttacatcg 30119

<210> 79

<211> 46
<212> DNA
<213> Artificial Sequence

<220>
<223> pGLAd3_5139Acc_CMV_AF_F

<400> 79
atttttaaagg gccacggtac gacattgatt attgactagt tattaa 46

<210> 80
<211> 46
<212> DNA
<213> Artificial Sequence

<220>
<223> pGLAd3_5139Acc_BGH_AF_R

<400> 80
tgtgtccatc cgtgtggtac ccatagagcc caccgcatcc ccagca 46

<210> 81
<211> 46
<212> DNA
<213> Artificial Sequence

<220>
<223> pGLAd3_7189Pci_BGH_AF_R

<400> 81
ttaaccccac tccccacatg gacattgatt attgactagt tattaa 46

<210> 82
<211> 46
<212> DNA
<213> Artificial Sequence

<220>
<223> pGLAd3_7189Pci_BGH_AF_R

<400> 82
gcatctgaac gaagcacatg ccatagagcc caccgcatcc ccagca 46

## Description

### Technical Field

The present disclosure relates to a helper plasmid-based gutless adenovirus (GLAd) production system, a gutless adenovirus production method using same, a gutless adenovirus produced using same, and a use of gutless adenovirus produced using same.

### Background Art

Gene therapy is emerging as an effective treatment option for various inherited genetic diseases. Gutless adenovirus (hereinafter referred to as "GLAd"), also known as helper-dependent adenovirus (hereinafter referred to "HDAd"), has many notable characteristics as a gene delivery vector for gene therapy, including broad tropism, high infectivity, a large transgene cargo capacity, and an absence of integration into the host genome. Additionally, GLAd ensures long-term transgene expression in host organisms due to its minimal immunogenicity, since it was constructed following the deletion of all the genes from an adenovirus.

However, unavoidable contamination of the highly immunogenic adenovirus used as a helper for GLAd production has remained as a problem that hampers the clinical use of GLAd for the treatment of inherited genetic diseases.

Gene therapy that recovers the normal function of a target gene by replacing a corresponding defective gene is emerging as an effective therapeutic option for various inherited genetic diseases, including Leber's congenital amaurosis (LCA) and spinal muscular atrophy (SMA). This particular type of gene therapy depends upon a vehicle referred to as a vector for delivery of a gene of normal functionality to target tissues or organs.

In gene therapy for the treatment of inherited genetic diseases, two types of gene delivery, that is, ex-vivo gene delivery and in-vivo gene delivery can be considered. Ex vivo gene delivery is a cell therapy product-based approach that utilizes genetically engineered cells. In this approach, vector safety can be continuously monitored at different stages prior to the implantation of genetically manipulated cells into the patient's body. In contrast, in-vivo gene delivery directly transfers a therapeutic transgene to patient tissues or organs as the final destined location. Thus, nothing is more important than vector safety in this approach. In addition to being safe, in-vivo gene delivery vectors should ensure long-term transgene expression to sustain the therapeutic efficacy of the delivered transgene for a long period of time.

Currently, the most commonly used in-vivo gene delivery vector for the clinical treatment of inherited genetic diseases is adeno-associated virus (AAV). The safety of AAV has been well established through a wide variety of clinical trials. AAV exhibits broad tropism for infection and ensures long-term transgene expression in various tissues and organs. These characteristics have drawn considerable attention toward AAV as an in-vivo gene delivery vector for various inherited genetic diseases. Nevertheless, AAV has two notable drawbacks: potential for insertional mutagenesis and a low packaging capacity for the transgene. AAV mainly remains as an episome, but can randomly integrate into the host genome although at a low frequency. This characteristic raises concern about insertional mutagenesis. AAV also possesses a small transgene cargo capacity (~4.5 kb) and can neither deliver large genes such as huntingtin (9.4 kb) or dystrophin (11 kb), nor can multiple genes be delivered at once by one virus entity. These characteristics of AAV indicates that an ideal in-vivo gene delivery vector must be of high safety profile and large transgene cargo capability, and inability to randomly integrated into a host genome, suggesting that given, the ideal vector could provide better opportunities for in-vivo gene therapy.

GLAd has been considered as a last-generation adenovirus. GLAd is constructed following the deletion of all the genes from an adenovirus, with the resultant expression of no adenoviral proteins. This structural characteristic minimizes the host immune response and allows long-term transgene expression in host tissues or organs. GLAd also shows broad tropism for infection and a high transduction efficiency in transgene delivery. In fact, GLAd is highly comparable to AAV in terms of many safety issues. Moreover, GLAd presents prominent advantages over AAV in regard to genome integration and transgene cargo capacity. GLAd does not integrate into the host genome, which eliminates concern about insertional mutagenesis. GLAd also exhibits a high accommodation capacity (up to 36 kb) for transgenes, hence making it possible to deliver large genes and multiple genes.

However, despite these evident beneficial features, there is a problem associated with the production of the currently available GLAd. Since GLAd is devoid of all adenoviral genes, the production of recombinant GLAd is absolutely dependent upon a helper adenovirus that provides all viral proteins for GLAd packaging. In the standard production process, the helper adenovirus actively replicates while providing helper function and thus remains as a contaminant in the final GLAd preparation. Although a significant reduction of contaminant helper adenovirus can be achieved through Cre-loxP-based excision of the $\Psi$ packaging signal, it is very difficult to completely remove the contaminant helper adenovirus in GLAd production. Moreover, the helper adenovirus can generate a replication-competent adenovirus (RCA) through homologous recombination between helper adenovirus and the E1 region present in packaging cells. These undesirable contaminant helper adenovirus and RCA can cause serious acute and chronic toxicity in host organisms. Furthermore, the host immune response against viral proteins expressed from these contaminant viruses can kill the cells co-infected with recombinant GLAd, and these contaminant viruses, which could result in insufficient expression of GLAd-mediated therapeutic transgenes. These unavoidable problems have raised safety concerns and hindered the clinical use of GLAd despite its unique features and tremendous advantages. Therefore, it is vital to establish a system that can produce recombinant GLAd in the absence of helper adenovirus and thus results in no contamination of helper adenovirus and RCA.

## Disclosure of Invention

## Technical Problem

The present disclosure pertains to a helper plasmid-based gutless adenovirus (GLAd) production system which can product GLAd in the absence of helper adenovirus, a method for production of gutless adenovirus, a gutless adenovirus produced using the same, and a use of the gutless adenovirus produced using the same.

Specifically in the present disclosure, the helper function for GLAd packaging and further amplification is provided by a helper plasmid that does not contain any cis-acting elements required for virus package. Due to the structural characteristic of being free of all *cis*-acting elements, the helper plasmid is impossible to convert into adenovirus.

Utilizing the helper plasmid, the present disclosure successfully produced large quantities of recombinant GLAd that was completely free of adenovirus and RCA contaminants. The recombinant GLAd that was produced efficiently delivered many target transgenes and exhibited a therapeutic potential for Huntington's disease (HD) and Duchenne muscular dystrophy (DMD). Accordingly, the GLAd production system of the present disclosure is expected to open a new way to the clinical application of GLAd-based gene therapy for various inherited genetic diseases.

Therefore, an aspect of the present disclosure is to provide a helper plasmid-based gutless adenovirus (GLAd) production system.

Another aspect of the present disclosure is to provide a method for production of gutless adenovirus

A further aspect of the present disclosure is to provide gutless adenovirus.

A further another aspect of the present disclosure is to provide a use of gutless adenovirus.

## Solution to Problem

The present disclosure pertains to a helper plasmid-based gutless adenovirus (GLAd) production system, a method for production of gutless adenovirus, a gutless adenovirus produced using the same, and a use of the gutless adenovirus produced using the same.

Below, a detailed description will be given of the present disclosure.

An aspect of the present disclosure is concerned with a gutless adenovirus (GLAd) production system.

The adenovirus genome, which consists of a linear molecule of double-stranded DNA, especially, the genome of adenovirus type 5 is well characterized. For example, there is general conservation associated with positions of E1, IX, E2, IVa2, E3, E4, L1, L2, L3, L4, and L5 genes in the overall structure of adenovirus genome. At each terminus of adenovirus genome is an inverted terminal repeat (ITR) which is necessary for viral replication. Subsequent to the 5' ITR at the 5' terminus, the cis-acting element Ψ packaging signal is located which is required for the packaging and encapsidation of adenovirus genome.

The structure of adenovirus genome is described on the basis of the expression order of viral genes after transduction into host cells. More specifically, viral genes are referred to as early (E) or late (L) genes according to whether transcription occurs prior to or after onset of DNA replication. At the early stage of infection, adenovirus expresses E1, E2, E3, and E4 genes in host cells to induce the transduction of host cells for viral replication. The E1 gene, which is considered a master switch, acts as a transcription activator and plays a critical role in both early and later gene transcription; E2 is involved in DNA replication; E3 is responsible for immune modulation; and E4 regulates viral mRNA metabolism.

The basic genome structure of adenovirus is as follows:

5' inverted terminal repeat (ITR) - Ψ packaging signal - E1 - other genes - 3' inverted terminal repeat (ITR).

Here, some elements, for example, 5' ITR and/or Ψ packaging signal may be somewhere else and then combined later. This recombination can recreate the basic genome structure. Some elements, for example, E1 may be transferred elsewhere and may not return back to the adenovirus genome. That is, the E1 region is delivered to a different cell strain which is then allowed to express the E1 protein and can thus be utilized as an adenovirus production cell strain. Some elements, for example, E3 may be completely eliminated because it does not have any influence on the construction and/or generation of adenovirus.

In the present disclosure, the gutless adenovirus production system may include a helper plasmid, a genome plasmid, and a virus packaging cell strain.

In the present disclosure, the helper plasmid may contain adenovirus genes other than the E1 and E3 regions, but with no limitations thereto. That is, a virus packaging cell strain may include the gene of E1 region, but may not include the gene of E3 region because the absence of the gene does not have any influence on generation of the virus. The helper plasmid supplies adenoviral proteins so that the final genome plasmid carrying a transgene to be expressed and elements necessary for transgene expression is packaged into viruses by using GLAd, that is, GLAdyated.

In the present disclosure, the helper plasmid may include a minimal number of adenovirus genes necessary for GLAd production, except for the adenovirus genes that the virus packaging cell strain expresses, but with no limitations thereto.

Therefore, the helper plasmid according to an embodiment of the present disclosure may have structural characteristics as follows:

None of the 5' inverted terminal repeat (ITR), the Ψ packaging signal, E1, and the 3' inverted terminal repeat are contained;

A region between E1 and E3 and a region between E3 and the 3' inverted terminal repeat are contained (A region stretching from the base immediately after E1 to the base immediately before E3 and a region stretching from the base immediately after E3 to the base immediately before the 3' inverted terminal repeat) are contained; and

Optionally, E3 may or may not be contained.

As such, the helper plasmid can perform the helper function only, with inability to convert into adenovirus, due to the structural characteristic of being devoid of the 5' inverted terminal repeat, Ψ packaging signal, E1, and 3' inverted terminal repeat. Although not including the genes of adenovirus, the helper plasmid free of the cis-acting elements necessary for virus generation cannot convert into adenovirus particles.

In the present disclosure, the helper plasmid may not be an infectious virus particle.

In the present disclosure, the helper plasmid may not convert into an infectious virus particle.

In the present disclosure, the helper plasmid may not include an ITR, but with no limitations thereto.

In the present disclosure, the helper plasmid may not include an ITR and a Ψ packaging signal, but with no limitations thereto.

In the present disclosure, the helper plasmid may further include an antibiotic-resistant gene, for example, a kanamycin-resistant gene, but with no limitations thereto.

In the present disclosure, the helper plasmid may further include an Ori replication origin, but with no limitations thereto.

In the present disclosure, the helper plasmid may be composed of at least one plasmid, for example, one to five plasmids, but with no limitations thereto.

In the present disclosure, the helper plasmid may be pAdBest_dITR, but with no limitations thereto.

In the present disclosure, the genome plasmid may include a 5' homologous stretch, a 3' homologous stretch, and a 3' inverted terminal repeat (ITR).

In the present disclosure, the genome plasmid may further include an antibiotic-resistant gene, for example, a kanamycin-resistant gene, but with no limitations thereto.

In the present disclosure, the genome plasmid may further include an Ori replication origin, but with no limitations thereto.

In the present disclosure, the genome plasma may further include a stuffer DNA (sDNA).

In the present disclosure, the stuffer DNA may further include a scaffold/matrix attachment element (SMAR), but is not limited thereto.

In the present disclosure, the genome plasmid may include a GLAd genome region packaged into the capsid of GLAd, but with no limitations thereto.

In the present disclosure, the genome plasmid may be a final genome plasmid including a transgene gene to be expressed using GLAd, and elements necessary for transgene expression, but with no limitations thereto.

In the present disclosure, the transgene to be expressed using GLAd and elements necessary for transgene expression in the final genome plasmid may be transferred from a cloning shuttle plasmid, but with no limitations thereto.

Therefore, the genome plasmid in the present disclosure may be a final genome plasmid including all cis-acting elements necessary for GLAd production, with or without a transgene and elements necessary for transgene expression being additionally included.

In the present disclosure, the final genome plasmid may be linearized by cutting a region not contained in GLAd genome with a restriction enzyme, but without limitations thereto.

In the present disclosure, the final genome plasmid may include a 5' inverted terminal repeat (ITR), a Ψ packaging signal, a promoter, an intron, a transgene, a poly(A) signal, a stuffer DNA (sDNA), and a 3' inverted terminal repeat (ITR).

In the present disclosure, the final genome plasmid may include a GLAd genome region to be packaged into the capsid of GLAd, but with no limitations thereto.

In the present disclosure, the transgene may be at least one gene, but with no limitations thereto.

In the present disclosure, the transgene may have a function of inhibiting expression of a specific gene product, but with no limitations thereto.

In the present disclosure, the transgene may be at least one selected from the group consisting of *factor IX* (R338L Padua mutant, for treatment of hemophilia B), *glucocerebrosidase* (GCR, for treatment of Gaucher's disease), *hexosaminidase* A (HEXA, for treatment of Tay-Sachs disease), *hypoxanthine phosphoribosyltransferase* 1 (HPRT1, for treatment of Lesch-Nyhan syndrome), *iduronate-2-sulfatase* (IDS, for treatment of Hunter syndrome), *methyl-CpG-binding protein* 2 (MECP2, for treatment of Rett syndrome), *survival of motor neuron* 1 (SMN1, for treatment of spinal muscular atrophy (SMA), and *dystrophin* gene [for treatment of Duchenne muscular dystrophy (DMD)], but is not limited thereto.

In the present disclosure, the genome plasmid may be pGLAd or pGLAd3, but is not limited thereto.

In the present disclosure, the system may further a cloning shuttle plasmid, but with no limitations thereto.

In the present disclosure, the cloning shuttle plasmid may include a 5' homologous stretch, a 5' inverted terminal repeat (ITR), a Ψ packaging signal, a promoter, a multi-cloning site (MCS), a poly(A) signal, and a 3' homologous stretch, but with no limitations thereto.

In the present disclosure, the promoter may be a CMV promoter or a chicken β-actin promoter, but with no limitations thereto.

In the present disclosure, the cloning shuttle plasmid may include an intron, but with no limitations thereto.

In the present disclosure, the intron may be derived from a rabbit β-globin gene, but with no limitations thereto.

In the present disclosure, the poly(A) signal sequence may be an SV40 poly(A) signal, but with no limitations thereto.

In the present disclosure, the cloning shuttle plasmid may further include an antibiotic-resistant gene, for example, a kanamycin-resistant gene, but with no limitations thereto.

In the present disclosure, the cloning shuttle plasmid may further include an Ori replication origin, but with no limitations thereto.

In the present disclosure, the cloning shuttle plasmid may include a transgene to be expressed using GLAd and elements necessary for transgene expression, but with no limitations thereto.

In the present disclosure, the transgene is at least one gene, but with no limitations thereto.

In the present disclosure, the transgene may have a function of inhibiting expression of a specific gene product, but with no limitations thereto.

In the present disclosure, the transgene may be cloned at a multi-cloning site, but with no limitations thereto.

In the present disclosure, the transgene may be at least one selected from the group consisting of *factor IX* (R338L Padua mutant, for treatment of hemophilia B), *glucocerebrosidase* (GCR, for treatment of Gaucher's disease), *hexosaminidase* A (HEXA, for treatment of Tay-Sachs disease), *hypoxanthine phosphoribosyltransferase* 1 (HPRT1, for treatment of Lesch-Nyhan syndrome), *iduronate-2-sulfatase* (IDS, for treatment of Hunter syndrome), *methyl-CpG-binding protein* 2 (MECP2, for treatment of Rett syndrome), *survival of motor neuron* 1 (SMN1, for treatment of spinal muscular atrophy (SMA), and *dystrophin* gene [for treatment of Duchenne muscular dystrophy (DMD)], but is not limited thereto.

In the present disclosure, the cloning shuttle plasmid may be pBest or pBest4, but is not limited thereto.

In the present disclosure, the virus packaging cell strain may a strain that express a protein of the E1 region of adenovirus, for example, HEK293 or HEK293T, but with no limitations thereto.

In the present disclosure, the system may further include a pAd5pTP expression plasmid, but with no limitations thereto. Given, the pAd5pTP expression plasmid may have an effect of remarkably increasing the production of GLAd.

In the present disclosure, the pAd5pTP expression plasmid may include the nucleotide sequence of SEQ ID NO: 76.

Another aspect of the present disclosure pertains to a method for production of gutless adenovirus (GLAd), the method comprising the steps of:

transfecting a final genome plasmid into a virus packaging cell strain; and
transfecting a helper plasmid into the virus packaging cell strain.

Here, the final genome plasmid of the method is as defined above, and thus the description thereof is omitted.

In the present disclosure, the final genome plasmid may be linearized with a restriction enzyme, but with no limitations thereto.

Here, the helper plasmid of the method is as defined above, and thus the description thereof is omitted.

In the present disclosure, the transfection may be carried out by a calcium phosphate precipitation method, but with no limitations thereto.

In the present disclosure, the production method may further comprise a step of transfecting a pAd5pTP expression plasmid into a virus packaging cell strain. Given, this step may lead to a remarkable increase in GLAd production.

In the present disclosure, the pAd5pTP expression plasmid may include the nucleotide sequence of SEQ ID NO: 76.

In the present disclosure, a step of transfecting a final genome plasmid into a virus packaging cell strain and a step of transfecting a helper plasmid into the virus packaging cell strain may be conducted irrespective of the order thereof and, for example, may be conducted simultaneously, but with no limitations thereto.

In the present disclosure, the GLAd produced by the production method may be free of contaminant virus species.

In the present disclosure, the contaminant virus species may be adenovirus or replication-competent adenovirus (RCA), but is not limited thereto.

In an embodiment of the present disclosure, the production method of the present disclosure may be conducted as follows:

A transgene expression cassette in the cloning shuttle plasmid including a transgene is transferred and integrated into a genome plasmid to form a final genome plasmid. The final genome plasmid is linearized with a restriction enzyme. Then, the linearized final genome plasmid and a helper plasmid are transfected into a virus packaging cell strain. GLAd is produced through the transfection step.

In another embodiment of the present disclosure, the production method of the present disclosure may be conducted as follows:

A transgene expression cassette in the cloning shuttle plasmid including a transgene is transferred and integrated into a genome plasmid to form a final genome plasmid. The final genome plasmid is linearized with a restriction enzyme. Then, the linearized final genome plasmid, a helper plasmid, and a pAd5pTP expression plasmid are transfected into a packaging cell strain. Through the transfection step, GLAd is produced.

In a further embodiment of the present disclosure, the production method of the present disclosure may be conducted as follows:

A helper plasmid is transfected into a packaging cell strain. After an adequate period of time, GLAd including a transgene expression cassette is additionally transfected into the transfected cell strain. GLAd is produced after an adequate time. In this regard, the GLAd may be produced in an amplification pattern.

In another embodiment of the present disclosure, the production method of the present disclosure may be conducted as follows:

A helper plasmid and a pAd5pTP expression plasmid are transfected into a virus packaging cell strain. After an adequate period of time, GLAd including a transgene expression cassette is additionally transfected into the transfected cell strain. After an adequate period of time, GLAd is produced. In this regard, the production of GLAd may be implemented in an amplified pattern.

Another aspect of the present disclosure pertains to a method for construction of a cloning shuttle plasmid, the method comprising the steps of:

- synthesizing a first DNA fragment corresponding to a $Kan^r$~ColE1 region in the presence of a primer set, with a pGT2 plasmid serving as a template;
- digesting the first DNA fragment with a restriction enzyme;
- preparing a second DNA fragment including a 5' homologous stretch, a 5' inverted terminal repeat (ITR), a $\psi$5 packaging signal, a CMV promoter, a multi-cloning site (MCS), an SV40 poly(A) signal, and a 3' homologous stretch;
- digesting the second DNA fragment with a restriction enzyme; and
- ligating the restriction digest of the first DNA fragment to the restriction digest of the second DNA fragment to construct pBest.

In the present disclosure, the pGT2 plasmid may include the nucleotide sequence of SEQ ID NO: 63 and may be, for example, composed of the nucleotide sequence of SEQ ID NO: 63, but is not limited thereto.

In the present disclosure, the primer set in the step of synthesizing a first DNA fragment may be a primer set consisting of the nucleotide sequences of SEQ ID NOS: 1 and 2, but is not limited thereto.

In the present disclosure, the step of synthesizing a first DNA fragment may be carried out by PCR, but with no limitations thereto.

In the present disclosure, the first DNA fragment may contain a restriction site, for example, SfiI and XcmI restriction sites, but with no limitations thereto.

In the present disclosure, the SfiI restriction site may be located at the 3' end in the first DNA fragment, but with no limitations thereto.

In the present disclosure, the XcmI restriction site may be located at the 5' end in the first DNA fragment, but with no limitations thereto.

In the present disclosure, the step of digesting the first DNA fragment with a restriction enzyme may be carried out using XcmI and SfiI restriction enzymes, with no limitations thereto.

In the present disclosure, the second DNA fragment may contain a restriction site, for example, SfiI and XcmI restriction sites, with no limitations thereto.

In the present disclosure, the SfiI restriction site may be located at the 5' end in the second DNA fragment, but with no limitations thereto.

In the present disclosure, the XcmI restriction site may be located at the 3' end in the second DNA fragment, but with no limitations thereto.

In the present disclosure, the step of digesting the second DNA fragment may be carried out using XcmI and SfiI restriction enzymes, but with no limitations thereto.

In the present disclosure, the step of constructing pBest may be carried out by ligating the restriction digest of the first DNA fragment to the restriction digest of the second DNA fragment, but with no limitations thereto.

In the present disclosure, the pBest may include the nucleotide sequence of SEQ ID NO: 64 and may be, for example, composed of the nucleotide sequence of SEQ ID NO: 64, but is not limited thereto.

Another aspect of the present disclosure pertains to a method for construction of a helper plasmid, the method comprising the following steps.

**1. Construction of ψ5-Left-Arm-2**

synthesizing a $Kan^r$~ColE1 region of ψ5-Left-Arm-I in the presence of a primer set, with pGT2 plasmid serving as a template;
digesting the $Kan^r$~ColE1 region of ψ5-Left-Arm-I with a restriction enzyme;
preparing a third DNA fragment corresponding to a BstZ17I~BamHI region of the ψ5 genome;
ligating the restriction digest of the third DNA fragment to the restriction digest of the $Kan^r$~ColE1 region of ψ5-Left-Arm-I to prepare ψ5-Left-Arm-1;
digesting the ψ5-Left-Arm-I with a restriction enzyme;
synthesizing a fourth DNA fragment in the presence of a primer set, with ψ5 serving as a template;
digesting the fourth DNA fragment with a restriction enzyme; and
ligating the restriction digest of the fourth DNA fragment to the restriction digest of the ψ5-Left-Arm-I to construct ψ5-Left-Arm-2.

In the present disclosure, the primer set in the step of synthesizing a $Kan^r$~ColE1 region of ψ5-Left-Arm-I may be a primer set including the nucleotide sequence of SEQ ID NOS: 3 and 4, but with no limitations thereto.

In the present disclosure, the step of synthesizing a $Kan^r$~ColE1 region of ψ5-Left-Arm-I may be carried out by PCR, but with no limitations thereto.

In the present disclosure, the $Kan^r$~ColE1 region of ψ5-Left-Arm-I may contain a restriction site, for example, BamHI and BstZ17I restriction sites, but with no limitations thereto.

In the present disclosure, the BamHI restriction site may be located at the 5' end in the $Kan^r$~ColE1 region of ψ5-Left-Arm-1, but with no limitations thereto.

In the present disclosure, the BstZ17I restriction site may be located at the 3' end in the $Kan^r$~ColE1 region of ψ5-Left-Arm-1, but with no limitations thereto.

In the present disclosure, the step of digesting the $Kan^r$~ColE1 region of ψ5-Left-Arm-I with a restriction enzyme may be carried out using BamHI and BstZ17I restriction enzymes, with no limitations thereto.

In the present disclosure, the third DNA fragment corresponding to the BstZ17I~BamHI region of the ψ5 genome may include the nucleotide sequence of SEQ ID NO: 65 and may be, for example, composed of the nucleotide sequence of SEQ ID NO: 65, but is not limited thereto.

In the present disclosure, the third DNA fragment may contain a restriction site, for example, BamHI and BstZ17I restriction sites, but with no limitations thereto.

In the present disclosure, the BamHI restriction site may be located at the 3' end in the third DNA fragment, but with no limitations thereto.

In the present disclosure, the BstZ17I restriction site may be located at the 5' end in the third DNA fragment, but with no limitations thereto.

In the present disclosure, the step of preparing a third DNA fragment may be carried out using BamHI and BstZ17I restriction enzymes, with no limitations thereto.

In the present disclosure, the step of preparing ψ5-Left-Arm-I may be carried out by ligating the restriction digest of the third DNA fragment to the restriction digest of the $Kan^r$~ColE1 region of ψ5-Left-Arm-1, but with no limitations thereto.

In the present disclosure, the step of digesting ψ5-Left-Arm-I with a restriction enzyme may be carried out using ClaI and BstZ17I restriction enzymes, but with no limitations thereto.

In the present disclosure, the fourth DNA fragment may be a DNA fragment stretching from '*' (asterisk) mark to the BstZ17I restriction site (b panel in FIG. 7) .

In the present disclosure, the primer set in the step of synthesizing a fourth DNA fragment may be a primer set including the nucleotide sequences of SEQ ID NOS: 5 and 6, but with no limitations thereto.
In the present disclosure, the step of synthesizing a fourth DNA fragment may be carried out by PCR, but with no limitations thereto.
In the present disclosure, the fourth DNA fragment may include the nucleotide sequence of SEQ ID NO: 66 and may be, for example, composed of the nucleotide sequence of SEQ ID NO: 66, but with no limitations thereto.
In the present disclosure, the fourth DNA fragment may contain a restriction site, for example, ClaI and BstZ17I enzyme sites, but with no limitations thereto.
In the present disclosure, the ClaI restriction site may be located at the 5' end in the fourth DNA fragment, but with no limitations thereto.
In the present disclosure, the BstZ17I restriction site may be located at the 3' end in the fourth DNA fragment, but with no limitations thereto.
In the present disclosure, the step of digesting the fourth DNA fragment with a restriction enzyme may be carried out using ClaI and BstZ17I restriction enzymes, with no limitations thereto.
In the present disclosure, the step of constructing ψ5-Left-Arm-2 may be carried out by ligating the restriction digest of the fourth DNA fragment to the restriction digest of the ψ5-Left-Arm-1, with no limitations thereto.
As used herein, the term "ψ5-Left-Arm-2" refers to a portion of Ad5 (adenovirus type 5, GenBank AC_000008) resulting from deletion of 5' ITR and ψ packaging signal, specifically nucleotides 1 to 3133.

**2. Construction of ψ5-Right-Arm-2**

the steps of:

synthesizing a Kan$^r$~ColE1 region of ψ5-Right-Arm-1 in the presence of a primer set, with pGT2 plasmid, ψ5-Left-Arm-1, or ψ5-Left-Arm-2serving as a template;
digesting the Kan$^r$~ColE1 region of ψ5-Right-Arm-1 with a restriction enzyme;
preparing a fifth DNA fragment corresponding to a BamHI~3' ITR region of the ψ5 genome;
ligating the restriction digest of the fifth DNA fragment to the restriction digest of the Kan$^r$~ColE1 region of ψ5-Right-Arm-1 to prepare ψ5-Right-Arm-1;
digesting the ψ5-Right-Arm-1 with a restriction enzyme;
synthesizing a sixth DNA fragment in the presence of a primer set, with ψ5 serving as a template;
digesting the sixth DNA fragment with a restriction enzyme; and
ligating the restriction digest of the sixth DNA fragment to the restriction digest of the ψ5-Right-Arm-1 to construct ψ5-Right-Arm-2.

In the present disclosure, the primer set in the step of synthesizing a Kan$^r$~ColE1 region of ψ5-Right-Arm-1 may be a primer set including the nucleotide sequence of SEQ ID NOS: 7 and 8, but with no limitations thereto.
In the present disclosure, the step of synthesizing a Kan$^r$~ColE1 region of ψ5-Right-Arm-1 may be carried out by PCR, but with no limitations thereto.
In the present disclosure, the Kan$^r$~ColE1 region of ψ5-Right-Arm-1 may contain a restriction site, for example, PacI and BamHI restriction sites, but with no limitations thereto.
In the present disclosure, the PacI restriction site may be located at the 5' end in the Kan$^r$~ColE1 region of ψ5-Right-Arm-1, but with no limitations thereto.
In the present disclosure, the BamHI restriction site may be located at the 3' end in the Kan$^r$~ColE1 region of ψ5-Right-Arm-1, but with no limitations thereto.
In the present disclosure, the step of digesting the Kan$^r$~ColE1 region of ψ5-Right-Arm-1 with a restriction enzyme may be carried out using BamHI restriction enzyme, with no limitations thereto.
In the present disclosure, the fifth DNA fragment corresponding to the BamHI~3'ITR region of the ψ5 genome may include the nucleotide sequence of SEQ ID NO: 67 and may be, for example, composed of the nucleotide sequence of SEQ ID NO: 67, but is not limited thereto.
In the present disclosure, the step of preparing a fifth DNA fragment corresponding to a BamHI~3' ITR region of the ψ5 genome may be carried out by digesting ψ5 DNA with BamHI restriction enzyme, with no limitations thereto.
In the present disclosure, the BamHI restriction site may be located at the 5' end in the fifth DNA fragment, but with no limitations thereto.
In the present disclosure, the step of preparing ψ5-Right-Arm-1 may be carried out by ligating the restriction digest of the fifth DNA fragment to the restriction digest of the Kan$^r$~ColE1 region of ψ5-Right-Arm-1, but with no limitations thereto.
In the present disclosure, the step of digesting ψ5-Right-Arm-1 with a restriction enzyme may be carried out using SpeI and NdeI restriction enzymes, but with no limitations thereto.

In the present disclosure, the primer set in the step of synthesizing a sixth DNA fragment in the presence thereof, with ψ5 serving as a template is a primer set including nucleotides sequences of SEQ ID NOS: 9 to 12, but with no limitations thereto.

In the present disclosure, the step of synthesizing a sixth DNA fragment may be carried out by PCR, but with no limitations thereto.

In the present disclosure, the sixth DNA fragment may include the nucleotide sequence of SEQ ID NO: 68 and may be, for example, composed of the nucleotide sequence of SEQ ID NO: 68, but with no limitations thereto.

In the present disclosure, the sixth DNA fragment may contain a restriction site, for example, SpeI and NdeI restriction sites, but with no limitations thereto.

In the present disclosure, the SpeI restriction site may be located at the 5' end in the sixth DNA fragment, but with no limitations thereto.

In the present disclosure, the NdeI restriction site may be located at the 3' end in the sixth DNA fragment, but with no limitations thereto.

In the present disclosure, the step of digesting the sixth DNA fragment with a restriction enzyme may be carried out using SpeI and NdeI restriction enzymes, with no limitations thereto.

In the present disclosure, the step of constructing **ψ5-Right-Arm-2** may be carried out by ligating the restriction digest of the sixth DNA fragment to the restriction enzyme-digested ψ5-Right-Arm-1, but with no limitations thereto.

As used herein, the term "ψ5-Right-Arm-2" refers to a portion of Ad5 (adenovirus type 5, GenBank

AC_000008) in which the partially remaining E3 region is completely deleted by removing nucleotides 27864 to 31000 of Ad5.

### 3. Construction of pAdBest

The steps of:

digesting ψ5-Right-Arm-2 with a restriction enzyme;
digesting ψ5-Left-Arm-2 with a restriction enzyme to obtain a larger fragment; and
ligating the restriction digest of the ψ5-Left-Arm-2 to the restriction digest of the ψ5-Right-Arm-2 to construct pAdBest.

In the present disclosure, the step of digesting ψ5-Right-Arm-2 with a restriction enzyme may be carried out using ClaI and BamHI restriction enzymes, but with no limitations thereto.

In the present disclosure, the step of digesting ψ5-Left-Arm-2 with a restriction enzyme to obtain a larger fragment may be carried out using ClaI and BamHI restriction enzymes.

In the present disclosure, the larger fragment DNA obtained by digesting ψ5-Left-Arm-2 with ClaI and BamHI restriction enzymes may include the nucleotide sequence of SEQ ID NO: 69 and may be, for example, composed of the nucleotide sequence of SEQ ID NO: 69, but with no limitations thereto.

In the present disclosure, the larger fragment obtained by digesting ψ5-Left-Arm-2 with a restriction enzyme may contain a restriction site, for example, ClaI and BamHI restriction sites, but with no limitations thereto.

In the present disclosure, the ClaI restriction site may be located at the 5' end in the larger fragment obtained by digesting ψ5-Left-Arm-2 with a restriction enzyme, but with no limitations thereto.

In the present disclosure, the BamHI restriction site may be located at the 3' end in the larger fragment obtained by digesting ψ5-Left-Arm-2 with a restriction enzyme, but with no limitations thereto.

In the present disclosure, the step of constructing pAdBest may be carried out by ligating the restriction digest of the ψ5-Right_Arm-2 with the larger fragment obtained by digesting ψ5-Left Arm-2 with a restriction enzyme, but with no limitations thereto.

### 4. Construction of pAdBest dITR

The steps of:

digesting pAdBest with a restriction enzyme to obtain a smaller fragment;
ligating an adaptor containing ClaI and EcoRI restriction sites to the smaller fragment obtained by digesting pAdBest with a restriction enzyme to prepare pAdBest_EcoR_Cla;
digesting pAdBest_EcoR_Cla with a restriction enzyme to obtain a larger fragment;
synthesizing a seventh DNA fragment in the presence of a primer set, with pAdBest serving as a template;
digesting the seventh DNA fragment with a restriction enzyme;
ligating the restriction digest of the seventh DNA fragment to the larger fragment obtained by digesting pAdBest_EcoR_Cla with a restriction enzyme to prepare pAdBest_EcoR_Cla_dITR;
digesting pAdBest_EcoR_Cla_dITR with a restriction enzyme;
digesting pAdBest with a restriction enzyme to obtain a larger fragment; and
ligating the restriction digest of the pAdBest_EcoR_Cla_dITR with the larger fragment obtained by digesting pAdBest with a restriction enzyme to construct pAdBest_dITR.

In the present disclosure, the step of digesting pAdBest with a restriction enzyme to obtain a smaller fragment may be carried out using ClaI and EcoRI restriction enzymes, but with no limitations thereto.

In the present disclosure, the smaller fragment obtained by digesting pAdBest with a restriction enzyme may contain a restriction site, for example, ClaI and EcoRI restriction site, but with no limitations thereto.

In the present disclosure, the ClaI restriction site may be located at the 3' end of the smaller fragment obtained by digesting pAdBest with a restriction enzyme, but with no limitations thereto.

In the present disclosure, the EcoRI restriction site may be located at 5' end of the smaller fragment obtained by digesting pAdBest with a restriction enzyme, but with no limitations thereto.

In the present disclosure, the adaptor containing ClaI and EcoRI restriction sites may be an adaptor including the nucleotide sequences of SEQ ID NOS: 13 and 14, but with no limitations thereto.

In the present disclosure, the step of preparing pAdBest_EcoR_Cla may be carried out by ligating the adaptor to the smaller fragment obtained by digesting pAdBest with a restriction enzyme, but with no limitations thereto.

In the present disclosure, the step of digesting pAdBest_EcoR_Cla with a restriction enzyme to obtain a larger fragment may be carried out using AvrII and RsrII restriction enzymes, but with no limitations thereto.

In the present disclosure, the larger fragment obtained by digesting pAdBest_EcoR_Cla with a restriction enzyme may contain a restriction site, for example, AvrII and RsrII restriction sites, but with no limitations thereto.

In the present disclosure, the AvrII restriction site may be located at the 3' end of the larger fragment obtained by digesting pAdBest_EcoR_Cla with a restriction enzyme, but with no limitations thereto.

In the present disclosure, the RsrII restriction site may be located at the 5' end of the larger fragment obtained by digesting pAdBest_EcoR_Cla with a restriction enzyme, but with no limitations thereto.

In the present disclosure, the primer set in the step of synthesizing a seventh DNA fragment may include nucleotide sequences of SEQ ID NOS: 15 to 18, but with no limitations thereto.

In the present disclosure, the step of preparing a seventh DNA fragment may be carried out by overlapping PCR, but with no limitations thereto.

In the present disclosure, the seventh DNA fragment may include the nucleotide sequence of SEQ ID NO: 70, for example, may be composed of the nucleotide sequence of SEQ ID NO: 70, but with no limitations thereto.

In the present disclosure, the seventh DNA fragment may contain a restriction site, for example, AvrII and RsrII restriction site, but with no limitations thereto.

In the present disclosure, the step of digesting the seventh DNA fragment with a restriction site may be carried out using AvrII and RsrII restriction enzymes, but with no limitations thereto.

In the present disclosure, the AVrII restriction site may be located at the 5' end in the seventh DNA fragment, but with no limitations thereto.

In the present disclosure, the RsrII restriction site may be located at 3' end in the seventh DNA fragment, but with no limitations thereto.

In the present disclosure, the step of preparing pAdBest_EcoR_Cla_dITR may be carried out by ligating the restriction digest of the seventh DNA fragment to the larger fragment obtained by digesting pAdBest_EcoR_Cla with a restriction enzyme, but with no limitations thereto.

In the present disclosure, the step digesting pAdBest_EcoR_Cla_dITR with a restriction enzyme may be carried out using ClaI and EcoRI restriction enzymes, but with no limitations thereto.

In the present disclosure, the step of digesting pAdBest with a restriction enzyme to obtain a larger fragment may be carried out using ClaI and EcoRI restriction enzymes, but with no limitations thereto.

In the present disclosure, the larger fragment obtained by digesting pAdBest with a restriction enzyme may contain a restriction site, for example, ClaI and EcoRI restriction sites, but with no limitations thereto.

In the present disclosure, the ClaI restriction site may be located at the 5' end in the larger fragment obtained by digesting pAdBest with a restriction enzyme, but with no limitations thereto.

In the present disclosure, the EcoRI restriction site may be located at the 3' end in the larger fragment obtained by digesting pAdBest with a restriction enzyme, but with no limitations thereto.

In the present disclosure, the step of constructing pAdBest_ITR may be carried out by ligating the restriction digest of the pAdBest_EcoR_Cla_dITR with the larger fragment obtained by digesting pAdBest with a restriction enzyme, but with no limitations thereto.

In the present disclosure, the pAdBest_dITR may include the nucleotide sequence of SEQ ID NO: 71, for example, may be composed of the nucleotide sequence of SEQ ID NO: 71, but with no limitations thereto.

Another aspect of the present disclosure pertains to a method for constructing a genome plasmid, the method comprising the steps of:

synthesizing an eighth DNA fragment in the presence of a primer set, with pAdBest plasmid serving as a template;
digesting the eighth DNA fragment with a restriction enzyme;
ligating a restriction digest of the eighth DNA fragment to an adaptor to prepare pAdBestGL1;
removing a restriction site from pAdBestGL1 to prepare pAdBestGL1_dCla;
digesting pAdBestGL1 and pAdBestGL1_dCla with respective restriction enzymes;
ligating restriction digests of pAdBestGL1 and pAdBestGL1_dCla to adaptors to prepare pAdBestGL2_wtCla and pAdBestGL2, respectively;
digesting pAdBestGL2 with a restriction enzyme;
mixing the restriction digest of pAdBestGL2 and a restriction digest of lambda phage to prepare pAdBestGL3;
digesting pAdBestGL3 with a restriction enzyme;
ligating the restriction digest of pAdBestGL3 to an adaptor to prepare pAdBestGL4_3H;
ligating the restriction digest of pAdBestGL3 to an adaptor to prepare pAdBestGL4_5H;
removing a restriction site from pAdBestGL4_3H to prepare pAdBestGL4_3H_2dCla;
removing a restriction site from pAdBestGL4_5H to prepare pAdBestGL4_5H_dCla;
digesting pAdBestGL4_3H_2dCla with a restriction enzyme;
digesting pAdBestGL4_5H_dCla with a restriction enzyme to obtain a larger fragment;
ligating the restriction digest of pAdBestGL4_3H_2dCla with the larger fragment obtained by digesting pAdBestGL4_5H_dCla with a restriction enzyme to prepare pAdBestGL5;
digesting pAdBestGL2_wtCla with a restriction enzyme;
digesting pAdBestGL5 with a restriction enzyme;
mixing the restriction digest of pAdBestGL2_wtCla with the restriction digest of pAdBestGL5 to prepare pAdBestGL;
digesting pAdBestGL with a restriction enzyme;
preparing a scaffold/matrix attachment (SMAR) element;
digesting the SMAR element with a restriction enzyme; and
ligating the restriction digest of pAdBestGL to the restriction digest of the SMAR element.

In the present disclosure, the primer set in the step of synthesizing an eighth DNA fragment may be a primer set including the nucleotide sequences of SEQ ID NOS: 19 and 20, but with no limitations thereto.

In the present disclosure, the step of synthesizing an eighth DNA fragment may be carried out using PCR, but with no limitations thereto.

In the present disclosure, the eighth DNA fragment may include the nucleotide sequence of SEQ ID NO: 72, for example, may be composed of the nucleotide sequence of SEQ ID NO: 72, but with no limitations thereto.

In the present disclosure, the eighth DNA fragment may contain a restriction site, for example, SacI and AscI restriction sites, but with no limitations thereto.

In the present disclosure, the step of digesting the eighth DNA fragment with a restriction enzyme may be carried out using SacI and AscI restriction enzymes, but with no limitations thereto.

In the present disclosure, the SacI restriction site may be located at the 3' end in the eighth DNA fragment, but with no limitations thereto.

In the present disclosure, the AscI restriction site may be located at the 5' end in the eighth DNA fragment, but with no limitations thereto.

In the present disclosure, the adaptor in the step of preparing pAdBestGL1 may include the nucleotide sequences of SEQ ID NOS: 21 and 22, but with no limitations thereto.

In the present disclosure, the step of preparing pAdBestGL1 by ligating the adaptor to the restriction digest of the eighth DNA fragment, but with no limitations thereto.

In the present disclosure, the restriction site in the step of preparing a restriction site from pAdBestGL1 to prepare pAdBestGLl_ dCla may be the ClaI restriction site, but is not limited thereto.

In the present disclosure, the step of preparing pAdBestGL1_dCla may be carried out by digesting pAdBestGL1 with ClaI restriction enzyme and filling the gap with Klenow, followed by allowing self-ligation, but with no limitations thereto.

In the present disclosure, the step of digesting pAdBestGL1 and pAdBestGL1_dCla with respective restriction enzymes may be carried out using SacI and AvrII restriction enzymes, but with no limitations thereto.

In the present disclosure, the adaptors in the step of preparing pAdBestGL2_wtCla and pAdBestGL2 may each include the nucleotide sequences of SEQ ID NOS: 23 and 24 (FIG. 8b), but with no limitations thereto.

In the present disclosure, the step of preparing pAdBestGL2_wtCla and pAdBestGL2 may be carried out by ligating the restriction digests of pAdBestGL1 and pAdBestGL1_dCla to the adaptors, respectively, but with no limitations thereto.

In the present disclosure, the step of digesting pAdBestGL2 with a restriction enzyme may be carried out using HpaI restriction enzyme, but with no limitations thereto.

In the present disclosure, the restriction digest of lambda phage DNA may be obtained by digesting lambda phage DNA with HindIII restriction enzyme, but with no limitations thereto.

In the present disclosure, the step of preparing pAdBestGL3 may be carried out by mixing the restriction digest of pAdBestGL2 and the restriction digest of lambda phage DNA and subjecting the mixture to in vitro homologous annealing (iHoA), but with no limitations thereto.

In the present disclosure, the step of digesting pAdBestGL3 with a restriction enzyme may be carried out using SacI and ApaI restriction enzymes or ApaI and AvrII restriction enzymes, but with no limitations thereto.

In the present disclosure, the restriction digest of pAdBestGL3 in the step of preparing pAdBestGL4_3H may be obtained by digesting pAdBestGL3 with SacI and ApaI restriction enzymes, but with no limitations thereto.

In the present disclosure, the adaptor in the step of preparing pAdBestGL4_3H may be an adaptor including the nucleotides of SEQ ID NOS: 25 and 26 (FIG. 8d), but is not limited thereto.

In the present disclosure, the step of preparing pAdBestGL4_3H may be carried out by ligating the adaptor to the restriction digest of pAdBestGL3, but with no limitations thereto.

In the present disclosure, the restriction digest of pAdBestGL3 in the step of preparing pAdBestGL4_5H may be obtained by digesting pAdBestGL3 with ApaI and AvrII restriction enzymes, but with no limitations thereto.

In the present disclosure, the adaptor in the step of preparing pAdBestGL4_5H may be an adaptor including the nucleotides of SEQ ID NOS: 27 and 28 (FIG. 8e), but with no limitations thereto.

In the present disclosure, the step of preparing pAdBestGL4_5H may be carried out by ligating the adaptor to the restriction digest of pAdBestGL3, but with no limitations thereto.

In the present disclosure, the restriction site to be removed in the step of removing a restriction site from pAdBestGL4_3H to prepare pAdBestGL4_3H_2dCla may be a ClaI restriction site, but is not limited thereto.

In the present disclosure, the step of preparing pAdBestGL4_3H_2dCla may comprise the steps of: digesting pAdBestGL4_3H with ClaI restriction enzyme, filling the gap with Klenow, subjecting the same to self-ligation, and transforming the resultant plasmid into Dam-/- bacterial cells; digesting the resulting pAdBestGL4_3H_dCla with ClaI restriction enzyme, again; filling the gap with Klenow; and subjecting the same to self-ligation.

In the present disclosure, the restriction site to be removed in the step of removing a restriction site from pAdBestGL4_5H to prepare pAdBestGL4_5H_2dCla may be a ClaI restriction site, but is not limited thereto.

In the present disclosure, the step of preparing pAdBestGL4_5H_dCla may be carried out by digesting pAdBestGL4_5H with ClaI restriction enzyme, filling the gap with Klenow, and subjecting the same to self-ligation, but with no limitations thereto.

In the present disclosure, the step of digesting pAdBestGL4_3H_2dCla with a restriction enzyme may be carried out using SacI and ApaI restriction enzymes, but with no limitations thereto.

In the present disclosure, the step of digesting pAdBestGL4_5H_dCla with a restriction enzyme to obtain a larger fragment may be carried out using SacI and ApaI restriction enzymes, but with no limitations thereto.

In the present disclosure, the larger fragment obtained by digesting pAdBestGL4_5H_dCla with a restriction enzyme may contain a restriction site, for example, SacI and ApaI restriction sites, but with no limitations thereto.

In the present disclosure, the SacI restriction site may be located at the 5' end in the larger fragment obtained by digesting pAdBestGL4_5H_dCla with a restriction enzyme, with no limitations thereto.

In the present disclosure, the ApaI restriction site may be located at the 3' end in the larger fragment obtained by digesting pAdBestGL4_5H_dCla with a restriction enzyme, but with no limitations thereto.

In the present disclosure, the step of preparing pAdBestGL5 may be carried out by ligating the restriction digest of pAdBestGL4_3H_2dCla with the larger fragment obtained by digesting pAdBestGL4_5H_dCla with a restriction enzyme, but with no limitations thereto.

In the present disclosure, the step of digesting pAdBestGL2_wtCla with a restriction step may be carried out using HpaI restriction enzyme, but with no limitations thereto.

In the present disclosure, the step of digesting pAdBestGL5 with a restriction enzyme may be carried out using SacI and AvrII restriction enzymes, but with no limitations thereto.

In the present disclosure, the step of preparing pAdBestGL may be carried out by mixing the restriction digest of pAdBestGL2_wtCla and the restriction digest of pAdBestGL5 and subjecting the mixture to iHoA, but with no limitations thereto.

In the present disclosure, the step of digesting pAdBestGL with a restriction enzyme may be carried out using ApaI and NsiI restriction enzymes, but with no limitations thereto.

In the present disclosure, the primer set in the step of preparing a scaffold/matrix attachment element may be a primer set including SEQ ID NOS: 29 and 30, but is not limited thereto.

In the present disclosure, the step of preparing a scaffold/matrix attachment element may be carried out PCR, but with no limitations thereto.

In the present disclosure, the scaffold/matrix attachment element may contain a restriction site, for example, ApaI and NsiI restriction sites, but with no limitations thereto.

In the present disclosure, the step of digesting the scaffold/matrix attachment element with a restriction enzyme may be carried out using ApaI and NsiI restriction enzymes, but with no limitations thereto.

In the present disclosure, the Apal restriction site may be located at the 5' end in the scaffold/matrix attachment element, but with no limitations thereto.

In the present disclosure, the NsiI restriction site may be located at the 3' end in the scaffold/matrix attachment element, but with no limitations thereto.

In the present disclosure, the step of constructing pGLAd may be carried out by ligating the restriction digest of the scaffold/matrix element to the restriction digest of pAdBestGL, with no limitations thereto.

In the present disclosure, the pGLAd may include the nucleotide sequence of SEQ ID NO: 73, for example, may be composed of the nucleotide sequence of SEQ ID NO: 73, but with no limitations thereto.

Another aspect of the present disclosure pertains to a gutless adenovirus including the following composition: 5' inverted terminal repeat (ITR), a ψ packaging signal, a promoter, an intron, a transgene, a poly(A) signal, a stuffer DNA (sDNA), and 3' inverted terminal repeat.

In the present disclosure, the promoter is a CMV promoter or a chicken β-actin promoter, but with no limitations thereto.

In the present disclosure, the intron may be derived from a rabbit β-globin gene, but with no limitations thereto.

In the present disclosure, the poly(A) signal may be an SV40 poly(A) signal, but with no limitations thereto.

In the present disclosure, the stuffer DNA may further include a scaffold/matrix attachment element (SMAR), but with no limitations thereto.

In the present disclosure, the transgene gene may be at least one gene, but with no limitations thereto.

In the present disclosure, the transgene may have a function of inhibiting expression of a specific gene, but with no limitations thereto.

In the present disclosure, the transgene may be at least one selected from the group consisting of *factor IX* (R338L Padua mutant, for treatment of hemophilia B), *glucocerebrosidase* (GCR, for treatment of Gaucher's disease), *hexosaminidase* A (HEXA, for treatment of Tay-Sachs disease), *hypoxanthine phosphoribosyltransferase* 1 (HPRT1, for treatment of Lesch-Nyhan syndrome), *iduronate-2-sulfatase* (IDS, for treatment of Hunter syndrome), *methyl-CpG-binding protein* 2 (MECP2, for treatment of Rett syndrome), *survival of motor neuron* 1 (SMN1, for treatment of spinal muscular atrophy (SMA), and *dystrophin* gene [for treatment of Duchenne muscular dystrophy (DMD)], but is not limited thereto.

In the present disclosure, the gutless adenovirus may be produced using the helper plasmid-based gutless adenovirus production system described above, but with no limitations thereto.

In the present disclosure, the gutless adenovirus may be produced by the gutless adenovirus production method described above, but with no limitations thereto.

## Advantageous Effects of Invention

The present disclosure pertains to a helper plasmid-based gutless adenovirus (GLAd) production system, a method for producing a gutless adenovirus, using same, a gutless adenovirus produced using same, and a use of the gutless adenovirus produced using same. The helper plasmid-based GLAd production system of the present disclosure can produce only the recombinant GLAd, without generating helper plasmid-derived adenovirus and replication-competent adenovirus (RCA). The recombinant GLAd produced by the production system can effectively deliver genes irrespective of sizes of the transgenes, exhibiting a therapeutic potential for Huntington's disease (HD) and Duchenne muscular dystrophy (DMD). Accordingly, the helper plasma-based GLAd production system of the present disclosure is suggested to be a new flatform for the clinical application of GLAd-based gene therapy for various inherited genetic diseases.

## Brief Description of Drawings

FIG. 1 shows construction of the pAdBest_dITR helper plasmid and the pGLAd genome plasmid and their use for producing GLAd in accordance with an embodiment of the present disclosure:

a. Structural characteristics of the most commonly used helper adenovirus. The black arrowheads indicate loxP sites;

b. Ad5 genome structure and transcription units. E and L indicate the early gene and the late gene, respectively.

The arrows indicate the orientation of transcription units;
c. Schematic illustration and comparison of Ad5, pAdBest_dITR helper plasmid, pGLAd genome plasmid, and GLAd genome structures. SMAR indicates the scaffold/matrix attachment element. Colors indicate the origin of the DNA backbones;
d. Schematic illustration of GLAd production. Cotransfection of the pAdBest_dITR helper plasmid and the PacI-linearized recombinant pGLAd_X genome plasmid into HEK293T cells produces recombinant GLAd.X virus; and
e. LacZ staining of HEK293 cells infected with the GLAd.LacZ virus. At 48 hours after the infection of HEK293 cells with the GLAd.LacZ virus, the cells were subjected to LacZ staining. Upper: untreated control (background dots are X-gal crystals, not stained cells); middle: 50 ul out of 10 ml viral medium was used for infection; bottom: 5 ul out of 1 ml viral lysate was used for infection

FIG. 2 shows construction of the pGLAd3, which is a novelgenome plasmid, and its use for producing GLAd in accordance with an embodiment of the present disclosure:

a. Schematic illustration and comparison of pGLAd and pGLAd3 genome plasmid and corresponding GLAd. Colors show the origin of the DNA backbones;
b. Cytopathic effect (CPE) observed during the production of recombinant GLAd3.LacZ virus. The pAdBest_dITR helper plasmid and the PacI-cut pGLAd3_LacZ genome plasmid were co-transfected into HEK293T cells. Forty-eight hours later, untreated control (left) and transfected cells (right) were photographed under the microscope;
c. LacZ staining of HEK293 cells infected with the GLAd3.LacZ virus. At 48 h after the infection of HEK293 cells with the GLAd3.LacZ virus, the cells were subjected to LacZ staining. The numbers indicate the viral lysate volume (0, 5, or 20 μl) used for infection from 1 ml viral lysate prepared from a 100 mm culture dish; and
d. Homologous regions identified in the helper adenovirus, E1-expressing packaging cells and our pAdBest_dITR helper plasmid. Homologous recombination can occur in colored boxed regions. RCA generation requires two homologous recombination events, through which E1 is transferred from the packaging cell to the helper adenovirus

FIG. 3 shows workflow for large-scale GLAd production in accordance with an embodiment of the present disclosure:

a. The entire process of the standard conventional GLAd production method. The pHDAd.LacZ is a GLAd genome plasmid containing the LacZ expression cassette. The titer indicates the total infectious GLAd particles (BFU determined by LacZ staining) produced in each round of amplification; and
b. The entire process for seed GLAd (P0) rescue, continuous amplification (P1 to P3) and large-scale GLAd production (P3, P4, or P5, depending on scale) is described (for details, see Materials and methods). The titer (BFU) was determined by LacZ staining in each round of amplification.

FIG. 4 shows analysis of adenovirus and RCA contaminants generated during GLAd production in accordance with an embodiment of the present disclosure:

a. Serial infection of HEK293 cells in 100 or 150 mm culture dishes with Ad.LacZ (positive control) or P3 GLAd3.LacZ ($3 \times 10^9$ BFU) (see FIG. 3 for production), respectively. Under these conditions, replicable adenovirus and RCA can be amplified. In the positive control, one infectious virus particle resulted in $5 \times 10^5$ infectious virus particles (1 BFU to $5 \times 10^5$ BFU) within less than two complete rounds of amplification. Benzonase was used to degrade the helper plasmid continuously used for the preparation of P3 GLAd3.LacZ (viral DNA packaged into the capsid shell is resistant to Benzonase). In each round of amplification, the cells were collected and processed to release adenovirus and RCA. In the final round of amplification, both the cells and culture medium were collected and processed for the analysis of adenovirus and RCA;
b. Analysis of adenovirus and RCA in the samples (a) by PCR. The samples were analyzed by PCR for the N-terminal DNA of the fiber gene, which is present in both the adenovirus and RCA but not in the GLAd genome or HEK293 cells. Ad5 DNA (10 pg) was used as a positive control for PCR. The Ad.LacZ sample was subjected to PCR before or after 100× dilution (in this dilution, only 500 virus particles were present in the sample). The GLAd3.LacZ sample was PCR-amplified without or with spiked Ad5 DNA (10 pg). The arrowhead indicates the target PCR product (484 bp). M is a 100 bp size marker;
c. Infection of HEK293 cells with P4 GLAd3.LacZ or P5 GLAd3.LacZ for the amplification of adenovirus and RCA, as shown in a. The prepared samples were subjected to PCR analysis (d); and
d. Analysis of adenovirus and RCA in samples (c) by PCR. PCR was carried out as described in b.

FIG. 5 shows expression of huntingtin mshRs, the codon-optimized synthetic huntingtin gene, or both by recombinant GLAd in accordance with an embodiment of the present disclosure:

a. Schematic illustration of the full-length human mature huntingtin mRNA and the locations of mshRs. The numbers indicate the locations of the target sites of mshR1, mshR2, and mshR3;
b. Template for mshRs. The 21NTs are sense and anti-sense sequences of 21 nucleotides in length;
c. Expression plasmid for mshRs. Individual mshR (Table S2) was cloned into this plasmid using the BamHI and EcoRI sites;
d. Inhibition of endogenous huntingtin expression by mshR expression plasmids. HEK293T cells were either left untreated or were transfected with pGT2, pGT2-mshR1, pGT2-mshR2, or pGT2-mshR3. Forty-eight hours later, the cells were harvested and subjected to western blotting analysis of endogenous huntingtin expression. HTT denotes huntingtin;
e. Schematic illustration of the full-length human mature huntingtin mRNA and the locations of mshRs. The upper black and lower red horizontal lines indicate the native and codon-optimized huntingtin mRNAs, respectively. The numbers indicate the locations of the target sites of mshR1, mshR2, and mshR3. For mshR1 and mshR3, the amino acids and their corresponding codons are shown. Nucleotides identified as different between the native (endogenous) and codon-optimized huntingtin mRNA are highlighted with gray;
f. pGLAd4 genome plasmid for cloning mshR1, mshR3, and the codon-optimized synthetic huntingtin gene (right orientation or reverse (R) orientation);
g. Recombinant GLAd delivering both huntingtin mshRs and the codon-optimized synthetic hungtingtin gene simultaneously. GLAd4.coHTT.HTTmshR1/3 delivers the right-oriented codon-optimized hungtingtin gene. The total length (28.3 kb) is the size from the 5' ITR to the 3' ITR;
h. Effects of mshR1/3 and the codon-optimized synthetic huntingtin gene on huntingtin expression when simultaneously delivered by recombinant GLAd. At 48 h after the infection of HEK293T cells with GLAd4.coHTT.HTTmshR1/3 or GLAd4.coHTT(R). HTTmshR1/3 (reverse oriented huntingtin gene), the cells were harvested and subjected to western blotting for the analysis of huntingtin expression. HTT denotes huntingtin; and
i. Action model of recombinant GLAd4.coHTT.HTTmshR1/3 in HD. wtHTT and mtHTT indicate wild type and mutant huntingtin mRNA, respectively. coHTT denotes mRNA transcribed from the codon-optimized synthetic huntingtin gene. Recombinant GLAd4.coHTT.HTTmshR1/ 3 simultaneously delivers both mshRs and the codon-optimized synthetic huntingtin gene to target HD tissues.

FIG. 6 shows recombinant GLAd delivers the dystrophin gene in vivo in accordance with an embodiment of the present disclosure in accordance with an embodiment of the present disclosure:

a. Schematic illustration of recombinant GLAd4.Dys. The total length (27.7 kb) is the size from the 5' ITR to the 3' ITR;
b. Time frame of animal experiments; and
c. Examination of dystrophin expression in dystrophin-knockout MDX mice. The focal gastrocnemius muscle of MDX mice was injected with PBS or 50 $\mu$l of the GLAd4.Dys virus (4 $\times$ 1010 viral particles). Four weeks later, muscle tissues biopsied from the wild-type control and MDX mice treated with PBS or the GLAd4. Dys virus were subjected to immunofluorescence staining and analyzed under a confocal microscope (magnification = $\times$ 200; scale bar = 20 $\mu$m).

FIG. 7 shows construction of the pAdBest_dITR helper plasmid and the pBest cloning shuttle plasmid in accordance with an embodiment of the present disclosure;

a. Schematic illustration of $\Psi$5, a derivative of Ad5. Vertical dotted lines in E1 and E3 indicate actual deletion locations in the corresponding gene. The unique BamHI is shown;
b-e. Construction scheme for the pAdBest_dITR helper plasmid. For details, see Materials and methods; and
f. Structural configuration of the cloning shuttle plasmid pBest. Black and gray boxes indicate the 5' and 3' homologous stretches for iHoA.

FIG. 8 shows construction of the pGLAd genome plasmid. For details see Materials and methods.
FIG. 9 shows schematic illustration of iHoA for the construction of recombinant pGLAd_LacZ in accordance with an embodiment of the present disclosure;

a. The black boxes indicate PmeI sites, and arrowheads indicate the cleavage position of PmeI. PmeIcleaved pBest_LacZ was mixed with ClaI-cut pGLAd and subjected to iHoA. 68 bp and 49 bp indicate the 5' and 3' homologous stretches, respectively, between pBest and pGLAd;

b. Intermediate result of iHoA. Aligned double-stranded DNAs (a) were converted from single-stranded ones. This process resulted in hybrid-annealing and transferred the 5' ITR, Ψ packaging signal and LacZ expression cassette to the pGLAd genome plasmid; and
c. Result of completed iHoA. The annealed strands are repaired in transformed bacterial cells.

FIG. 10 shows efficiency of iHoA in the construction of recombinant pGLAd_X in accordance with an embodiment of the present disclosure;

a. Bacterial colonies formed on an agar plate following iHoA. The arrows indicate smaller colonies, potentially containing the correct recombinant pGLAd_X plasmids;
b. The restriction map for pGLAd_LacZ. E and B indicate EcoRI and BamHI sites, respectively. Numbers denote the locations of the corresponding restriction sites;
c, Colony screening result. Five smaller colonies were picked and subjected to plasmid purification. The plasmids were digested with EcoRI and resolved on an agarose gel. M is the lambda HindIII size marker; and
d, Additional restriction digestion results. Clones 1 and 2 were doubly digested with EcoRI and BamHI. M is the lambda HindIII size marker.

FIG. 11 shows sequence verification of iHoA junctions in accordance with an embodiment of the present disclosure:

a. iHoA result. The colored arrowheads indicate sequencing primers. The arrowhead points to the starting nucleotide of the ClaI site (5'-ATCGAT-3', destroyed after iHoA) used for the linearization of pGLAd in iHoA. The arrows indicate the ends of the homologous stretches used for iHoA; and
b and c. Sequencing results. The junction points are boxed. The arrows and arrowhead indicate the corresponding position described in a.

FIG. 12 shows construction of the pGLAd3, which is a novelgenome plasmid in accordance with an embodiment of the present disclosure;

a. Schematic illustration of the mouse E-cadherin intron 2 region. The vertical black boxes indicate exons of the E-cadherin gene. The horizontal lines show the PCR products obtained using the primer sets (Table 20). N, C and F1-F5 represent the names of the PCR products. The numbers on the horizontal lines indicate the length of the PCR products.
b. Construction scheme for the pGLAd3. For details, see Materials and methods; and
c. Schematic illustration of the pBest4 cloning shuttle plasmid. Black and gray boxes indicate the 5' and 3' homologous stretches for iHoA. MCS represents the multi-cloning sites for transgenes.

FIG. 13 shows two kinds of helpers for GLAd production and their effects on the generation of adenovirus and RCA contaminants in accordance with an embodiment of the present disclosure through schematic representation of GLAd production with helpers in HEK293T or HEK293 packaging cells. Homologous recombination can occur in the colored boxed regions. RCA represents replication-competent adenovirus.
FIG. 14 shows conversion of the pGLAd3 into the pGLAd4 genome plasmid in accordance with an embodiment of the present disclosure. The pGLAd3 contains two BssHII sites. The pGLAd3 was digested with BssHII and self-ligated. This process deleted the portion indicated with the asterisk, decreasing the total length from 26,597 bp to 16,392 bp.

## Best Mode for Carrying out the Invention

The present disclosure pertains to a helper plasmid-based gutless adenovirus (GLAd) production system.

## Mode for Carrying Out the Invention

A better understanding of the present disclosure may be obtained through the following examples, which are set forth to illustrate, but are not to be construed as limiting the present disclosure.

**Materials and Methods**

**Reagents, kits, experimental mice, and general cloning techniques**

All the restriction enzymes, Klenow fragment and HindIII-digested lambda phage DNA were purchased from New England Biolabs (MA, USA). AnyFusion and Pfu polymerase were obtained from Genenmed (Seoul, Korea). ψ5, which is a kind of Ad5, was as described previously. Chemical reagents were obtained from Sigma (MO, USA). Dulbecco's modified Eagle's medium and fetal bovine serum (FBS) were purchased from Welgene (Gyeongsangbukdo, Korea) and CellSera (NSW, Australia), respectively. Chemically competent XL-1 Blue and DH10b cells were purchased from RBC (Taipei, Taiwan). The human dystrophin gene, the codon-optimized human huntingtin gene, and mshRs were synthesized by GenScript (NJ, USA). Polymerase chain reaction (PCR) primers and synthetic oligos were obtained from Cosmogenetech (Seoul, Korea). Nucleotide sequence analysis was also performed by Cosmogenetech. The T-blunt PCR cloning kit and LaboPass Tissue Genomic DNA Isolation Kit were purchased from SolGent (Daejeon, Korea) and Cosmogenetech, respectively. Q Sepharose XL and Chelating Sepharose FF resin for column chromatography were obtained from GE Healthcare (IL, USA). The Vivaspin Turbo ultrafiltration spin column (100 kDa cut-off) was purchased from Sartorius (Goettingen, Germany). Benzonase was obtained from Merck (Darmstadt, Germany). Dystrophin-knockout MDX (C57BL/10ScSn-Dmdmdx/J) and wild-type mice (C57BL/10J) were obtained from the Jackson Laboratory (ME, USA). A dystrophin antibody (ab15277) and a huntingtin antibody (sc-47757) were purchased from Abcam (Cambridge, UK) and Santa Cruz Biotechnology (CA, USA), respectively. A β-actin antibody (Abc-2002) was obtained from AbClon (Seoul, Korea). SuperSignal West Pico Chemiluminescent Substrate solution was purchased from Fisher Scientific (NH, USA). HEK293T and HEK293 cells were obtained from ATCC (VA, USA). For the cloning and engineering of DNA sequences, standard DNA manipulation techniques were employed.

**EXAMPLE 1. Construction of pBest Cloning Shuttle Plasmid**

The DNA fragment corresponding to the $Kan^r$~ColE1 region was synthesized by PCR using the following primer set, with the pGT2 plasmid (SEQ ID NO: 63) serving as a template:

TABLE 1

| SEQ ID NO: | Name | Sequence (5' -> 3') | Note |
|---|---|---|---|
| 1 | KanColE1_1F | GGGCCAAGGATCTGATGGCGCAGGGGA | |
| 2 | KanColE1_1R | CTTGGCCGCAGCGGCCGAGCAAAAGGCCAGCAAAAGGCCA | |

A DNA fragment encompassing the 5' homologous stretch, 5' inverted terminal repeat (ITR), ψ5 packaging signal, CMV promoter, a multi-cloning site (MCS), an SV40 poly(A) signal, and the 3' homologous stretch (FIG. 7f) was chemically synthesized by GenScript. This synthetic DNA included an SfiI site at the 5' end and an XcmI site at the 3' site. The PCR product and synthetic DNA were ligated together using the SfiI and XcmI restriction sites to generate the pBest plasmid (SEQ ID NO: 64)

**EXAMPLE 2: Construction of pAdBest_dITR Helper Plasmid**

A PCR product was obtained in the same manner as in Example 1, with the exception of using the following primer set:

TABLE 2

| SEQ ID NO: | Name | Sequence (5' -> 3') | Note |
|---|---|---|---|
| 3 | KanColE1_2F | CCCGGATCCGCAGTGGGCTTACATGGCGATAGC | |
| 4 | KanColE1_2R | CCCGTATACATCGATTTAATTAAGAGCAAAAGGCCAGC | |

The PCR product (Kan~ColE1 region) was digested with BamHI/BstZ17I and ligated with the BstZ17I- BamHI fragment (SEQ ID NO: 65) of the ψ5 genome. Then, the "asterisk mark to BstZ17I" DNA fragment (SEQ ID NO: 66 Fig. 7a) was prepared by PCR using the primer set and inserted into ψ5-Left-Arm-1 to produce ψ5-Left-Arm-2.

TABLE 3

| SEQ ID NO: | Name | Sequence (5' -> 3') | Note |
|---|---|---|---|
| 5 | Cla_delE1_F | GGGATCGATTTAAGGGTGGGAAAGAATATATAAG | |
| 6 | BstZ_R | CCCGTATACGGGGACACGGACAGCCTTTTCGTC | |

ψ5-Left-Arm-2 is devoid of nucleotides 1-3133 (portion of the 5' ITR and ψ packaging signal) of Ad5 (adenovirus type 5, GenBank AC_000008).

To construct ψ5-Right-Arm-1 (FIG. 7c), the DNA fragment corresponding to the $Kan^r$~ColE1 region was prepared by PCR using the following primer set

TABLE 4

| SEQ ID NO: | Name | Sequence (5' -> 3') | Note |
|---|---|---|---|
| 7 | KanColE1_3F | TTAATTAAGCAGTGGGCTTACATGGCGATAGC | |
| 8 | KanColE1_3R | CCCGGATCCATCGATTTAATTAAGAGCAAAAGGCCAGC | |

The PCR product was digested with BamHI and ligated with the DNA fragment (SEQ ID NO: 67) corresponding to the BamHI-3' ITR of the ψ5 genome.

The partially remaining E3 region in the ψ5 genome was completely deleted to reduce the genome size of the adenovirus by overlapping PCR using the unique SpeI (27,082) and NdeI (31,089) restriction sites and the primer set of Table 5, with Ad ψ5 serving as a template.

TABLE 5

| SEQ ID NO: | Name | Sequence (5' -> 3') | Note |
|---|---|---|---|
| 9 | E3_Spe_F | GGGACTAGTTTCGCGCCCTTTCTCAAATTTAAGC | |
| 10 | delE3_R | GCGGATGGACAGGAACTTATAACATTCAGTCGTAG | |
| 11 | delE3_F | CTACGACTGAATGTTATAAGTTCCTGTCCATCCGC | |
| 12 | E3_Nde_R | GGGCATATGGATACACGGGGTTGAAGGTATCTTC | |

The resultant ψ**5-Right-Arm-2** construct was devoid of a portion corresponding to nucleotides 27,864-31,000 of Ad5.

Thereafter, the ClaI-BamHI viral DNA (SEQ ID NO: 69) was cleaved out from ψ5-Left-Arm-2 and ligated to ψ**5-Right-Arm-2** to construct pAdBest (FIG. 7d).

Removal of the 3' ITR from pAdBest was carried out via the following steps:
pAdBest was first cleaved with ClaI/EcoRI, and a smaller fragment was isolated and ligated with the following adaptor containing ClaI and EcoRI sites to afford pAdBest_EcoR_Cla.

TABLE 6

| SEQ ID NO: | Name | Sequence (5' -> 3') | Note |
|---|---|---|---|
| 13 | Cla_EcoR_S | CGATCCGGAGGCCCTTG | |
| 14 | Cla_EcoR_AS | AATTCAAGGGCCTCCGGAT | |

Subsequently, pAdBest_EcoR_Cla was cut with AvrII/RsrII, and a larger fragment was isolated. Overlapping PCR was performed using the following primer sets, with pAdBest serving as a template.

TABLE 7

| SEQ ID NO: | Name | Sequence (5' -> 3') | Note |
|---|---|---|---|
| 15 | Avr_F | CTGCCTAGGCAAAATAGCACCC | |
| 16 | Avr_dITR_R | AACGATGTAAGTTTTAGGGCGGAGTAACTTGTATG | |

(continued)

| SEQ ID NO: | Name | Sequence (5' -> 3') | Note |
|---|---|---|---|
| 17 | Avr_dITR_F | GCCCTAAAACTTACATCGTTAATTAAGCAGTGGGC | |
| 18 | Rsr_R | TAGCGGTCCGCCACACCCAGCC | |

The PCR product (SEQ ID NO: 70) was digested with AvrII/RsrII and ligated with the larger fragment of pAdBest_EcoR _Cla cleaved with AvrII/RsrII, resulting in the production of pAdBest_EcoR_Cla_dITR with complete removal of the 3' ITR from pAdBest. Then, the ClaI-EcoRI fragment of pAdBest was ligated back to the corresponding sites of pAdBest_EcoR_Cla_dITR to construct pAdBest_dITR (SEQ ID NO: 71) (FIG. 7e).

**EXAMPLE 3. Construction of pGLAd Genome Plasmid**

PCR was performed using the following primer set, with pAdBest serving as a template:

TABLE 8

| SEQ ID NO: | Name | Sequence (5' -> 3') | Note |
|---|---|---|---|
| 19 | KanColE1_4F | GGTTGGCGCGCCCTACGTCACCCGCCCCGTTCCCAC | |
| 20 | KanColE1_4R | CCCGAGCTCAAACTACATAAGACCCCCACCTTAT | |

The PCR product (SEQ ID NO: 72) thus obtained was cut with SacI/AscI and then ligated to the following adaptor to produce pAdBestGL1 (FIG. 8a).

TABLE 9

| SEQ ID NO: | Name | Sequence (5' -> 3') | Note |
|---|---|---|---|
| 21 | Sac_Pst_Avr_Asc_S | CTTAACCTGCAGATCCTCCTAGGTTTTTGG | |
| 22 | Sac_Pst_Avr_Asc_AS | CGCGCCAAAAACCTAGGAGGATCTGCAGGTTAAGAGC T | |

To remove the unique ClaI site from pAdBestGL1, pAdBestGL1 was cleaved with ClaI, filled-in with Klenow, and then self-ligated, which resulted in pAdBestGL1_dCla. Both pAdBestGL1 and pAdBestGL1_dCla were digested with SacI/AvrII and ligated with a synthetic HpaI-site-containing adaptor (Fig. 8b), which generated pAdBestGL2_wtCla and pAdBestGL2, respectively.

Table 10

| SEQ ID NO: | Name | Sequence (5' -> 3') | Note |
|---|---|---|---|
| 23 | Sac_Hpa_Avr_S | CGGGCGGCGACCTCGCGGGTTAACCGTCCTTTAAAAAA GTCGTTTCTGCAAGCTC | |
| 24 | Sac_Hpa_Avr_AS | CTAGGAGCTTGCAGAAACGACTTTTTTAAAGGACGGTT AACCCGCGAGGTCGCCGCCCGAGCT | |

Then, HpaI-digested pAdBestGL2 was mixed with HindIII-digested lambda phage DNA (arrowhead, FIG. 8c) and subjected to in vitro homologous annealing (iHoA) to construct pAdBestGL3. pAdBestGL3 was cleaved with SacI/ApaI or ApaI/AvrII and ligated with a SacI/ApaI adaptor (FIG. 8d) or an ApaI/AvrII adaptor (FIG. 8e) to produce pAdBestGL4_3H and pAdBestGL4_5H, respectively.

Table 11

| SEQ ID NO: | Name | Sequence (5' -> 3') | Note |
|---|---|---|---|
| 25 | SacI/ApaI_S | CATGGTTCCAAAATGCCCCTTAACCGGGT TGGGCC | |
| 26 | SacI/ApaI_A S | CAACCCGGTTAAGGGGCATTTTGGAACCA TGAGCT | |

Table 12

| SEQ ID NO: | Name | Sequence (5' -> 3') | Note |
|---|---|---|---|
| 27 | ApaI/AvrII_S | CATGGTTCCAAAATGCCCCTTAACCGGGTTC | |
| 28 | ApaI/AvrII_A S | CTAGGAACCCGGTTAAGGGGCATTTTGGAAC CATGGGCC | |

pAdBest4_3H_2dCla was constructed by consecutively removing the ClaI sites from pAdBestGL4_3H. In detail, pAdBestGL4_3H was cleaved with ClaI, filled-in with Klenow, self-ligated, and transformed into Dam-/- bacterial cells. The resultant pAdBestGL4_3H_dCla construct was cut again with ClaI, filled-in with Klenow and self-ligated to generate pAdBestGL4_3H_2dCla. Similarly, pAdBestGL4_5H was digested with ClaI, filled-in with Klenow and self-ligated to produce pAdBestGL4_5H_dCla. Then, the SacI-ApaI fragment (5' half portion) of pAdBestGL4_5H_dCla was transferred to SacI/ApaI-cleaved pAdBestGL4_3H_2dCla to construct pAdBestGL5 (FIG. 8f). To produce pAdBestGL (FIG. 8g), pAdBestGL2_wtCla was cut with HpaI, mixed with SacI/AvrII-digested pAdBestGL5 (FIG. 8f) and subjected to iHoA. Then, pAdBestGL5 was cleaved with ApaI/NsiI and ligated with the SMAR element prepared by PCR using the following primer set, to finally construct pGLAd (FIG. 8h) .

Table 13

| SEQ ID NO: | Name | Sequence (5' -> 3') | Note |
|---|---|---|---|
| 29 | pGLAdSMAR_F | TCTGGGCCCAAATAAACTTATAAATTGTGAGAG | |
| 30 | pGLAdSMAR_R | CCCATGCATATATTTAAAGAAAAAAAAATTGTA | |

**EXAMPLE 4. In Vitro Homologous Annealing (iHoA)**

iHoA was performed using AnyFusion. The entire procedure followed the manufacturer's instructions with slight modification: incubation was performed for 10 min at 55°C, followed by further incubation for 20 min at room temperature. Then, the reaction mixture was transformed into chemically competent XL-1 Blue or DH10b cells and spread over an antibiotic-containing agar plate

**EXAMPLE 5. Generation of GLAd.LacZ and GLAd3.LacZ**

The pGLAd_LacZ or pGLAd3_LacZ plasmid (10 $\mu$g) was cut with the PacI restriction enzyme, and PacI activity was heat-inactivated. This PacI-linearized pGLAd_LacZ or pGLAd3_LacZ genome plasmid and 30 $\mu$g of the pAdBest _dITR helper plasmid were co-transfected into HEK293T cells plated in a 100 mm culture dish using the calcium phosphate precipitation method. After 6 hours of incubation, the culture medium (10% FBS) was changed to a fresh medium (5% FBS). Forty-eight hours later, the transfected cells and media were harvested (in this step, the culture medium was saved as the viral medium), and the cells were resuspended in 1 ml of fresh culture medium (5% FBS) and disrupted through three cycles of freezing and thawing (the cleared lysate was referred to as the viral lysate). The viral lysate was harvested and used to infect HEK293 cells (recombinant GLAd.LacZ and GLA3.LaZ viruses cannot induce lytic cell death in treated HEK293 cells because the GLAd virus alone cannot be amplified in HEK293 cells). Forty-eight hours after treatment, the cells were stained for LacZ expression.

**EXAMPLE 6. LacZ Staining**

The culture medium was removed from the HEK293 cells, and the cells were fixed with fresh fixation solution (2% formaldehyde/methanol and 0.1% glutaraldehyde in PBS) for 2 min at room temperature. After two careful washes with PBS, the cells were incubated with staining solution [1 mg/ml X-gal, 2 mM magnesium chloride ($MgCl_2$), 5 mM potassium ferri-cyanide, and 5 mM K ferro-cyanide in PBS) at 37°C until LacZ staining was evident

**EXAMPLE 7. Purification of Genomic DNA from Mouse Tail**

Genomic DNA was purified from the tail of a C57BL/6J mouse using the LaboPass Tissue Genomic DNA Isolation Kit. The entire procedure followed the manufacturer's instructions with slight modification. Briefly, the mouse tail (2 x 2 mm piece) was incubated with lysis buffer containing proteinase K until the tissue was completely lysed. The sample was mixed with 100% ethanol and passed through a mini spin column. Bound genomic DNA was thoroughly washed with two different wash buffers and eluted with distilled water.

**Cloning of Mouse E-Cadherin Intron 2 Region as pGLAd3 Genome Backbone**

Genomic DNA purified from the mouse tail was used as a PCR template. The primer sets and sequences are described in Table 20. PCR amplification of the F1, F2, F3, F4, or F5 fragment was performed for 45 cycles (30 seconds at 95°C, 30 seconds at 60-65°C, 4 min at 72°C) with each primer set using Pfu polymerase. Each PCR product was cloned into the T-Blunt vector of the T-Blunt PCR cloning kit. The resultant T-F1, T-F2, T-F3, T-F4, and T-F5 products (FIG. 12b) were sequenced for verification.

**EXAMPLE 9. Construction of pGLAd3 Genome Plasmid**

Overlapping PCR was carried out for 50 cycles (30 seconds at 95°C, 30 seconds at 62°C, 60 seconds at 72°C) using the primer sets N-F/N-R and C-F/C-R (Table 20), with the genomic DNA from the mouse tail serving as a PCR template. The resultant NC fragment PCR product, containing sites for restriction enzymes such as BspEI, XhoI, and NsiI, was subjected to iHoA with pGLAd cut with SacI/AvrII (FIG. 12b) to produce pGLAd_NC. Next, pGLAd_NC was cleaved with NsiI/XhoI and ligated with the annealed synthetic Nsi-Xho fragment (Table 12b) to construct pGLAd_NC_PU, which was the final recipient harboring the F12345 fragment. In parallel, T-F1_dPac was generated by removing the unique PacI site of T-F1 with PacI, blunting with Klenow and self-ligation. T-F2_SMAR was prepared following the ligation of SfiI/SalI-cleaved T-F2 with the SMAR element prepared by PCR using the SMAR-F/SMAR-R primers (table 20), with pGLAd serving as a template. Then, T-F12 was generated by transferring the NotI/RsrII-cut T-F1_dPac fragment to NotI/RsrII-cleaved T-F2_SMAR. The construction of F-T34 was carried out following the transfer of the T-F4 fragment, which was processed with cutting with PvuI, blunting with Klenow, heat-inactivating and additionally cutting with PciI, to the T-F3 that was obtained through processing via cutting with SpeI, blunting with Klenow, heat-inactivating and additionally cutting with PciI. T-F345 was produced by ligating the MluI-XbaI fragment of T-F5 with T-F34 cut with SpeI/MluI (the SpeI site can be ligated to the XbaI site). T-F12345 was generated by transferring the SalI-RsrII fragment of T-F12 to the T-F345 cut with SalI/RsrII. Ultimately, pGLAd3 was constructed utilizing iHoA between PstI-cut pGLAd_NC_PU and XhoI-cut T-F12345. In every step of the construction procedure, sequencing was performed for verification

**EXAMPLE 10. Construction of pBest4 Cloning Shuttle Plasmid**

The pCAG portion of the plasmid was prepared by PCR using the following primer set and then cutting the PCR product with AseI and BsaI.

Table 14

| SEQ ID NO: | Name | Sequence (5' -> 3') | Note |
|---|---|---|---|
| 31 | pCAG_F | GTTATTAATAGTAATCAATTACG | |
| 32 | pCAG_R | CTTGGGTCTCCCTATCGCCCGCCGCGCTTCGCTTTTTATAGG | |

The β-globin intron region was also prepared by PCR with the following primers and the cutting the PCR productt with BsaI and HindIII.

Table 15

| SEQ ID NO: | Name | Sequence (5' -> 3') | Note |
|---|---|---|---|
| 33 | bGint_F | GGCGATAGGGAGACCCAAGCTGGTGAGTTTGGGGACCC | |
| 34 | bGint_R | GGGAAGCTTGGGTCCCCTGTAGGAAAAGAAGAAGGCATGAAC | |

The pBest cleaved with AseI/HindIII was ligated with the prepared PCR products to construct the pBest4 shuttle plasmid (SEQ ID NO: 75)

**EXAMPLE 11. Plaque Assay**

HEK293 cells (80-90% confluency) plated in a 100 mm culture dish were treated with Ad.LacZ (first-generation Ad as a positive control; 1 x $10^3$ infectious viral particles), GLAd3.LacZ (1.12 x $10^7$ -1.80 $\times$ $10^7$ BFU) or the cell lysate prepared from HEK293T cells transfected with the helper plasmid alone (Table 18). Twenty-four hours later, the culture medium was aspirated, carefully overlaid with 12 ml of a sterilie culture medium containing agarose (0.3%) and incubated in a $CO_2$ incubator for 10-15 days. A 10 ml aliquot of the diluted MTT solution was added to the agarose layer and incubated for 5 hours. Viral plaques were counted on a light box.

**EXAMPLE 12. Construction of pAd5pTP expression plasmid**

The pTP gene of adenovirus type 5 (Ad5) was prepared by PCR using the following primer set, with the Ad $\Psi$5 DNA serving as a template:

Table 16

| SEQ ID NO: | Name | Sequence (5' -> 3') | Note |
|---|---|---|---|
| 35 | Ad5pTP_F | GGG**AAGCTT**ACCATGGCCTTGAGCGTCAACGATTGCGCGCGCCTGACC | |
| 36 | Ad5pTP_R | GGC**GAATTC**CTAAAAGCGGTGACGCGGGCGAGCC | |

The bold portion in SEQ ID NO: 21 is the HindIII site.

The bold portion in SEQ ID NO: 22 is the EcoRI site.

PCR was performed with 40 cycles (30 seconds at 95°C, 30 seconds at 60°C, 120 30 seconds at 72°C). The PCR product thus obtained was digested with HindIII and EcoRI and cloned into the pLV_XL plasmid prepared by clevage of pLV_VSVG_XL with HindIII and EcoRI to construct pAd5pTP (SEQ ID NO: 76) expression plasmid.

**EXAMPLE 13. Large-scale production of recombinant GLAd**

To produce recombinant GLAd at a large scale, continuous amplification was employed as demonstrated in the standard method for conventional GLAd production. In brief, HEK293T cells (50-70% confluency) plated in a 100 mm dish were transfected with a mixture of 10 $\mu$g of pGLAd3_LacZ (linearized with PacI and heat-inactivated), 30 $\mu$g of pAdBest_dITR, and 2.5 $\mu$g of pAd5pTP using the calcium phosphate precipitation method. After 6 hours of incubation, the culture medium (10% FBS) was changed to fresh medium (5% FBS). Forty-eight hours later, the transfected cells were harvested, resuspended in 1 ml of fresh culture medium (5% FBS) and disrupted through three cycles of freezing and thawing to rescue the GLAd3. LacZ virus (P0 seed GLAd). For the first round of amplification (P1), HEK293T cells (50-70% confluency) plated in a 100 mm dish were transfected with a mixture of 45 $\mu$g of pAdBest_dITR and 3.75 $\mu$g of pAd5pTP using the calcium phosphate precipitation method. After 6 h of incubation, the culture medium (10% FBS) was changed to fresh medium (5% FBS) containing P0 seed GLAd. Forty-eight hours later, the transfected cells and media were harvested (in this step, the culture medium was saved as the viral medium), and the cells were resuspended with 1 ml of fresh culture medium (5% FBS) and disrupted through three cycles of freezing and thawing (the cleared lysate was referred to as the viral lysate). The viral lysate was harvested and combined with the viral medium (total 10 ml) and used to infect HEK293T cells (plated in 10 x 150 mm dishes) for the next round of amplification (P2) (see FIG. 3). Additional rounds of amplification (P3, P4, P5 and so on) can be carried out to increase the GLAd production scale. The flow chart for each round of amplification, the obtained viral titers, the number of plates needed and the required amounts of the helper and pAd5pTP plasmids are described in detail in FIG. 3.

**EXAMPLE 14. Amplification of Adenovirus and RCA Contaminant Generated During GLAd Production**

Unlike GLAd, adenovirus and RCA can replicate in HEK293 cells. Thus, HEK293 cells were infected with Ad. LacZ (positive control) or GLAd3.LacZ (P3, 3 x $10^9$ BFU; P4 or P5, 1 x $10^8$ BFU), and the potential contaminant adenovirus and RCA in GLAd3.LacZ were allowed to replicate during one (P4 or P5), approximately two (positive control), or three (P3) rounds of amplification. Because an MOI (multiplicity of infection) of GLAd3.LacZ that is too high, resulting in overexpression of LacZ, can kill infected cells, two 150 mm dishes were used for the initial infection of GLAd3.LacZ (P3, 3 x $10^9$ BFU). Benzonase was employed to degrade the helper plasmid that was continuously used for the preparation of P3, P4, or P5 GLAd3.LacZ (the helper plasmid contains the target gene for PCR-based analysis; the viral DNA packaged into the capsid shell is resistant to Benzonase). To examine its effects on the infectivity of adenovirus and RCA, Benzonase treatment was also applied to the positive control (Ad.LacZ). For each round of amplification, cells were infected with the corresponding virus and then harvested after 72 h. These cells were resuspended in a volume of 1 ml (for a 100 mm dish) or 2.5 ml (for each 150 mm dish) and disrupted via three cycles of freezing and thawing (the cleared lysate was referred to as viral lysate). Each viral lysate was used for further amplifications, and the cell lysate and culture medium were finally harvested together. During these amplification processes, CPE (cytopathic effect) was examined under a microscope (for the entire workflow, see FIGS. 4a and 4c).

**EXAMPLE 15. Analysis of Adenovirus and RCA Contaminant by PCR**

The serially amplified samples from HEK293 cells (FIGS. 4a and 4c) were first heat-treated for 10 min at 95°C (if this step is omitted, endogenous cellular DNase degrades the spiked Ad5 DNA) and then analyzed by PCR for N-terminal DNA of the fiber gene, which is present in both adenovirus and RCA but not in the GLAd genome or HEK293 cells. Ad5 DNA (10 pg, Ad Ψ5 DNA) was used as a positive control for PCR. Ad.LacZ samples were subjected to PCR before or after 100× dilution, in which only 500 virus particles are contained in the sample. GLAd3. LacZ samples were PCR-amplified in the presence or absence of spiked Ad5 DNA (10 pg). PCR was carried out for 40 cycles (30 seconds at 95 °C, 30 seconds at 60 °C, 30 seconds at 72°C) in the presence of Pfu polymerase using the following primer set, and the results were analyzed by agarose gel electrophoresis (FIGS. 4b and 4d):

Table 17

| SEQ ID NO: | Name | Sequence (5' -> 3') | Note |
|---|---|---|---|
| 37 | Ad5Fiber F | CGCGCAAGACCGTCTGAAGATACC | |
| 38 | Ad5Fiber R | GGCCTGATGTTTGCAGGGCTAGC | |

**EXAMPLE 16. Construction of the pGLAd4 Genome Plasmid**

The pGLAd3 contains two BssHII restriction sites (FIG. 14). Treatment of pGLAd3 with BssHII and self-ligation resulted in pGLAd4 (SEQ ID NO: 77), which decreased in the length of plasmid from 26,597 bp (pGLAd3) to 16,392 bp (pGLAd4). After completion, the BssHII site was sequenced for verification.

**EXAMPLE 17. Construction of Huntingtin mshR Expression Plasmid**

The template for mshR expression, which was confirmed to be fully functional, was described previously. Based on these conserved sequence and structural characteristics, the corresponding DNAs were synthesized (Table 21) and cloned into the pGT2 plasmid (SEQ ID NO: 63) using the BamHI and EcoRI sites.

**EXAMPLE 18. Knockdown of Endogenous Huntingtin Expression by mshR**

HEK293T cells plated in a 100 mm culture dish were transfected with 15 μg of pGT2, pGT2-mshR1, pGT2-mshR2, or pGT2-mshR3. Forty-eight hours later, untreated control and transfected cells were harvested and subjected to western blotting analysis for endogenous huntingtin expression.

**EXAMPLE 19. Western Blotting**

Whole-cell lysates prepared using RIPA lysis buffer were resolved in an SDS gel and transferred to a nitrocellulose membrane. The membrane was blocked with Blotto A solution (TBST, 5% milk) for 1 hour at room temperature and further incubated with Blotto B solution (TBST, 1% milk) containing the primary antibody (1:500-1000) at 4°C overnight. After one 5 min wash with TBST (TBS, 0.05% Tween-20; TBS (Tris-buffered saline): 10 mM Tris-Cl (pH 8.0), 150 mM NaCl), the membrane was incubated with an HRP-conjugated secondary antibody (1:5000-100,000) in Blotto B solution for 2 hours at room temperature. Following two washes (5 min each) with TBST, the membrane was briefly treated with the SuperSignal West Pico Chemiluminescent Substrate solution, and the resulting image was analyzed with a Bio Imaging System.

**EXAMPLE 20. Construction of the pGLAd4_HTTmshR1/3**

HTTmshR1 and HTTmshR3 encompassing pCMV, mshR and BGHpA (FIG. 5d) were prepared by PCR using appropriate primer sets (Table 22), with pGT2-mshR1 or pGT-mshR3 serving as templates (FIGS. 5c and 5d). To construct pGLAd4_HTTmshR1/3, the resultant HTTmshR1 and HTTmshR3 constructs were subjected to iHoA with Acc65I-cut pGLAd4 and PciI-cut pGLAd4, respectively (FIG. 5f). After completion, the regions adjacent to the Acc65I and PciI sites were sequenced for verification.

**EXAMPLE 21. Construction of pGLAd4_coHTT.HTTmshR1/3 and pGLAd4_coHTT(R).HTTmshR1/3**

The full-length codon-optimized synthetic huntingtin gene (9.4 kb) containing Q22 was cloned into the pBest4 shuttle plasmid in different orientations. In an approach previously demonstrated as a standard procedure, pBest4_coHTT and pBest4_coHTT(R) were cut with PmeI and subjected to iHoA with ClaI-cleaved pGLAd4_HTTmshR1/3. This iHoA process produced the pGLAd4_coHTT.HTTmshR1/3 or pGLAd4_coHTT(R). HTTmshR1/3 construct. Successful completion was sequenced for verification.

**EXAMPLE 22. Production of Recombinant GLAd4.coHTT.HTTmshR1/3 and GLAd4.coHTT(R).HTTmshR1/3 Viruses**

The same procedure used for producing the recombinant GLAd.LacZ and GLAd3.LacZ viruses was applied.

**EXAMPLE 23. Purification of recombinant GLAd4.Dys Virus**

The recombinant GLAd4.Dys virus was produced as shown in FIG. 3 and purified from a P3 preparation. For purification, two column-based chromatography methods were employed as reported previously, with slight modification. Briefly, the cell lysate and the harvested culture medium were combined (P3), treated with Benzonase, filtered and subjected to Q Sepharose column chromatography as a first step of purification. After washing, the bound virus was eluted and diluted with buffer and loaded onto a Zn-chelated chromatography column. The column was thoroughly washed, and the bound virus was eluted. Buffer change to a formulation buffer and concentration of virus were simultaneously carried out utilizing Vivaspin Turbo ultrafiltration spin column. The formulation buffer was described previously.

**EXAMPLE 24. Determination of GLAd4.Dys Viral Particles**

The purified recombinant GLAd4.Dys virus was serially diluted using virus lysis buffer [0.1% SDS, 10 mM Tris-Cl (pH 7.4), 1 mM EDTA] and incubated for 10 min at 56°C with gentle shaking. Then, the $OD_{260}$ was determined and subjected to the calculation of the virus particle concentration using the following equation:

[Equation]

$$\text{Virus particles (VP/ml)} = (OD_{260}) \times (\text{virus dilution factor}) \times (1.1 \times 10^{12})$$

In this calculation, the blank solution consisted of a mixture of a virus lysis buffer and a virus formulation buffer. The extinction coefficient was used as established:

[Extinction coefficient]

$$OD_{260} \text{ unit} = 1.1 \times 10^{12} \text{ virus particles/ml.}$$

**EXAMPLE 25. Animal Study**

All animal experiments were conducted according to the protocol (KNU2018-0134) approved by the Institutional Animal Use and Care Committee (IAUCC) of Kyungpook National University (Daegu, Korea). Eight-week-old male wild-type control mice (C57BL/10J) and dystrophin-knockout MDX mice (C57BL/10ScSn-Dmdmdx/J) were housed under 12-hour light-dark cycles and given water freely in accordance with the Kyungpook National University Animal Facility regulations. The focal gastrocnemius muscles of the MDX mice were injected intramuscularly with PBS (n = 3) or with 50 $\mu$l of recombinant GLAd4.Dys virus (4 x $10^{10}$ particles) (n = 3). Four weeks later, muscle tissues were biopsied and subjected to analysis.

**EXAMPLE 26. Immunofluorescence Staining**

The immunofluorescence staining of dystrophin was performed as described previously[30] with slight modification. Biopsied muscle tissues were fixed at 4°C overnight with freshly prepared 4% PFA in PBS. Then, the tissues were incubated with 5% sucrose in PBS at 4°C for 6 h and further incubated with 20% sucrose in PBS at 4°C overnight. The processed tissues were embedded in OCT, frozen by dipping in liquid nitrogen-chilled isopentane, and stored at -70°C. Four-micron-thick cross-sections were produced, then placed in PBS for 10 min and washed with PBST (0.1% Triton X-100 in PBS) three times for 10 min each at room temperature. The tissue sections were blocked with 10% horse serum in PBST at 4°C overnight, incubated with a dystrophin antibody (1:100 in blocking buffer) at 4°C overnight, and then washed with PBST three times for 10 min each at room temperature. Finally, the tissue sections were incubated with a TRITC-conjugated secondary antibody (1:100 in blocking buffer) at 4°C overnight and washed with PBST three times for 10 min each at room temperature. The stained tissue sections were covered with mounting medium containing DAPI and analyzed under a confocal microscope.

**Results**

**Construction of pAdBest_dITR Helper Plasmid and pGLAd Genomic Plasmid and Use thereof for Producing GLAd**

Currently, the most commonly used helper adenovirus contains loxP sites flanking the $\Psi$ packaging signal21,22 (FIG. 1a). Its structural characteristics allow this particular adenovirus to efficiently produce recombinant GLAd with reduced contamination of adenovirus in Cre-expressing packaging cells. Nevertheless, contamination of helper adenovirus is unavoidable even in this sophisti cated production system. Moreover, homologous recombination between the helper adenovirus and the E1 region present in packaging cells generates RCA, although RCA has not been intensively analyzed in this system. The present inventors hypothesized that the conversion of the helper adenovirus into a helper plasmid following the deletion of the region involved in homologous recombination might prevent the generation of both adenovirus and RCA contaminants in GLAd preparations. To achieve this goal, the present inventors attempted to generate the helper plasmid that supplies all viral proteins for GLAd packaging but is securely unpackageable into active viral particles, resulting in no generation of RCA.

The helper adenovirus-free recombinant GLAd production system of the present inventors requires two independent plasmids: one serving as a helper for GLAd packaging and the other as the GLAd genome.

As the helper plasmid, the present inventors constructed pAdBest_dITR (~31 kb) (FIG. 7). Based on the overlapping transcription units and multiple transcripts governed by a single promoter (FIG. 1b), the $\Psi$5 genome25, a derivative of Ad5 (FIG. 7a), was manipulated to obtain the helper plasmid. The helper plasmid contains neither ITRs nor $\Psi$ packaging signal (FIG. 1c), both of which are essential for virus packaging. Thus, this plasmid only serves as a helper but is securely unpackageable into active viral particles. During this manipulation, the 5' ITR and $\Psi$ packaging signal were transferred to pBest, a shuttle plasmid (FIG. 7f) that is used for transgene cloning.

As the GLAd genome plasmid, the present inventors constructed pGLAd (FIG. 1c). This pGLAd is composed of stuffer DNA from lambda phage and only the 3' ITR of Ad5. The scaffold matrix attachment (SMAR) element was used to stabilize the GLAd genome in cells while enhancing transgene expression (for entire construction processes, see FIG. 8).

Generally, it is a tedious process to insert a transgene into a large plasmid such as the pGLAd genome plasmid (~27 kb). To facilitate this process, short homologous stretches were added to both pBest and pGLAd when constructing these plasmids. These homologous stretches expedited the transfer of the transgene expression cassette from pBest to pGLAd.

As an example of this process, the LacZ gene was first cloned into pBest, which was then linearized with the rare-cutting enzyme PmeI (Fig. 9a). Simultaneously, pGLAd was cut at the unique ClaI site. When linearized pBest_LacZ and linearized pGLAd were mixed and treated with AnyFusion, a nuclease that can convert doublestranded DNA to single-stranded DNA, both linearized homologous regions were efficiently annealed in vitro (the entire process is referred to as "iHoA" for in vitro homologous annealing) (Fig. 9b). The length of the homologous stretches annealed in vitro results in sufficient stability to transform bacterial cells. The resultant recombinant pGLAd_LacZ plasmid recovered both the ITRs and the $\Psi$ packaging signal, and the ITRs were flanked by rare-cutting PacI restriction sites (Fig. 9c). The entire process is quick and efficient in the transfer of a transgene expression cassette to the pGLAd genome plasmid. Moreover, in the transformation step, the bacterial cells containing the correct pGLAd_LacZ plasmid formed colonies that were markedly smaller in size than those of other undesired clones (Fig. 10a). This visually observable characteristic accelerated the screening process (FIGS. 10b to 10d). After the completion of this process, the junctions of the homologous stretches of the resultant recombinant plasmid were sequenced. The nucleotide sequences perfectly matched the homologous stretches (FIG. 11), indicating that the iHoA process operated as designed.

After the construction of both the pAdBest_dITR helper plasmid and the recombinant pGLAd_LacZ genome plasmid, the present inventors tested the two-plasmid-based recombinant GLAd production system. HEK293T cells were transfected with the pAdBest_dITR helper plasmid and the PacI-linearized pGLAd_LacZ genome (Figs. 1d and 1e). Co-transfected HEK293T cells successfully produced recombinant GLAd.LacZ virus (Fig. 1e), demonstrating that all the constructs and processes involved in the production of recombinant GLAd worked properly as designed.

**Construction of New Genome Plasmid pGLAd3**

Interestingly, the nature of the stuffer in GLAd has been shown to negatively affect transgene expression. In particular, a lambda DNA stuffer showed this undesirable characteristic, although this finding is controversial. Nevertheless, under the initial conditions, the present inventors utilized lambda DNA to quickly examine the validity of the designed plasmid construction schemes and to determine whether the helper plasmid-based GLAd production system would function properly as expected. Since the GLAd production system operated as designed, the present inventors prepared a new stuffer to reduce concern about the decreased expression of transgenes in the presence of lambda stuffer. The present inventors cloned genomic fragments from the mouse E-cadherin intron 2 region (Fig. 12a) and generated pGLAd3 as a new GLAd genome plasmid (Figs. 2a and 12b). The present inventors also constructed another cloning shuttle plasmid, pBest4 (Fig. 12c), which was upgraded with a strong CAG promoter and an intron. Similar to the original system, this new system tested with the LacZ gene also effectively produced recombinant GLAd3.LacZ virus (Table 18, Figs. 2b and 2c) with a cytopathic effect (CPE) (Fig. 2b), indicating that all the elements and processes, such as pCAG, the intron, iHoA, and GLAd packaging, performed correctly as designed. In sharp contrast, HEK293T cells transfected with the pAdBest_dITR helper plasmid alone did not produce any viral particles (Table 18). Additionally, recombinant GLAd produced by the two-plasmid-based system did not contain any other viral species, such as adenovirus or RCA (Table 18). It is clear that the helper plasmid only provides helper function for GLAd packaging but is securely unpackageable into active viral particles. Furthermore, these results indicate that in accordance with its structural characteristics (Fig. 2d), the helper plasmid does not generate RCA (Fig. 13).

**Large-Scale Production of GLAd**

The optimized standard method for conventional large scale GLAd production involves a serial amplification process (Fig. 3a). Every round of this process requires freshly added helper adenovirus since GLAd can be amplified only in the presence of the helper adenovirus. Seed GLAd is repeatedly amplified over 4-6 rounds prior to the final round of amplification in large-scale cell culture (~$1 \times 10^9$ cells). This entire amplification process routinely produces ~$1 \times 10^{12}$ BFU (~1000 BFU/cell; BFU, blue-forming units determined by LacZ staining)[22]. This relatively high yield of GLAd is attributed to the use of a replicable helper adenovirus that can supply a sufficient amount of viral proteins for GLAd packaging and amplification.

In an attempt to produce GLAd at a large scale utilizing the helper plasmid, the present inventors followed the standard amplification procedure established for conventional large-scale GLAd production, although the helper plasmid cannot replicate in GLAd packaging cells. The present inventors have already shown successful production of seed GLAd (~$2 \times 10^7$ BFU/ 100 mm dish) by transfecting the PacI-cut GLAd genome plasmid and the helper plasmid (Table 18). This seed GLAd was well amplified by the helper plasmid, and its levels were scaled up (data not shown), even though the amplification efficiency was not yet optimized.

In previous studies of other researchers, it was shown that the additional expression of adenoviral proteins such as E1A, pTP, and IVa2, either individually or in combination, increases adenovirus production. The present inventors tested these adenoviral proteins individually and in combination to improve amplification efficiency in GLAd production. The additional expression of only precursor terminal protein (pTP) increased GLAd production (~4-fold) (data not shown). Furthermore, the present inventors observed that a greater amount of helper plasmid (45 $\mu$g/ 100 mm dish rather than 30 $\mu$g/100 mm dish) resulted in a higher yield in GLAd production (in P1 and thereafter, not in P0; data not shown).

For each amplification procedure, the pAdBest_dITR helper plasmid and the pTP-expressing plasmid were co-transfected into 293T packaging cells, similar to the conventional amplification procedure utilizing helper adenovirus. Through optimization, the present inventors successfully established a standard procedure for the helper plasmid-based large-scale GLAd production (Fig. 3b). P3 routinely produced $5\times10^{10}$- $1\times10^{11}$ BFU (50-100 BFU/cell), which is only a 10-20-fold-lower yield compared to the conventional large-scale production method utilizing replicable helper adenovirus (for example, conventional method: new method = 1-2 $\times$ $10^{11}$ BFU: 1 $\times$ 1010 BFU). P5 is expected to produce 5 $\times$ $10^{12}$- 1 $\times$ $10^{13}$ BFU from 1 $\times$ $10^{11}$ cells.

**Absence of Adenovirus and RCA Contaminant Generation During GLAd Production**

Preventing contamination of adenovirus and RCA in GLAd preparation is crucial for considering the clinical application of GLAd. The present inventors have demonstrated that GLAd can be produced by the transfection of the PacI-cut GLAd genome plasmid and the helper plasmid, and none of the three independent GLAd preparations contained any adenovirus and/or RCA (Table 18). Nevertheless, the present inventors analyzed adenovirus and RCA more intensively again in GLAd preparations (P3, P4, or P5) (Fig. 3) whose scales were large enough and, thus, might be used to produce the clinical materials in the future.

Adenovirus (Ad.LacZ) used as a positive control was efficiently amplified in HEK293 cells (Fig. 4a). One infectious viral particle (1 BFU) generated ~5 $\times$ $10^5$ BFU in less than two complete rounds of amplification. This robust amplification activity of adenovirus caused a severe CPE, which was easily observable with the naked eye. These results suggest that even a single infectious contaminant adenovirus (or RCA) particle in GLAd preparations results in numerous viral particles after a few rounds of amplification. The present inventorse analyzed P3 GLAd3.LacZ (Fig. 3) containing 3 $\times$ $10^9$ BFU (Fig. 4a). Following infecting HEK293 cells with this GLAd, the amplification of adenovirus and RCA, if any, was allowed for three consecutive rounds. These amplification conditions were sufficient for 1 BFU to generate at least 5 $\times$ $10^5$ BFU, causing a severe CPE in HEK293 cells in the final round. However, any CPE could not be observed in infected cells. Accordingly, any adenoviral species (either adenovirus or RCA) could not be detected in the PCR-based analysis (Fig. 4b). These data indicate that the preparation of P3 GLAd (at least 3 $\times$ $10^9$ BFU) did not contain any adenovirus and/or RCA contaminants.

Additionally, the present inventors analyzed the presence of adenovirus and RCA contaminants in P4 and P5 GLAd3.LacZ (1 $\times$ $10^8$ BFU for each). P4 and P5 GLAd were prepared utilizing P3 and P4 GLAd, respectively, as described (Fig. 3). HEK293 cells were infected with P4 or P5 GLAd pretreated with Benzonase, which removes the helper plasmid but not the capsid-protected adenovirus or RCA, and allowed the continued amplification of any potential adenovirus and RCA for additional rounds (P4: one round from P3; P5: two rounds from P3) (Fig. 4c). Similar to P3 GLAd, it was impossible to detect any adenoviral species (either adenovirus or RCA) in the PCR-based analysis (Fig. 4d). Taken together, the data clearly demonstrate that the helper plasmid-based GLAd production system does not generate adenovirus and/or RCA as undesirable contaminants.

**New GLAd as Efficient in Vitro and in Vivo Gene Delivery Vector**

Following all the successes described above, the present inventors attempted to test the gene delivery activity of ther recombinant GLAd in vitro and in vivo. The present inventors chose huntingtin (9.4 kb) and dystrophin (11 kb) as target transgenes, as it is very difficult for other viral vectors to deliver these large genes and gene therapy has long been pursued as a treatment option for the associated diseases [Huntington's disease (HD) and Duchenne muscular dystrophy (DMD)].

For proper packaging, GLAd genome size from the 5' ITR to the 3' ITR should be within the range of 27-37.8 kb, which is 75-105% of the original genome size. Thus, the present inventors first converted the pGLAd3 genome plasmid to pGLAd4 (Fig. 148) to reduce its size because the length of pGLAd3 harboring large genes is near the upper length limit for virus packaging.

Huntington's disease, which is inherited in a dominant fashion, is a fatal neurodegenerative disease caused by a poly-CAG (a codon for glutamine) repeat expansion in huntingtin gene[40]. Huntintun's disease patients possess one mutant copy of this gene, and the disease conditions can be ameliorated when the expression of mutant huntingtin is inhibited by an antisense oligonucleotide or RNAi. Although the normal function of huntingtin in adult nerve cells remains unknown, it is important to note that huntingtin is essential for early embryonic development (huntingtin knockout causes embryonic lethality in mouse). Additionally, overexpression of wild-type huntingtin has been shown to reduce the cellular toxicity of mutant huntingtin. These results suggest that a more appropriate recombinant GLAd for testing is to deliver the RNAi and huntingtin gene sequence together concurrently rather than delivering the huntingtin gene alone.

For RNAi, the present inventors established miRNA-based shRNAs (mshRs hereinafter) instead of conventional shRNAs to knock down the expression of huntingtin since mshRs have been proven to be safer than shRNAs for the knockdown of huntingtin. The present inventors chose three target sites[48] (Fig. 5a) that have been confirmed to be functional and constructed mshR expression constructs using synthetic mshRs (Fig. 5b and Table 21) and pGT2 plasmids (Fig. 5c). As expected, the transfection of HEK293T cells with each of these constructs resulted in decreased expression of endogenous huntingtin (Fig. 5d). Thus, two mshR expression cassettes for mshR1 and mshR3 were inserted into the pGLAd4 genome plasmid via PCR with appropriate primer sets (Table 22). For the huntingtin gene, it was taken into consideration that the poly-CAG repeats are the only difference between wild-type and mutant huntingtin genes. Thus, a knockdown approach can simultaneously reduce the expression of both endogenous wild type and mutant huntingtin proteins. To decrease concern regarding undesirable toxicity that might result from decreased expression of endogenous wild-type huntingtin, and also to compensate for the decrease in wild-type huntingtin, the present inventors utilized a codon-optimized synthetic huntingtin gene whose expression cannot be attenuated by mshRs (Fig. 5e). Through iHoA, the present inventors successfully transferred a codon-optimized synthetic gene expression cassette in different orientations into the pGLAd4 genome plasmid, in which both the mshR1 and mshR3 expression cassettes had already been inserted (Fig. 5f). Then recombinant GLAd4.coHTT. HTTmshR1/3 (Fig. 5g) and GLAd4.coHTT(R). HTTmshR1/3 viruses were produced.

The GLAd4.coHTT.HTTmshR1/3 virus overexpressed huntingtin, whereas the GLAd4. coHTT(R).HTTmshR1/3 virus inhibited the expression of endogenous huntingtin (Fig. 5h). These research data indicate that the recombinant GLAd successfully delivered both the mshRs and the codon-optimized synthetic huntingtin gene (~13 kb in total length) simultaneously (Fig. 5i). This recombinant GLAd can potentially be utilized as a gene therapy for the treatment of Huntington's disease and is currently being investigated for clinical application.

In addition to recombinant GLAd delivering the large huntingtin gene, the present inventors have successfully constructed recombinant GLAds for many other small-sized transgenes, such as factor IX (R338L Padua mutant, for hemophilia B), glucocerebrosidase (GCR, for Gaucher's disease), hexosaminidase A (HEXA, for Tay-Sachs disease), hypoxanthine phosphoribosyltransferase 1 (HPRT1, for Lesch-Nyhan syndrome), iduronate-2-sulfatase (IDS, for Hunter syndrome), methyl-CpG-binding protein 2 (MECP2, for Rett syndrome), and survival of motor neuron 1 (SMN1, for spinal muscular atrophy (SMA)). Each of these recombinant GLAds efficiently overexpressed the transgene in vitro (data not shown) and is also currently under investigation for possible clinical applications.

For in vivo study, the present inventors produced a recombinant GLAd that delivers the dystrophin gene (~11 kb), a target for DMD. First the human dystrophin gene was cloned into pBest4 and a recombinant pGLAd4_Dys plasmid were constructed through iHoA, followed by producing the recombinant GLAd4. Dys virus (Fig. 6a). Then the GLAd4.Dys virus were injected into the focal gastrocnemius muscle of a dystrophin-knockout MDX mouse. Four weeks later (Fig. 6b), the muscle tissue at the injected site was biopsied and analyzed for dystrophin expression (Fig. 6c). The recombinant GLAd4.Dys virus evidently expressed dystrophin in the target tissue. Although improvement of the disease condition was unobservable (unlike humans, MDX mice do not show manifestations of the disease), the data indicate that the GLAd4.Dys virus maintained the expression of dystrophin in the target tissue for at least 4 weeks.

**Discussion**

An ideal in vivo gene delivery vector for gene therapy is recommended to exhibit the following characteristics: absence of random integration into the host genome (eliminating concern about insertional mutagenesis), no expression of viral proteins (requiring gutless viral genome, thus resulting in limited host immune response), broad tropism, high transduction efficiency in transgene delivery. and a sufficient cargo capacity for transgenes. Among the many gene delivery vectors currently available, GLAd is the only one that exhibits all of these recommended features. Surprisingly, however, GLAd has only been used in animal studies so far, and no clinical applications have been attempted, thus, no clinical data for GLAd are currently available14-20. It is believed that this unexpected consequence is associated with the safety concerns raised by adenovirus, which is required for GLAd packaging and further amplification as a helper in the conventional GLAd production process, remaining as a contaminant in prepared GLAd. An additional hurdle to overcome for the clinical use of GLAd is the RCA contaminant[21] generated during conventional GLAd production procedures or large-scale preparation of helper adenovirus.

The commonly used replication-incompetent first-generation adenovirus is highly immunogenic and can cause cellular toxicity in host organisms20. Thus, this virus has been widely used as a gene delivery vector for anticancer therapy, in which its immunogenic activity provides an adjuvant function and helps to remove tumor tissues more effectively through cooperation with the delivered therapeutic anti-cancer gene. In contrast, adenovirus is harmful in gene therapy, especially for the treatment of inherited genetic diseases requiring long-term transgene expression, since the host immune response caused by adenovirus can result in decreased or short-term transgene expression and toxicity in the patient. In this regard, it is important to note that the best result regarding adenovirus contamination, even in the most advanced helper adenovirus-based GLAd production system, is 0.01%, which corresponds to $1 \times 10^6$ out of $1 \times 10^{10}$ viral particles.

Regarding safety measures, compared with the replication-incompetent first-generation adenovirus (the helper adenovirus used for GLAd production is usually a first-generation adenovirus), RCA is more dangerous. Unlike the first-generation adenovirus, which is devoid of E1, thus allowing replication only in E1-expressing packaging cells such as HEK293 and HEK293T cells, RCA can replicate autonomously in any cell types following its infection. In particular, when gene therapy contaminated with RCA is administered to an immune-suppressed (or immune-weakened) patient, RCA can easily replicate by itself, which could result in fatal toxicity in the patient. Furthermore, the E1 protein expressed from RCA can support the robust replication/amplification of the replication-incompetent adenovirus by complementing E1 deficiency of this adenovirus. Thus, the Food and Drug Administration (FDA) heavily controls RCA and requires that RCA particles should be quantified in every batch of clinical adenovirus (and possibly also GLAd), and a single clinical dose should contain less than one infectious RCA particle in $3 \times 10^{10}$ adenoviral particles. Therefore, it is clear that for the clinical use of GLAd, helper adenovirus should be replaced with a new helper and that a new GLAd production system completely free of concerns about adenovirus and RCA contaminants should be established, as demonstrated for the three plasmid-based AAV production system[49], a standard procedure for preparing clinical AAV.

In the present disclosure, the present inventors report a novel helper plasmid for GLAd production for the first time. The helper plasmid does not contain any ITRs and Ψ packaging signal, both of which are essential for viral packaging. Additionally, unlike helper adenovirus, the helper plasmid possesses only a single homologous region [nucleotides 3034-5015 (1482 bp) of adenovirus type 5, based on GenBank AC_000008] for homologous recombination, which can occur in HEK293 or HEK293T GLAd packaging cells. These structural characteristics not only eliminate the possibility of the conversion of the helper plasmid to active viral particles but also inhibit the generation of RCA during GLAd production. Actually, the present inventors were unable to detect any adenovirus or RCA contaminants even in the large-scale production of GLAd and in high-passage GLAd prepared through consecutive amplifications. The data thus obtained clearly indicate that the new helper plasmid-based GLAd system exclusively produces target recombinant GLAd completely free of adenovirus and RCA contaminants, which therefore significantly decreases the safety concerns raised by these contaminant viruses.

Although the helper plasmid of the present disclosure cannot replicate in GLAd packaging cells, its helper function for GLAd packaging and further amplification is adequate to produce a sufficient amount of recombinant GLAd. In large-scale GLAd production, the system of the present disclosure exhibited a moderately reduced efficiency compared with a conventional helper adenovirus-based system. Nevertheless, the system allowed large-scale GLAd production with no difficulty. In drug development, safety is generally far more important than production yields. Such a consideration fully justifies the somewhat disadvantageous characteristics regarding the production yield of the GLAd system of the present disclosure.

The recombinant GLAd of the present disclosure effectively delivered many transgenes in vitro and in vivo. As expected, the GLAd efficiently transferred large genes such as huntingtin (9.4 kb) and dystrophin (11 kb) or even multiple expression cassettes (~13 kb in total length) composed of the huntingtin gene and two mshRs. In many studies, the AAV-mediated delivery of the dystrophin gene has been explored, but with a focus only on the mini- or microdystrophin gene, which are smaller versions of the full-length dystrophin gene exceeding the packaging capacity of AAV. It is important to note that the successful GLAd-mediated delivery of the multiple expression cassettes containing of huntingtin genes and two mshRs represents the first demonstration of a possible therapeutic approach for HD. These examples recapitulate a unique characteristic of GLAd regarding its versatility for gene delivery, particularly for the delivery of large genes.

The use of viral vectors in in vivo gene therapy is a double-edged sword. Viral vectors transfer transgenes more efficiently but can cause toxicity in host organisms. Gutless viral vectors such as AAV and GLAd do not contain any viral genes in their genome backbones; hence, they are safer than other viral vectors expressing viral proteins. Nevertheless, capsid protein-mediated toxicity of these vectors is unpreventable, as observed even for AAV, which exhibits severe acute toxicity at a high dose[54]. However, it is possible to significantly decrease concern about virus capsid-mediated toxicity by utilizing a GLAd that is not contaminated with highly immunogenic adenoviral species or by using a significantly reduced amount of GLAd based on the safe delivery procedure demonstrated by balloon occlusion catheter-mediated intervention for GLAd-based liver-directed transgene expression[18,19]. This procedure exhibits a high safety profile and achieves long-term transgene expression for as long as 7 years. In this context, the work of the present inventors needs to be evaluated, as a new platform for GLAd is presented in regard to contamination of helper adenovirus and RCA for the first time, along with the description of its outstanding characteristics as an in vivo gene delivery vector (Table 19).

In conclusion, the data obtained in the present disclosure clearly demonstrate that the helper plasmid-based system of thepresent disclosure efficiently produces recombinant GLAd that is free of adenovirus and RCA contaminants. Currently, gene therapy is addressing unmet needs for the treatment of various inherited genetic diseases. In this particular type of gene therapy, delivery vectors play a pivotal role, and it is hopeful that theur helper plasmid and GLAd production system of the present disclosure will pave the way for the successful development of future GLAd-based gene therapy.

Table 18

| GLAd yield and other viruses generated during GLAd production | | | |
|---|---|---|---|
| | | GLAd yield[a] (total BFU) | Other viruses[b,c] |
| Helper plasmid + pGLAd3_LacZ genome[d] | GLAd3.LacZ #1 | $1.20\times10^7$ | None |
| | GLAd3.LacZ #2 | $1.12\times10^7$ | None |
| | GLAd3.LacZ #3 | $1.80\times10^7$ | None |
| Helper plasmid[d] | #1 | N/A | None |
| | #2 | N/A | None |
| | #3 | N/A | None |
| Ad.LacZ[e] ($1\times10^3$ used) | | | ~$10^3$ |

FU blue-forming units, N/A not applicable
[a]Determined by LacZ staining (total BFU)
[b]Adenovirus and/or RCA
[c]Determined by plaque assay
[d]Transfected into HEK293T cells plated on 100 mm culture dish
[e]Positive control for plaque assay

Table 19

| Comparison of AAV and Conventional GLAd with Novel GLAd | | |
|---|---|---|
| | Conventional GLAd | Novel GLAd |
| Use of helper adenovirus in production | Yes | No |
| Use of helper plasmid in production | N/A | Yes |
| Helper adenovirus contamination | Yes | No |
| Replication-competent adenovirus (RCA) contamination | Yes | No |
| | AAV | Novel GLAd |
| Delivery capacity for transgene | ~4.5 kb | ~36 kb |
| Transduction efficiency[a] | +++ (70%) | +++++ (100%) |
| Broad tropism | Yes | Yes |
| Random integration into host genome (potential of insertional mutagenesis) | Yes [b] | No |
| Expression of viral proteins | No | No |
| In vivo acute toxicity by viral capsid | Yes | Yes |
| In vivo chronic toxicity | No | No |
| Long-term in vivo transgene expression | Yes | Yes |

N/A not applicable
[a]Relative activity
[b]Although frequency is low

Table 20

| Primers used for pGLAd3 backbone cloning | | | |
|---|---|---|---|
| SEQ ID NO: | Name | Sequence (5’ -> 3’) | Note |
| 39 | N-F | TCTTATGTAGTTTGAGCTCGGCTTTGGTTATTTGATGGA TTGACC | |
| 40 | N-R | TCCGGAGGGGCTCGAGGCTGGCTTAAAACAGTAACTCAA TATG | |
| 41 | C-F | CTCGAGCCCCTCCGGAGGAGTGGCCAGGGCGTTCTGGAG GTAG | |
| 42 | C-R | CGCCAAAAACCTAGGTGGGCCCAGGAAGACTCACTGGTT GG | |
| 43 | Nsi-Xho-S | TCGAGCTAGACTCTGGGGCTAAAGCTGCAGTATCCATCA CACTGGCGGCCGC | |
| 44 | Nsi-Xho-AS | TCGAGCGGCCGCCAGTGTGATGGATACTGCAGCTTTAGC CCCAGAGTCTAGCTCGATGCA | |
| 45 | F1-F | CCTCCTCGAGCTAGACTCTGGGGCTAAAGCAATGAG | |
| 46 | F1-R | CCCACGCGTGCGGCCGCTTGAGAGGCAGAGGCAAAGGCA AGTG | |
| 47 | F2-F | TCAAGCGGCCGCGTGCTGAGACTAAAGGGATGTGCTAC | |
| 48 | F2-R | CCCGTCGACCATCATTCTAAGGCCTGCCTGAGCTA | |
| 49 | F3-F | CCCGTCGACGCACTGGCTTTACAGGGGCCGTCTGC | |
| 50 | F3-R | GGGCGATCGGGAAGGAAACCTACCTAGCCTACAAG | |
| 51 | F4-F | GGGCGATCGGTAGACTAGGATAGCCTCAAACTCCT | |
| 52 | F4-R | GGGACGCGTGTTAACATACTATCAGAATAGTGATA | |
| 53 | F5-F | GGGACGCGTTGGGTGCAATCTTACCAGAGCCTTAC | |
| 54 | F5-R | ACGGCAGTTCAAAATCAAGTAATAC | |
| 55 | SMAR-F | CGGCCTGGGTGGCCAAATAAACTTATAAATTGTGAGAGA AA | |
| 56 | SMAR-R | CAGGTCGACATATTTAAAGAAAAAAAAATTGTATC | |

Table 21

| Synthetic DNA used for huntingtin mshR expression | | | |
|---|---|---|---|
| SEQ ID NO: | Name | Sequence (5’ -> 3’) | Note |
| 57 | HTTmshR1_S | **GATCC**TGCTGTTGACAGTGAGCGAGACCGTGTGAATCATTGT CTATAGTGAAGCCACAGATGTATAGACAATGATTCACACGGT CGTGCCTACTGCCTCGGA**G** | |
| 58 | HTTmshR1_A_S | **AATTC**TCCGAGGCAGTAGGCACGACCGTGTGAATCATTGTCT ATACATCTGTGGCTTCACTATAGACAATGATTCACACGGTCT CGCTCACTGTCAACAGCA**G** | |

(continued)

| Synthetic DNA used for huntingtin mshR expression | | | |
|---|---|---|---|
| SEQ ID NO: | Name | Sequence (5' -> 3') | Note |
| 59 | HTTmshR2_S | **GATCC**TGCTGTTGACAGTGAGCGACAGCTTGTCCAGGTTTATGAATAGTGAAGCCACAGATGTATTCATAAACCTGGACAAGCTGGTGCCTACTGCCTCGGA**G** | |
| 60 | HTTmshR2_A S | **AATTC**TCCGAGGCAGTAGGCACCAGCTTGTCCAGGTTTATGAATACATCTGTGGCTTCACTATTCATAAACCTGGACAAGCTGTCGCTCACTGTCAACAGCA**G** | |
| 61 | HTTmshR3_S | **GATCC**TGCTGTTGACAGTGAGCGAGGATACCTGAAATCCTGCTTTTAGTGAAGCCACAGATGTAAAAGCAGGATTTCAGGTATCCGTGCCTACTGCCTCGGA**G** | |
| 62 | HTTmshR3_A_S | **AATTC**TCCGAGGCAGTAGGCACGGATACCTGAAATCCTGCTTTTACATCTGTGGCTTCACTAAAAGCAGGATTTCAGGTATCCTCGCTCACTGTCAACAGCA**G** | |

The nucleotides in bold letters at 5' and 3' ends account for adhesive ends for BamHI and EcoRI sites, respectively.

Table 22

| PCR primers used for cloning huntingtin mshR into pGLAd3 backbone | | | |
|---|---|---|---|
| SEQ ID NO: | Name | Sequence (5' -> 3') | Note |
| 63 | pGLAd3_5139Acc_CMV_AF_F | ATTTTAAAGGGCCACGGTACGACATTGATTATTGACTAGTTATTAA | |
| 64 | pGLAd3_5139Acc_BGH_AF_R | TGTGTCCATCCGTGTGGTACCCATAGAGCCCACCGCATCCCCAGCA | |
| 65 | pGLAd3_7189Pci_CMV_AF_F | TTAACCCCACTCCCCACATGGACATTGATTATTGACTAGTTATTAA | |
| 66 | pGLAd3_7189Pci_BGH_AF_R | GCATCTGAACGAAGCACATGCCATAGAGCCCACCGCATCCCCAGCA | |

Underlined nucleotide sequences are portions homologous to pGLAd3 genome plasmids.

**Industrial Applicability**

The present disclosure pertains to a helper plasmid-based gutless adenovirus (GLAd) production method and system.

## Claims

1. A gutless adenovirus (GLAd) production system, comprising a helper plasmid, a genome plasmid, and a virus packaging cell strain.

2. The gutless adenovirus (GLAd) production system of claim 1, wherein the helper plasmid

3. The gutless adenovirus (GLAd) production system of claim 1, wherein the helper plasmid does not undergo conversion into a virus particle.

4. The gutless adenovirus (GLAd) production system of claim 1, wherein the helper plasmid is free of an inverted terminal repeat (ITR).

5. The gutless adenovirus (GLAd) production system of claim 1, wherein the helper plasmid is free of an ITR and a Ψ packaging signal.

6. The gutless adenovirus (GLAd) production system of claim 1, wherein the helper plasmid comprises one to five plasmids.

7. The gutless adenovirus (GLAd) production system of claim 1, wherein the genome plasmid comprises a 5' homologous stretch, a 3' homologous stretch, and a 3' inverted terminal repeat (ITR).

8. The gutless adenovirus (GLAd) production system of claim 7, wherein the genome plasmid further comprises an antibiotic-resistant gene.

9. The gutless adenovirus (GLAd) production system of claim 7, wherein the genome plasmid further comprise an Ori reprication origin.

10. The gutless adenovirus (GLAd) production system of claim 7, wherein the genome plasmid further comprises a stuffer DNA (sDNA).

11. The gutless adenovirus (GLAd) production system of claim 10, wherein the stuffer DNA further comprises scaffold/matrix attachment element (SMAR).

12. The gutless adenovirus (GLAd) production system of claim 1, wherein the genome plasmid comprises a GLAd genome portion to be packaged into a capsid of GLAd.

13. The gutless adenovirus (GLAd) production system of claim 1, wherein the genome plasmid is a final genome plasmid comprising a transgene to be expressed using GLAd, and elements necessary for transgene expression.

14. The gutless adenovirus (GLAd) production system of claim 1, wherein the system further comprises a cloning shuttle plasmid.

15. The gutless adenovirus (GLAd) production system of claim 14, wherein the cloning shuttle plasmid comprises a 5' homologous stretch, a 5' inverted terminal repeat (ITR), a Ψ packaging signal, a promoter, a multi-cloning site (MCS), a poly(A) signal, and a 3' homologous stretch.

16. The gutless adenovirus (GLAd) production system of claim 15, wherein the cloning shuttle plasmid comprises an intron.

17. The gutless adenovirus (GLAd) production system of claim 15, wherein the cloning shuttle plasmid further comprises an antibiotic-resistant gene.

18. The gutless adenovirus (GLAd) production system of claim 15, wherein the cloning shuttle plasmid further comprises an Ori replication origin.

19. The gutless adenovirus (GLAd) production system of claim 14, wherein the cloning shuttle plasmid comprises a transgene to be expressed using GLAd, and elements necessary for transgene expresseion.

20. The gutless adenovirus (GLAd) production system of claim 1, wherein the virus packaging cell strain is a cell strain expressing a protein belonging to an E1 region of adenovirus.

21. The gutless adenovirus (GLAd) production system of claim 1, wherein the system further comprises a pAd5pTP expression plasmid.

22. The gutless adenovirus (GLAd) production system of claim 21, wherein the pAd5pTP expression plasmid comprises the nucleotide sequence of SEQ ID NO: 76.

**23.** A method for producing gutless adenovirus (GLAd), the method comprising the steps of:

transfecting a final genome plasmid into a virus packaging cell strain; and
transfecting a helper plasmid into the virus packaging cell strain.

**24.** The method of claim 23, wherein the final genome plasmid is linearized by a restriction enzyme.

**25.** The method of claim 23, wherein the transfection is carried out by a calcium phosphate precipitation method.

**26.** The method of claim 23, wherein the method further comprises a step of transfecting a pAd5pTP expression plasmid into a virus packaging cell strain.

**27.** The method of claim 23, wherein the helper plasmid is not an infectious virus particle.

**28.** The method of claim 23, wherein the helper plasmid does not undergo conversion into an infectious virus particle.

**29.** The method of claim 23, wherein the helper plasmid is free of an inverted terminal repeat (ITR).

**30.** The method of claim 23, wherein the helper plasmid is free of an ITR and a Ψ packaging signal.

**31.** The method of claim 23, wherein the helper plasmid comprises one to five plasmids.

**32.** The method of claim 23, wherein the final genome plasmid comprises a 5' inverted terminal repeat (ITR), a Ψ packaging signal, a promoter, an intron, a transgene, a poly(A) signal, a stuffer DNA (sDNA), and a 3' inverted terminal repeat.

**33.** The method of claim 23, wherein the final genome plasmid further comprises an antibiotic-resistant gene.

**34.** The method of claim 23, wherein the final genome plasmid further comprises an Ori replication origin.

**35.** The method of claim 23, wherein the stuffer DNA further comprises a scaffold/matrix attachment element (SMAR).

**36.** The method of claim 23, wherein the final genome plasmid comprises a GLAd genome portion to be packaged into a capside of GLAd.

**37.** The method of claim 23, wherein the final genome plasmid comprises a transgene to be expressed using GLAd, and elements necessary for transgene expression.

**38.** The method of claim 23, wherein the virus packaging cell strain expresses a protein belonging to an E1 region of adenovirus.

**39.** The method of claim 23, wherein the GLAd produced by the production method is free of a contaminant virus species.

**40.** The method of claim 39, wherein the contaminant virus species is adenovirus or replication-competent adenovirus (RCA).

**41.** The method of claim 26, wherein the pAd5pTP expression plasmid comprises the nucleotide sequence of SEQ ID NO: 76.

**42.** A gutless adenovirus, comprising a 5' inverted terminal repeat (ITR), a Ψ packaging signal, a promoter, an intron, a transgene, a poly(A) signal, a stuffer DNA (sDNA), and a 3' inverted terminal repeat (ITR).

【Fig 1】

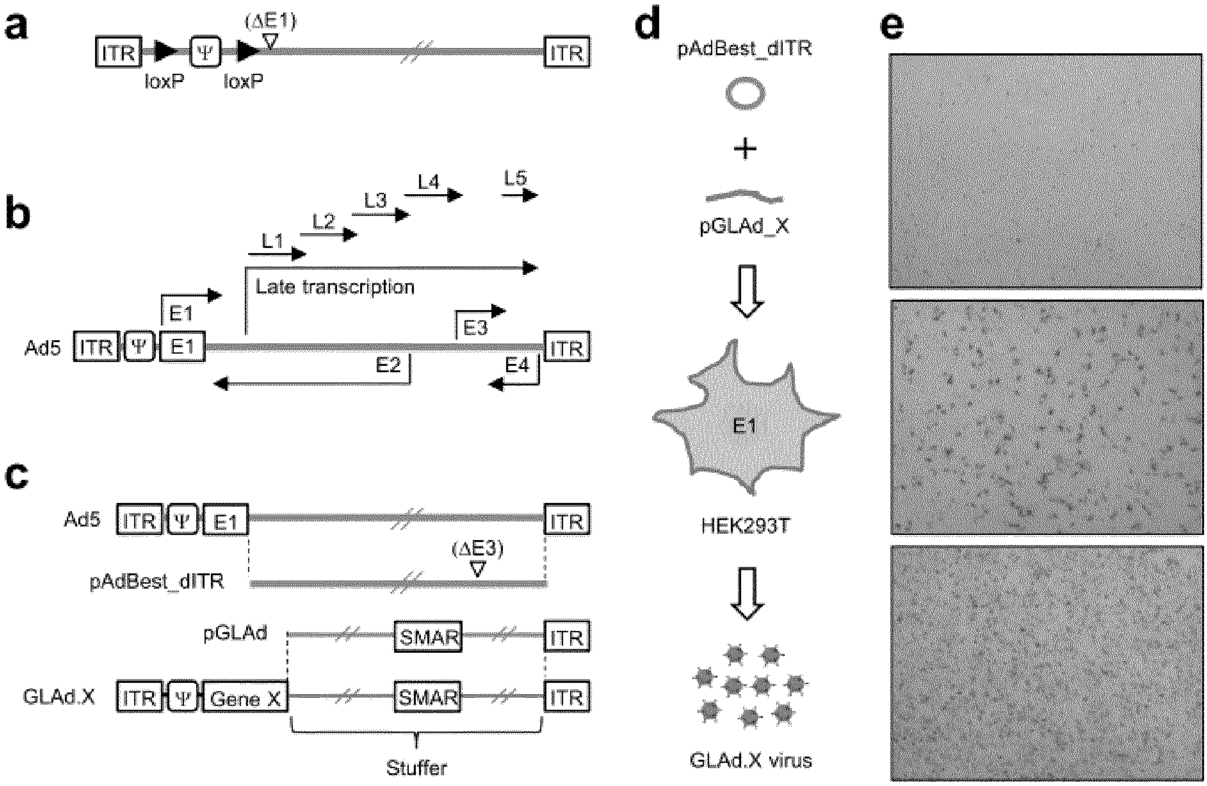
a
(ΔE1)
ITR
loxP
Ψ
loxP
ITR
b
L1
L2
L3
L4
L5
Late transcription
E1
E3
Ad5
ITR
Ψ
E1
ITR
E2
E4
c
Ad5
ITR
Ψ
E1
ITR
(ΔE3)
pAdBest_dITR
pGLAd
SMAR
ITR
GLAd.X
ITR
Ψ
Gene X
SMAR
ITR
Stuffer
d
pAdBest_dITR
+
pGLAd_X
E1
HEK293T
GLAd.X virus
e

【Fig 2】

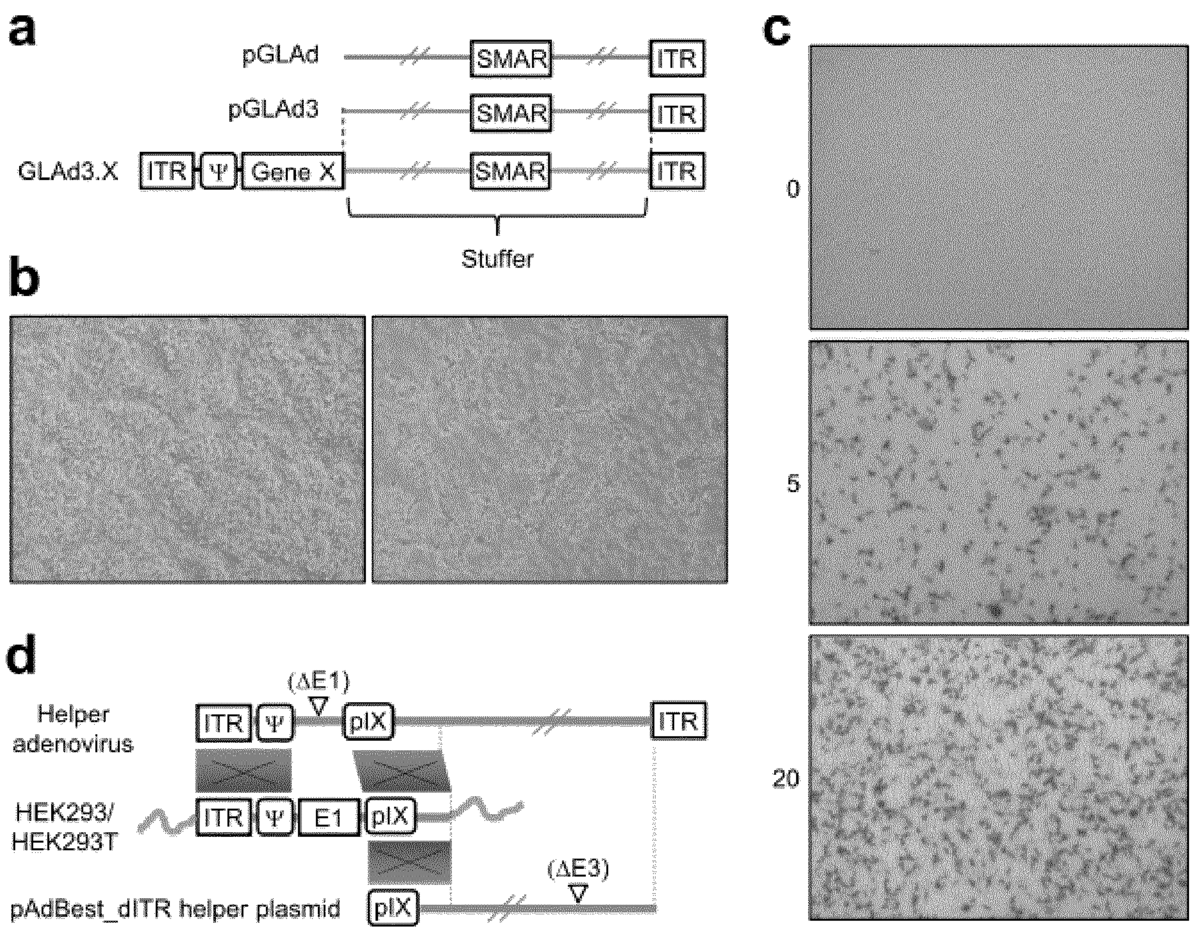
a
pGLAd
pGLAd3
GLAd3.X
ITR
Ψ
Gene X
SMAR
ITR
Stuffer
b
c
0
5
20
d
(ΔE1)
Helper adenovirus
ITR
Ψ
pIX
ITR
HEK293/ HEK293T
E1
(ΔE3)
pAdBest_dITR helper plasmid

【Fig 3】

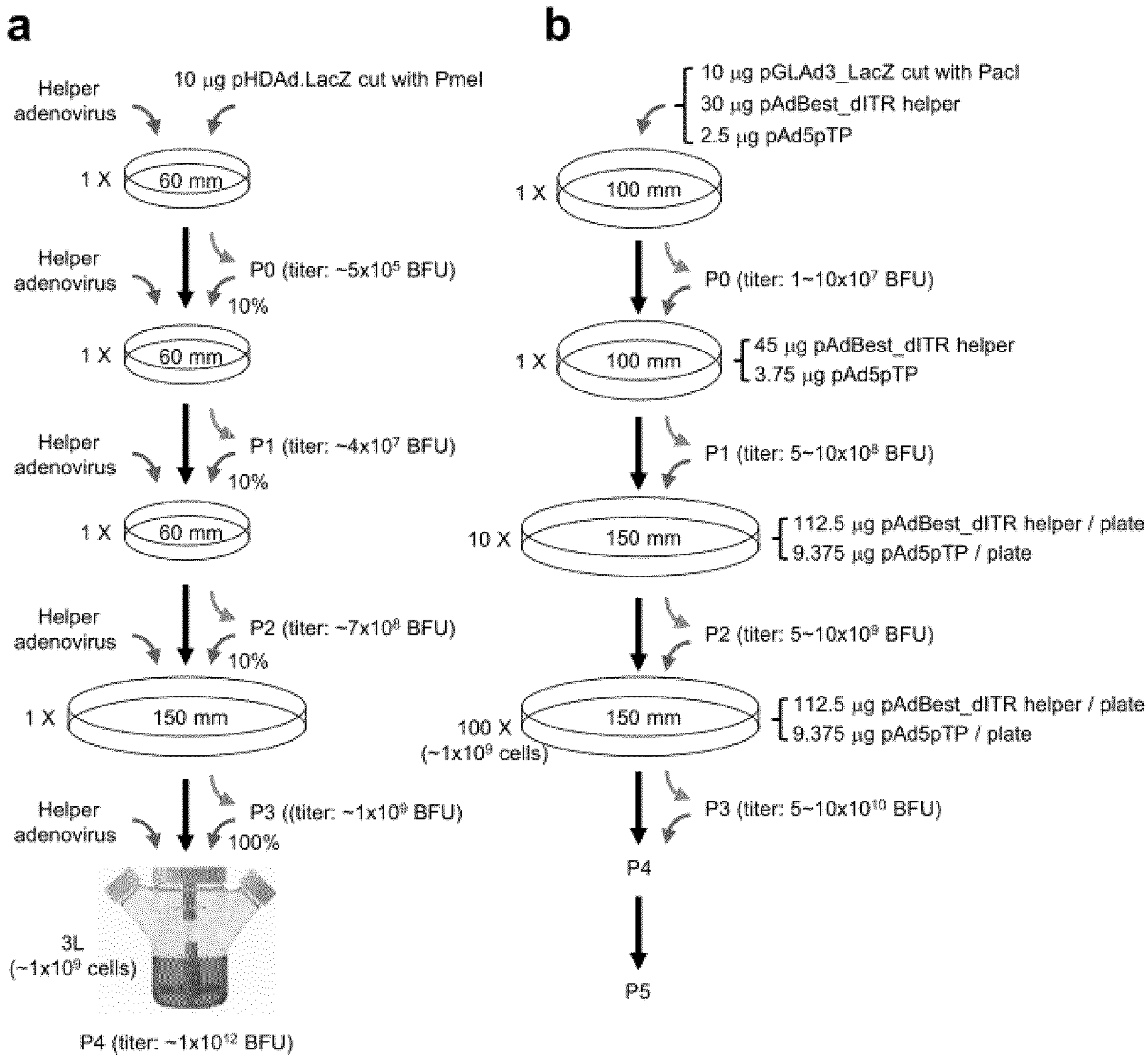
a
Helper adenovirus
10 μg pHDAd.LacZ cut with PmeI
1 X
60 mm
Helper adenovirus
P0 (titer: ~5x10^5 BFU)
10%
1 X
60 mm
Helper adenovirus
P1 (titer: ~4x10^7 BFU)
10%
1 X
60 mm
Helper adenovirus
P2 (titer: ~7x10^8 BFU)
10%
1 X
150 mm
Helper adenovirus
P3 ((titer: ~1x10^9 BFU)
100%
3L
(~1x10^9 cells)
P4 (titer: ~1x10^12 BFU)
b
10 μg pGLAd3_LacZ cut with PacI
30 μg pAdBest_dITR helper
2.5 μg pAd5pTP
1 X
100 mm
P0 (titer: 1~10x10^7 BFU)
1 X
100 mm
45 μg pAdBest_dITR helper
3.75 μg pAd5pTP
P1 (titer: 5~10x10^8 BFU)
10 X
150 mm
112.5 μg pAdBest_dITR helper / plate
9.375 μg pAd5pTP / plate
P2 (titer: 5~10x10^9 BFU)
100 X
(~1x10^9 cells)
150 mm
112.5 μg pAdBest_dITR helper / plate
9.375 μg pAd5pTP / plate
P3 (titer: 5~10x10^10 BFU)
P4
P5

【Fig 4】

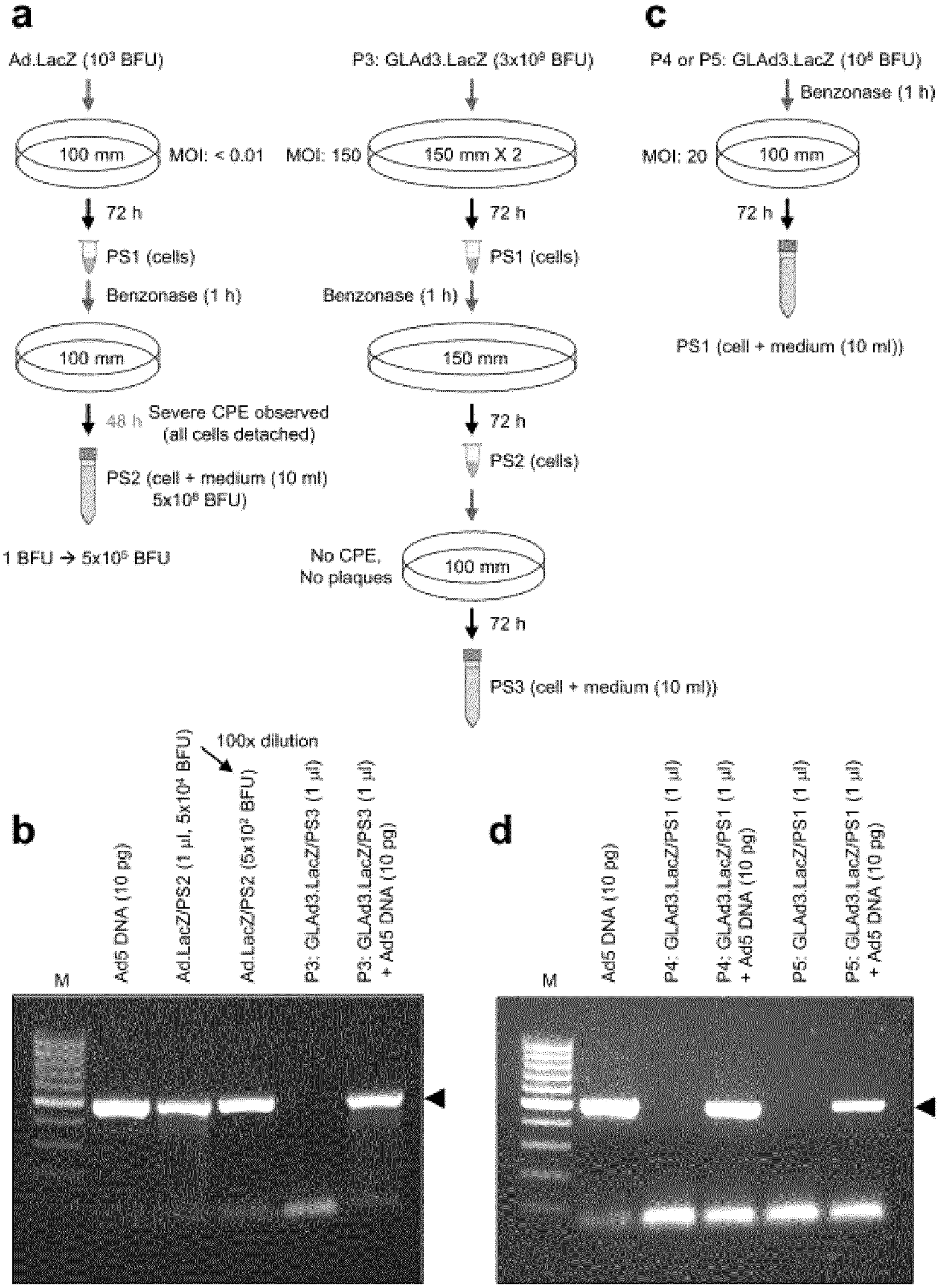
a
Ad.LacZ (10³ BFU)
100 mm
MOI: < 0.01
72 h
PS1 (cells)
Benzonase (1 h)
100 mm
48 h
Severe CPE observed (all cells detached)
PS2 (cell + medium (10 ml) 5x10⁸ BFU)
1 BFU → 5x10⁵ BFU
P3: GLAd3.LacZ (3x10⁹ BFU)
MOI: 150
150 mm X 2
72 h
PS1 (cells)
Benzonase (1 h)
150 mm
72 h
PS2 (cells)
No CPE, No plaques
100 mm
72 h
PS3 (cell + medium (10 ml))
c
P4 or P5: GLAd3.LacZ (10⁸ BFU)
Benzonase (1 h)
MOI: 20
100 mm
72 h
PS1 (cell + medium (10 ml))
b
M
Ad5 DNA (10 pg)
Ad.LacZ/PS2 (1 μl, 5x10⁴ BFU)
Ad.LacZ/PS2 (5x10² BFU)
100x dilution
P3: GLAd3.LacZ/PS3 (1 μl)
P3: GLAd3.LacZ/PS3 (1 μl) + Ad5 DNA (10 pg)
d
M
Ad5 DNA (10 pg)
P4: GLAd3.LacZ/PS1 (1 μl)
P4: GLAd3.LacZ/PS1 (1 μl) + Ad5 DNA (10 pg)
P5: GLAd3.LacZ/PS1 (1 μl)
P5: GLAd3.LacZ/PS1 (1 μl) + Ad5 DNA (10 pg)

【Fig 5】

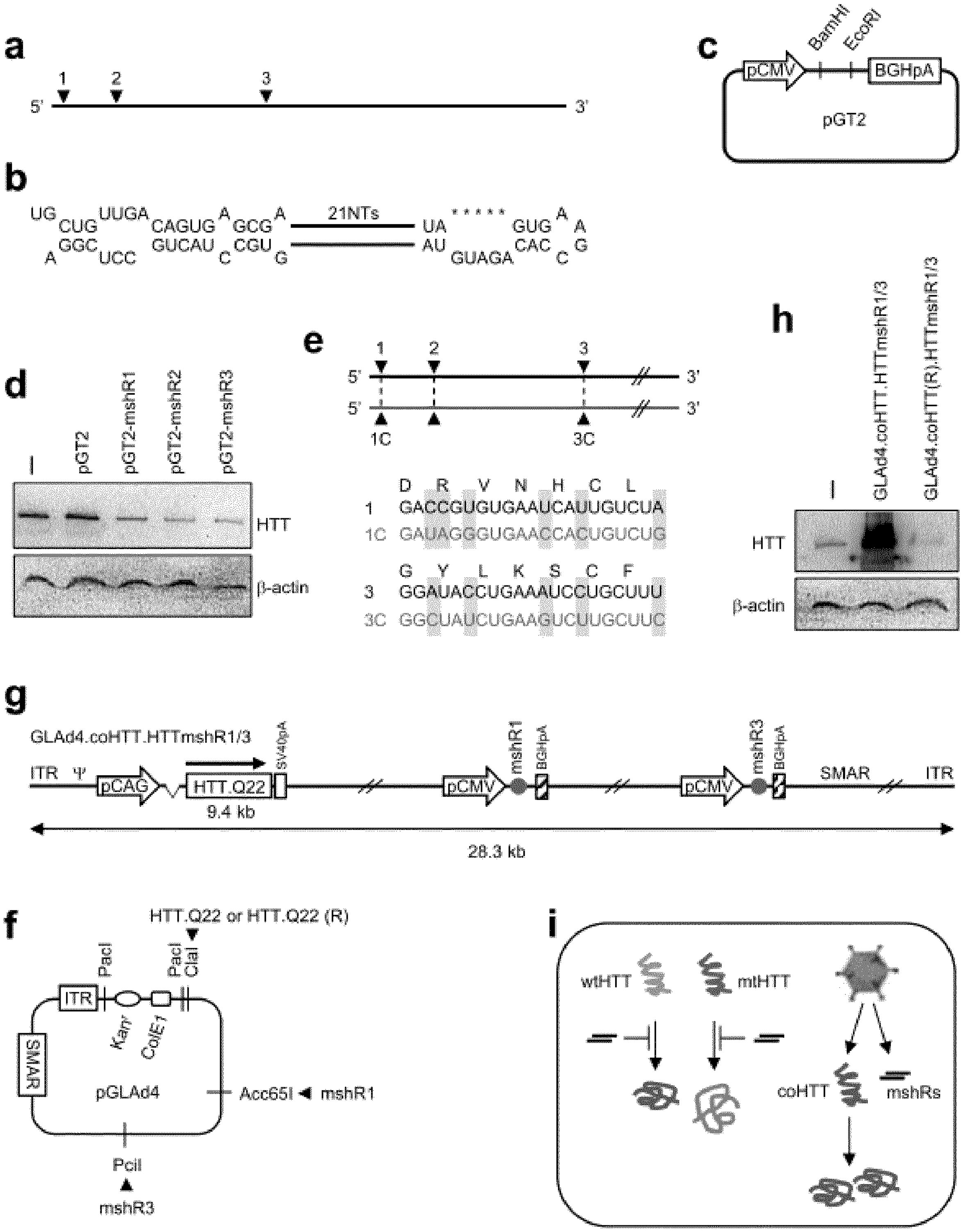
a
1
2
3
5'
3'
b
21NTs
c
pCMV
BamHI
EcoRI
BGHpA
pGT2
d
pGT2
pGT2-mshR1
pGT2-mshR2
pGT2-mshR3
HTT
β-actin
e
1C
3C
D R V N H C L
1 GACCGUGUGAAUCAUUGUCUA
1C GAUAGGGUGAACCACUGUCUG
G Y L K S C F
3 GGAUACCUGAAAUCCUGCUUU
3C GGCUAUCUGAAGUCUUGCUUC
h
GLAd4.coHTT.HTTmshR1/3
GLAd4.coHTT(R).HTTmshR1/3
HTT
β-actin
g
GLAd4.coHTT.HTTmshR1/3
ITR
Ψ
pCAG
HTT.Q22
SV40pA
pCMV
mshR1
BGHpA
mshR3
SMAR
9.4 kb
28.3 kb
f
HTT.Q22 or HTT.Q22 (R)
PacI
ClaI
ITR
Kanr
ColE1
SMAR
pGLAd4
Acc65I
mshR1
PciI
mshR3
i
wtHTT
mtHTT
coHTT
mshRs

【Fig 6】

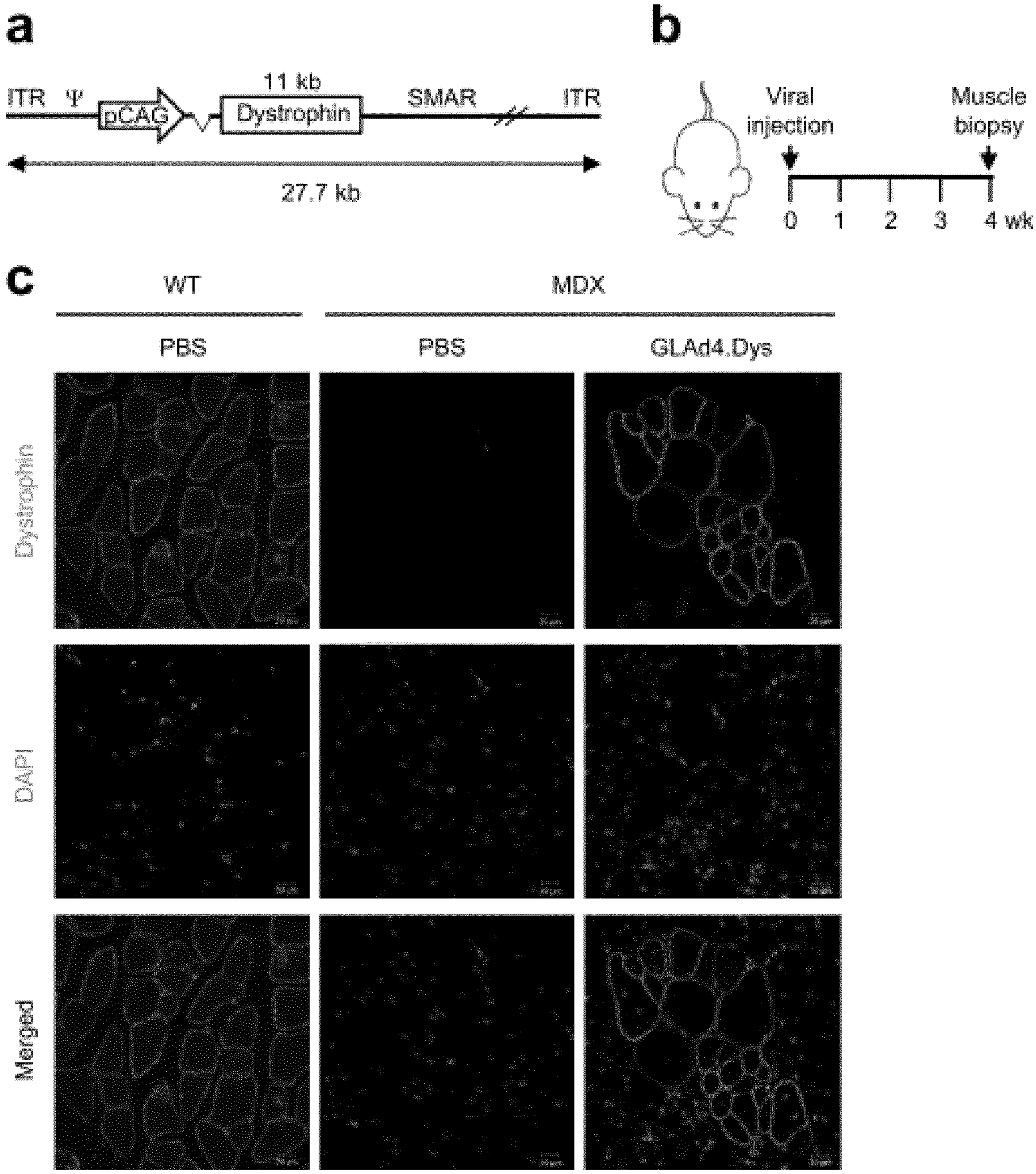
a
ITR
Ψ
pCAG
11 kb
Dystrophin
SMAR
ITR
27.7 kb
b
Viral
injection
Muscle
biopsy
0
1
2
3
4 wk
c
WT
MDX
PBS
PBS
GLAd4.Dys
Dystrophin
DAPI
Merged

【Fig 7】

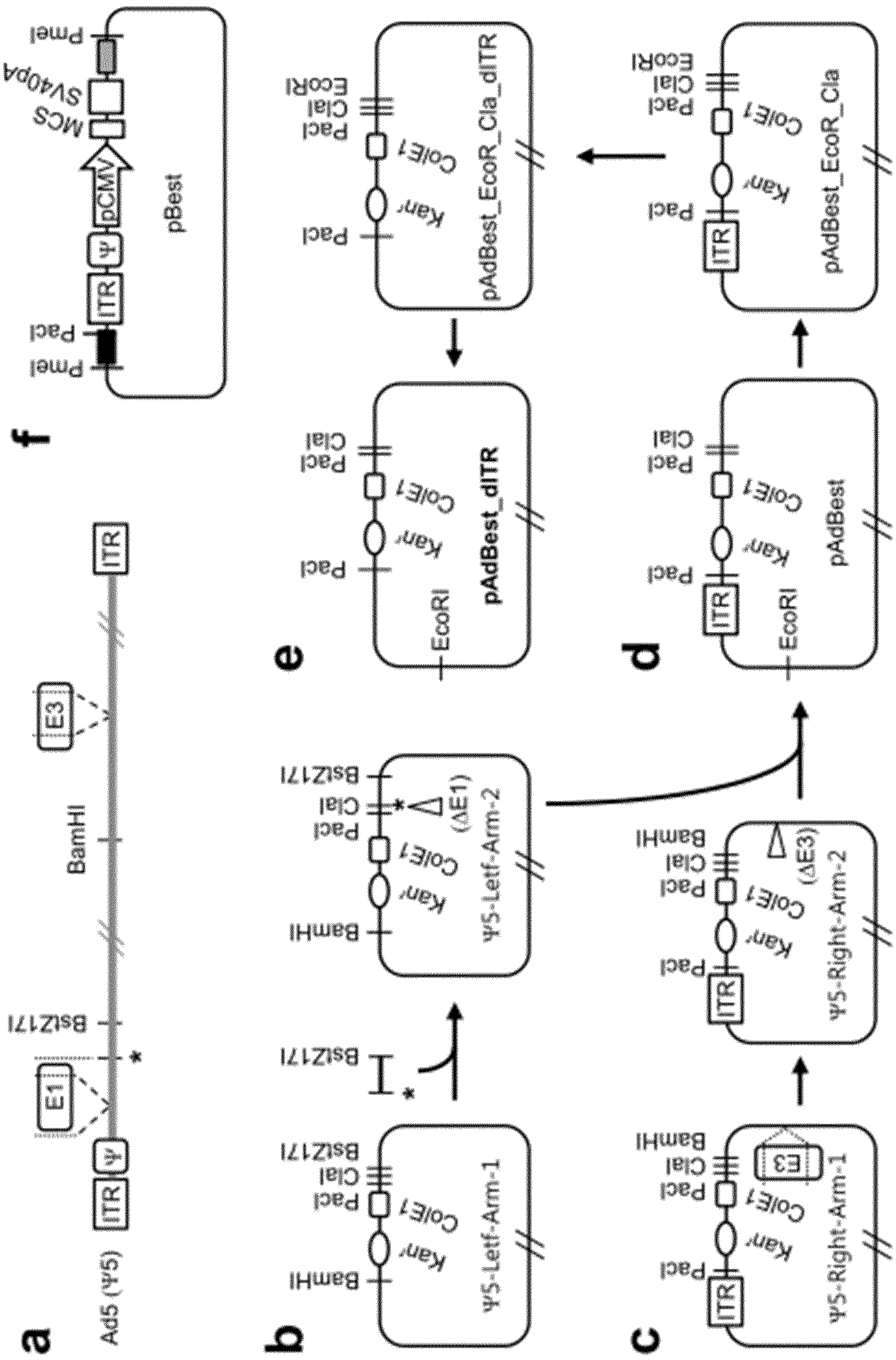
a
Ad5 (Ψ5)
ITR
Ψ
E1
BstZ17I
BamHI
E3
ITR
f
PmeI
PacI
ITR
Ψ
pCMV
MCS
SV40pA
PmeI
pBest
b
BamHI
Kan^r
ColE1
PacI
ClaI
BstZ17I
Ψ5-Letf-Arm-1
BstZ17I
Ψ5-Letf-Arm-2
(ΔE1)
e
EcoRI
pAdBest_dITR
pAdBest_EcoR_Cla_dITR
EcoRI
c
Ψ5-Right-Arm-1
E3
Ψ5-Right-Arm-2
(ΔE3)
d
pAdBest
pAdBest_EcoR_Cla

【Figure】

【Fig 8】

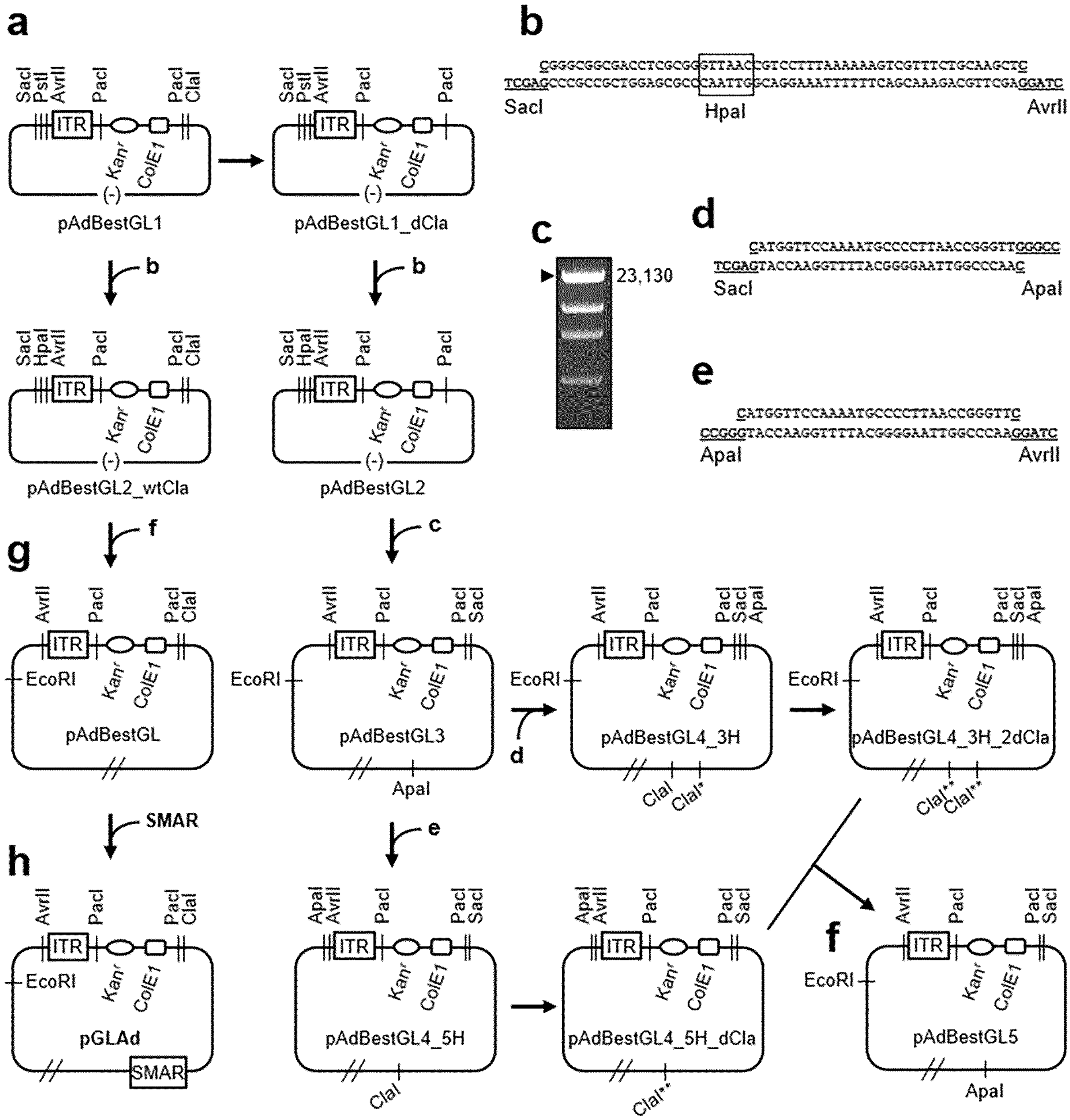
a
b
CGGGCGGCGACCTCGCGGGTTAACCGTCCTTTAAAAAAGTCGTTTCTGCAAGCTC
TCGAGCCCGCCGCTGGAGCGCCCAATTGGCAGGAAATTTTTTCAGCAAAGACGTTCGAGGATC
SacI
HpaI
AvrII
c
23,130
d
CATGGTTCCAAAATGCCCCTTAACCGGGTTGGGCC
TCGAGTACCAAGGTTTTACGGGGAATTGGCCCAAC
SacI
ApaI
e
CATGGTTCCAAAATGCCCCTTAACCGGGTTC
CCGGGTACCAAGGTTTTACGGGGAATTGGCCCAAGGATC
ApaI
AvrII
pAdBestGL1
pAdBestGL1_dCla
pAdBestGL2_wtCla
pAdBestGL2
g
pAdBestGL
pAdBestGL3
pAdBestGL4_3H
pAdBestGL4_3H_2dCla
SMAR
h
pGLAd
pAdBestGL4_5H
pAdBestGL4_5H_dCla
f
pAdBestGL5
ITR
Kan^r
ColE1
EcoRI
ClaI
ClaI*
ClaI**
ApaI
PacI
PstI

【Fig 9a】

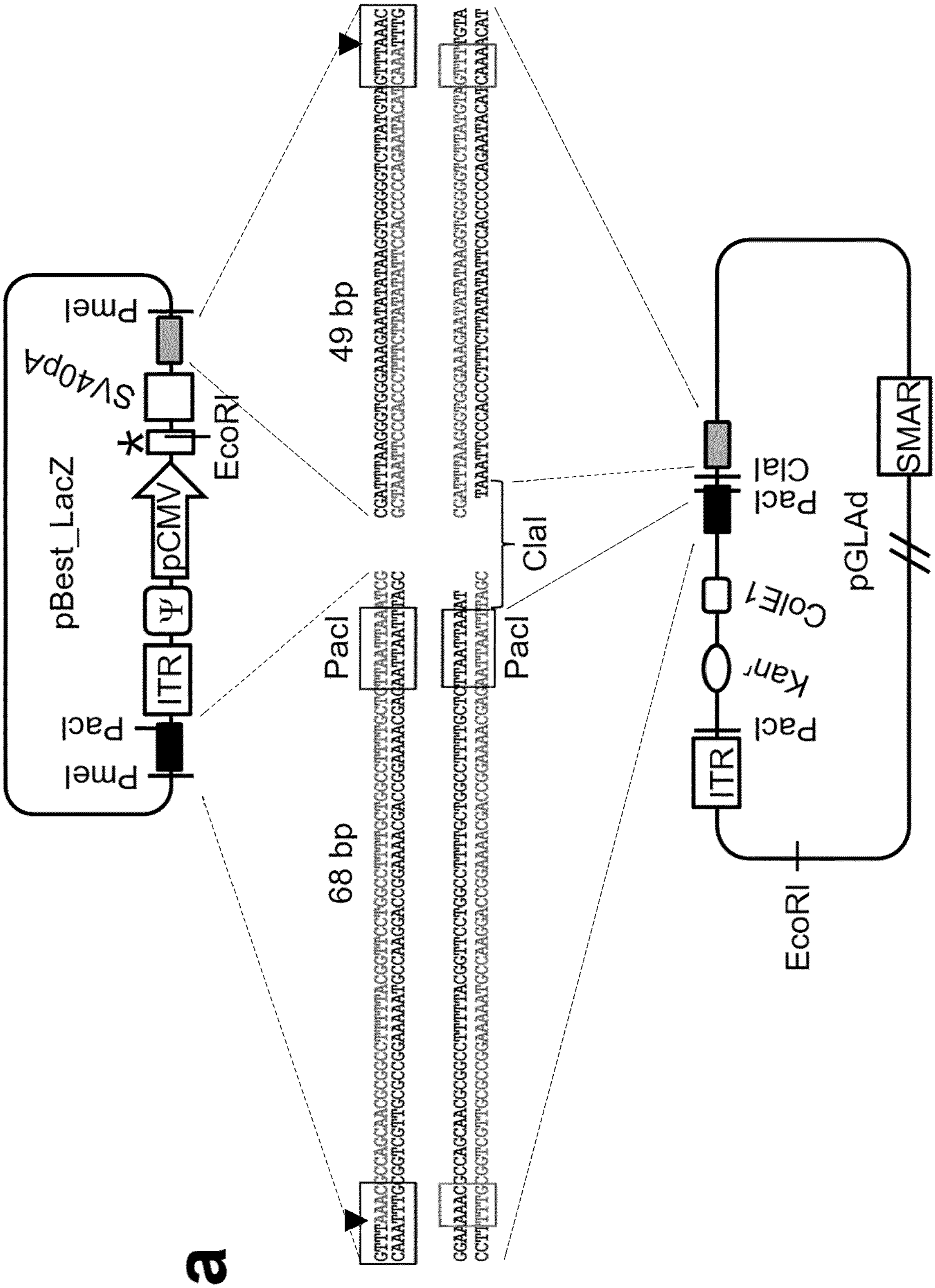
a
pBest_LacZ
PmeI
PacI
ITR
Ψ
pCMV
EcoRI
SV40pA
49 bp
68 bp
PacI
ClaI
pGLAd
ColE1
Kan^r
SMAR

【Fig 9b】

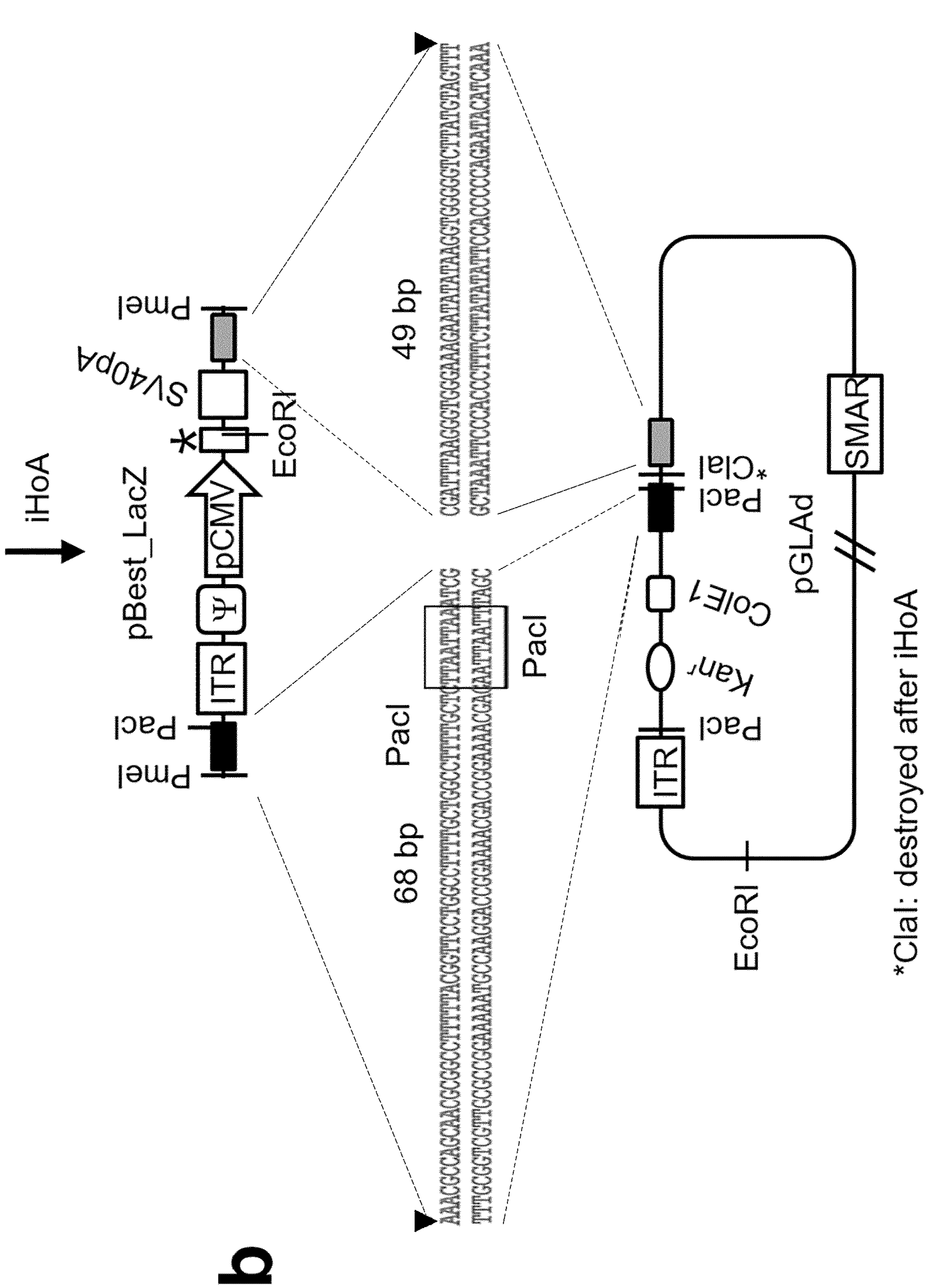
b
iHoA
pBest_LacZ
PmeI
PacI
ITR
Ψ
pCMV
*
EcoRI
SV40pA
PmeI
68 bp
PacI
PacI
49 bp
ITR
PacI
Kanr
ColE1
PacI
*ClaI
pGLAd
SMAR
EcoRI
*ClaI: destroyed after iHoA

【Fig 9c】

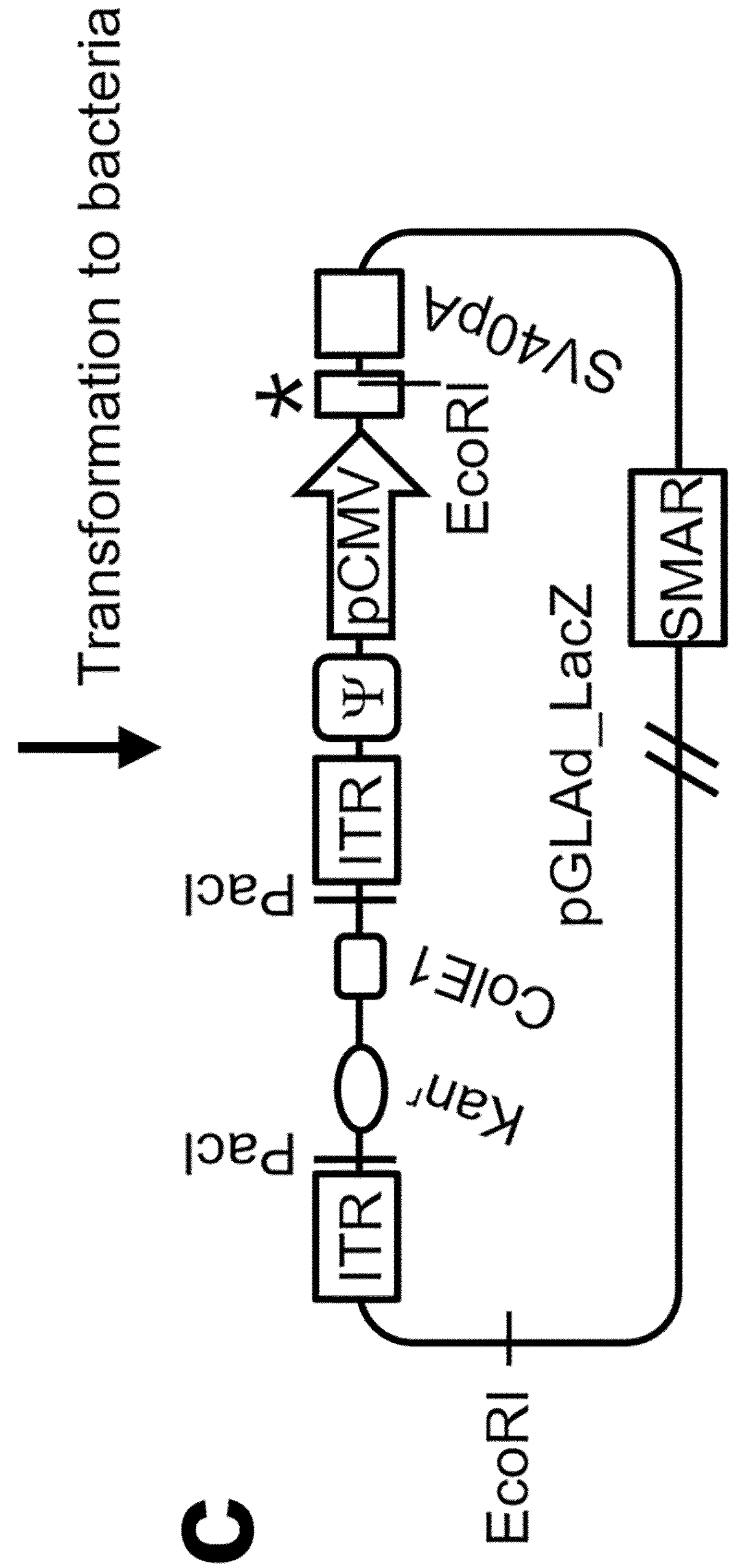
Transformation to bacteria
C
EcoRI
ITR
PacI
Kanr
ColE1
PacI
ITR
Ψ
pCMV
EcoRI
*
SV40pA
pGLAd_LacZ
SMAR

【Fig 10】

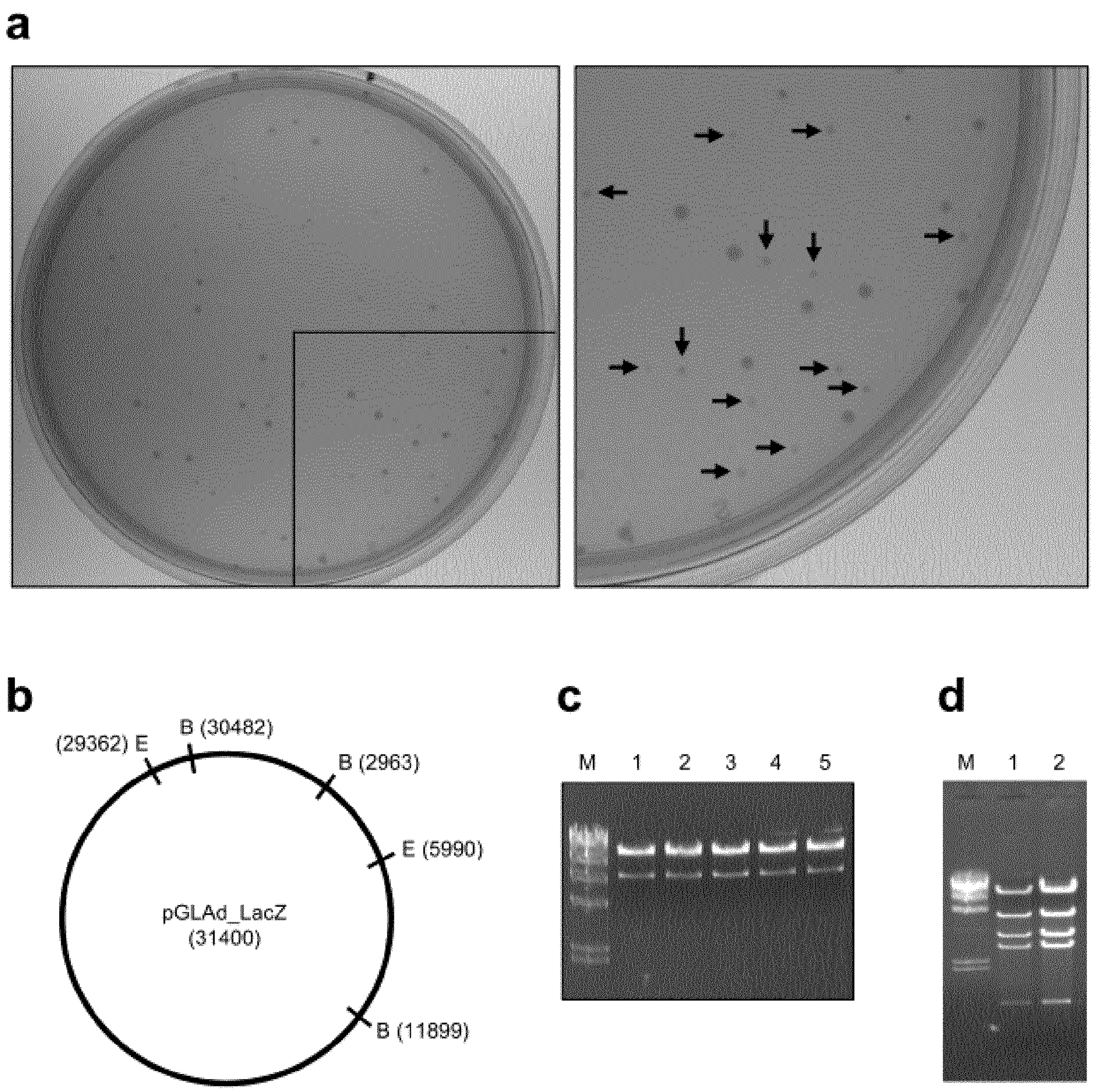
a
b
(29362) E
B (30482)
B (2963)
E (5990)
pGLAd_LacZ
(31400)
B (11899)
c
M 1 2 3 4 5
d
M 1 2

【Fig 11a】

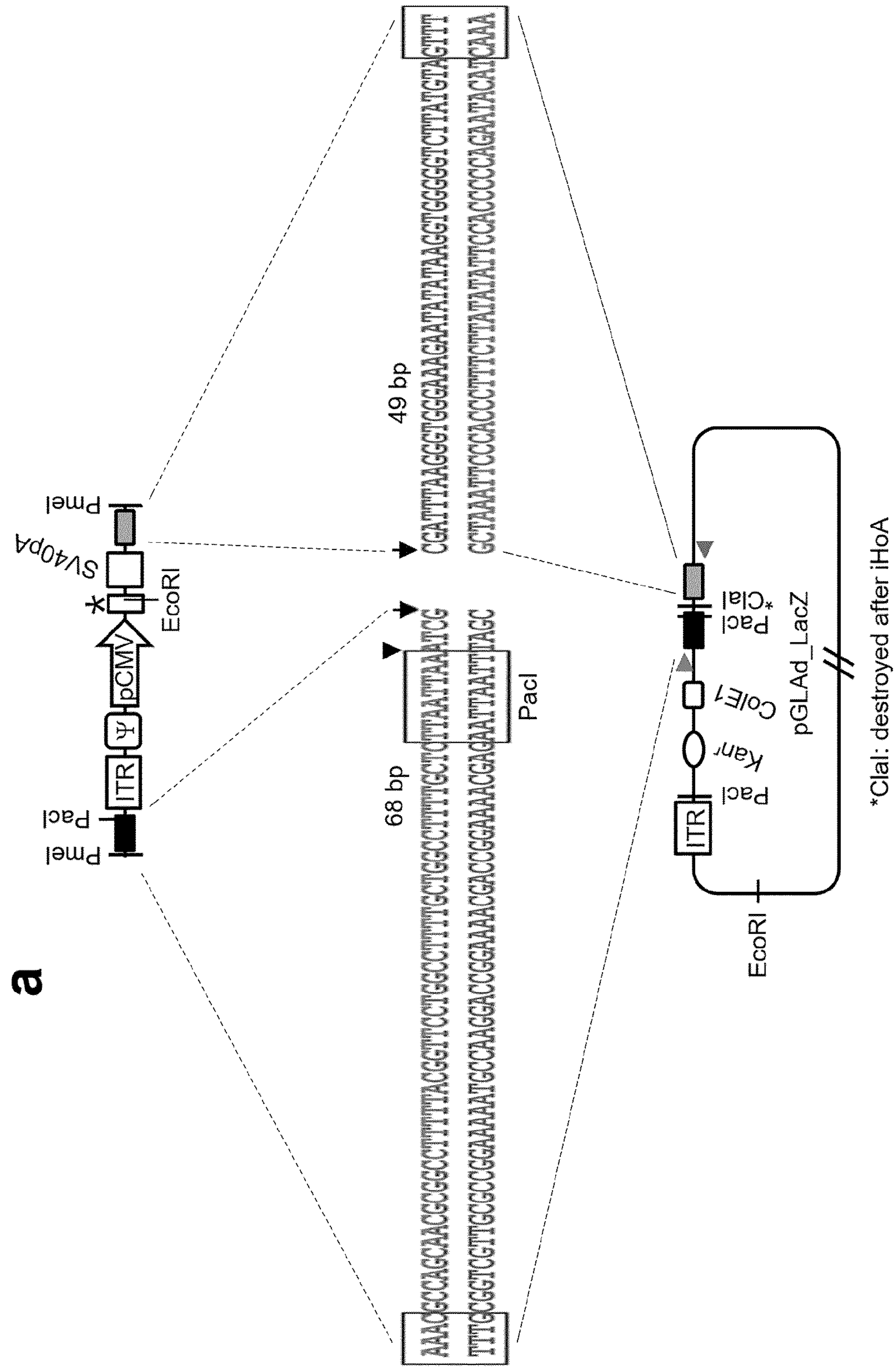
a
PmeI
PacI
ITR
Ψ
pCMV
EcoRI
SV40pA
PmeI
49 bp
68 bp
PacI
pGLAd_LacZ
ITR
PacI
Kanr
ColE1
PacI
*ClaI
EcoRI
*ClaI: destroyed after iHoA

【Fig 11b】

b

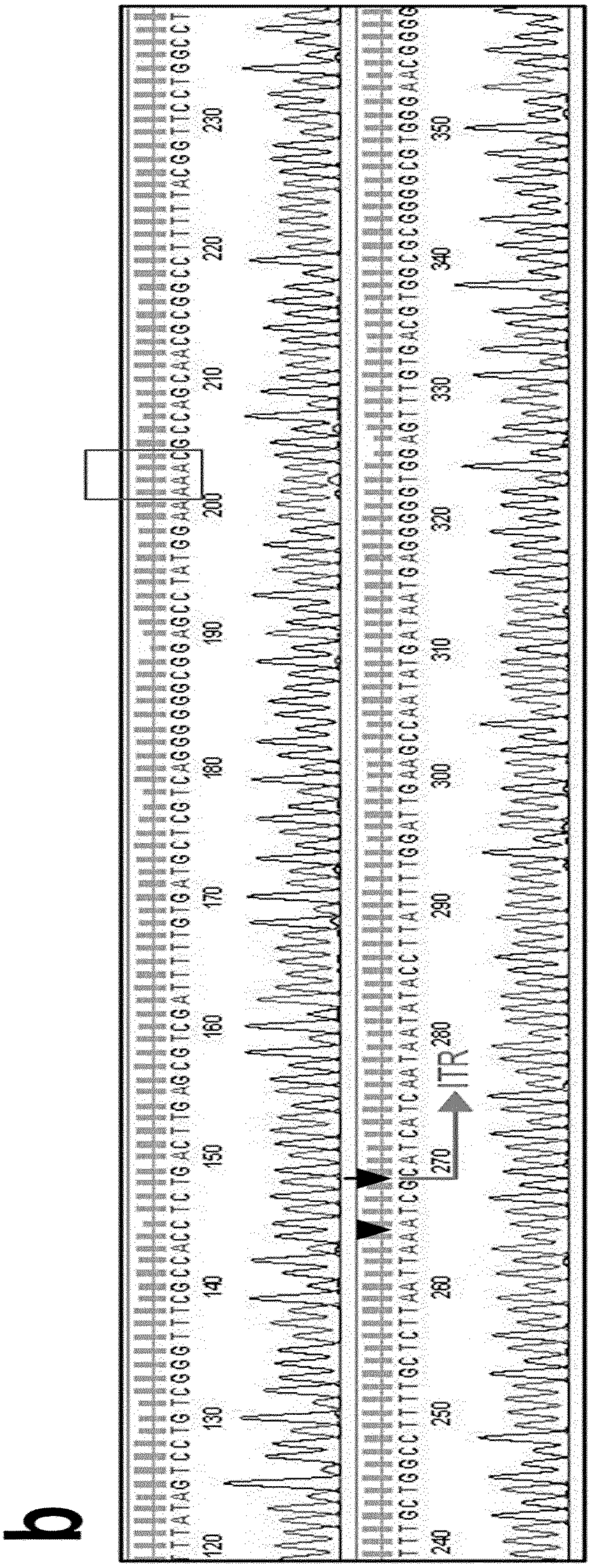

【Fig 11c】

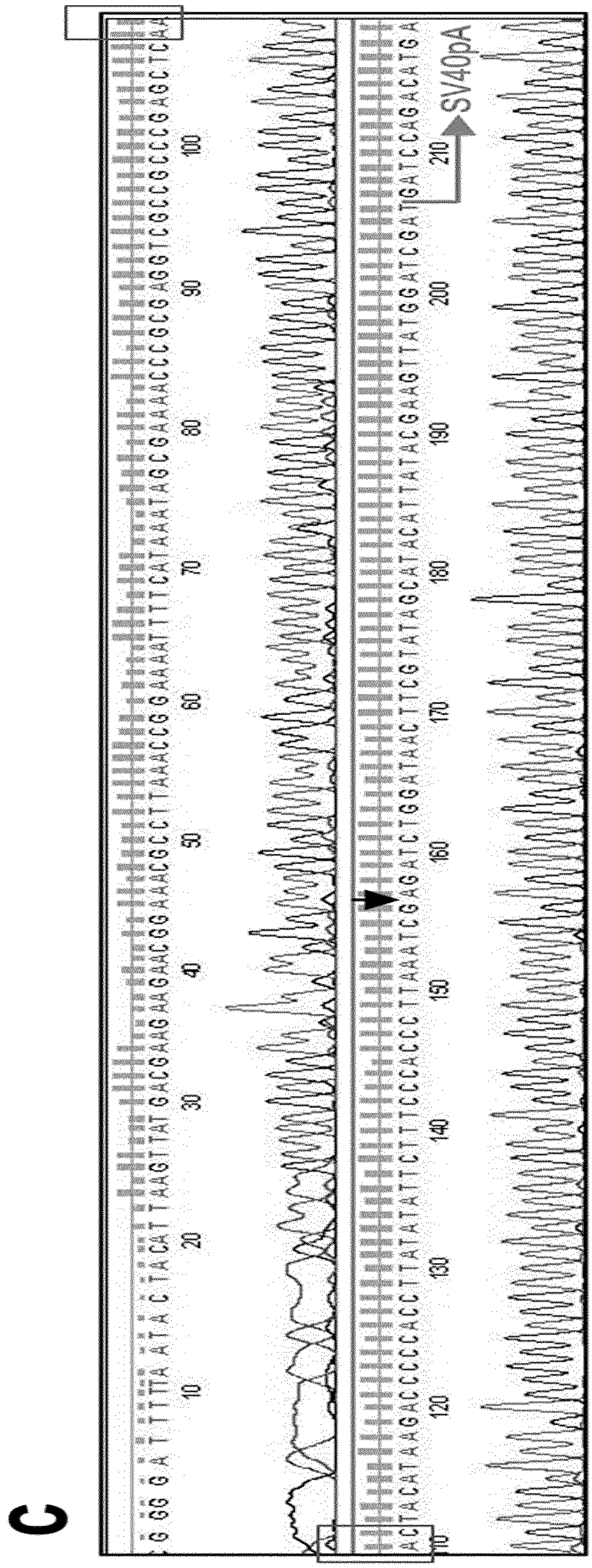

【Fig 12】

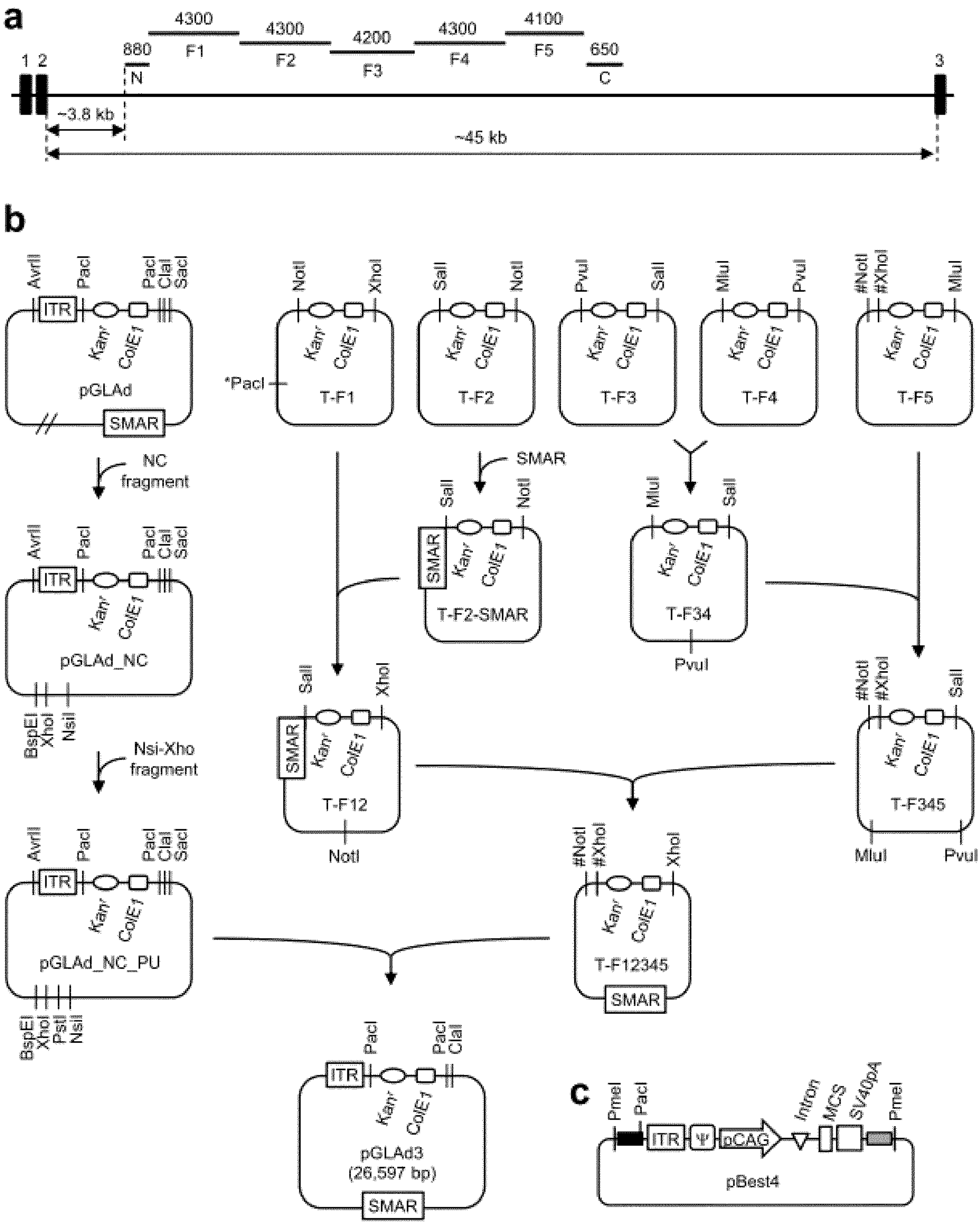
a
1 2
880
N
4300
F1
4300
F2
4200
F3
4300
F4
4100
F5
650
C
3
~3.8 kb
~45 kb
b
AvrII PacI PacI ClaI SacI
ITR
Kanr ColE1
pGLAd
SMAR
NC fragment
pGLAd_NC
BspEI XhoI NsiI
Nsi-Xho fragment
pGLAd_NC_PU
BspEI XhoI PstI NsiI
NotI XhoI
*PacI
T-F1
SalI NotI
T-F2
PvuI SalI
T-F3
MluI PvuI
T-F4
#NotI #XhoI MluI
T-F5
SMAR
T-F2-SMAR
T-F34
T-F12
T-F345
T-F12345
pGLAd3
(26,597 bp)
c
PmeI PacI
ITR
Ψ
pCAG
Intron
MCS
SV40pA
PmeI
pBest4

【Fig 13】

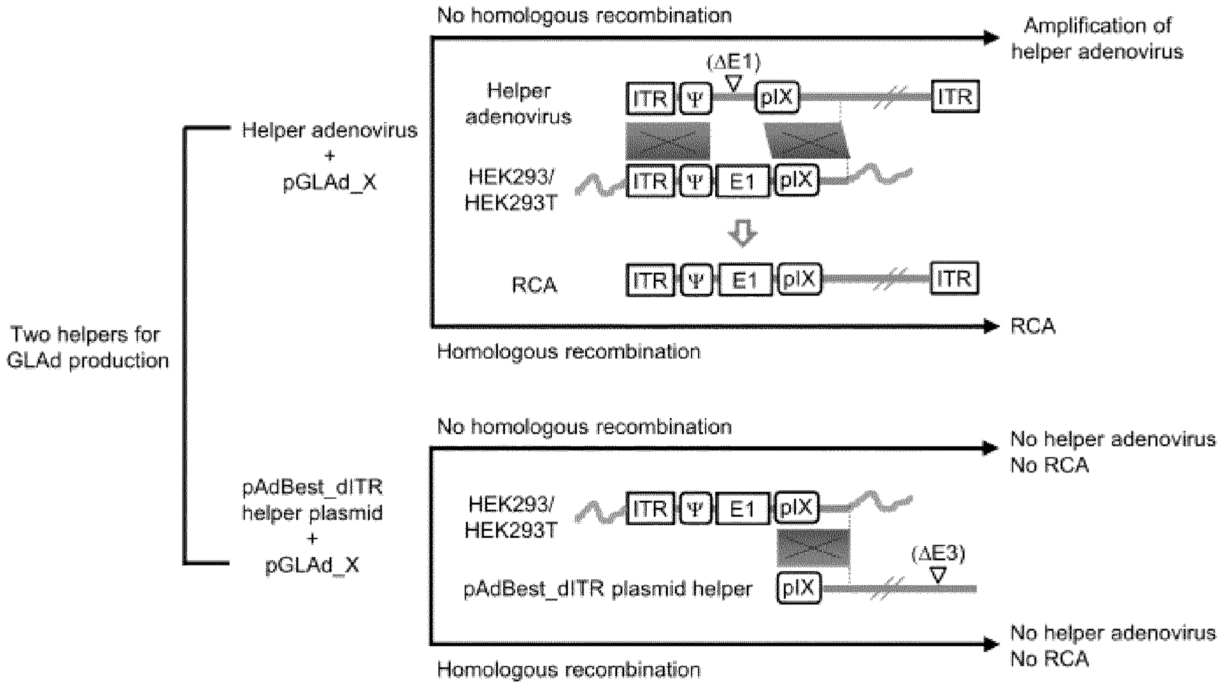
No homologous recombination
Amplification of helper adenovirus
(ΔE1)
Helper adenovirus
ITR
Ψ
pIX
ITR
Helper adenovirus
+
pGLAd_X
HEK293/ HEK293T
ITR
Ψ
E1
pIX
RCA
ITR
Ψ
E1
pIX
ITR
RCA
Two helpers for GLAd production
Homologous recombination
No homologous recombination
No helper adenovirus
No RCA
pAdBest_dITR helper plasmid
+
pGLAd_X
HEK293/ HEK293T
ITR
Ψ
E1
pIX
(ΔE3)
pAdBest_dITR plasmid helper
pIX
No helper adenovirus
No RCA
Homologous recombination

【Fig 14】

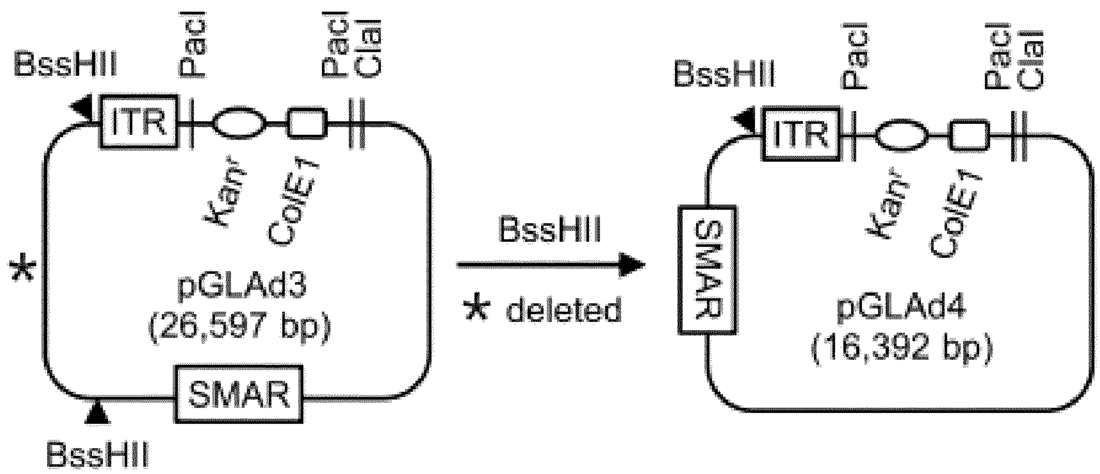
BssHII
PacI
PacI
ClaI
ITR
Kan^r
ColE1
pGLAd3
(26,597 bp)
SMAR
BssHII
BssHII
* deleted
BssHII
PacI
PacI
ClaI
ITR
SMAR
Kan^r
ColE1
pGLAd4
(16,392 bp)

**INTERNATIONAL SEARCH REPORT**

International application No.
**PCT/KR2020/014585**

**A. CLASSIFICATION OF SUBJECT MATTER**

**C12N 7/00**(2006.01)i

According to International Patent Classification (IPC) or to both national classification and IPC

**B. FIELDS SEARCHED**

Minimum documentation searched (classification system followed by classification symbols)

C12N 7/00(2006.01); A01K 63/00(2006.01); A61K 48/00(2006.01); C12N 15/86(2006.01); C12N 15/861(2006.01); C12Q 1/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 가틀리스 아데노바이러스(gutless adenovirus, GLAd), 아데노바이러스(adenovirus), 헬퍼 플라스미드(helper plasmid), 게놈 플라스미드(genome plasmid), 클로닝 셔틀 플라스미드(cloning shuttle plasmid)

**C. DOCUMENTS CONSIDERED TO BE RELEVANT**

| Category* | Citation of document, with indication, where appropriate, of the relevant passages | Relevant to claim No. |
|---|---|---|
| X | US 8709778 B2 (DANTHINNE, Xavier) 29 April 2014 (2014-04-29) See columns 5-25. | 1,6,12-14,20,23-25,31,32,35-40,42 |
| Y | | 2-5,27-30,33,34 |
| A | | 7-11,15-19,21,22,26,41 |
| Y | WO 96-40955 A1 (GRAHAM, Frank L. et al.) 19 December 1996 (1996-12-19) See pages 12-16. | 2-5,27-30,33,34 |
| A | US 8236300 B2 (SEHGAL, Lakshman R. et al.) 07 August 2012 (2012-08-07) See claims 1-11. | 1-42 |
| A | US 2007-0077226 A1 (LIU, Xinyuan et al.) 05 April 2007 (2007-04-05) See claims 1-11. | 1-42 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 February 2021** | **19 February 2021** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**

International application No.
**PCT/KR2020/014585**

**C. DOCUMENTS CONSIDERED TO BE RELEVANT**

| Category* | Citation of document, with indication, where appropriate, of the relevant passages | Relevant to claim No. |
|---|---|---|
| A | ALBA, R. et al. Gutless adenovirus: last-generation adenovirus for gene therapy. Gene Therapy. 2005, vol. 12, pp. S18-S27. See pages S18-S27. | 1-42 |
| PX | LEE, Dongwoo et al. No more helper adenovirus: production of gutless adenovirus (GLAd) free of adenovirus and replication-competent adenovirus (RCA) contaminants. Experimental & Molecular Medicine. 28 October 2019, vol. 51, inner pp. 1-18. See inner pages 1-18. | 1-42 |

Form PCT/ISA/210 (second sheet) (July 2019)

**INTERNATIONAL SEARCH REPORT**

International application No.
**PCT/KR2020/014585**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**
**Information on patent family members**

International application No.
**PCT/KR2020/014585**

| Patent document cited in search report | Publication date (day/month/year) | Patent family member(s) | Publication date (day/month/year) |
|---|---|---|---|
| US 8709778 B2 | 29 April 2014 | US 2010-0105110 A1 | 29 April 2010 |
| | | WO 2010-050997 A1 | 06 May 2010 |
| WO 96-40955 A1 | 19 December 1996 | AT 213022 T | 15 February 2002 |
| | | AT 304604 T | 15 September 2005 |
| | | AU 2000-40761 A1 | 23 October 2000 |
| | | AU 2004-201152 B2 | 08 March 2007 |
| | | AU 687829 B2 | 05 March 1998 |
| | | AU 724161 B2 | 14 September 2000 |
| | | AU 765093 B2 | 11 September 2003 |
| | | AU 769735 B2 | 05 February 2004 |
| | | CA 2166118 C | 17 April 2007 |
| | | CA 2220997 A1 | 19 December 1996 |
| | | CA 2220997 C | 24 April 2007 |
| | | CA 2266855 A1 | 02 April 1998 |
| | | CA 2366914 C | 11 May 2010 |
| | | CA 2367390 A1 | 12 October 2000 |
| | | DE 69434486 T2 | 06 July 2006 |
| | | DE 69619110 T2 | 18 July 2002 |
| | | EP 0699236 B2 | 14 June 2006 |
| | | EP 0705344 A1 | 10 April 1996 |
| | | EP 0705344 B1 | 14 September 2005 |
| | | EP 0832267 A1 | 01 April 1998 |
| | | EP 0832267 B1 | 06 February 2002 |
| | | EP 0938579 A1 | 01 September 1999 |
| | | EP 0992584 A3 | 07 June 2000 |
| | | EP 1159439 A2 | 05 December 2001 |
| | | EP 1159439 B1 | 08 January 2014 |
| | | EP 1165817 A1 | 02 January 2002 |
| | | ES 2249761 T3 | 01 April 2006 |
| | | JP 06-335393 A | 06 December 1994 |
| | | JP 09-501822 A | 25 February 1997 |
| | | JP 11-506334 A | 08 June 1999 |
| | | JP 2000-514312 A | 31 October 2000 |
| | | JP 2002-509422 A | 26 March 2002 |
| | | JP 2003-519464 A | 24 June 2003 |
| | | JP 2004-501602 A | 22 January 2004 |
| | | JP 2534968 B2 | 18 September 1996 |
| | | JP 3532566 B2 | 31 May 2004 |
| | | JP 3803689 B2 | 02 August 2006 |
| | | US 105919676 A | 06 July 1999 |
| | | US 106140087 A | 31 October 2000 |
| | | US 2003-0228280 A1 | 11 December 2003 |
| | | US 2007-0128165 A1 | 07 June 2007 |
| | | US 7135187 B2 | 14 November 2006 |
| | | US 7537932 B1 | 26 May 2009 |
| | | WO 00-52187 A9 | 11 July 2002 |
| | | WO 00-60106 A1 | 12 October 2000 |
| | | WO 94-26891 A3 | 19 January 1995 |
| | | WO 95-00655 A1 | 05 January 1995 |
| | | WO 98-13510 A1 | 02 April 1998 |

Form PCT/ISA/210 (patent family annex) (July 2019)

**INTERNATIONAL SEARCH REPORT**
**Information on patent family members**

International application No.
**PCT/KR2020/014585**

| Patent document cited in search report | Publication date (day/month/year) | Patent family member(s) | Publication date (day/month/year) |
|---|---|---|---|
| US 8236300 B2 | 07 August 2012 | AU 2003-298768 A1 | 23 June 2004 |
| | | AU 2003-298768 A8 | 23 June 2004 |
| | | CA 2508514 A1 | 17 June 2004 |
| | | CA 2674563 A1 | 24 July 2008 |
| | | CA 2674563 C | 30 December 2014 |
| | | EP 1573005 A2 | 14 September 2005 |
| | | EP 2099438 A2 | 16 September 2009 |
| | | EP 2393497 A1 | 14 December 2011 |
| | | EP 2393497 B1 | 02 April 2014 |
| | | GB 2410747 A | 10 August 2005 |
| | | US 2004-0198683 A1 | 07 October 2004 |
| | | US 2006-0286083 A1 | 21 December 2006 |
| | | US 2007-0184027 A1 | 09 August 2007 |
| | | US 2007-0212334 A1 | 13 September 2007 |
| | | US 2007-0238685 A1 | 11 October 2007 |
| | | US 2009-0105180 A1 | 23 April 2009 |
| | | US 2009-0123440 A1 | 14 May 2009 |
| | | US 2009-0238795 A1 | 24 September 2009 |
| | | US 2010-0240740 A1 | 23 September 2010 |
| | | US 2010-0247487 A1 | 30 September 2010 |
| | | US 2010-0249221 A1 | 30 September 2010 |
| | | US 2011-0196022 A1 | 11 August 2011 |
| | | US 2011-0196023 A1 | 11 August 2011 |
| | | US 2011-0307076 A1 | 15 December 2011 |
| | | US 7179459 B2 | 20 February 2007 |
| | | US 7481998 B2 | 27 January 2009 |
| | | US 7501114 B2 | 10 March 2009 |
| | | US 7670597 B2 | 02 March 2010 |
| | | US 7687058 B2 | 30 March 2010 |
| | | US 7713522 B2 | 11 May 2010 |
| | | US 7803365 B2 | 28 September 2010 |
| | | US 8048410 B2 | 01 November 2011 |
| | | US 8242095 B2 | 14 August 2012 |
| | | US 8367056 B2 | 05 February 2013 |
| | | US 8420075 B2 | 16 April 2013 |
| | | US 9353383 B2 | 31 May 2016 |
| | | US 9388427 B2 | 12 July 2016 |
| | | WO 2004-050844 A2 | 17 June 2004 |
| | | WO 2004-050844 A3 | 22 July 2004 |
| | | WO 2008-088358 A2 | 24 July 2008 |
| | | WO 2008-088358 A3 | 18 December 2008 |
| | | WO 2010-085262 A1 | 29 July 2010 |
| US 2007-0077226 A1 | 05 April 2007 | CN 1453361 A | 05 November 2003 |
| | | CN 1788081 A | 14 June 2006 |
| | | CN 1788081 C | 14 June 2006 |
| | | WO 2004-106505 A1 | 09 December 2004 |

Form PCT/ISA/210 (patent family annex) (July 2019)